# EUROPEAN PATENT APPLICATION

(11) **EP 4 361 269 A1**
(43) Date of publication of application: **01.05.2024**
(21) Application number: 22828501.1
(22) Date of filing: 23.06.2022
(51) Int. Cl.: C12N 15/113, A61K 31/711, A61K 31/712, A61K 31/7125, A61K 48/00, A61P 21/04

(54) **COMBINATION OF ANTISENSE OLIGOMERS**

(30) Priority: 23.06.2021 JP 2021104145
(71) Applicant: Nippon Shinyaku Co., Ltd., Kyoto-shi Kyoto 601-8550 (JP); NATIONAL CENTER OF NEUROLOGY AND PSYCHIATRY, Kodaira-shi Tokyo 187-8551 (JP)
(72) Inventor: TONE, Yuichiro, Kyoto-shi, Kyoto 601-8550 (JP); AOKI, Yoshitsugu, Kodaira-shi, Tokyo 187-8551 (JP); MOTOHASHI, Norio, Kodaira-shi, Tokyo 187-8551 (JP)
(74) Representative: Hoffmann Eitle
(86) International application number: PCT/JP2022/025120
(87) International publication number: WO 2022/270585

(57) **Abstract**

Herein, a combination of antisense oligomers or pharmaceutically acceptable salts thereof, or hydrates thereof which cause simultaneous skipping of any two or more numerically consecutive exons selected from the group consisting of the 45th exon to the 55th exon in human dystrophin pre-mRNA is provided.

## Description

### Technical Field

The present invention relates to a pharmaceutical composition or a pharmaceutical combination for use in treatment of muscular dystrophy, a method for treatment of muscular dystrophy, and the like.

### Background Art

In recent years, exon skipping therapy has received attention which involves causing exon skipping of a gene having a mutation that causes a disease so that a protein having partial functions arises, thereby treating the disease. Examples of the disease that may be treated by such exon skipping therapy include Duchenne muscular dystrophy (DMD).

DMD is the most frequent form of hereditary progressive muscular disease that affects one in about 3,500 newborn boys. Although DMD patients exhibit motor functions rarely different from healthy humans in their infancy and childhood, muscle weakness is observed in children from around 4 to 5 years old. Then, muscle weakness in DMD patients progresses with age to the loss of ambulation by about 12 years old and death due to cardiac or respiratory insufficiency in the twenties. Therefore, it has been strongly desired to develop an effective therapeutic agent.

DMD is known to be caused by a mutation in the dystrophin gene. The dystrophin gene is located on X chromosome and is a huge gene consisting of 2.2 million DNA base pairs. DNA is transcribed into pre-mRNA, and introns are removed by splicing to synthesize mRNA of 13,993 bases in which 79 exons are joined together. This mRNA is translated into 3,685 amino acids to produce dystrophin protein. The dystrophin protein is associated with the maintenance of membrane stability in muscle cells and necessary to make muscle cells less fragile. Patients with DMD have a mutation in the dystrophin gene and hence, the functional dystrophin protein is rarely expressed in muscle cells of the patients. Therefore, the structure of muscle cells cannot be maintained at the time of muscle contraction in the body of the patients with DMD, leading to a large influx of calcium ions into muscle cells. Consequently, muscle cell necrosis and fibrosis progress so that muscle cells can be eventually regenerated only with difficulty.

Becker muscular dystrophy (BMD) is also caused by a mutation in the dystrophin gene. The symptoms involve muscle weakness but are typically mild and slow in the progress of muscle weakness, when compared to DMD. In many cases, its onset is in adulthood. Differences in clinical symptoms between DMD and BMD are considered to reside in whether the reading frame for amino acids on the translation of dystrophin mRNA into the dystrophin protein is disrupted by the mutation or not (Non Patent Literature 1). More specifically, in DMD, the presence of mutation shifts the amino acid reading frame so that the expression of functional dystrophin protein is abolished, whereas in BMD the dystrophin protein that is capable of functioning, though imperfectly, is produced because the amino acid reading frame is preserved, while a part of the exons are deleted by the mutation.

Exon skipping is expected to serve as a method for treating DMD. This method involves modifying splicing to restore the amino acid reading frame of dystrophin mRNA and induce expression of the dystrophin protein having the function partially restored (Non Patent Literature 2). The amino acid sequence part to be translated from an exon, which is a target for exon skipping, will be lost. For this reason, the dystrophin protein expressed by this treatment becomes shorter than normal one but since the amino acid reading frame is maintained, the function to stabilize muscle cells is partially retained. Consequently, it is expected that exon skipping will lead DMD to the similar symptoms to that of BMD which is milder. The exon skipping approach has passed the animal tests using mice or dogs and now is currently assessed in clinical trials on human DMD patients.

The skipping of an exon can be induced by binding of antisense nucleic acids targeting site(s) surrounding either 5' or 3' splice site or both sites, or exon-internal sites. An exon will only be included in the mRNA when both splice sites thereof are recognized by the spliceosome complex. Thus, exon skipping can be induced by targeting the sites surrounding the splice sites with antisense nucleic acids. Furthermore, the binding of an SR protein rich in serine and arginine to an exonic splicing enhancer (ESE) is considered necessary for an exon to be recognized by the splicing mechanism. Accordingly, exon skipping can also be induced by targeting ESE.

Since a mutation of the dystrophin gene may vary depending on DMD patients, antisense nucleic acids need to be designed based on the site or type of respective genetic mutation. There are a plurality of reports on an antisense nucleic acid that induces exon skipping targeting one sequence of consecutive bases for a single exon in the dystrophin gene (Patent Literatures 1 to 6 and Non Patent Literatures 1 and 2). It has also been reported that when two types of antisense nucleic acids that target the same exon in the dystrophin gene are mixed and allowed to act (dual targeting), skipping activity may be enhanced as compared to use of each antisense nucleic acid alone (Patent Literature 7).

A method called multi-exon skipping has received attention which involves causing skipping of a plurality of exons (exon group), not one exon as described above. This method enables a wide range of mutations in the dystrophin gene to be treated by exon skipping. For example, exons 45 to 55 in the dystrophin gene are known as hot spots of genetic mutation, and it has been reported that skipping of these 11 exons enables about 60% of DMD patients having a deletion mutation to be treated (Non Patent Literature 3). Most of patients congenitally lacking exons 45 to 55 are known to manifest no or mild symptoms, though developing BMD (Non Patent Literature 4). Thus, it is expected that drugs capable of inducing exon 45 to 55 skipping are promising as therapeutic agents for DMD.

For example, a method using antisense nucleic acids respectively targeting all exons in a region which is the target of exon skipping (Non Patent Literatures 5, 7, 8, and 10), a method using antisense nucleic acids respectively targeting two different exons on the 3' side and 5' side of a region which is the target of exon skipping (Non Patent Literatures 6 and 9 and Patent Literatures 8, 9, and 11), and a method using an antisense nucleic acid targeting only an exon on the 5' side of a region which is the target of exon skipping (Patent Literature 10) have been reported as methods for inducing multi-exon skipping.

### Citation List

### Patent Literature

Patent Literature 1: International Publication WO2004/048570
Patent Literature 2: International Publication W2009/139630
Patent Literature 3: International Publication W2010/048586
Patent Literature 4: U.S. Patent Publication Nos. 2010/0168212
Patent Literature 5: International Publication W2011/057350
Patent Literature 6: International Publication W2006/000057
Patent Literature 7: International Publication W2007/135105
Patent Literature 8: International Publication W2004/083446

### Non Patent Literature

Patent Literature 9: International Publication W2014/007620
Patent Literature 10: International Publication W2019/200185
Patent Literature 11: International Publication W2020/219820

### Non Patent Literature

Non Patent Literature 1: Annemieke Aartsma-Rus et al., (2002) Neuromuscular Disorders 12: S71-S77
Non Patent Literature 2: Wilton S. D., e t al., Molecular Therapy 2007: 15: p. 1288-96
Non Patent Literature 3: Christophe Beroud et al., Human Mutation, 28(2), 2007, 196-202
Non Patent Literature 4: Yusuke Echigoya et al., Molecular Therapy-Nucleic Acids, 4(2), 2015, e225
Non Patent Literature 5: Yoshitsugu Aoki et al., PNAS, 109(34), 2012, 13763-13768
Non Patent Literature 6: Laura van Vliet et al., BMC Medical Genetics, 9, 105, 2008
Non Patent Literature 7: Joshua Lee et al., PLoS ONE, 13(5), e0197084, 2018
Non Patent Literature 8: Joshua Lee et al., Methods in Molecular Biology, 1828, 141-150, 2018
Non Patent Literature 9: Annemieke Aartsma-Rus et al, Am. J. Hum. Genet. 74(1), 83-92, 2004
Non Patent Literature 10: Yusuke Echigoya et al., Molecular Therapy, 27(11), 1-13, 2019

### Summary of Invention

### Technical Problem

The effects of drugs causing simultaneous skipping a plurality of exons (exon group) in objective pre-mRNA are not always sufficient. Under the foregoing circumstances, medicaments for treating patients having various mutations by causing simultaneous skipping of a plurality of exons (exon group) in objective pre-mRNA have been desired.

### Solution to Problem

The present invention provides a combination of antisense oligomers or pharmaceutically acceptable salts thereof, or hydrates thereof, a pharmaceutical composition, a pharmaceutical combination, a method for treatment of muscular dystrophy, and the like as follows:
(1) A combination of antisense oligomers or pharmaceutically acceptable salts thereof, or hydrates thereof which cause simultaneous skipping of any two or more numerically consecutive exons selected from the group consisting of the 45th exon to the 55th exon in human dystrophin pre-mRNA,
   the combination comprising:
   (i) a first antisense oligomer or a pharmaceutically acceptable salt thereof, or a hydrate thereof, comprising:
      a first unit oligomer comprising a base sequence complementary to a base sequence consisting of a base sequence of 11 bases in the upstream direction from the 3' end of the 44th intron and a base sequence of 69 bases in the downstream direction from the 5' end of the 45th exon in the human dystrophin pre-mRNA, or a partial base sequence thereof; and
      a second unit oligomer comprising a base sequence complementary to a base sequence of from the 52nd to 75th bases in the upstream direction from the 3' end of the 44th intron in the human dystrophin pre-mRNA, or a partial base sequence thereof; and
   (ii) a second antisense oligomer or a pharmaceutically acceptable salt thereof, or a hydrate thereof, comprising a base sequence complementary to a base sequence consisting of a base sequence of 33 bases in the upstream direction from the 3' end of the 54th intron and a base sequence of 53 bases in the downstream direction from the 5' end of the 55th exon in the human dystrophin pre-mRNA, or a partial base sequence thereof.
(2) The combination according to (1), wherein
   the first unit oligomer comprises a base sequence complementary to consecutive 15 to 30 bases of a base sequence consisting of a base sequence of 11 bases in the upstream direction from the 3' end of the 44th intron and a base sequence of 69 bases in the downstream direction from the 5' end of the 45th exon in the human dystrophin pre-mRNA,
   the second unit oligomer comprises a base sequence complementary to consecutive 1 to 10 bases of a base sequence of from the 52nd to 75th bases in the upstream direction from the 3' end of the 44th intron in the human dystrophin pre-mRNA, and
   the second antisense oligomer comprises a base sequence complementary to consecutive 15 to 30 bases of a base sequence consisting of a base sequence of 33 bases in the upstream direction from the 3' end of the 54th intron and a base sequence of 53 bases in the downstream direction from the 5' end of the 55th exon in the human dystrophin pre-mRNA.
(3) The combination according to (1) or (2), wherein
   the first unit oligomer comprises a base sequence complementary to:
      (a) any one base sequence selected from the group consisting of SEQ ID NOs: 211 to 906;
      (b) a base sequence that hybridizes under stringent conditions to a base sequence complementary to any one base sequence selected from the group consisting of SEQ ID NOs: 211 to 906;
      (c) a base sequence that has at least 85% identity with any one base sequence selected from the group consisting of SEQ ID NOs: 211 to 906, and has a length within ±15% of the length of the any one base sequence selected; or
      (d) a partial base sequence of any one base sequence selected from the group consisting of the base sequences (a), (b), and (c), and/or
   the second unit oligomer comprises a base sequence complementary to:
      (a) any one base sequence selected from the group consisting of SEQ ID NOs: 1 to 105;
      (b) a base sequence that hybridizes under stringent conditions to a base sequence complementary to any one base sequence selected from the group consisting of SEQ ID NOs: 1 to 105;
      (c) a base sequence that has at least 85% identity with any one base sequence selected from the group consisting of SEQ ID NOs: 1 to 105, and has a length within ±15% of the length of the any one base sequence selected; or
      (d) a partial base sequence of any one base sequence selected from the group consisting of the base sequences (a), (b), and (c) .
(4) The combination according to any one of (1) to (3), wherein
   the second antisense oligomer comprises a base sequence complementary to:
   (a) any one base sequence selected from the group consisting of SEQ ID NOs: 3507 to 4298;
   (b) a base sequence that hybridizes under stringent conditions to a base sequence complementary to any one base sequence selected from the group consisting of SEQ ID NOs: 3507 to 4298;
   (c) a base sequence that has at least 85% identity with any one base sequence selected from the group consisting of SEQ ID NOs: 3507 to 4298, and has a length within ±15% of the length of the any one base sequence selected; or
   (d) a partial base sequence of any one base sequence selected from the group consisting of the base sequences (a), (b), and (c) .
(5) The combination according to any one of (1) to (4), wherein the first antisense oligomer comprises the first unit oligomer and the second unit oligomer from the 5' ends in this order, the first unit oligomer comprises any one base sequence selected from SEQ ID NOs: 907 to 1602, the second unit oligomer comprises any one base sequence selected from SEQ ID NOs: 106 to 210, and the second antisense oligomer comprises any one base sequence selected from SEQ ID NOs: 4299 to 5090.
(6) The combination according to any one of (1) to (5), wherein the first unit oligomer comprises any one base sequence selected from the group consisting of SEQ ID NOs: 1180, 1190, 1201, 1212, 1222, 1224, and 1239.
(7) The combination according to any one of (1) to (6), wherein the second unit oligomer comprises any one base sequence selected from the group consisting of SEQ ID NOs: 114, 124, 151, 201, 203, and 205.
(8) The combination according to (6) or (7), wherein
   the first antisense oligomer comprises the first unit oligomer and the second unit oligomer from the 5' ends in this order, and
   the first unit oligomer comprises a base sequence of SEQ ID NO: 1201, and the second unit oligomer comprises a base sequence of SEQ ID NO: 151,
   the first unit oligomer comprises a base sequence of SEQ ID NO: 1201, and the second unit oligomer comprises a base sequence of SEQ ID NO: 201,
   the first unit oligomer comprises a base sequence of SEQ ID NO: 1201, and the second unit oligomer comprises a base sequence of SEQ ID NO: 203,
   the first unit oligomer comprises a base sequence of SEQ ID NO: 1201, and the second unit oligomer comprises a base sequence of SEQ ID NO: 205,
   the first unit oligomer comprises a base sequence of SEQ ID NO: 1239, and the second unit oligomer comprises a base sequence of SEQ ID NO: 114,
   the first unit oligomer comprises a base sequence of SEQ ID NO: 1224, and the second unit oligomer comprises a base sequence of SEQ ID NO: 124,
   the first unit oligomer comprises a base sequence of SEQ ID NO: 1180, and the second unit oligomer comprises a base sequence of SEQ ID NO: 151,
   the first unit oligomer comprises a base sequence of SEQ ID NO: 1190, and the second unit oligomer comprises a base sequence of SEQ ID NO: 151,
   the first unit oligomer comprises a base sequence of SEQ ID NO: 1212, and the second unit oligomer comprises a base sequence of SEQ ID NO: 151, or
   the first unit oligomer comprises a base sequence of SEQ ID NO: 1222, and the second unit oligomer comprises a base sequence of SEQ ID NO: 151.
(9) The combination according to any one of (1) to (8), wherein the second antisense oligomer comprises a base sequence selected from the group consisting of SEQ ID NOs: 4698, 4702, 4752, 4923, 4926, 4936, and 4977.
(10) The combination according to any one of (1) to (9), wherein
   the first antisense oligomer comprises the first unit oligomer and the second unit oligomer from the 5' ends in this order, and
   the first unit oligomer comprises a base sequence of SEQ ID NO: 1201, the second unit oligomer comprises a base sequence of SEQ ID NO: 151, and the second antisense oligomer comprises a base sequence of SEQ ID NO: 4950,
   the first unit oligomer comprises a base sequence of SEQ ID NO: 1201, the second unit oligomer comprises a base sequence of SEQ ID NO: 201, and the second antisense oligomer comprises a base sequence of SEQ ID NO: 4950,
   the first unit oligomer comprises a base sequence of SEQ ID NO: 1201, the second unit oligomer comprises a base sequence of SEQ ID NO: 203, and the second antisense oligomer comprises a base sequence of SEQ ID NO: 4950,
   the first unit oligomer comprises a base sequence of SEQ ID NO: 1201, the second unit oligomer comprises a base sequence of SEQ ID NO: 205, and the second antisense oligomer comprises a base sequence of SEQ ID NO: 4950,
   the first unit oligomer comprises a base sequence of SEQ ID NO: 1239, the second unit oligomer comprises a base sequence of SEQ ID NO: 114, and the second antisense oligomer comprises a base sequence of SEQ ID NO: 4950,
   the first unit oligomer comprises a base sequence of SEQ ID NO: 1224, the second unit oligomer comprises a base sequence of SEQ ID NO: 124, and the second antisense oligomer comprises a base sequence of SEQ ID NO: 4950,
   the first unit oligomer comprises a base sequence of SEQ ID NO: 1180, the second unit oligomer comprises a base sequence of SEQ ID NO: 151, and the second antisense oligomer comprises a base sequence of SEQ ID NO: 4950,
   the first unit oligomer comprises a base sequence of SEQ ID NO: 1190, the second unit oligomer comprises a base sequence of SEQ ID NO: 151, and the second antisense oligomer comprises a base sequence of SEQ ID NO: 4950,
   the first unit oligomer comprises a base sequence of SEQ ID NO: 1212, the second unit oligomer comprises a base sequence of SEQ ID NO: 151, and the second antisense oligomer comprises a base sequence of SEQ ID NO: 4950,
   the first unit oligomer comprises a base sequence of SEQ ID NO: 1222, the second unit oligomer comprises a base sequence of SEQ ID NO: 151, and the second antisense oligomer comprises a base sequence of SEQ ID NO: 4950,
   the first unit oligomer comprises a base sequence of SEQ ID NO: 1201, the second unit oligomer comprises a base sequence of SEQ ID NO: 151, and the second antisense oligomer comprises a base sequence of SEQ ID NO: 4698,
   the first unit oligomer comprises a base sequence of SEQ ID NO: 1201, the second unit oligomer comprises a base sequence of SEQ ID NO: 151, and the second antisense oligomer comprises a base sequence of SEQ ID NO: 4702,
   the first unit oligomer comprises a base sequence of SEQ ID NO: 1201, the second unit oligomer comprises a base sequence of SEQ ID NO: 151, and the second antisense oligomer comprises a base sequence of SEQ ID NO: 4752,
   the first unit oligomer comprises a base sequence of SEQ ID NO: 1201, the second unit oligomer comprises a base sequence of SEQ ID NO: 151, and the second antisense oligomer comprises a base sequence of SEQ ID NO: 4923,
   the first unit oligomer comprises a base sequence of SEQ ID NO: 1201, the second unit oligomer comprises a base sequence of SEQ ID NO: 151, and the second antisense oligomer comprises a base sequence of SEQ ID NO: 4926,
   the first unit oligomer comprises a base sequence of SEQ ID NO: 1201, the second unit oligomer comprises a base sequence of SEQ ID NO: 151, and the second antisense oligomer comprises a base sequence of SEQ ID NO: 4936,
   the first unit oligomer comprises a base sequence of SEQ ID NO: 1201, the second unit oligomer comprises a base sequence of SEQ ID NO: 151, and the second antisense oligomer comprises a base sequence of SEQ ID NO: 4977, or
   the first unit oligomer comprises a base sequence of SEQ ID NO: 1180, the second unit oligomer comprises a base sequence of SEQ ID NO: 151, and the second antisense oligomer comprises a base sequence of SEQ ID NO: 4977.
(11) The combination according to any one of (5) to (10), wherein the first unit oligomer comprises a base sequence of SEQ ID NO: 1201, the second unit oligomer comprises a base sequence of SEQ ID NO: 151, and the second antisense oligomer comprises a base sequence of SEQ ID NO: 4950 or 4880.
(12) The combination according to any one of (1) to (11), further comprising:
   (iii) a third antisense oligomer or a pharmaceutically acceptable salt thereof, or a hydrate thereof, comprising a base sequence complementary to a base sequence consisting of a base sequence of 23 bases in the upstream direction from the 3' end of the 45th exon and a base sequence of 73 bases in the downstream direction from the 5' end of the 45th intron in the human dystrophin pre-mRNA, or a partial base sequence thereof.
(13) The combination according to (12), wherein the third antisense oligomer comprises a base sequence complementary to consecutive 15 to 30 bases of a base sequence consisting of a base sequence of 23 bases in the upstream direction from the 3' end of the 45th exon and a base sequence of 73 bases in the downstream direction from the 5' end of the 45th intron in the human dystrophin pre-mRNA.
(14) The combination according to (12) or (13), wherein
   the third antisense oligomer comprises a base sequence complementary to:
   (a) any one base sequence selected from the group consisting of SEQ ID NOs: 1603 to 2554;
   (b) a base sequence that hybridizes under stringent conditions to a base sequence complementary to any one base sequence selected from the group consisting of SEQ ID NOs: 1603 to 2554;
   (c) a base sequence that has at least 85% identity with any one base sequence selected from the group consisting of SEQ ID NOs: 1603 to 2554, and has a length within ±15% of the length of the any one base sequence selected; or
   (d) a partial base sequence of any one base sequence selected from the group consisting of the base sequences (a), (b), and (c) .
(15-1) The combination according to (14), wherein the third antisense oligomer comprises a base sequence complementary to:
   (a) any one base sequence selected from the group consisting of SEQ ID NOs: 1611 to 1654, 1664 to 1707, 1718 to 1761, 1773 to 1816, 1829 to 1872, 1886 to 1929, 1944 to 1987, 2003 to 2046, 2063 to 2106, 2124 to 2167, 2186 to 2229, 2249 to 2292, 2313 to 2356, 2378 to 2421, 2444 to 2487, and 2511 to 2554;
   (b) a base sequence that hybridizes under stringent conditions to a base sequence complementary to any one base sequence selected from the group consisting of SEQ ID NOs: 1611 to 1654, 1664 to 1707, 1718 to 1761, 1773 to 1816, 1829 to 1872, 1886 to 1929, 1944 to 1987, 2003 to 2046, 2063 to 2106, 2124 to 2167, 2186 to 2229, 2249 to 2292, 2313 to 2356, 2378 to 2421, 2444 to 2487, and 2511 to 2554;
   (c) a base sequence that has at least 85% identity with any one base sequence selected from the group consisting of SEQ ID NOs: 1611 to 1654, 1664 to 1707, 1718 to 1761, 1773 to 1816, 1829 to 1872, 1886 to 1929, 1944 to 1987, 2003 to 2046, 2063 to 2106, 2124 to 2167, 2186 to 2229, 2249 to 2292, 2313 to 2356, 2378 to 2421, 2444 to 2487, and 2511 to 2554, and has a length within ±15% of the length of the any one base sequence selected; or
   (d) a partial base sequence of any one base sequence selected from the group consisting of the base sequences (a), (b), and (c).
(15-2) The combination according to (14), wherein the third antisense oligomer comprises a base sequence complementary to:
   (a) any one base sequence selected from the group consisting of SEQ ID NOs: 1614 to 1654, 1667 to 1707, 1721 to 1761, 1776 to 1816, 1832 to 1872, 1889 to 1929, 1947 to 1987, 2006 to 2046, 2066 to 2106, 2127 to 2167, 2189 to 2229, 2252 to 2292, 2316 to 2356, 2381 to 2421, 2447 to 2487, and 2514 to 2554;
   (b) a base sequence that hybridizes under stringent conditions to a base sequence complementary to any one base sequence selected from the group consisting of SEQ ID NOs: 1614 to 1654, 1667 to 1707, 1721 to 1761, 1776 to 1816, 1832 to 1872, 1889 to 1929, 1947 to 1987, 2006 to 2046, 2066 to 2106, 2127 to 2167, 2189 to 2229, 2252 to 2292, 2316 to 2356, 2381 to 2421, 2447 to 2487, and 2514 to 2554;
   (c) a base sequence that has at least 85% identity with any one base sequence selected from the group consisting of SEQ ID NOs: 1614 to 1654, 1667 to 1707, 1721 to 1761, 1776 to 1816, 1832 to 1872, 1889 to 1929, 1947 to 1987, 2006 to 2046, 2066 to 2106, 2127 to 2167, 2189 to 2229, 2252 to 2292, 2316 to 2356, 2381 to 2421, 2447 to 2487, and 2514 to 2554, and has a length within ±15% of the length of the any one base sequence selected; or
   (d) a partial base sequence of any one base sequence selected from the group consisting of the base sequences (a), (b), and (c).
(16) The combination according to (14), wherein the third antisense oligomer comprises a base sequence complementary to:
   (a) any one base sequence selected from the group consisting of SEQ ID NOs: 1617 to 1654, 1670 to 1707, 1724 to 1761, 1779 to 1816, 1835 to 1872, 1892 to 1929, 1950 to 1987, 2009 to 2046, 2069 to 2106, 2130 to 2167, 2192 to 2229, 2255 to 2292, 2319 to 2356, 2384 to 2421, 2450 to 2487, and 2517 to 2554;
   (b) a base sequence that hybridizes under stringent conditions to a base sequence complementary to any one base sequence selected from the group consisting of SEQ ID NOs: 1617 to 1654, 1670 to 1707, 1724 to 1761, 1779 to 1816, 1835 to 1872, 1892 to 1929, 1950 to 1987, 2009 to 2046, 2069 to 2106, 2130 to 2167, 2192 to 2229, 2255 to 2292, 2319 to 2356, 2384 to 2421, 2450 to 2487, and 2517 to 2554;
   (c) a base sequence that has at least 85% identity with any one base sequence selected from the group consisting of SEQ ID NOs: 1617 to 1654, 1670 to 1707, 1724 to 1761, 1779 to 1816, 1835 to 1872, 1892 to 1929, 1950 to 1987, 2009 to 2046, 2069 to 2106, 2130 to 2167, 2192 to 2229, 2255 to 2292, 2319 to 2356, 2384 to 2421, 2450 to 2487, and 2517 to 2554, and has a length within ±15% of the length of the any one base sequence selected; or
   (d) a partial base sequence of any one base sequence selected from the group consisting of the base sequences (a), (b), and (c) .
(17-1) The combination according to any one of (1) to (14), wherein the third antisense oligomer comprises a base sequence selected from the group consisting of SEQ ID NOs: 3060, 3065, 3077, 3082, 3087, 3090, 3096, 3108, 3119, and 3320.
(17-2) The combination according to any one of (1) to (14), wherein the third antisense oligomer comprises a base sequence selected from the group consisting of SEQ ID NOs: 3077, 3082, 3087, 3090, 3096, 3108, and 3119.
(17-3) The combination according to any one of (1) to (14), wherein the third antisense oligomer comprises a base sequence selected from the group consisting of SEQ ID NOs: 3082, 3087, 3090, 3096, 3108, and 3119.
(18) The combination according to any one of (12) to (17), wherein the first antisense oligomer comprises the first unit oligomer and the second unit oligomer from the 5' ends in this order, the first unit oligomer comprises any one base sequence selected from SEQ ID NOs: 907 to 1602, the second unit oligomer comprises any one base sequence selected from SEQ ID NOs: 106 to 210, the second antisense oligomer comprises any one base sequence selected from SEQ ID NOs: 4299 to 5090, and the third antisense oligomer comprises any one base sequence selected from SEQ ID NOs: 2555 to 3506.
(19) The combination according to any one of (1) to (18), wherein
   the first antisense oligomer comprises the first unit oligomer and the second unit oligomer from the 5' ends in this order, and
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3082,
   the first unit oligomer comprises a base sequence of SEQ ID NO: 1201, the second unit oligomer comprises a base sequence of SEQ ID NO: 201, the second antisense oligomer comprises a base sequence of SEQ ID NO: 4950, and the third antisense oligomer comprises a base sequence of SEQ ID NO: 3082,
   the first unit oligomer comprises a base sequence of SEQ ID NO: 1201, the second unit oligomer comprises a base sequence of SEQ ID NO: 203, the second antisense oligomer comprises a base sequence of SEQ ID NO: 4950, and the third antisense oligomer comprises a base sequence of SEQ ID NO: 3082,
   the first unit oligomer comprises a base sequence of SEQ ID NO: 1201, the second unit oligomer comprises a base sequence of SEQ ID NO: 205, the second antisense oligomer comprises a base sequence of SEQ ID NO: 4950, and the third antisense oligomer comprises a base sequence of SEQ ID NO: 3082,
   the first unit oligomer comprises a base sequence of SEQ ID NO: 1239, the second unit oligomer comprises a base sequence of SEQ ID NO: 114, the second antisense oligomer comprises a base sequence of SEQ ID NO: 4950, and the third antisense oligomer comprises a base sequence of SEQ ID NO: 3082,
   the first unit oligomer comprises a base sequence of SEQ ID NO: 1224, the second unit oligomer comprises a base sequence of SEQ ID NO: 124, the second antisense oligomer comprises a base sequence of SEQ ID NO: 4950, and the third antisense oligomer comprises a base sequence of SEQ ID NO: 3082,
   the first unit oligomer comprises a base sequence of SEQ ID NO: 1180, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3082,
   the first unit oligomer comprises a base sequence of SEQ ID NO: 1190, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3082,
   the first unit oligomer comprises a base sequence of SEQ ID NO: 1212, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3082,
   the first unit oligomer comprises a base sequence of SEQ ID NO: 1222, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3082,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3060,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3065,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3077,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3087,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3090,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3096,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3108,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3119,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3320,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4698, and the third antisense oligomer comprises a base sequence of SEQ ID NO: 3082,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4702, and the third antisense oligomer comprises a base sequence of SEQ ID NO: 3082,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4752, and the third antisense oligomer comprises a base sequence of SEQ ID NO: 3082,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4923, and the third antisense oligomer comprises a base sequence of SEQ ID NO: 3082,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4926, and the third antisense oligomer comprises a base sequence of SEQ ID NO: 3082,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4936, and the third antisense oligomer comprises a base sequence of SEQ ID NO: 3082,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4977, and the third antisense oligomer comprises a base sequence of SEQ ID NO: 3082,
   the first unit oligomer comprises a base sequence of SEQ ID NO: 1201, the second unit oligomer comprises a base sequence of SEQ ID NO: 151, the second antisense oligomer comprises a base sequence of SEQ ID NO: 4977, and the third antisense oligomer comprises a base sequence of SEQ ID NO: 3096, or
   the first unit oligomer comprises a base sequence of SEQ ID NO: 1180, the second unit oligomer comprises a base sequence of SEQ ID NO: 151, the second antisense oligomer comprises a base sequence of SEQ ID NO: 4977, and the third antisense oligomer comprises a base sequence of SEQ ID NO: 3096.
(20) The combination according to (18) or (19), wherein the first unit oligomer comprises a base sequence of SEQ ID NO: 1201, the second unit oligomer comprises a base sequence of SEQ ID NO: 151, the second antisense oligomer comprises a base sequence of SEQ ID NO: 4950 or 4880, and the third antisense oligomer comprises a base sequence of SEQ ID NO: 3082, 3090, or 3096.
(21) The combination according to any one of (1) to (20), the combination causing skipping of all exons from the 45th exon to the 55th exon in the human dystrophin pre-mRNA.
(22) The combination according to any one of (1) to (11), wherein the first and second antisense oligomers are oligonucleotides, or the combination according to any one of (12) to (21), wherein the first to third antisense oligomers are oligonucleotides.
(23) The combination according to (22), wherein a sugar moiety and/or a phosphate-binding region of at least one nucleotide constituting the oligonucleotide is modified.
(24) The combination according to (22) or (23), wherein the sugar moiety of at least one nucleotide constituting the oligonucleotide is a ribose in which the 2'-OH group is replaced by any one group selected from the group consisting of -OR, -R, -R'OR, -SH, -SR, -NH₂, -NHR, -NR₂, -N₃, -CN, -F, -Cl, -Br, and -I
   (wherein R is an alkyl or an aryl and R' is an alkylene).
(25) The combination according to any one of (22) to (24), wherein the phosphate-binding region of at least one nucleotide constituting the oligonucleotide is any one selected from the group consisting of a phosphorothioate bond, a phosphorodithioate bond, an alkylphosphonate bond, a phosphoramidate bond and a boranophosphate bond.
(26) The combination according to any one of (1) to (11), wherein the first and second antisense oligomers are morpholino oligomers, or the combination according to any one of (12) to (21), wherein the first to third antisense oligomers are oligonucleotides.
(27) The combination according to (26), wherein the first to third antisense oligomers are phosphorodiamidate morpholino oligomers.
(28) The combination according to (26) or (27), wherein the 5' end of each of the first to third antisense oligomers is a group represented by any one of the following chemical formulae (1) to (3): 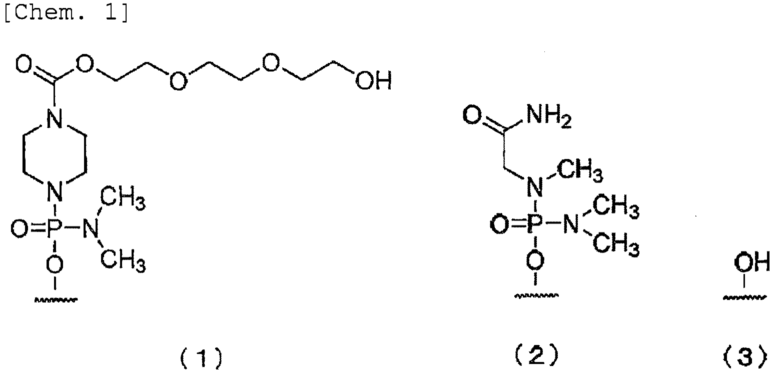
(29)
   (a) A pharmaceutical composition comprising the first and second antisense oligomers according to any one of (1) to (28), or pharmaceutically acceptable salts thereof, or hydrates thereof, or
   (b) a pharmaceutical combination comprising (i) a pharmaceutical composition comprising the first antisense oligomer according to any one of (1) to (28), or a pharmaceutically acceptable salt thereof, or a hydrate thereof, and (ii) a pharmaceutical composition comprising the second antisense oligomer according to any one of (1) to (28), or a pharmaceutically acceptable salt thereof, or a hydrate thereof.
(30)
   (a) A pharmaceutical composition comprising the first to third antisense oligomers according to any one of (12) to (28), or pharmaceutically acceptable salts thereof, or hydrates thereof, or
   (b) a pharmaceutical combination comprising (i) a pharmaceutical composition comprising the first antisense oligomer according to any one of (12) to (28), or a pharmaceutically acceptable salt thereof, or a hydrate thereof, (ii) a pharmaceutical composition comprising the second antisense oligomer according to any one of (12) to (28), or a pharmaceutically acceptable salt thereof, or a hydrate thereof, and (iii) a pharmaceutical composition comprising the third antisense oligomer according to any one of (12) to (28), or a pharmaceutically acceptable salt thereof, or a hydrate thereof.
(31) The pharmaceutical composition or the pharmaceutical combination according to (29) or (30), wherein the pharmaceutical composition further comprises a pharmaceutically acceptable carrier.
(32) The pharmaceutical composition or the pharmaceutical combination according to any one of (29) to (31), for treatment of muscular dystrophy.
(33) The pharmaceutical composition or the pharmaceutical combination according to any one of (29) to (32), for being administered to a human patient.
(34) A method for treatment of muscular dystrophy, comprising administering to a patient with muscular dystrophy (i) the first and second antisense oligomers according to any one of (1) to (28), or pharmaceutically acceptable salts thereof, or hydrates thereof, (ii) the first to third antisense oligomers according to any one of (12) to (28), or pharmaceutically acceptable salts thereof, or hydrates thereof, or (iii) the pharmaceutical composition or the pharmaceutical combination according to any one of (29) to (33).
(35) The method for treatment according to (34), wherein the muscular dystrophy patient is a patient with a mutation that is a target of exon 45 to 55 skipping in dystrophin gene.
(36) The method for treatment according to (34) or (35), wherein the patient is a human.

The present invention provides a combination of antisense oligomers that cause simultaneous skipping of a plurality of exons in a target. Another aspect of the present invention provides a pharmaceutical composition or combination for treating muscular dystrophy patients having various mutations by causing simultaneous skipping of a plurality of exons in objective pre-mRNA. An alternative aspect of the present invention enables simultaneous skipping of exons 45 to 55 in human dystrophin pre-mRNA to be caused with a high efficiency.

### Brief Description of Drawings

[Figure 1] Figure 1 is a diagram showing results of studying exon 45 to 55 skipping in mouse dystrophin pre-mRNA in H2K-mdx52 cells by RT-PCR (total concentration of added PMO: 30 µM).
[Figure 2] Figure 2 is a diagram showing results of studying exon 45 skipping in mouse dystrophin pre-mRNA in H2K-mdx52 cells by RT-PCR (total concentration of added PMO: 30 µM).
[Figure 3] Figure 3 is a diagram showing results of studying exon 45 to 55 skipping in mouse dystrophin pre-mRNA in H2K-mdx52 cells by RT-PCR. In the drawing, "2-2" indicates a result obtained by treatment with Mixture 2 + PMO No. 3 (1:1), "2-4" indicates a result obtained by treatment with Mixture 2 + PMO No. 3 (1:2), "2-5" indicates a result obtained by treatment with PMO No. 3 singly, "2-7" indicates a result obtained by treatment with Mixture 2 singly, and "NT" means "not treated" (total concentration of added PMO: 15 µM).
[Figure 4] Figure 4 is a diagram showing results of studying exon 45 skipping in mouse dystrophin pre-mRNA in H2K-mdx52 cells by RT-PCR. In the drawing, "2-2" indicates a result obtained by treatment with Mixture 2 + PMO No. 3 (1:1), "2-4" indicates a result obtained by treatment with Mixture 2 + PMO No. 3 (1:2), "2-5" indicates a result obtained by treatment with PMO No. 3 singly, "2-7" indicates a result obtained by treatment with Mixture 2 singly, and NT means "not treated" (total concentration of added PMO: 15 µM).
[Figure 5] Figure 5 is a diagram showing results of studying exon 45 to 55 skipping in mouse dystrophin pre-mRNA in H2K-mdx52 cells by RT-PCR. In the drawing, "3-2" indicates a result obtained by treatment with Mixture 2 + PMO No. 4 (1:1), "3-4" indicates a result obtained by treatment with Mixture 2 + PMO No. 4 (1:2), "3-5" indicates a result obtained by treatment with PMO No. 4 singly, "3-7" indicates a result obtained by treatment with Mixture 2 singly, and "NT" means "not treated" (total concentration of added PMO: 15 µM).
[Figure 6] Figure 6 is a diagram showing results of studying exon 45 skipping in mouse dystrophin pre-mRNA in H2K-mdx52 cells by RT-PCR. In the drawing, "3-2" indicates a result obtained by treatment with Mixture 2 + PMO No. 4 (1:1), "3-4" indicates a result obtained by treatment with Mixture 2 + PMO No. 4 (1:2), "3-5" indicates a result obtained by treatment with PMO No. 4 singly, "3-7" indicates a result obtained by treatment with Mixture 2 singly, and NT means "not treated" (total concentration of added PMO: 15 µM).
[Figure 7] Figure 7 is a diagram showing results of studying exon 45 to 55 skipping in mouse dystrophin pre-mRNA in H2K-mdx52 cells by RT-PCR. In the drawing, "2-1" indicates a result obtained by treatment with Mixture 2 singly, "2-2" indicates a result obtained by treatment with Mixture 2 + PMO No. 3 (1:1), "2-3" indicates a result obtained by treatment with Mixture 2 + PMO No. 3 (2:1), "2-4" indicates a result obtained by treatment with Mixture 2 + PMO No. 3 (3:1), and "NT" means "not treated" (total concentration of added PMO: 15 µM).
[Figure 8] Figure 8 is a diagram showing results of studying exon 45 skipping in mouse dystrophin pre-mRNA in H2K-mdx52 cells by RT-PCR. In the drawing, "2-1" indicates a result obtained by treatment with Mixture 2 singly, "2-2" indicates a result obtained by treatment with Mixture 2 + PMO No. 3 (1:1), "2-3" indicates a result obtained by treatment with Mixture 2 + PMO No. 3 (2:1), "2-4" indicates a result obtained by treatment with Mixture 2 + PMO No. 3 (3:1), and "NT" means "not treated" (total concentration of added PMO: 15 µM).

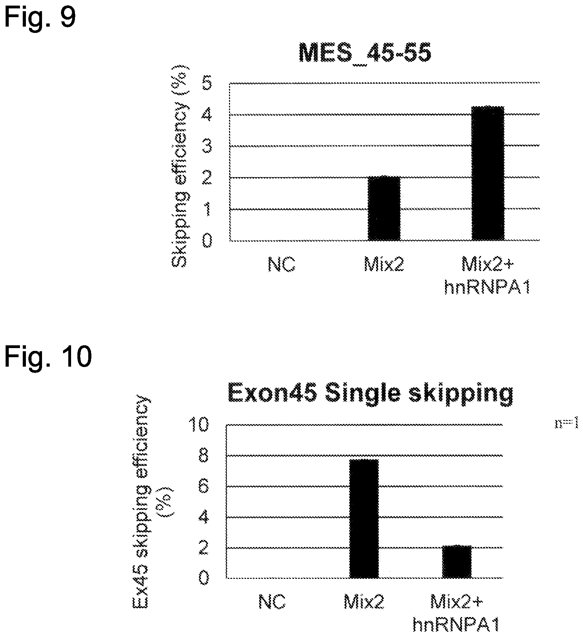

[Figure 9] Figure 9 is a diagram showing results of studying exon 45 to 55 skipping in mouse dystrophin pre-mRNA in H2K-mdx52 cells by RT-PCR. In the drawing, "NC" indicates a result obtained by treatment with Endo-porter singly, "Mix 2" indicates a result obtained by treatment with a mixture of PMO No. 1 and PMO No. 2 both in a final concentration of 25 µM, and "Mix 2 + hnRNP A1" indicates a result obtained by treatment with a mixture of PMO No. 1 and PMO No. 2 both in a final concentration of 18.75 µM, and PMO No. 3 in a final concentration of 12.5 µM (total concentration of added PMO: 50 µM).
[Figure 10] Figure 10 is a diagram showing results of studying exon 45 skipping in mouse dystrophin pre-mRNA in H2K-mdx52 cells by RT-PCR. In the drawing, "NC" indicates a result obtained by treatment with Endo-porter singly, "Mix 2" indicates a result obtained by treatment with a mixture of PMO No. 1 and PMO No. 2 both in a final concentration of 25 µM, and "Mix 2 + hnRNP A1" indicates a result obtained by treatment with a mixture of PMO No. 1 and PMO No. 2 both in a final concentration of 18.75 µM, and PMO No. 3 in a final concentration of 12.5 µM (total concentration of added PMO: 50 µM).

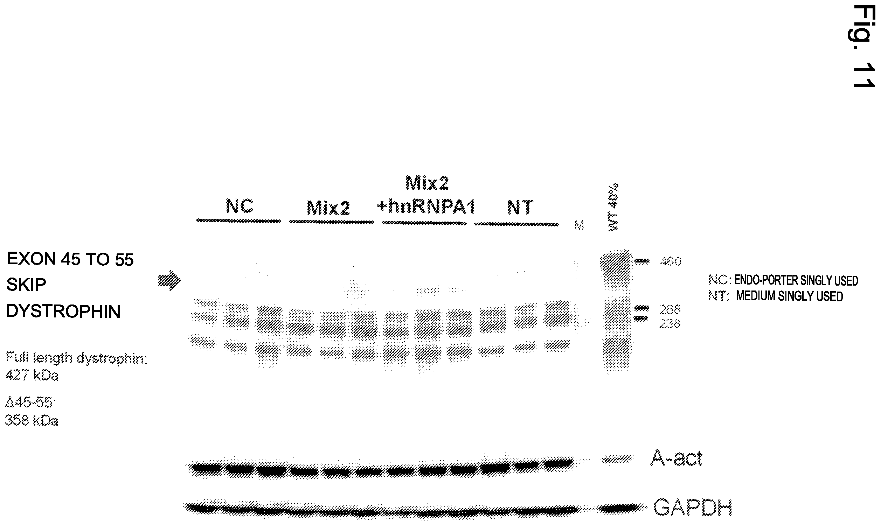

[Figure 11] Figure 11 is a diagram showing results of studying, by Western blotting, expression of dystrophin protein by exon 45 to 55 skipping in mouse dystrophin pre-mRNA in H2K-mdx52 cells. In the drawing, "NC" indicates a result obtained by treatment with Endo-porter singly, "Mix 2" indicates a result obtained by treatment with a mixture of PMO No. 1 and PMO No. 2 both in a final concentration of 25 µM, "Mix 2 + hnRNP A1" indicates a result obtained by treatment with a mixture of PMO No. 1 and PMO No. 2 both in a final concentration of 18.75 µM, and PMO No. 3 in a final concentration of 12.5 µM, and "NT" means "not treated" (total concentration of added PMO: 50 µM).
[Figure 12] Figure 12 is a diagram showing results of studying exon 45 to 55 multi-exon skipping in normal human-derived myoblasts by RT-PCR.
[Figure 13] Figure 13 is a diagram showing results of studying exon 45 skipping in normal human-derived myoblasts by RT-PCR.
[Figure 14] Figure 14 is a diagram showing results of studying exon 45 to 55 multi-exon skipping in DMD patient-derived myoblasts with exon 48 to 50 deletion by RT-PCR.
[Figure 15] Figure 15 is a diagram showing results of studying exon 45 skipping in DMD patient-derived myoblasts with exon 48 to 50 deletion by RT-PCR.
[Figure 16] Figure 16 is a diagram showing results of studying exon 45 to 55 multi-exon skipping in DMD patient-derived myoblasts with exon 48 to 50 deletion by RT-PCR.
[Figure 17] Figure 17 is a diagram showing results of studying exon 45 skipping in DMD patient-derived myoblasts with exon 48 to 50 deletion by RT-PCR.
[Figure 18] Figure 18 is a diagram showing results of studying exon 45 to 55 multi-exon skipping in DMD patient-derived myoblasts with exon 48 to 50 deletion by Western blotting.
[Figure 19] Figure 19 is a diagram showing results of studying exon 45 to 55 multi-exon skipping in DMD patient-derived myoblasts with exon 46 to 51 deletion by RT-PCR.
[Figure 20] Figure 20 is a diagram showing results of studying exon 45 skipping in DMD patient-derived myoblasts with exon 46 to 51 deletion by RT-PCR.
[Figure 21] Figure 21 is a diagram showing results of studying exon 45 to 55 multi-exon skipping in DMD patient-derived myoblasts with exon 46 to 51 deletion by RT-PCR.
[Figure 22] Figure 22 is a diagram showing results of studying exon 45 skipping in DMD patient-derived myoblasts with exon 46 to 51 deletion by RT-PCR.
[Figure 23] Figure 23 is a diagram showing results of studying exon 45 to 55 multi-exon skipping in DMD patient-derived myoblasts with exon 46 to 51 deletion by Western blotting.
[Figure 24] Figure 24 is a diagram showing results of studying exon 45 to 55 multi-exon skipping in DMD patient-derived myoblasts with exon 51 deletion by RT-PCR.
[Figure 25] Figure 25 is a diagram showing results of studying exon 45 skipping in DMD patient-derived myoblasts with exon 51 deletion by RT-PCR.
[Figure 26] Figure 26 is a diagram showing results of studying exon 45 to 55 multi-exon skipping in DMD patient-derived myoblasts with exon 51 deletion by RT-PCR.
[Figure 27] Figure 27 is a diagram showing results of studying exon 45 skipping in DMD patient-derived myoblasts with exon 51 deletion by RT-PCR.
[Figure 28] Figure 28 is a diagram showing results of studying exon 45 to 55 multi-exon skipping in DMD patient-derived myoblasts with exon 51 deletion by RT-PCR.
[Figure 29] Figure 29 is a diagram showing results of studying exon 45 skipping in DMD patient-derived myoblasts with exon 51 deletion by RT-PCR.
[Figure 30] Figure 30 is a diagram showing results of studying exon 45 to 55 multi-exon skipping in DMD patient-derived myoblasts with exon 51 deletion by RT-PCR.
[Figure 31] Figure 31 is a diagram showing results of studying exon 45 skipping in DMD patient-derived myoblasts with exon 51 deletion by RT-PCR.
[Figure 32] Figure 32 is a diagram showing results of studying exon 45 to 55 multi-exon skipping in DMD patient-derived myoblasts with exon 51 deletion by RT-PCR.
[Figure 33] Figure 33 is a diagram showing results of studying exon 45 skipping in DMD patient-derived myoblasts with exon 51 deletion by RT-PCR.
[Figure 34] Figure 34 is a diagram showing results of studying exon 45 to 55 multi-exon skipping in DMD patient-derived myoblasts with exon 51 deletion by RT-PCR.
[Figure 35] Figure 35 is a diagram showing results of studying exon 45 skipping in DMD patient-derived myoblasts with exon 51 deletion by RT-PCR.
[Figure 36] Figure 36 is a diagram showing results of studying exon 45 to 55 multi-exon skipping in DMD patient-derived myoblasts with exon 51 deletion by RT-PCR.
[Figure 37] Figure 37 is a diagram showing results of studying exon 45 skipping in DMD patient-derived myoblasts with exon 51 deletion by RT-PCR.
[Figure 38] Figure 38 is a diagram showing results of studying exon 45 to 55 multi-exon skipping in DMD patient-derived myoblasts with exon 51 deletion by Western blotting.
[Figure 39] Figure 39 is a diagram showing results of studying exon 45 to 55 multi-exon skipping in DMD patient-derived myoblasts with exon 51 deletion by Western blotting.

### Description of Embodiments

Hereinafter, the present invention is described in detail. The embodiments described below are intended to be presented by way of example merely to describe the invention but not to limit the invention only to the following embodiments. The present invention may be implemented in various ways without departing from the gist of the invention.

### 1. Combination of antisense oligomers

The present invention provides a combination of antisense oligomers or pharmaceutically acceptable salts thereof, or hydrates thereof which cause simultaneous skipping of two or more numerically consecutive exons selected from the group consisting of the 45th exon to the 55th exon in human dystrophin pre-mRNA, the combination comprising:
(i) a first antisense oligomer or a pharmaceutically acceptable salt thereof, or a hydrate thereof, comprising:
   a first unit oligomer comprising a base sequence complementary to a base sequence consisting of a base sequence of 11 bases in the upstream direction from the 3' end of the 44th intron and a base sequence of 69 bases in the downstream direction from the 5' end of the 45th exon in the human dystrophin pre-mRNA, or a partial base sequence thereof; and
   a second unit oligomer comprising a base sequence complementary to a base sequence of from the 52nd to 75th bases in the upstream direction from the 3' end of the 44th intron in the human dystrophin pre-mRNA, or a partial base sequence thereof; and
(ii) a second antisense oligomer or a pharmaceutically acceptable salt thereof, or a hydrate thereof, comprising a base sequence complementary to a base sequence consisting of a base sequence of 33 bases in the upstream direction from the 3' end of the 54th intron and a base sequence of 53 bases in the downstream direction from the 5' end of the 55th exon in the human dystrophin pre-mRNA, or a partial base sequence thereof. The foregoing combination is hereinafter referred to also as the "combination of the present invention".

As used herein, the term "combination" means a substance combination, a pharmaceutical combination, an agent combination, and the like. In one embodiment, respective antisense oligomers in the combination of the present invention are comprised in one pharmaceutical composition, and simultaneously administered. In another embodiment, respective antisense oligomers in the combination of the present invention are comprised in a plurality of pharmaceutical compositions, and separately (simultaneously or sequentially) administered. As used herein, the term "simultaneously" administering a plurality of pharmaceutical compositions means that a plurality of pharmaceutical compositions are administered at the same time. As used herein, the term "sequentially" administering a plurality of pharmaceutical compositions means that these are administered at different times. Specifically, one pharmaceutical composition may be administered before or after another pharmaceutical composition, and an administration interval in this case is not limited, but may be, for example, a few minutes, a few hours, or a few days.

Hereinafter, a first antisense oligomer or a pharmaceutically acceptable salt thereof, or a hydrate thereof, and a second antisense oligomer or a pharmaceutically acceptable salt thereof, or a hydrate thereof (and optionally a third antisense oligomer or a pharmaceutically acceptable salt thereof, or a hydrate thereof described herein) may be collectively referred to as the "antisense oligomer of the present invention". The antisense oligomer of the present invention may refer to each of antisense oligomers or pharmaceutically acceptable salts thereof, or hydrates thereof. A first antisense oligomer or a pharmaceutically acceptable salt thereof, or a hydrate thereof described above as (i) may be referred to as the "first antisense oligomer of the present invention", and a second antisense oligomer or a pharmaceutically acceptable salt thereof, or a hydrate thereof described above as (ii) may be referred to as the "second antisense oligomer of the present invention".

As used herein, the term "gene" is intended to mean a genomic gene and also include cDNA, pre-mRNA and mRNA. Preferably, the gene is pre-mRNA. As used herein, the term "pre-mRNA" is an RNA molecule comprising an exon and an intron transcribed from a target gene on the genome and is a mRNA precursor.

The human dystrophin pre-mRNA is an RNA molecule comprising an exon and an intron transcribed from the human dystrophin gene on the genome and is a mRNA precursor. Those skilled in the art can obtain information on the base sequence of the human dystrophin pre-mRNA by analogy from the genomic sequence of the human dystrophin gene (GenBank Accession Nos. NG _012232.1).

In the human genome, the human dystrophin gene locates at locus Xp21.2. The human dystrophin gene has a size of about 3.0 Mbp and is the largest gene among known human genes. However, the coding regions of the human dystrophin gene are only about 14 kb, distributed as 79 exons throughout the human dystrophin gene (Roberts, RG, et al., Genomics, 16: 536-538 (1993)). The pre-mRNA, which is the transcript of the human dystrophin gene, undergoes splicing to generate mature mRNA of about 14 kb. The base sequence of mature mRNA of human wild-type dystrophin gene is known (GenBank Accession Nos. NM_004006).

The first antisense oligomer of the present invention comprises the first unit oligomer and the second unit oligomer, or consists of the first unit oligomer and the second unit oligomer.

The first unit oligomer targets a base sequence of 11 bases in the upstream direction from the 3' end of the 44th intron and a base sequence of 69 bases in the downstream direction from the 5' end of the 45th exon in the human dystrophin pre-mRNA. As used herein, the term "targeting" means that an intended base sequence is a base sequence complementary to the base sequence of a target region or a partial base sequence of the target sequence.

A target sequence of the first unit oligomer can be indicated by the range of -11 bases to +69 bases when the boundary between the 3' end of intron 44 and the 5' end of exon 45 is defined as basing point 0, a base sequence region on the 5' side (upstream) from the basing point in the dystrophin gene is indicated by "-" (minus), and a base sequence region on the 3' side (downstream) therefrom is indicated by "+". In this respect, the region indicated by the range of -11 bases to -1 base belongs to intron 44, and the region indicated by the range of +1 base to +69 bases belongs to exon 45.

The first unit oligomer comprises a base sequence complementary to a base sequence consisting of a base sequence of 11 bases in the upstream direction from the 3' end of the 44th intron and a base sequence of 69 bases in the downstream direction from the 5' end of the 45th exon in the human dystrophin pre-mRNA, or a partial base sequence thereof.

The second unit oligomer targets a base sequence of from the 52nd to 75th bases in the upstream direction from the 3' end of the 44th intron in the human dystrophin pre-mRNA.

A target sequence of the second unit oligomer can be indicated by the range of -75 bases to -52 bases when the boundary between the 3' end of intron 44 and the 5' end of exon 45 is defined as basing point 0, a base sequence region on the 5' side (upstream) from the basing point in the dystrophin gene is indicated by "-" (minus), and a base sequence region on the 3' side (downstream) therefrom is indicated by "+". In this respect, the region indicated by the range of -75 bases to -52 bases belongs to intron 44.

The second unit oligomer comprises a base sequence complementary to a base sequence of from the 52nd to 75th bases in the upstream direction from the 3' end of the 44th intron in the human dystrophin pre-mRNA, or a partial base sequence thereof.

The second antisense oligomer of the present invention targets a base sequence consisting of a base sequence of 33 bases in the upstream direction from the 3' end of the 54th intron and a base sequence of 53 bases in the downstream direction from the 5' end of the 55th exon in the human dystrophin pre-mRNA.

A target sequence of the second antisense oligomer can be indicated by the range of -33 bases to +53 bases when the boundary between the 3' end of intron 54 and the 5' end of exon 55 is defined as basing point 0, a base sequence region on the 5' side (upstream) from the basing point in the dystrophin gene is indicated by "-" (minus), and a base sequence region on the 3' side (downstream) therefrom is indicated by "+". In this respect, the region indicated by the range of -33 bases to -1 base belongs to intron 54, and the region indicated by the range of +1 base to +53 bases belongs to exon 55.

The second antisense oligomer comprises a base sequence complementary to a base sequence of 33 bases in the upstream direction from the 3' end of the 54th intron and a base sequence of 53 bases in the downstream direction from the 5' end of the 55th exon in the human dystrophin pre-mRNA, or a partial base sequence thereof.

The combination of the present invention may further comprise, in addition to the first antisense oligomer and the second antisense oligomer of the present invention, a third antisense oligomer or a pharmaceutically acceptable salt thereof, or a hydrate thereof, comprising a base sequence complementary to a base sequence consisting of a base sequence of 23 bases in the upstream direction from the 3' end of the 45th exon, and a base sequence of 73 bases in the downstream direction from the 5' end of the 45th intron in the human dystrophin pre-mRNA, or a partial base sequence thereof. Hereinafter, a third antisense oligomer or a pharmaceutically acceptable salt thereof, or a hydrate thereof is referred to also as the "third antisense oligomer of the present invention".

The third antisense oligomer of the present invention targets a base sequence consisting of a base sequence of 23 bases in the upstream direction from the 3' end of the 45th exon and a base sequence of 73 bases in the downstream direction from the 5' end of the 45th intron in the human dystrophin pre-mRNA.

A target sequence of the third antisense oligomer can be indicated by the range of -23 bases to +73 bases when the boundary between the 3' end of exon 45 and the 5' end of intron 46 is defined as basing point 0, a base sequence region on the 5' side (upstream) from the basing point in the dystrophin gene is indicated by "-" (minus), and a base sequence region on the 3' side (downstream) therefrom is indicated by "+". In this respect, the region indicated by the range of -23 bases to -1 base belongs to exon 45, and the region indicated by the range of +1 base to +73 bases belongs to intron 46.

The third antisense oligomer comprises a base sequence complementary to a base sequence consisting of a base sequence of 23 bases in the upstream direction from the 3' end of the 45th exon and a base sequence of 73 bases in the downstream direction from the 5' end of the 45th intron in the human dystrophin pre-mRNA, or a partial base sequence thereof.

Specific examples of surrounding sequences of the target sequences of the first unit oligomer and the second unit oligomer comprised in the first antisense oligomer, the second antisense oligomer, and the third antisense oligomer of the present invention include those shown in Table 1 below.

### [Table 1]

**Table 1**

| **Target surrounding sequence** | |
|---|---|
| **Range of -600 to +69 bases based on basing point of 3' end of intron 44 (including H45_ (-75)-(-52): range of -75 to -52 bases based on basing point of 3' end of intron 44, and H45_(-11)-(69): range of - 11 to +69 bases based on basing point of 3' end of intron 44)** | **SEQ ID NO:** |
| TCTTGATGGGATGCTCCTGAAAGCAATTAATTCTCAGTTTTTTGTGGCTTCTAATGCAAAATACATTGACGCAGACAGAATTTGAA ATGAATTTTCTTCTAATATAGCAATTAATTTTATTTAAATATCTCTAGAGTTTTTTTTTAATACTGTGACTAACCTATGTTTGTTC TTTTTCACCTCTCGTATCCACGATCACTAAGAAACCCAAATACTTTGTTCATGTTTAAATTTTACAACATTTCATAGACTATTAAA CATGGAACATCCTTGTGGGGACAAGAAATCGAATTTGCTCTTGAAAAGGTTTCCAACTAATTGATTTGTAGGACATTATAACATCC TCTAGCTGACAAGCTTACAAAAATAAAAACTGGAGCTAACCGAGAGGGTGCTTTTTTCCCTGACACATAAAAGGTGTCTTTCTGTC TTGTATCCTTTGGATATGGGCATGTCAGTTTCATAGGGAAATTTTCACATGGAGCTTTTGTATTTCTTTCTTTGCCAGTACAACTG CATGTGGTAGCACACTGTTTAATCTTTTCTCAAATAAAAAGACATGGGGCTTCATTTTTGTTTTGCCTTTTTGGTATCTTACAGGA ACTCCAGGATGGCATTGGGCAGCGGCAAACTGTTGTCAGAACATTGAATGCAACTGGGGAAGAAATA | **5091** |

| **Ragne of -23 to +400 bases based on basing point of 5' end of intron 45 (including H45_(154)-(249): range of -23 to +73 bases based on basing point of 5' end of intron 45)** | |
|---|---|
| CAGCTGTCAGACAGAAAAAAGAGGTAGGGCGACAGATCTAATAGGAATGAAAACATTTTAGCAGACTTTTTAAGCTTTCTTTAGAA GAATATTTCATGAGAGATTATAAGCAGGGTGAAAGGCACTAACATTAAAGAACCTATCAACCATTAATCAACAGCAGTAAAGAAAT TTTTTATTTCTTTTTTTCATATACTAAAATATATACTTGTGGCTAGTTAGTGGTTTTCTGCTATTTTAAACTTGAAGTTTGCTTTA AAAATCACCCATGATTGCTTAAAGGTGAATATCTTCAATATATTTTAACTTCAACAAGCTGAATCTCAGTTGTTTTTCAAGAAGAT TTTAGAAAGCAATTATAAATGATTGTTTTGTAGGAAAGACAGATCTTTGCTTAGTTTTAAAAATAGCTATGAATATGAC | **5092** |

| **Range of -400 to +53 bases based on basing point of 3' and of intron 54 (including H55_(-33)-(+53): range of -33 to +53 bases based on basing point of 3' end of intron 54)** | |
|---|---|
| TCTCAAATTTGGCAGTATATTAAAAATAAGCTTTCAAAATTGACCAACAAAAACTACAAAATTGAAAAAAAGGTACTTTGAACTTT CACATGTTCAAATATATGTATATATATTTCACATATATATATGAAACCTCCTCTGTGGAGAGGGGTTTATAGAAATCTGTAATTGT CATTCTTGCATGCCTTCCCCCATACAAACGCCTTTAAGTTAAATAAAAATGAAAGTAAATAGACTGCACAATATTATAGTTGTTGC TTAAAGGAAGAGCTGTAGCAACAACTCACCCCATTGTTGGTATATTACAATTTAGTTCCTCCATCTTTCTCTTTTTATGGAGTTCA CTAGGTGCACCATTCTGATATTTAATAATTGCATCTGAACATTTGGTCCTTTGCAGGGTGAGTGAGCGAGAGGCTGCTTTGGAAGA AACTCATAGATTACTGCAACAGT | **5093** |

Specific examples of the target sequences of the first unit oligomer and the second unit oligomer comprised in the first antisense oligomer, the second antisense oligomer, and the third antisense oligomer of the present invention include those shown in Table 2 below.

### [Table 2]

**Table 2**

| **Target sequence** | **SEQ ID NO:** |
|---|---|
| **H45_(-75)-(-52) (range of -75 to -52 bases based on basing point of 3' end of intron 44)** | |
| GCACACTGTTTAATCTTTTCTCAA | 5094 |
| **H45_(-11)-(+69) (range of -11 to +69 bases based on basing point of 3' end of intron 44)** | 5095 |
| GTATCTTACAGGAACTCCAGGATGGCATTGGGCAGCGGCAAACTGTTGTCAGAACATTGAATGCAACTGGGGAAGAAATA | |
| **H45_(+154)-(+249) (range of -23 to +73 bases based on basing point of 5' end of intron 45)** | 5096 |
| CAGCTGTCAGACAGAAAAAAGAGGTAGGGCGACAGATCTAATAGGAATGAAAACATTTTAGCAGACTTTTTAAGCTTTCTTTAGAAGAATATTTCA | |
| **H55_(-33)-(+53) (range of -33 to +53 bases based on basing point of 3' end of intron 54)** | 5097 |
| AATAATTGCATCTGAACATTTGGTCCTTTGCAGGGTGAGTGAGCGAGAGGCTGCTTTGGAAGAAACTCATAGATTACTGCAACAGT | |

As used herein, thymine "T" and uracil "U" are interchangeable with each other. Neither "T" nor "U" essentially influences the exon skipping activity of the antisense oligomer of the present invention. Therefore, as used herein, identical base sequences except for "T" or "U" are represented by the same SEQ ID NO. In the tables below, "U" may be described as "T" even in the base sequence of pre-mRNA. Those skilled in the art can understand an RNA sequence by appropriately replacing "T" with "U".

Herein, a target base sequence is described as "Ha_b-c".

"Ha" represents the ath exon of the human dystrophin gene, "b" represents the 5'-terminal base of the target base sequence, and "c" represents the 3'-terminal base of the target base sequence.

When "b" and "c" are positive integers, "b" and "c" each represent a base number in the downstream direction when the 5'-terminal base of the ath exon is counted as the 1st base. On the other hand, when "b" and "c" are negative integers, "b" and "c" each represent a base number in the upstream direction when the 3'-terminal base of the (a - 1)th intron is counted as the 1st base.

For example, "H55_(-75)-(-52)" means a base sequence in which the 5' end of the target base sequence is the 75th base in the upstream direction from the 3' end of the 54th intron and the 3' end of the target base sequence is the 52nd base in the upstream direction from the 3' end of the 54th intron.

The surrounding sequence of the target region or the target sequence of the antisense oligomer of the present invention includes both wild (e.g., the base sequences represented by SEQ ID NOs: 5021 to 5027) and mutant types in relation to the human dystrophin pre-mRNA. Such a mutant type has, for example, any one base sequence selected from the group consisting of base sequences (B0) and (B1) to (B16) below:
(B0) a base sequence that hybridizes under stringent conditions to a base sequence complementary to any one base sequence selected from the group consisting of SEQ ID NOs: 5021 to 5027;
(B1) a base sequence that has at least 85% identity with any one base sequence selected from the group consisting of SEQ ID NOs: 5021 to 5027, and has a length within ±15% of the length of the any one base sequence selected;
(B2) a base sequence that has at least 86% identity with any one base sequence selected from the group consisting of SEQ ID NOs: 5021 to 5027, and has a length within ±14% of the length of the any one base sequence selected;
(B3) a base sequence that has at least 87% identity with any one base sequence selected from the group consisting of SEQ ID NOs: 5021 to 5027, and has a length within ±13% of the length of the any one base sequence selected;
(B4) a base sequence that has at least 88% identity with any one base sequence selected from the group consisting of SEQ ID NOs: 5021 to 5027, and has a length within ±12% of the length of the any one base sequence selected;
(B5) a base sequence that has at least 89% identity with any one base sequence selected from the group consisting of SEQ ID NOs: 5021 to 5027, and has a length within ±11% of the length of the any one base sequence selected;
(B6) a base sequence that has at least 90% identity with any one base sequence selected from the group consisting of SEQ ID NOs: 5021 to 5027, and has a length within ±10% of the length of the any one base sequence selected;
(B7) a base sequence that has at least 91% identity with any one base sequence selected from the group consisting of SEQ ID NOs: 5021 to 5027, and has a length within ±9% of the length of the any one base sequence selected;
(B8) a base sequence that has at least 92% identity with any one base sequence selected from the group consisting of SEQ ID NOs: 5021 to 5027, and has a length within ±8% of the length of the any one base sequence selected;
(B9) a base sequence that has at least 93% identity with any one base sequence selected from the group consisting of SEQ ID NOs: 5021 to 5027, and has a length within ±7% of the length of the any one base sequence selected;
(B10) a base sequence that has at least 94% identity with any one base sequence selected from the group consisting of SEQ ID NOs: 5021 to 5027, and has a length within ±6% of the length of the any one base sequence selected;
(B11) a base sequence that has at least 95% identity with any one base sequence selected from the group consisting of SEQ ID NOs: 5021 to 5027, and has a length within ±5% of the length of the any one base sequence selected;
(B12) a base sequence that has at least 96% identity with any one base sequence selected from the group consisting of SEQ ID NOs: 5021 to 5027, and has a length within ±4% of the length of the any one base sequence selected;
(B13) a base sequence that has at least 97% identity with any one base sequence selected from the group consisting of SEQ ID NOs: 5021 to 5027, and has a length within ±3% of the length of the any one base sequence selected;
(B14) a base sequence that has at least 98% identity with any one base sequence selected from the group consisting of SEQ ID NOs: 5021 to 5027, and has a length within ±2% of the length of the any one base sequence selected;
(B15) a base sequence that has at least 99% identity with any one base sequence selected from the group consisting of SEQ ID NOs: 5021 to 5027, and has a length within ±1% of the length of the any one base sequence selected; and
(B16) a base sequence that has at least 99.5% identity with any one base sequence selected from the group consisting of SEQ ID NOs: 5021 to 5027, and has a length within ±0.5% of the length of the any one base sequence selected.

As used herein, the term "base sequence that hybridizes under stringent conditions" refers to, for example, a base sequence obtained by colony hybridization, plaque hybridization, Southern hybridization or the like, using as a probe all or part of a base sequence complementary to, e.g., any one base sequence selected from the group consisting of SEQ ID NOs: 5021 to 5027. The hybridization method which may be used includes methods described in, for example, "Sambrook & Russell, Molecular Cloning: A Laboratory Manual Vol. 3, Cold Spring Harbor, Laboratory Press, 2001," "Ausubel, Current Protocols in Molecular Biology, John Wiley & Sons, 1987-1997," etc.

As used herein, the term "complementary base sequence" is not limited to a base sequence that forms Watson-Crick pairs with an intended base sequence, and also includes a base sequence that forms wobble base pairs therewith. Herein, the Watson-Crick pair means a base pair that forms a hydrogen bond between adenine and thymine, between adenine and uracil, or between guanine and cytosine, and the wobble base pair means a base pair that forms a hydrogen bond between guanine and uracil, between inosine and uracil, between inosine and adenine, or between inosine and cytosine. The term "complementary base sequence" does not have to have 100% complementarity with the intended base sequence and may contain, for example, 1, 2, 3, 4, or 5 noncomplementary bases based on the intended base sequence or may be a base sequence shorter by 1 base, 2 bases, 3 bases, 4 bases, or 5 bases than the intended base sequence.

As used herein, the term "stringent conditions" may be any of low stringent conditions, moderate stringent conditions or high stringent conditions. The term "low stringent condition" is, for example, 5 × SSC, 5 × Denhardt's solution, 0.5% SDS, 50% formamide at 32°C. The term "moderate stringent condition" is, for example, 5 × SSC, 5 × Denhardt's solution, 0.5% SDS, 50% formamide at 42°C, or 5 × SSC, 1% SDS, 50 mM Tris-HCl (pH 7.5), 50% formamide at 42°C. The term "high stringent condition" is, for example, 5 × SSC, 5 × Denhardt's solution, 0.5% SDS, 50% formamide at 50°C, or 0.2 × SSC, 0.1% SDS at 65°C. Under these conditions, base sequences with higher homology are expected to be obtained efficiently at higher temperatures, although multiple factors are involved in hybridization stringency including temperature, probe concentration, probe length, ionic strength, time, salt concentration and others, and those skilled in the art may approximately select these factors to achieve similar stringency.

When commercially available kits are used for hybridization, for example, an Alkphos Direct Labelling and Detection System (GE Healthcare) may be used. In this case, according to the attached protocol, after cultivation with a labeled probe overnight, the membrane can be washed with a primary wash buffer containing 0.1% (w/v) SDS at 55°C, thereby detecting hybridization. Alternatively, when the probe is labeled with digoxigenin (DIG) using a commercially available reagent (e.g., a PCR Labelling Mix (Roche Diagnostics), etc.) in producing a probe based on all or part of the complementary sequence to any one base sequence selected from the group consisting of SEQ ID NOs: 233 to 256, 341 to 369, and 385 to 389, hybridization can be detected with a DIG Nucleic Acid Detection Kit (Roche Diagnostics) or the like.

The identity between base sequences may be determined using algorithm BLAST (Basic Local Alignment Search Tool) by Karlin and Altschul (Proc. Natl. Acad. Sci. USA 872264-2268, 1990; Proc. Natl. Acad. Sci. USA 90: 5873, 1993). Programs called BLASTN and BLASTX based on the BLAST algorithm have been developed (Altschul SF, et al: J. Mol. Biol. 215: 403, 1990). When a base sequence is sequenced using BLASTN, the parameters are, for example, score = 100 and wordlength = 12. When BLAST and Gapped BLAST programs are used, the default parameters for each program are employed.

The antisense oligomer of the present invention comprises a base sequence complementary to a base sequence of the target regions of the present invention, or a partial base sequence thereof. The term "partial" means a region, except for the full length, of the target regions, i.e., a partial region of the target regions. The partial region may be 10 to 60 bases long, 10 to 55 bases long, 10 to 50 bases long, 10 to 45 bases long, 10 to 40 bases long, 10 to 35 bases long, 10 to 30 bases long, 10 to 25 bases long, 15 to 60 bases long, 15 to 55 bases long, 15 to 50 bases long, 15 to 45 bases long, 15 to 40 bases long, 15 to 35 bases long, 15 to 30 bases long, 15 to 25 bases long, 16 to 60 bases long, 16 to 55 bases long, 16 to 50 bases long, 16 to 45 bases long, 16 to 40 bases long, 16 to 35 bases long, 16 to 30 bases long, 16 to 25 bases long, 17 to 60 bases long, 17 to 55 bases long, 17 to 50 bases long, 17 to 45 bases long, 17 to 40 bases long, 17 to 35 bases long, 17 to 30 bases long, 17 to 25 bases long, 18 to 60 bases long, 18 to 55 bases long, 18 to 50 bases long, 18 to 45 bases long, 18 to 40 bases long, 18 to 35 bases long, 18 to 30 bases long, 18 to 25 bases long, 19 to 60 bases long, 19 to 55 bases long, 19 to 50 bases long, 19 to 45 bases long, 19 to 40 bases long, 19 to 35 bases long, 19 to 30 bases long, 19 to 25 bases long, 20 to 60 bases long, 20 to 55 bases long, 20 to 50 bases long, 20 to 45 bases long, 20 to 40 bases long, 20 to 35 bases long, 20 to 30 bases long, 20 to 25 bases long, 15 to 30 bases long, 15 to 29 bases long, 15 to 28 bases long, 15 to 27 bases long, 15 to 26 bases long, 15 to 25 bases long, 15 to 24 bases long, 15 to 23 bases long, 15 to 22 bases long, 15 to 21 bases long, 15 to 20 bases long, 15 to 19 bases long, 15 to 18 bases long, 16 to 30 bases long, 16 to 29 bases long, 16 to 28 bases long, 16 to 27 bases long, 16 to 26 bases long, 16 to 25 bases long, 16 to 24 bases long, 16 to 23 bases long, 16 to 22 bases long, 16 to 21 bases long, 16 to 20 bases long, 16 to 19 bases long, 16 to 18 bases long, 17 to 30 bases long, 17 to 29 bases long, 17 to 28 bases long, 17 to 27 bases long, 17 to 26 bases long, 17 to 25 bases long, 17 to 24 bases long, 17 to 23 bases long, 17 to 22 bases long, 17 to 21 bases long, 17 to 20 bases long, 17 to 19 bases long, 17 to 18 bases long, 18 to 30 bases long, 18 to 29 bases long, 18 to 28 bases long, 18 to 27 bases long, 18 to 26 bases long, 18 to 25 bases long, 18 to 24 bases long, 18 to 23 bases long, 18 to 22 bases long, 18 to 21 bases long, 18 to 20 bases long, 18 to 19 bases long, 19 to 30 bases long, 19 to 29 bases long, 19 to 28 bases long, 19 to 27 bases long, 19 to 26 bases long, 19 to 25 bases long, 19 to 24 bases long, 19 to 23 bases long, 19 to 22 bases long, 19 to 21 bases long, 19 to 20 bases long, 20 to 30 bases long, 20 to 29 bases long, 20 to 28 bases long, 20 to 27 bases long, 20 to 26 bases long, 20 to 25 bases long, 20 to 24 bases long, 20 to 23 bases long, 20 to 22 bases long, 20 to 21 bases long, 5 to 25 bases long, 5 to 24 bases long, 5 to 23 bases long, 5 to 22 bases long, 5 to 21 bases long, 5 to 20 bases long, 5 to 19 bases long, 5 to 18 bases long, 5 to 17 bases long, 5 to 16 bases long, 5 to 15 bases long, 5 to 14 bases long, 5 to 13 bases long, 5 to 12 bases long, 7 to 25 bases long, 7 to 24 bases long, 7 to 23 bases long, 7 to 22 bases long, 7 to 21 bases long, 7 to 20 bases long, 7 to 19 bases long, 7 to 18 bases long, 7 to 17 bases long, 7 to 16 bases long, 7 to 15 bases long, 7 to 14 bases long, 7 to 13 bases long, 7 to 12 bases long, 9 to 25 bases long, 9 to 24 bases long, 9 to 23 bases long, 9 to 22 bases long, 9 to 21 bases long, 9 to 20 bases long, 9 to 19 bases long, 9 to 18 bases long, 9 to 17 bases long, 9 to 16 bases long, 9 to 15 bases long, 9 to 14 bases long, 9 to 13 bases long, 9 to 12 bases long, 10 to 25 bases long, 10 to 24 bases long, 10 to 23 bases long, 10 to 22 bases long, 10 to 21 bases long, 10 to 20 bases long, 10 to 19 bases long, 10 to 18 bases long, 10 to 17 bases long, 10 to 16 bases long, 10 to 15 bases long, 10 to 14 bases long, 10 to 13 bases long, 10 to 12 bases long, 60 bases long, 59 bases long, 58 bases long, 57 bases long, 56 bases long, 55 bases long, 54 bases long, 53 bases long, 52 bases long, 51 bases long, 50 bases long, 49 bases long, 48 bases long, 47 bases long, 46 bases long, 45 bases long, 44 bases long, 43 bases long, 42 bases long, 41 bases long, 40 bases long, 39 bases long, 38 bases long, 37 bases long, 36 bases long, 35 bases long, 34 bases long, 33 bases long, 32 bases long, 31 bases long, 30 bases long, 29 bases long, 28 bases long, 27 bases long, 26 bases long, 25 bases long, 24 bases long, 23 bases long, 22 bases long, 21 bases long, 20 bases long, 19 bases long, 18 bases long, 17 bases long, 16 bases long, 15 bases long, 14 bases long, 13 bases long, 12 bases long, 11 bases long, 10 bases long, 9 bases long, 8 bases long, 7 bases long, 6 bases long, or 5 bases long, but not limited thereto. These lengths may be increased or decreased by 1, 2, or 3 bases.

The antisense oligomer of the present invention has an activity to cause simultaneous skipping of any two or more numerically consecutive exons selected from the group consisting of the 45th exon to the 55th exon in human dystrophin pre-mRNA. As used herein, such skipping of two or more numerically consecutive exons from objective pre-mRNA is referred to as "multi-exon skipping" or "multi-skipping", and this activity is referred to as "multi-exon skipping activity" or "multi-skipping activity".

As used herein, the term "cause simultaneous skipping" of two or more numerically consecutive exons includes not only removal of the respective exons from pre-mRNA at completely the same timings but also sequential removal of the respective exons within a period from pre-mRNA to mature mRNA. Specifically, the term "cause simultaneous skipping" of two or more numerically consecutive exons refers to removal of a plurality of (two or more) numerically consecutive exons from pre-mRNA.

As used herein, the term "two or more numerically consecutive exons" means a plurality of exons that increase one by one in exon number among exons (the total number of exons is referred to as Texon) contained in objective pre-mRNA. The exon number means a number assigned to exons in order from the 5' end to the 3' end with an exon at the most upstream position of pre-mRNA defined as the first exon, followed by the second, the third, .... In the case of skipping of two or more numerically consecutive exons in a certain gene, its exon numbers a₁, ..., aⱼ can be represented by the sequence {aⱼ}. The general term aⱼ in the sequence {aⱼ} is represented by the expression below: ${a}_{j} = m + \left(j-1\right)$ wherein m is a given natural number that satisfies 1 ≤ m ≤ (Texon - 1), and j is a natural number that satisfies 2 ≤ (m + j) ≤ Texon + 1.

When the objective pre-mRNA is, for example, human dystrophin pre-mRNA, Texon is 79.

In a certain aspect, j is a given natural number selected from 1 to 11. In another aspect, j is 11, j is 10, j is 9, j is 8, j is 7, j is 6, j is 5, j is 4, j is 3, j is 2, or j is 1.

Herein, the any two or more numerically consecutive exons selected from the group consisting of the 45th exon to the 55th exon mean a plurality of exons that increase one by one in exon number among 11 exons from the 45th exon to the 55th exon contained in pre-mRNA. The exon number means a number assigned to exons in order from the 5' end to the 3' end with an exon at the most upstream position of pre-mRNA defined as the first exon, followed by the second, the third, ..., and the 79th exons among 79 exons contained in human dystrophin pre-mRNA. An intron is numbered as the same number as that of an exon positioned on the 5' side thereof. Specifically, the 45th intron is flanked by the 45th exon positioned on the 5' side thereof and the 46th exon positioned on the 3' side thereof. As used herein, the "nth" exon or intron means the nth exon or intron counted from the 5' end toward the 3' end in pre-mRNA.

Table 3 shows combinations of exons included in the any two or more numerically consecutive exons selected from the group consisting of the 45th exon to the 55th exon.

### [Table 3]

**Table 3**

| Combination | Exons included | Combination | Exons included |
|---|---|---|---|
| Combination 1 | 45, 46 | Combination 29 | 48 ∼ 50 |
| Combination 2 | 45 ∼ 47 | Combination 30 | 48 ∼ 51 |
| Combination 3 | 45 ∼ 48 | Combination 31 | 48 ∼ 52 |
| Combination 4 | 45 ∼ 49 | Combination 32 | 48 ∼ 53 |
| Combination 5 | 45 ∼ 50 | Combination 33 | 48 ∼ 54 |
| Combination 6 | 45 ∼ 51 | Combination 34 | 48 ∼ 55 |
| Combination 7 | 45 ∼ 52 | Combination 35 | 49, 50 |
| Combination 8 | 45 ∼ 53 | Combination 36 | 49 ∼ 51 |
| ⁻Combination 9 | 45 ∼ 54 | Combination 37 | 49 ∼ 52 |
| Combination 10 | 45 ∼ 55 | Combination 38 | 49 ∼ 53 |
| Combination 11 | 46 ∼ 47 | Combination 39 | 49 ∼ 54 |
| Combination 12 | 46 ∼ 48 | Combination 40 | 49 ∼ 55 |
| Combination 13 | 46 ∼ 49 | Combination 41 | 50 ∼ 51 |
| Combination 14 | 46 ∼ 50 | Combination 42 | 50 ∼ 52 |
| Combination 15 | 46 ∼ 51 | Combination 43 | 50 ∼ 53 |
| Combination 16 | 46 ∼ 52 | Combination 44 | 50 ∼ 54 |
| Combination 17 | 46 ∼ 53 | Combination 45 | 50 ∼ 55 |
| Combination 18 | 46 ∼ 54 | Combination 46 | 51 ∼ 52 |
| Combination 19 | 46 ∼ 55 | Combination 47 | 51 ∼ 53 |
| Combination 20 | 47, 48 | Combination 48 | 51 ∼ 54 |
| Combination 21 | 47 ∼ 49 | Combination 49 | 51 ∼ 55 |
| Combination 22 | 47 ∼ 50 | Combination 50 | 52, 3 |
| Combination 23 | 47 ∼ 51 | Combination 51 | 52 ∼ 54 |
| Combination 24 | 47 ∼ 52 | Combination 52 | 52 ∼ 55 |
| Combination 25 | 47 ∼ 53 | Combination 53 | 53, 54 |
| Combination 26 | 47 ∼ 54 | Combination 54 | 53 ∼ 55 |
| Combination 27 | 47 ∼ 55 | Combination 55 | 54, 55 |
| Combination 28 | 48, 49 | | |

Among the combinations of exons described in Table 3, for example, the combination 1, 2, 3, 4, 6, 8, 10, 18, 20, 21, 23, 25, 27, 28, 30, 32, 34, 36, 38, 40, 41, 43, 45, 46, 50, 52, or 55 is a skipping pattern expected to exert higher therapeutic effects on DMD. Multi-exon skipping in such a combination is expected to exert therapeutic effects on more patients with DMD. In one embodiment, the combination of the present invention causes skipping of all exons from the 45th exon to the 55th exon in human dystrophin pre-mRNA.

The any two or more numerically consecutive exons selected from the group consisting of the 45th exon to the 55th exon may include a plurality of groups of consecutive exons and may be, for example, but not limited to, (example 1) exons 45 and 46 (first exon group) and exons 48 to 53 (second exon group), or (example 2) exons 46 and 47 (first exon group), exons 49 and 50 (second exon group), and exons 52 to 54 (third exon group).

In the present invention, the term "activity to cause skipping" (i.e., multi-skipping activity) means, when human dystrophin pre-mRNA is taken as an example, an activity to produce human dystrophin mRNA having deletion of any two or more numerically consecutive exons selected from the group consisting of the 45th exon to the 55th exon in the human dystrophin pre-mRNA.

In other words, this activity means that by binding of the antisense oligomer of the present invention to a target site in human dystrophin pre-mRNA, the 5'-terminal nucleotide of an exon immediately downstream of the exons to be deleted is linked to the 3'-terminal nucleotide of an exon immediately upstream of the exons to be deleted when the pre-mRNA undergoes splicing, thus resulting in formation of mature mRNA which is free of codon frame shift (i.e., mature mRNA having deletion of the exons without frame shift).

The antisense oligomer of the present invention exhibits a multi-skipping activity under physiological conditions. The term "under physiological conditions" refers to conditions set to mimic the *in vivo* environment in terms of pH, salt composition and temperature. The conditions are, for example, 25 to 40°C, preferably 37°C, pH 5 to 8, preferably pH 7.4 and 150 mM of sodium chloride concentration.

Whether multi-skipping is caused or not can be confirmed by introducing the combination of the present invention into a dystrophin expression cell (e.g., human rhabdomyosarcoma cells), amplifying the region surrounding exons 45 to 55 of mRNA of the human dystrophin gene from the total RNA of the dystrophin expression cell by RT-PCR, and performing nested PCR or sequence analysis on the PCR amplified product. The multi-skipping efficiency can be determined as follows. The mRNA for the human dystrophin gene is collected from test cells; in the mRNA, the polynucleotide level "A" of the band where any two or more numerically consecutive exons among exons 45 to 55 are skipped, the polynucleotide level "B" of the band where any one exon among exons 45 to 55 is skipped, and the polynucleotide level "C" of the band where no skipping is caused are measured. Using these measurement values of "A", "B", and "C", the efficiency is calculated by the following equation. $Skipping efficiency \left(%\right) = A / \left(A+B+C\right) \times 100$

For example, the multi-skipping efficiency of exons 45 to 55 can be determined by using a forward primer for exon 44 and a reverse primer for exon 56 to measure the polynucleotide level "A" of the band where exons 45 to 55 are multi-skipped, using the forward primer for exon 44 and a reverse primer for exon 46 to measure the polynucleotide level "B" of the band where exon 45 is single-skipped, and using the forward primer for exon 44 and the reverse primer for exon 46 to measure the polynucleotide level "C" of the band where no skipping is caused, followed by calculation by the equation using these measurement values of "A", "B", and "C".

The number of exons to be deleted in human dystrophin mRNA by the antisense oligomer of the present invention is 2, 3, 4, 5, 6, 7, 8, 9, 10, or 11. This is referred to as a deletion pattern, and various deletion patterns may exist in admixture in results obtained in one skipping experiment or skipping treatment. For example, mRNA admixture having deletion of 2, 3, 4, 5, 6, 7, 8, 9, 10, or 11 exons is obtained by introducing the antisense oligomer of the present invention to cells expressing human dystrophin pre-mRNA, and collecting its mRNA.

In a certain aspect, the term "activity to cause skipping" can be defined as (C1) to (C10) below.

(C1) Any two numerically consecutive exons selected from the group consisting of the 45th exon to the 55th exon in human dystrophin pre-mRNA are skipped with the efficiency of 50 or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the two numerically consecutive exons may be the 45th and the 46th exons, the 46th and the 47th exons, the 47th and the 48th exons, the 48th and the 49th exons, the 49th and the 50th exons, the 50th and the 51st exons, the 51st and the 52nd exons, the 52nd and the 53rd exons, the 53rd and the 54th exons, or the 54th and the 55th exons.

(C2) Any three numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the three numerically consecutive exons may be the 45th to the 47th exons, the 46th to the 48th exons, the 47th to the 49th exons, the 48th to the 50th exons, the 49th to the 51st exons, the 50th to the 52nd exons, the 51st to the 53rd exons, the 52nd to the 54th exons, or the 53rd to the 55th exons.

(C3) Any four numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the four numerically consecutive exons may be the 45th to the 48th exons, the 46th to the 49th exons, the 47th to the 50th exons, the 48th to the 51st exons, the 49th to the 52nd exons, the 50th to the 53rd exons, the 51st to the 54th exons, or the 52nd to the 55th exons.

(C4) Any five numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the five numerically consecutive exons may be the 45th to the 49th exons, the 46th to the 50th exons, the 47th to the 51st exons, the 48th to the 52nd exons, the 49th to the 53rd exons, the 50th to the 54th exons, or the 51st to the 55th exons.

(C5) Any six numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the six numerically consecutive exons may be the 45th to the 50th exons, the 46th to the 51st exons, the 47th to the 52nd exons, the 48th to the 53rd exons, the 49th to the 54th exons, or the 50th to the 55th exons.

(C6) Any seven numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the seven numerically consecutive exons may be the 45th to the 51st exons, the 46th to the 52nd exons, the 47th to the 53rd exons, the 48th to the 54th exons, or the 49th to the 55th exons.

(C7) Any eight numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the eight numerically consecutive exons may be the 45th to the 52nd exons, the 46th to the 53rd exons, the 47th to the 54th exons, or the 48th to the 55th exons.

(C8) Any nine numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the nine numerically consecutive exons may be the 45th to the 53rd exons, the 46th to the 54th exons, or the 47th to the 55th exons.

(C9) Any ten numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% or higher.

Herein, the ten numerically consecutive exons may be the 45th to the 54th exons, or the 46th to the 55th exons.

(C10) Eleven numerically consecutive exons selected from the group consisting of the 45th exon to the 55th exon in human dystrophin pre-mRNA are skipped with the efficiency of 5% or higher, 10% or higher, 15% or higher, 20% or higher, 25% or higher, 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, or 95% higher.

Herein, the eleven numerically consecutive exons may be the 45th to the 55th exons.

The antisense oligomer of the present invention may be 10 to 60 bases long, 10 to 55 bases long, 10 to 50 bases long, 10 to 45 bases long, 10 to 40 bases long, 10 to 35 bases long, 10 to 30 bases long, 10 to 25 bases long, 15 to 60 bases long, 15 to 55 bases long, 15 to 50 bases long, 15 to 45 bases long, 15 to 40 bases long, 15 to 35 bases long, 15 to 30 bases long, 15 to 25 bases long, 16 to 60 bases long, 16 to 55 bases long, 16 to 50 bases long, 16 to 45 bases long, 16 to 40 bases long, 16 to 35 bases long, 16 to 30 bases long, 16 to 25 bases long, 17 to 60 bases long, 17 to 55 bases long, 17 to 50 bases long, 17 to 45 bases long, 17 to 40 bases long, 17 to 35 bases long, 17 to 30 bases long, 17 to 25 bases long, 18 to 60 bases long, 18 to 55 bases long, 18 to 50 bases long, 18 to 45 bases long, 18 to 40 bases long, 18 to 35 bases long, 18 to 30 bases long, 18 to 25 bases long, 19 to 60 bases long, 19 to 55 bases long, 19 to 50 bases long, 19 to 45 bases long, 19 to 40 bases long, 19 to 35 bases long, 19 to 30 bases long, 19 to 25 bases long, 20 to 60 bases long, 20 to 55 bases long, 20 to 50 bases long, 20 to 45 bases long, 20 to 40 bases long, 20 to 35 bases long, 20 to 30 bases long, 20 to 25 bases long, 15 to 30 bases long, 15 to 29 bases long, 15 to 28 bases long, 15 to 27 bases long, 15 to 26 bases long, 15 to 25 bases long, 15 to 24 bases long, 15 to 23 bases long, 15 to 22 bases long, 15 to 21 bases long, 15 to 20 bases long, 15 to 19 bases long, 15 to 18 bases long, 16 to 30 bases long, 16 to 29 bases long, 16 to 28 bases long, 16 to 27 bases long, 16 to 26 bases long, 16 to 25 bases long, 16 to 24 bases long, 16 to 23 bases long, 16 to 22 bases long, 16 to 21 bases long, 16 to 20 bases long, 16 to 19 bases long, 16 to 18 bases long, 17 to 30 bases long, 17 to 29 bases long, 17 to 28 bases long, 17 to 27 bases long, 17 to 26 bases long, 17 to 25 bases long, 17 to 24 bases long, 17 to 23 bases long, 17 to 22 bases long, 17 to 21 bases long, 17 to 20 bases long, 17 to 19 bases long, 17 to 18 bases long, 18 to 30 bases long, 18 to 29 bases long, 18 to 28 bases long, 18 to 27 bases long, 18 to 26 bases long, 18 to 25 bases long, 18 to 24 bases long, 18 to 23 bases long, 18 to 22 bases long, 18 to 21 bases long, 18 to 20 bases long, 18 to 19 bases long, 19 to 30 bases long, 19 to 29 bases long, 19 to 28 bases long, 19 to 27 bases long, 19 to 26 bases long, 19 to 25 bases long, 19 to 24 bases long, 19 to 23 bases long, 19 to 22 bases long, 19 to 21 bases long, 19 to 20 bases long, 20 to 30 bases long, 20 to 29 bases long, 20 to 28 bases long, 20 to 27 bases long, 20 to 26 bases long, 20 to 25 bases long, 20 to 24 bases long, 20 to 23 bases long, 20 to 22 bases long, 20 to 21 bases long, 60 bases long, 59 bases long, 58 bases long, 57 bases long, 56 bases long, 55 bases long, 54 bases long, 53 bases long, 52 bases long, 51 bases long, 50 bases long, 49 bases long, 48 bases long, 47 bases long, 46 bases long, 45 bases long, 44 bases long, 43 bases long, 42 bases long, 41 bases long, 40 bases long, 39 bases long, 38 bases long, 37 bases long, 36 bases long, 35 bases long, 34 bases long, 33 bases long, 32 bases long, 31 bases long, 30 bases long, 29 bases long, 28 bases long, 27 bases long, 26 bases long, 25 bases long, 24 bases long, 23 bases long, 22 bases long, 21 bases long, 20 bases long, 19 bases long, 18 bases long, 17 bases long, 16 bases long, 15 bases long, 14 bases long, 13 bases long, 12 bases long, 11 bases long, or 10 bases long, but not limited thereto. These lengths may be increased or decreased by 1, 2, or 3 bases.

The first antisense oligomer of the present invention is a linked-type antisense oligomer configured to comprise a plurality of unit oligomers linked to each other, a pharmaceutically acceptable salt thereof, or a hydrate thereof (hereinafter, also referred to as the "linked-type antisense oligomer of the present invention"). The unit oligomers mean respective oligomers constituting the linked-type antisense oligomer of the present invention. Specifically, the unit oligomers mean moieties (units) comprising base sequences that hybridize with target base sequences having consecutive base sequences when the linked-type antisense oligomer of the present invention binds to the target base sequences in human dystrophin pre-mRNA.

The unit oligomers may be linked via a linker that does not contribute to hybridization, or may be linked directly without the mediation of a linker. When the unit oligomers are linked directly to each other, the 3' end of the unit positioned on the 5' side and the 5' end of the unit positioned on the 3' side form a phosphate bond or any one of the following groups. 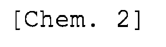 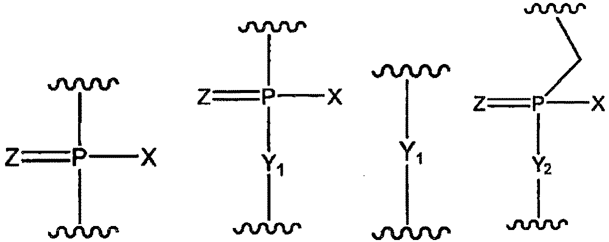 wherein X represents -OH, -CH₂R¹, -O-CH₂R¹, -S-CH₂R¹, - NR²R³ or F;
R¹ represents H or an alkyl;
R² and R³, which may be the same or different, each represents H, an alkyl, a cycloalkyl or an aryl;
Y₁ represents O, S, CH₂, or NR¹;
Y₂ represents O, S, or NR¹;
Z represents O or S.

The first unit oligomer constituting the linked-type antisense oligomer of the present invention may comprise a base sequence complementary to a base sequence consisting of a base sequence of 11 bases in the upstream direction from the 3' end of the 44th intron and a base sequence of 69 bases in the downstream direction from the 5' end of the 45th exon in human dystrophin pre-mRNA, or a partial base sequence thereof. The second unit oligomer constituting the linked-type antisense oligomer of the present invention may comprise a base sequence complementary to a base sequence of from the 52nd to 75th bases in the upstream direction from the 3' end of the 44th intron in human dystrophin pre-mRNA, or a partial base sequence thereof.

In relation to the target sequence of the unit oligomer, the term "partial" means a partial region of consecutive bases, except for the full length, of the target sequence. The partial region may be 5 to 30 bases long, 5 to 29 bases long, 5 to 28 bases long, 5 to 27 bases long, 5 to 26 bases long, 5 to 25 bases long, 5 to 24 bases long, 5 to 23 bases long, 5 to 22 bases long, 5 to 21 bases long, 5 to 20 bases long, 5 to 19 bases long, 5 to 18 bases long, 5 to 17 bases long, 5 to 16 bases long, 5 to 15 base long, 5 to 14 bases long, 5 to 13 bases long, 5 to 12 bases long, 7 to 30 bases long, 7 to 29 bases long, 7 to 28 bases long, 7 to 27 bases long, 7 to 26 bases long, 7 to 25 bases long, 7 to 24 bases long, 7 to 23 bases long, 7 to 22 bases long, 7 to 21 bases long, 7 to 20 bases long, 7 to 19 bases long, 7 to 18 bases long, 7 to 17 bases long, 7 to 16 bases long, 7 to 15 bases long, 7 to 14 bases long, 7 to 13 bases long, 7 to 12 bases long, 9 to 30 bases long, 9 to 29 bases long, 9 to 28 bases long, 9 to 27 bases long, 9 to 26 bases long, 9 to 25 bases long, 9 to 24 bases long, 9 to 23 bases long, 9 to 22 bases long, 9 to 21 bases long, 9 to 20 bases long, 9 to 19 bases long, 9 to 18 bases long, 9 to 17 bases long, 9 to 16 bases long, 9 to 15 bases long, 9 to 14 bases long, 9 to 13 bases long, 9 to 12 bases long, 10 to 30 bases long, 10 to 29 bases long, 10 to 28 bases long, 10 to 27 bases long, 10 to 26 bases long, 10 to 25 bases long, 10 to 24 bases long, 10 to 23 bases long, 10 to 22 bases long, 10 to 21 bases long, 10 to 20 bases long, 10 to 19 bases long, 10 to 18 bases long, 10 to 17 bases long, 10 to 16 bases long, 10 to 15 bases long, 10 to 14 bases long, 10 to 13 bases long, 10 to 12 bases long, 30 bases long, 29 bases long, 28 bases long, 27 bases long, 26 bases long, 25 bases long, 24 bases long, 23 bases long, 22 bases long, 21 bases long, 20 bases long, 19 bases long, 18 bases long, 17 bases long, 16 bases long, 15 bases long, 14 bases long, 13 bases long, 12 bases long, 11 bases long, 10 bases long, 9 bases long, 8 bases long, 7 bases long, 6 bases long, or 5 bases long, but is not limited thereto. These lengths may be increased or decreased by 1, 2, or 3 bases.

The size of each unit oligomer may be 5 to 30 bases long, 5 to 29 bases long, 5 to 28 bases long, 5 to 27 bases long, 5 to 26 bases long, 5 to 25 bases long, 5 to 24 bases long, 5 to 23 bases long, 5 to 22 bases long, 5 to 21 bases long, 5 to 20 bases long, 5 to 19 bases long, 5 to 18 bases long, 5 to 17 bases long, 5 to 16 bases long, 5 to 15 bases long, 5 to 14 bases long, 5 to 13 bases long, 5 to 12 bases long, 7 to 30 bases long, 7 to 29 bases long, 7 to 28 bases long, 7 to 27 bases long, 7 to 26 bases long, 7 to 25 bases long, 7 to 24 bases long, 7 to 23 bases long, 7 to 22 bases long, 7 to 21 bases long, 7 to 20 bases long, 7 to 19 bases long, 7 to 18 bases long, 7 to 17 bases long, 7 to 16 bases long, 7 to 15 bases long, 7 to 14 bases long, 7 to 13 bases long, 7 to 12 bases long, 9 to 30 bases long, 9 to 29 bases long, 9 to 28 bases long, 9 to 27 bases long, 9 to 26 bases long, 9 to 25 bases long, 9 to 24 bases long, 9 to 23 bases long, 9 to 22 bases long, 9 to 21 bases long, 9 to 20 bases long, 9 to 19 bases long, 9 to 18 bases long, 9 to 17 bases long, 9 to 16 bases long, 9 to 15 bases long, 9 to 14 bases long, 9 to 13 bases long, 9 to 12 bases long, 10 to 30 bases long, 10 to 29 bases long, 10 to 28 bases long, 10 to 27 bases long, 10 to 26 bases long, 10 to 25 bases long, 10 to 24 bases long, 10 to 23 bases long, 10 to 22 bases long, 10 to 21 bases long, 10 to 20 bases long, 10 to 19 bases long, 10 to 18 bases long, 10 to 17 bases long, 10 to 16 bases long, 10 to 15 bases long, 10 to 14 bases long, 10 to 13 bases long, 10 to 12 bases long, 30 bases long, 29 bases long, 28 bases long, 27 bases long, 26 bases long, 25 bases long, 24 bases long, 23 bases long, 22 bases long, 21 bases long, 20 bases long, 19 bases long, 18 bases long, 17 bases long, 16 bases long, 15 bases long, 14 bases long, 13 bases long, 12 bases long, 11 bases long, 10 bases long, 9 bases long, 8 bases long, 7 bases long, 6 bases long, 5 bases long, but not limited thereto. These lengths may be increased or decreased by 1, 2, or 3 bases. The unit oligomers may have the same size or different sizes.

In the first antisense oligomer, the order of the first unit oligomer and the second unit oligomer is not limited. The first antisense oligomer may comprise the first unit oligomer and the second unit oligomer from the 5' ends in this order, or may comprise the second unit oligomer and the first unit oligomer from the 5' ends in this order.

In one embodiment, the first unit oligomer comprises or consists of a base sequence complementary to consecutive 15 to 30 bases of a base sequence consisting of a base sequence 11 bases in the upstream direction from the 3' end of the 44th intron and a base sequence of 69 bases in the downstream direction from the 5' end of the 45th exon in human dystrophin pre-mRNA. In one embodiment, the second unit oligomer comprises or consists of a base sequence complementary to consecutive 1 to 10 bases of a base sequence of from the 52nd to 75th bases in the upstream direction from the 3' end of the 44th intron in human dystrophin pre-mRNA. In one embodiment, the second antisense oligomer comprises a base sequence complementary to consecutive 15 to 30 bases of a base sequence consisting of a base sequence of 33 bases in the upstream direction from the 3' end of the 54th intron and a base sequence of 53 bases in the downstream direction from the 5' end of the 55th exon in human dystrophin pre-mRNA. In one embodiment, the third antisense oligomer comprises or consists of a base sequence complementary to consecutive 15 to 30 bases of a base sequence consisting of a base sequence of 23 bases in the upstream direction from the 3' end of the 45th exon and a base sequence of 73 bases in the downstream direction from the 5' end of the 45th intron in the human dystrophin pre-mRNA.

Table 4 below shows examples of the target sequence of the first unit oligomer, and the complementary sequence (antisense sequence) thereof.

**[Table 4-1]**

| **Table 4** | | | | | |
|---|---|---|---|---|---|
| **Length mer** | **Target site** | **Targe sequence** | **SEQ ID NO:** | **Antisense sequence (5' to 3')** | **SEQ ID NO:** |
| 15 | H45_5-19 | TCCAGGATGGCATTG | 211 | CAATGCCATCCTGGA | 907 |
| 15 | H45_6-20 | CCAGGATGGCATTGG | 212 | CCAATGCCATCCTGG | 908 |
| 15 | H45_7-21 | CAGGATGGCATTGGG | 213 | CCCAATGCCATCCTG | 909 |
| 15 | H45_8-22 | AGGATGGCATTGGGC | 214 | GCCCAATGCCATCCT | 910 |
| 15 | H45_9-23 | GGATGGCATTGGGCA | 215 | TGCCCAATGCCATCC | 911 |
| 15 | H45_10-24 | GATGGCATTGGGCAG | 216 | CTGCCCAATGCCATC | 912 |
| 15 | H45_11-25 | ATGGCATTGGGCAGC | 217 | GCTGCCCAATGCCAT | 913 |
| 15 | H45_12-26 | TGGCATTGGGCAGCG | 218 | CGCTGCCCAATGCCA | 914 |
| 15 | H45_13-27 | GGCATTGGGCAGCGG | 219 | CCGCTGCCCAATGCC | 915 |
| 15 | H45_14-28 | GCATTGGGCAGCGGC | 220 | GCCGCTGCCCAATGC | 916 |
| 15 | H45_15-29 | CATTGGGCAGCGGCA | 221 | TGCCGCTGCCCAATG | 917 |
| 15 | H45_16-30 | ATTGGGCAGCGGCAA | 222 | TTGCCGCTGCCCAAT | 918 |
| 15 | H45_17-31 | TTGGGCAGCGGCAAA | 223 | TTTGCCGCTGCCCAA | 919 |
| 15 | H45_18-32 | TGGGCAGCGGCAAAC | 224 | GTTTGCCGCTGCCCA | 920 |
| 15 | H45_19-33 | GGGCAGCGGCAAACT | 225 | AGTTTGCCGCTGCCC | 921 |
| 15 | H45_20-34 | GGCAGCGGCAAACTG | 226 | CAGTTTGCCGCTGCC | 922 |
| 15 | H45_21-35 | GCAGCGGCAAACTGT | 227 | ACAGTTTGCCGCTGC | 923 |
| 15 | H45_22-36 | CAGCGGCAAACTGTT | 228 | AACAGTTTGCCGCTG | 924 |
| 15 | H45_23-37 | AGCGGCAAACTGTTG | 229 | CAACAGTTTGCCGCT | 925 |
| 15 | H45_24-38 | GCGGCAAACTGTTGT | 230 | ACAACAGTTTGCCGC | 926 |
| 15 | H45_25-39 | CGGCAAACTGTTGTC | 231 | GACAACAGTTTGCCG | 927 |
| 15 | H45_26-40 | GGCAAACTGTTGTCA | 232 | TGACAACAGTTTGCC | 928 |

**[Table 4-3]**

| | | | | | |
|---|---|---|---|---|---|
| 15 | H45_27-41 | GCAAACTGTTGTCAG | 233 | CTGACAACAGTTTGC | 929 |
| 15 | H45_28-42 | CAAACTGTTGTCAGA | 234 | TCTGACAACAGTTTG | 930 |
| 15 | H45_29-43 | AAACTGTTGTCAGAA | 235 | TTCTGACAACAGTTT | 931 |
| 15 | H45_30-44 | AACTGTTGTCAGAAC | 236 | GTTCTGACAACAGTT | 932 |
| 15 | H45_31-45 | ACTGTTGTCAGAACA | 237 | TGTTCTGACAACAGT | 933 |
| 15 | H45_32-46 | CTGTTGTCAGAACAT | 238 | ATGTTCTGACAACAG | 934 |
| 15 | H45_33-47 | TGTTGTCAGAACATT | 239 | AATGTTCTGACAACA | 935 |
| 15 | H45_34-48 | GTTGTCAGAACATTG | 240 | CAATGTTCTGACAAC | 936 |
| 15 | H45_35-49 | TTGTCAGAACATTGA | 241 | TCAATGTTCTGACAA | 937 |
| 15 | H45_36-50 | TGTCAGAACATTGAA | 242 | TTCAATGTTCTGACA | 938 |
| 15 | H45_37-51 | GTCAGAACATTGAAT | 243 | ATTCAATGTTCTGAC | 939 |
| 15 | H45_38-52 | TCAGAACATTGAATG | 244 | CATTCAATGTTCTGA | 940 |
| 15 | H45_39-53 | CAGAACATTGAATGC | 245 | GCATTCAATGTTCTG | 941 |
| 15 | H45_40-54 | AGAACATTGAATGCA | 246 | TGCATTCAATGTTCT | 942 |
| 16 | H45_4-19 | CTCCAGGATGGCATTG | 247 | CAATGCCATCCTGGAG | 943 |
| 16 | H45_5-20 | TCCAGGATGGCATTGG | 248 | CCAATGCCATCCTGGA | 944 |
| 16 | H45_6-21 | CCAGGATGGCATTGGG | 249 | CCCAATGCCATCCTGG | 945 |
| 16 | H45_7-22 | CAGGATGGCATTGGGC | 250 | GCCCAATGCCATCCTG | 946 |
| 16 | H45_8-23 | AGGATGGCATTGGGCA | 251 | TGCCCAATGCCATCCT | 947 |
| 16 | H45_9-24 | GGATGGCATTGGGCAG | 252 | CTGCCCAATGCCATCC | 948 |
| 16 | H45_10- | GATGGCATTGGGCAGC | 253 | GCTGCCCAATGCCATC | 949 |

**[Table 4-4]**

| | | | | | |
|---|---|---|---|---|---|
| | 25 | | | | |
| 16 | H45_11-26 | ATGGCATTGGGCAGCG | 254 | CGCTGCCCAATGCCAT | 950 |
| 16 | H45_12-27 | TGGCATTGGGCAGCGG | 255 | CCGCTGCCCAATGCCA | 951 |
| 16 | H45_13-28 | GGCATTGGGCAGCGGC | 256 | GCCGCTGCCCAATGCC | 952 |
| 16 | H45_14-29 | GCATTGGGCAGCGGCA | 257 | TGCCGCTGCCCAATGC | 953 |
| 16 | H45_15-30 | CATTGGGCAGCGGCAA | 258 | TTGCCGCTGCCCAATG | 954 |
| 16 | H45_16-31 | ATTGGGCAGCGGCAAA | 259 | TTTGCCGCTGCCCAAT | 955 |
| 16 | H45_17-32 | TTGGGCAGCGGCAAAC | 260 | GTTTGCCGCTGCCCAA | 956 |
| 16 | H45_18-33 | TGGGCAGCGGCAAACT | 261 | AGTTTGCCGCTGCCCA | 957 |
| 16 | H45_19-34 | GGGCAGCGGCAAACTG | 262 | CAGTTTGCCGCTGCCC | 958 |
| 16 | H45_20-35 | GGCAGCGGCAAACTGT | 263 | ACAGTTTGCCGCTGCC | 959 |
| 16 | H45_21-36 | GCAGCGGCAAACTGTT | 264 | AACAGTTTGCCGCTGC | 960 |
| 16 | H45_22-37 | CAGCGGCAAACTGTTG | 265 | CAACAGTTTGCCGCTG | 961 |
| 16 | H45_23-38 | AGCGGCAAACTGTTGT | 266 | ACAACAGTTTGCCGCT | 962 |
| 16 | H45_24-39 | GCGGCAAACTGTTGTC | 267 | GACAACAGTTTGCCGC | 963 |
| 16 | H45_25-40 | CGGCAAACTGTTGTCA | 268 | TGACAACAGTTTGCCG | 964 |
| 16 | H45_26-41 | GGCAAACTGTTGTCAG | 269 | CTGACAACAGTTTGCC | 965 |
| 16 | H45_27-42 | GCAAACTGTTGTCAGA | 270 | TCTGACAACAGTTTGC | 966 |

**[Table 4-5]**

| | | | | | |
|---|---|---|---|---|---|
| 16 | H45_28-43 | CAAACTGTTGTCAGAA | 271 | TTCTGACAACAGTTTG | 967 |
| 16 | H45_29-44 | AAACTGTTGTCAGAAC | 272 | GTTCTGACAACAGTTT | 968 |
| 16 | H45_30-45 | AACTGTTGTCAGAACA | 273 | TGTTCTGACAACAGTT | 969 |
| 16 | H45_31-46 | ACTGTTGTCAGAACAT | 274 | ATGTTCTGACAACAGT | 970 |
| 16 | H45_32-47 | CTGTTGTCAGAACATT | 275 | AATGTTCTGACAACAG | 971 |
| 16 | H45_33-48 | TGTTGTCAGAACATTG | 276 | CAATGTTCTGACAACA | 972 |
| 16 | H45_34-49 | GTTGTCAGAACATTGA | 277 | TCAATGTTCTGACAAC | 973 |
| 16 | H45_35-50 | TTGTCAGAACATTGAA | 278 | TTCAATGTTCTGACAA | 974 |
| 16 | H45_36-51 | TGTCAGAACATTGAAT | 279 | ATTCAATGTTCTGACA | 975 |
| 16 | H45_37-52 | GTCAGAACATTGAATG | 280 | CATTCAATGTTCTGAC | 976 |
| 16 | H45_38-53 | TCAGAACATTGAATGC | 281 | GCATTCAATGTTCTGA | 977 |
| 16 | H45_39-54 | CAGAACATTGAATGCA | 282 | TGCATTCAATGTTCTG | 978 |
| 16 | H45_40-55 | AGAACATTGAATGCAA | 283 | TTGCATTCAATGTTCT | 979 |
| 17 | H45_3-19 | ACTCCAGGATGGCATTG | 284 | CAATGCCATCCTGGAGT | 980 |
| 17 | H45_4-20 | CTCCAGGATGGCATTGG | 285 | CCAATGCCATCCTGGAG | 981 |
| 17 | H45_5-21 | TCCAGGATGGCATTGGG | 286 | CCCAATGCCATCCTGGA | 982 |
| 17 | H45_6-22 | CCAGGATGGCATTGGGC | 287 | GCCCAATGCCATCCTGG | 983 |
| 17 | H45_7-23 | CAGGATGGCATTGGGCA | 288 | TGCCCAATGCCATCCTG | 984 |
| 17 | H45_8-24 | AGGATGGCATTGGGCAG | 289 | CTGCCCAATGCCATCCT | 985 |
| 17 | H45_9-25 | GGATGGCATTGGGCAGC | 290 | GCTGCCCAATGCCATCC | 986 |
| 17 | H45_10-26 | GATGGCATTGGGCAGCG | 291 | CGCTGCCCAATGCCATC | 987 |

**[Table 4-6]**

| | | | | | |
|---|---|---|---|---|---|
| 17 | H45_11-27 | ATGGCATTGGGCAGCGG | 292 | CCGCTGCCCAATGCCAT | 988 |
| 17 | H45_12-28 | TGGCATTGGGCAGCGGC | 293 | GCCGCTGCCCAATGCCA | 989 |
| 17 | H45_13-29 | GGCATTGGGCAGCGGCA | 294 | TGCCGCTGCCCAATGCC | 990 |
| 17 | H45_14-30 | GCATTGGGCAGCGGCAA | 295 | TTGCCGCTGCCCAATGC | 991 |
| 17 | H45_15-31 | CATTGGGCAGCGGCAAA | 296 | TTTGCCGCTGCCCAATG | 992 |
| 17 | H45_16-32 | ATTGGGCAGCGGCAAAC | 297 | GTTTGCCGCTGCCCAAT | 993 |
| 17 | H45_17-33 | TTGGGCAGCGGCAAACT | 298 | AGTTTGCCGCTGCCCAA | 994 |
| 17 | H45_18-34 | TGGGCAGCGGCAAACTG | 299 | CAGTTTGCCGCTGCCCA | 995 |
| 17 | H45_19-35 | GGGCAGCGGCAAACTGT | 300 | ACAGTTTGCCGCTGCCC | 996 |
| 17 | H45_20-36 | GGCAGCGGCAAACTGTT | 301 | AACAGTTTGCCGCTGCC | 997 |
| 17 | H45_21-37 | GCAGCGGCAAACTGTTG | 302 | CAACAGTTTGCCGCTGC | 998 |
| 17 | H45_22-38 | CAGCGGCAAACTGTTGT | 303 | ACAACAGTTTGCCGCTG | 999 |
| 17 | H45_23-39 | AGCGGCAAACTGTTGTC | 304 | GACAACAGTTTGCCGCT | 1000 |
| 17 | H45_24-40 | GCGGCAAACTGTTGTCA | 305 | TGACAACAGTTTGCCGC | 1001 |
| 17 | H45_25-41 | CGGCAAACTGTTGTCAG | 306 | CTGACAACAGTTTGCCG | 1002 |
| 17 | H45_26-42 | GGCAAACTGTTGTCAGA | 307 | TCTGACAACAGTTTGCC | 1003 |
| 17 | H45_27-43 | GCAAACTGTTGTCAGAA | 308 | TTCTGACAACAGTTTGC | 1004 |
| 17 | H45_28-44 | CAAACTGTTGTCAGAAC | 309 | GTTCTGACAACAGTTTG | 1005 |

**[Table 4-7]**

| | | | | | |
|---|---|---|---|---|---|
| 17 | H45_29-45 | AAACTGTTGTCAGAACA | 310 | TGTTCTGACAACAGTTT | 1006 |
| 17 | H45_30-46 | AACTGTTGTCAGAACAT | 311 | ATGTTCTGACAACAGTT | 1007 |
| 17 | H45_31-47 | ACTGTTGTCAGAACATT | 312 | AATGTTCTGACAACAGT | 1008 |
| 17 | H45_32-48 | CTGTTGTCAGAACATTG | 313 | CAATGTTCTGACAACAG | 1009 |
| 17 | H45_33-49 | TGTTGTCAGAACATTGA | 314 | TCAATGTTCTGACAACA | 1010 |
| 17 | H45_34-50 | GTTGTCAGAACATTGAA | 315 | TTCAATGTTCTGACAAC | 1011 |
| 17 | H45_35-51 | TTGTCAGAACATTGAAT | 316 | ATTCAATGTTCTGACAA | 1012 |
| 17 | H45_36-52 | TGTCAGAACATTGAATG | 317 | CATTCAATGTTCTGACA | 1013 |
| 17 | H45_37-53 | GTCAGAACATTGAATGC | 318 | GCATTCAATGTTCTGAC | 1014 |
| 17 | H45_38-54 | TCAGAACATTGAATGCA | 319 | TGCATTCAATGTTCTGA | 1015 |
| 17 | H45_39-55 | CAGAACATTGAATGCAA | 320 | TTGCATTCAATGTTCTG | 1016 |
| 17 | H45_40-56 | AGAACATTGAATGCAAC | 321 | GTTGCATTCAATGTTCT | 1017 |
| 18 | H45_2-19 | AACTCCAGGATGGCATTG | 322 | CAATGCCATCCTGGAGTT | 1018 |
| 18 | H45_3-20 | ACTCCAGGATGGCATTGG | 323 | CCAATGCCATCCTGGAGT | 1019 |
| 18 | H45_4-21 | CTCCAGGATGGCATTGGG | 324 | CCCAATGCCATCCTGGAG | 1020 |
| 18 | H45_5-22 | TCCAGGATGGCATTGGGC | 325 | GCCCAATGCCATCCTGGA | 1021 |
| 18 | H45_6-23 | CCAGGATGGCATTGGGCA | 326 | TGCCCAATGCCATCCTGG | 1022 |
| 18 | H45_7-24 | CAGGATGGCATTGGGCAG | 327 | CTGCCCAATGCCATCCTG | 1023 |
| 18 | H45_8-25 | AGGATGGCATTGGGCAGC | 328 | GCTGCCCAATGCCATCCT | 1024 |
| 18 | H45_9-26 | GGATGGCATTGGGCAGCG | 329 | CGCTGCCCAATGCCATCC | 1025 |
| 18 | H45_10-27 | GATGGCATTGGGCAGCGG | 330 | CCGCTGCCCAATGCCATC | 1026 |
| 18 | H45_11- | ATGGCATTGGGCAGCGGC | 331 | GCCGCTGCCCAATGCCAT | 1027 |

**[Table 4-8]**

| | | | | | |
|---|---|---|---|---|---|
| | 28 | | | | |
| 18 | H45_12-29 | TGGCATTGGGCAGCGGCA | 332 | TGCCGCTGCCCAATGCCA | 1028 |
| 18 | H45_13-30 | GGCATTGGGCAGCGGCAA | 333 | TTGCCGCTGCCCAATGCC | 1029 |
| 18 | H45_14-31 | GCATTGGGCAGCGGCAAA | 334 | TTTGCCGCTGCCCAATGC | 1030 |
| 18 | H45_15-32 | CATTGGGCAGCGGCAAAC | 335 | GTTTGCCGCTGCCCAATG | 1031 |
| 18 | H45_16-33 | ATTGGGCAGCGGCAAACT | 336 | AGTTTGCCGCTGCCCAAT | 1032 |
| 18 | H45_17-34 | TTGGGCAGCGGCAAACTG | 337 | CAGTTTGCCGCTGCCCAA | 1033 |
| 18 | H45_18-35 | TGGGCAGCGGCAAACTGT | 338 | ACAGTTTGCCGCTGCCCA | 1034 |
| 18 | H45_19-36 | GGGCAGCGGCAAACTGTT | 339 | AACAGTTTGCCGCTGCCC | 1035 |
| 18 | H45_20-37 | GGCAGCGGCAAACTGTTG | 340 | CAACAGTTTGCCGCTGCC | 1036 |
| 18 | H45_21-38 | GCAGCGGCAAACTGTTGT | 341 | ACAACAGTTTGCCGCTGC | 1037 |
| 18 | H45_22-39 | CAGCGGCAAACTGTTGTC | 342 | GACAACAGTTTGCCGCTG | 1038 |
| 18 | H45_23-40 | AGCGGCAAACTGTTGTCA | 343 | TGACAACAGTTTGCCGCT | 1030 |
| 18 | H45_24-41 | GCGGCAAACTGTTGTCAG | 344 | CTGACAACAGTTTGCCGC | 1040 |
| 18 | H45_25-42 | GGGCAAACTGTTGTCAGA | 345 | TCTGACAACAGTTTGCCG | 1041 |
| 18 | H45_26-43 | GGCAAACTGTTGTCAGAA | 346 | TTCTGACAACAGTTTGCC | 1042 |
| 18 | H45_27-44 | GCAAACTGTTGTCAGAAC | 347 | GTTCTGACAACAGTTTGC | 1043 |
| 18 | H45_28-45 | CAAACTGTTGTCAGAACA | 348 | TGTTCTGACAACAGTTTG | 1044 |

**[Table 4-9]**

| | | | | | |
|---|---|---|---|---|---|
| 18 | H45_29-46 | AAACTGTTGTCAGAACAT | 349 | ATGTTCTGACAACAGTTT | 1045 |
| 18 | H45_30-47 | AACTGTTGTCAGAACATT | 350 | AATGTTCTGACAACAGTT | 1046 |
| 18 | H45_31-48 | ACTGTTGTCAGAACATTG | 351 | CAATGTTCTGACAACAGT | 1047 |
| 18 | H45_32-49 | CTGTTGTCAGAACATTGA | 352 | TCAATGTTCTGACAACAG | 1048 |
| 18 | H45_33-50 | TGTTGTCAGAACATTGAA | 353 | TTCAATGTTCTGACAACA | 1049 |
| 18 | H45_34-51 | GTTGTCAGAACATTGAAT | 354 | ATTCAATGTTCTGACAAC | 1050 |
| 18 | H45_35-52 | TTGTCAGAACATTGAATG | 355 | CATTCAATGTTCTGACAA | 1051 |
| 18 | H45_36-53 | TGTCAGAACATTGAATGC | 356 | GCATTCAATGTTCTGACA | 1052 |
| 18 | H45_37-54 | GTCAGAACATTGAATGCA | 357 | TGCATTCAATGTTCTGAC | 1053 |
| 18 | H45_38-55 | TCAGAACATTGAATGCAA | 358 | TTGCATTCAATGTTCTGA | 1054 |
| 18 | H45_39-56 | CAGAACATTGAATGCAAC | 359 | GTTGCATTCAATGTTCTG | 1055 |
| 18 | H45_40-57 | AGAACATTGAATGCAACT | 360 | AGTTGCATTCAATGTTCT | 1056 |
| 19 | H45_1-19 | GAACTCCAGGATGGCATTG | 361 | CAATGCCATCCTGGAGTTC | 1057 |
| 19 | H45_2-20 | AACTCCAGGATGGCATTGG | 362 | CCAATGCCATCCTGGAGTT | 1058 |
| 19 | H45_3-21 | ACTCCAGGATGGCATTGGG | 363 | CCCAATGCCATCCTGGAGT | 1059 |
| 19 | H45_4-22 | CTCCAGGATGGCATTGGGC | 364 | GCCCAATGCCATCCTGGAG | 1060 |
| 19 | H45_5-23 | TCCAGGATGGCATTGGGCA | 365 | TGCCCAATGCCATCCTGGA | 1061 |
| 19 | H45_6-24 | CCAGGATGGCATTGGGCAG | 366 | CTGCCCAATGCCATCCTGG | 1062 |
| 19 | H45_7-25 | CAGGATGGCATTGGGCAGC | 367 | GCTGCCCAATGCCATCCTG | 1063 |
| 19 | H45_8-26 | AGGATGGCATTGGGCAGCG | 368 | CGCTGCCCAATGCCATCCT | 1064 |
| 19 | H45_9-27 | GGATGGCATTGGGCAGCGG | 369 | CCGCTGCCCAATGCCATCC | 1065 |
| 19 | H45_10-28 | GATGGCATTGGGCAGCGGC | 370 | GCCGCTGCCCAATGCCATC | 1066 |

**[Table 4-10]**

| | | | | | |
|---|---|---|---|---|---|
| 19 | H45_11-29 | ATGGCATTGGGCAGCGGCA | 371 | TGCCGCTGCCCAATGCCAT | 1067 |
| 19 | H45_12-30 | TGGCATTGGGCAGCGGCAA | 372 | TTGCCGCTGCCCAATGCCA | 1068 |
| 19 | H45_13-31 | GGCATTGGGCAGCGGCAAA | 373 | TTTGCCGCTGCCCAATGCC | 1069 |
| 19 | H45_14-32 | GCATTGGGCAGCGGCAAAC | 374 | GTTTGCCGCTGCCCAATGC | 1070 |
| 19 | H45_15-33 | CATTGGGCAGCGGCAAACT | 375 | AGTTTGCCGCTGCCCAATG | 1071 |
| 19 | H45_16-34 | ATTGGGCAGCGGCAAACTG | 376 | CAGTTTGCCGCTGCCCAAT | 1072 |
| 19 | H45_17-35 | TTGGGCAGCGGCAAACTGT | 377 | ACAGTTTGCCGCTGCCCAA | 1073 |
| 19 | H45_18-36 | TGGGCAGCGGCAAACTGTT | 378 | AACAGTTTGCCGCTGCCCA | 1074 |
| 19 | H45_19-37 | GGGCAGCGGCAAACTGTTG | 379 | CAACAGTTTGCCGCTGCCC | 1075 |
| 19 | H45_20-38 | GGCAGCGGCAAACTGTTGT | 380 | ACAACAGTTTGCCGCTGCC | 1076 |
| 19 | H45_21-39 | GCAGCGGCAAACTGTTGTC | 381 | GACAACAGTTTGCCGCTGC | 1077 |
| 19 | H45_22-40 | CAGCGGCAAACTGTTGTCA | 382 | TGACAACAGTTTGCCGCTG | 1078 |
| 19 | H45_23-41 | AGCGGCAAACTGTTGTCAG | 383 | CTGACAACAGTTTGCCGCT | 1079 |
| 19 | H45_24-42 | GCGGCAAACTGTTGTCAGA | 384 | TCTGACAACAGTTTGCCGC | 1080 |
| 19 | H45_25-43 | CGGCAAACTGTTGTCAGAA | 385 | TTCTGACAACAGTTTGCCG | 1081 |
| 19 | H45_26-44 | GGCAAACTGTTGTCAGAAC | 386 | GTTCTGACAACAGTTTGCC | 1082 |
| 19 | H45_27-45 | GCAAACTGTTGTCAGAACA | 387 | TGTTCTGACAACAGTTTGC | 1083 |
| 19 | H45_28-46 | CAAACTGTTGTCAGAACAT | 388 | ATGTTCTGACAACAGTTTG | 1084 |

**[Table 4-11]**

| | | | | | |
|---|---|---|---|---|---|
| 19 | H45_29-47 | AAACTGTTGTCAGAACATT | 389 | AATGTTCTGACAACAGTTT | 1085 |
| 19 | H45_30-48 | AACTGTTGTCAGAACATTG | 390 | CAATGTTCTGACAACAGTT | 1086 |
| 19 | H45_31-49 | ACTGTTGTCAGAACATTGA | 391 | TCAATGTTCTGACAACAGT | 1087 |
| 19 | H45_32-50 | CTGTTGTCAGAACATTGAA | 392 | TTCAATGTTCTGACAACAG | 1088 |
| 19 | H45_33-51 | TGTTGTCAGAACATTGAAT | 393 | ATTCAATGTTCTGACAACA | 1089 |
| 19 | H45_34-52 | GTTGTCAGAACATTGAATG | 394 | CATTCAATGTTCTGACAAC | 1090 |
| 19 | H45_35-53 | TTGTCAGAACATTGAATGC | 395 | GCATTCAATGTTCTGACAA | 1091 |
| 19 | H45_36-54 | TGTCAGAACATTGAATGCA | 396 | TGCATTCAATGTTCTGACA | 1092 |
| 19 | H45_37-55 | GTCAGAACATTGAATGCAA | 397 | TTGCATTCAATGTTCTGAC | 1093 |
| 19 | H45_38-56 | TCAGAACATTGAATGCAAC | 398 | GTTGCATTCAATGTTCTGA | 1094 |
| 19 | H45_39-57 | CAGAACATTGAATGCAACT | 399 | AGTTGCATTCAATGTTCTG | 1095 |
| 19 | H45_40-58 | AGAACATTGAATGCAACTG | 400 | CAGTTGCATTCAATGTTCT | 1096 |
| 20 | H45_(-1)-19 | GGAACTCCAGGATGGCATTG | 401 | CAATGCCATCCTGGAGTTCC | 1097 |
| 20 | H45_1-20 | GAACTCCAGGATGGCATTGG | 402 | CCAATGCCATCCTGGAGTTC | 1098 |
| 20 | H45_2-21 | AACTCCAGGATGGCATTGGG | 403 | CCCAATGCCATCCTGGAGTT | 1099 |
| 20 | H45_3-22 | ACTCCAGGATGGCATTGGGC | 404 | GCCCAATGCCATCCTGGAGT | 1100 |
| 20 | H45_4-23 | CTCCAGGATGGCATTGGGCA | 405 | TGCCCAATGCCATCCTGGAG | 1101 |
| 20 | H45_5-24 | TCCAGGATGGCATTGGGCAG | 406 | CTGCCCAATGCCATCCTGGA | 1102 |
| 20 | H45_6-25 | CCAGGATGGCATTGGGCAGC | 407 | GCTGCCCAATGCCATCCTGG | 1103 |
| 20 | H45_7-26 | CAGGATGGCATTGGGCAGCG | 408 | CGCTGCCCAATGCCATCCTG | 1104 |
| 20 | H45_8-27 | AGGATGGCATTGGGCAGCGG | 409 | CCGCTGCCCAATGCCATCCT | 1105 |
| 20 | H45_9-28 | GGATGGCATTGGGCAGCGGC | 410 | GCCGCTGCCCAATGCCATCC | 1106 |

**[Table 4-12]**

| | | | | | |
|---|---|---|---|---|---|
| 20 | H45_10-29 | GATGGCATTGGGCAGCGGCA | 411 | TGCCGCTGCCCAATGCCATC | 1107 |
| 20 | H45_11-30 | ATGGCATTGGGCAGCGGCAA | 412 | TTGCCGCTGCCCAATGCCAT | 1108 |
| 20 | H45_12-31 | TGGCATTGGGCAGCGGCAAA | 413 | TTTGCCGCTGCCCAATGCCA | 1109 |
| 20 | H45_13-32 | GGCATTGGGCAGCGGCAAAC | 414 | GTTTGCCGCTGCCCAATGCC | 1110 |
| 20 | H45_14-33 | GCATTGGGCAGCGGCAAACT | 415 | AGTTTGCCGCTGCCCAATGC | 1111 |
| 20 | H45_15-34 | CATTGGGCAGCGGCAAACTG | 416 | CAGTTTGCCGCTGCCCAATG | 1112 |
| 20 | H45_16-35 | ATTGGGCAGCGGCAAACTGT | 417 | ACAGTTTGCCGCTGCCCAAT | 1113 |
| 20 | H45_17-36 | TTGGGCAGCGGCAAACTGTT | 418 | AACAGTTTGCCGCTGCCCAA | 1114 |
| 20 | H45_18-37 | TGGGCAGCGGCAAACTGTTG | 419 | CAACAGTTTGCCGCTGCCCA | 1115 |
| 20 | H45_19-38 | GGGCAGCGGCAAACTGTTGT | 420 | ACAACAGTTTGCCGCTGCCC | 1116 |
| 20 | H45_20-39 | GGCAGCGGCAAACTGTTGTC | 421 | GACAACAGTTTGCCGCTGCC | 1117 |
| 20 | H45_21-40 | GCAGCGGCAAACTGTTGTCA | 422 | TGACAACAGTTTGCCGCTGC | 1118 |
| 20 | H45_22-41 | CAGCGGCAAACTGTTGTCAG | 423 | CTGACAACAGTTTGCCGCTG | 1119 |
| 20 | H45_23-42 | AGCGGCAAACTGTTGTCAGA | 424 | TCTGACAACAGTTTGCCGCT | 1120 |
| 20 | H45_24-43 | GCGGCAAACTGTTGTCAGAA | 425 | TTCTGACAACAGTTTGCCGC | 1121 |
| 20 | H45_25-44 | CGGCAAACTGTTGTCAGAAC | 426 | GTTCTGACAACAGTTTGCCG | 1122 |
| 20 | H45_26-45 | GGCAAACTGTTGTCAGAACA | 427 | TGTTCTGACAACAGTTTGCC | 1123 |
| 20 | H45_27-46 | GCAAACTGTTGTCAGAACAT | 428 | ATGTTCTGACAACAGTTTGC | 1124 |

**[Table 4-13]**

| | | | | | |
|---|---|---|---|---|---|
| 20 | H45_28-47 | CAAACTGTTGTCAGAACATT | 429 | AATGTTCTGACAACAGTTTG | 1125 |
| 20 | H45_29-48 | AAACTGTTGTCAGAACATTG | 430 | CAATGTTCTGACAACAGTTT | 1126 |
| 20 | H45_30-49 | AACTGTTGTCAGAACATTGA | 431 | TCAATGTTCTGACAACAGTT | 1127 |
| 20 | H45_31-50 | ACTGTTGTCAGAACATTGAA | 432 | TTCAATGTTCTGACAACAGT | 1128 |
| 20 | H45_32-51 | CTGTTGTCAGAACATTGAAT | 433 | ATTCAATGTTCTGACAACAG | 1129 |
| 20 | H45_33-52 | TGTTGTCAGAACATTGAATG | 434 | CATTCAATGTTCTGACAACA | 1130 |
| 20 | H45_34-53 | GTTGTCAGAACATTGAATGC | 435 | GCATTCAATGTTCTGACAAC | 1131 |
| 20 | H45_35-54 | TTGTCAGAACATTGAATGCA | 436 | TGCATTCAATGTTCTGACAA | 1132 |
| 20 | H45_36-55 | TGTCAGAACATTGAATGCAA | 437 | TTGCATTCAATGTTCTGACA | 1133 |
| 20 | H45_37-56 | GTCAGAACATTGAATGCAAC | 438 | GTTGCATTCAATGTTCTGAC | 1134 |
| 20 | H45_38-57 | TCAGAACATTGAATGCAACT | 439 | AGTTGCATTCAATGTTCTGA | 1135 |
| 20 | H45_39-58 | CAGAACATTGAATGCAACTG | 440 | CAGTTGCATTCAATGTTCTG | 1136 |
| 20 | H45_40-59 | AGAACATTGAATGCAACTGG | 441 | CCAGTTGCATTCAATGTTCT | 1137 |
| 21 | H45_(-2)-19 | AGGAACTCCAGGATGGCATTG | 442 | CAATGCCATCCTGGAGTTCC T | 1138 |
| 21 | H45_(-1)-20 | GGAACTCCAGGATGGCATTGG | 443 | CCAATGCCATCCTGGAGTTC C | 1139 |
| 21 | H45_1-21 | GAACTCCAGGATGGCATTGGG | 444 | CCCAATGCCATCCTGGAGTT C | 1140 |
| 21 | H45_2-22 | AACTCCAGGATGGCATTGGGC | 445 | GCCCAATGCCATCCTGGAGT T | 1141 |
| 21 | H45_3-23 | ACTCCAGGATGGCATTGGGCA | 446 | TGCCCAATGCCATCCTGGAG T | 1142 |

**[Table 4-14]**

| | | | | | |
|---|---|---|---|---|---|
| 21 | H45_4-24 | CTCCAGGATGGCATTGGGCAG | 447 | CTGCCCAATGCCATCCTGGA G | 1143 |
| 21 | H45_5-25 | TCCAGGATGGCATTGGGCAGC | 448 | GCTGCCCAATGCCATCCTGG A | 1144 |
| 21 | H45_6-26 | CCAGGATGGCATTGGGCAGCG | 449 | CGCTGCCCAATGCCATCCTG G | 1145 |
| 21 | H45_7-27 | CAGGATGGCATTGGGCAGCGG | 450 | CCGCTGCCCAATGCCATCCT G | 1146 |
| 21 | H45_8-28 | AGGATGGCATTGGGCAGCGGC | 451 | GCCGCTGCCCAATGCCATCC T | 1147 |
| 21 | H45_9-29 | GGATGGCATTGGGCAGCGGCA | 452 | TGCCGCTGCCCAATGCCATC C | 1148 |
| 21 | H45_10-30 | GATGCCATTGGGCAGCGGCAA | 453 | TTGCCGCTGCCCAATGCCAT C | 1149 |
| 21 | H45_11-31 | ATGGCATTGGGCAGCGGCAAA | 454 | TTTGCCGCTGCCCAATGCCA T | 1150 |
| 21 | H45_12-32 | TGGCATTGGGCAGCGGCAAAC | 455 | GTTTGCCGCTGCCCAATGCC A | 1151 |
| 21 | H45_13-33 | GGCATTGGGCAGCGGCAAACT | 456 | AGTTTGCCGCTGCCCAATGC C | 1152 |
| 21 | H45_14-34 | GCATTGGGCAGCGGCAAACTG | 457 | CAGTTTGCCGCTGCCCAATG C | 1153 |
| 21 | H45_15-35 | CATTGGGCAGCGGCAAACTGT | 458 | ACAGTTTGCCGCTGCCCAAT G | 1154 |
| 21 | H45_16-36 | ATTGGGCAGCGGCAAACTGTT | 459 | AACAGTTTGCCGCTGCCCAA T | 1155 |
| 21 | H45_17-37 | TTGGGCAGCGGCAAACTGTTG | 460 | CAACAGTTTGCCGCTGCCCA A | 1156 |
| 21 | H45_18-38 | TGGGCAGCGGCAAACTGTTGT | 461 | ACAACAGTTTGCCGCTGCCC A | 1157 |
| 21 | H45_19-39 | GGGCAGCGGCAAACTGTTGTC | 462 | GACAACAGTTTGCCGCTGCC C | 1158 |
| 21 | H45_20-40 | GGCAGCGGCAAACTGTTGTCA | 463 | TGACAACAGTTTGCCGCTGC C | 1159 |
| 21 | H45_21-41 | GCAGCGGCAAACTGTTGTCAG | 464 | CTGACAACAGTTTGCCGCTG C | 1160 |

**[Table 4-15]**

| | | | | | |
|---|---|---|---|---|---|
| 21 | H45_22-42 | CAGCGGCAAACTGTTGTCAGA | 466 | TCTGACAACAGTTTGCCGCT G | 1161 |
| 21 | H45_23-43 | AGCGGCAAACTGTTGTCAGAA | 466 | TTCTGACAACAGTTTGCCGC T | 1162 |
| 21 | H45_24-44 | GCGGCAAACTGTTGTCAGAAC | 467 | GTTCTGACAACAGTTTGCCG C | 1163 |
| 21 | H45_25-45 | CGGCAAACTGTTGTCAGAACA | 468 | TGTTCTGACAACAGTTTGCC G | 1164 |
| 21 | H45_26-46 | GGCAAACTGTTGTCAGAACAT | 469 | ATGTTCTGACAACAGTTTGC C | 1165 |
| 21 | H45_27-47 | GCAAACTGTTGTCAGAACATT | 470 | AATGTTCTGACAACAGTTTG C | 1166 |
| 21 | H45_28-48 | CAAACTGTTGTCAGAACATTG | 471 | CAATGTTCTGACAACAGTTT G | 1167 |
| 21 | H45_29-49 | AAACTGTTGTCAGAACATTGA | 472 | TCAATGTTCTGACAACAGTT T | 1168 |
| 21 | H45_30-50 | AACTGTTGTCAGAACATTGAA | 473 | TTCAATGTTCTGACAACAGT T | 1169 |
| 21 | H45_31-51 | ACTGTTGTCAGAACATTGAAT | 474 | ATTCAATGTTCTGACAACAG T | 1170 |
| 21 | H45_32-52 | CTGTTGTCAGAACATTGAATG | 475 | CATTCAATGTTCTGACAACA G | 1171 |
| 21 | H45_33-53 | TGTTGTCAGAACATTGAATGC | 476 | GCATTCAATGTTCTGACAAC A | 1172 |
| 21 | H45_34-54 | GTTGTCAGAACATTGAATGCA | 477 | TGCATTCAATGTTCTGACAA C | 1173 |
| 21 | H45_35-55 | TTGTCAGAACATTGAATGCAA | 478 | TTGCATTCAATGTTCTGACA A | 1174 |
| 21 | H45_36-56 | TGTCAGAACATTGAATGCAAC | 479 | GTTGCATTCAATGTTCTGAC A | 1175 |
| 21 | H45_37-57 | GTCAGAACATTGAATGCAACT | 480 | AGTTGCATTCAATGTTCTGA C | 1176 |
| 21 | H45_38-58 | TCAGAACATTGAATGCAACTG | 481 | CAGTTGCATTCAATGTTCTG A | 1177 |
| 21 | H45_39-59 | CAGAACATTGAATGCAACTGG | 482 | CCAGTTGCATTCAATGTTCT G | 1178 |

**[Table 4-16]**

| | | | | | |
|---|---|---|---|---|---|
| 21 | H45_40-60 | AGAACATTGAATGCAACTGGG | 483 | CCCAGTTGCATTCAATGTTC T | 1179 |
| 22 | H45_(-3)-19 | CAGGAACTCCAGGATGGCATTG | 484 | CAATGCCATCCTGGAGTTCC TG | 1180 |
| 22 | H45_(-2)-20 | AGGAACTCCAGGATGGCATTGG | 485 | CCAATGCCATCCTGGAGTTC CT | 1181 |
| 22 | H45_(-1)-21 | GGAACTCCAGGATGGCATTGGG | 486 | CCCAATGCCATCCTGGAGTT CC | 1182 |
| 22 | H45_1-22 | GAACTCCAGGATGGCATTGGGC | 487 | GCCCAATGCCATCCTGGAGT TC | 1183 |
| 22 | H45_2-23 | AACTCCAGGATGGCATTGGGCA | 488 | TGCCCAATGCCATCCTGGAG TT | 1184 |
| 22 | H45_3-24 | ACTCCAGGATGGCATTGGGCAG | 489 | CTGCCCAATGCCATCCTGGA GT | 1185 |
| 22 | H45_4-25 | CTCCAGGATGGCATTGGGCAGC | 490 | GCTGCCCAATGCCATCCTGG AG | 1186 |
| 22 | H45_5-26 | TCCAGGATGGCATTGGGCAGCG | 491 | CGCTGCCCAATGCCATCCTG GA | 1187 |
| 22 | H45_6-27 | CCAGGATGGCATTGGGCAGCGG | 492 | CCGCTGCCCAATGCCATCCT GG | 1188 |
| 22 | H45_7-28 | CAGGATGGCATTGGGCAGCGGC | 493 | GCCGCTGCCCAATGCCATCC TG | 1189 |
| 22 | H45_8-29 | AGGATGGCATTGGGCAGCGGCA | 494 | TGCCGCTGCCCAATGCCATC CT | 1190 |
| 22 | H45_9-30 | GGATGGCATTGGGCAGCGGCAA | 495 | TTGCCGCTGCCCAATGCCAT CC | 1191 |
| 22 | H45_10-31 | GATGGCATTGGGCAGCGGCAAA | 496 | TTTGCCGCTGCCCAATGCCA TC | 1192 |
| 22 | H45_11-32 | ATGGCATTGGGCAGCGGCAAAC | 497 | GTTTGCCGCTGCCCAATGCC AT | 1193 |
| 22 | H45_12-33 | TGGCATTGGGCAGCGGCAAACT | 498 | AGTTTGCCGCTGCCCAATGC CA | 1194 |
| 22 | H45_13-34 | GGCATTGGGCAGCGGCAAACTG | 499 | CAGTTTGCCGCTGCCCAATG CC | 1195 |
| 22 | H45_14-35 | GCATTGGGCAGCGGCAAACTGT | 500 | ACAGTTTGCCGCTGCCCAAT GC | 1196 |

**[Table 4-17]**

| | | | | | |
|---|---|---|---|---|---|
| 22 | H45_15-36 | CATTGGGCAGCGGCAAACTGTT | 501 | AACAGTTTGCCGCTGCCCAA TG | 1197 |
| 22 | H45_16-37 | ATTGGGCAGCGGCAAACTGTTG | 502 | CAACAGTTTGCCGCTGCCCA AT | 1198 |
| 22 | H45_17-38 | TTGGGCAGCGGCAAACTGTTGT | 503 | ACAACAGTTTGCCGCTGCCC AA | 1199 |
| 22 | H45_18-39 | TGGGCAGCGGCAAACTGTTGTC | 504 | GACAACAGTTTGCCGCTGCC CA | 1200 |
| 22 | H45_19-40 | GGGCAGCGGCAAACTGTTGTCA | 505 | TGACAACAGTTTGCCGCTGC CC | 1201 |
| 22 | H45_20-41 | GGCAGCGGCAAACTGTTGTCAG | 506 | CTGACAACAGTTTGCCGCTG CC | 1202 |
| 22 | H45_21-42 | GCAGCGGCAAACTGTTGTCAGA | 507 | TCTGACAACAGTTTGCCGCT GC | 1203 |
| 22 | H45_22-43 | CAGCGGCAAACTGTTGTCAGAA | 508 | TTCTGACAACAGTTTGCCGC TG | 1204 |
| 22 | H45_23-44 | AGCGGCAAACTGTTGTCAGAAC | 509 | GTTCTGACAACAGTTTGCCG CT | 1205 |
| 22 | H45_24-45 | GCGGCAAACTGTTGTCAGAACA | 510 | TGTTCTGACAACAGTTTGCC GC | 1206 |
| 22 | H45_25-46 | CGGCAAACTGTTGTCAGAACAT | 511 | ATGTTCTGACAACAGTTTGC CG | 1207 |
| 22 | H45_26-47 | GGCAAACTGTTGTCAGAACATT | 512 | AATGTTCTGACAACAGTTTG CC | 1208 |
| 22 | H45_27-48 | GCAAACTGTTGTCAGAACATTG | 513 | CAATGTTCTGACAACAGTTT GC | 1209 |
| 22 | H45_28-49 | CAAACTGTTGTCAGAACATTGA | 514 | TCAATGTTCTGACAACAGTT TG | 1210 |
| 22 | H45_29-50 | AAACTGTTGTCAGAACATTGAA | 515 | TTCAATGTTCTGACAACAGT TT | 1211 |
| 22 | H45_30-51 | AACTGTTGTCAGAACATTGAAT | 516 | ATTCAATGTTCTGACAACAG TT | 1212 |
| 22 | H45_31-52 | ACTGTTGTCAGAACATTGAATG | 517 | CATTCAATGTTCTGACAACA GT | 1213 |
| 22 | H45_32-53 | CTGTTGTCAGAACATTGAATGC | 518 | GCATTCAATGTTCTGACAAC AG | 1214 |

**[Table 4-18]**

| | | | | | |
|---|---|---|---|---|---|
| 22 | H45_33-54 | TGTTGTCAGAACATTGAATGCA | 519 | TGCATTCAATGTTCTGACAA CA | 1215 |
| 22 | H45_34-55 | GTTGTCAGAACATTGAATGCAA | 520 | TTGCATTCAATGTTCTGACA AC | 1216 |
| 22 | H45_35-56 | TTGTCAGAACATTGAATGCAAC | 521 | GTTGCATTCAATGTTCTGAC AA | 1217 |
| 22 | H45_36-57 | TGTCAGAACATTGAATGCAACT | 522 | AGTTGCATTCAATGTTCTGA CA | 1218 |
| 22 | H45_37-58 | GTCAGAACATTGAATGCAACTG | 523 | CAGTTGCATTCAATGTTCTG AC | 1219 |
| 22 | H45_38-59 | TCAGAACATTGAATGCAACTGG | 524 | CCAGTTGCATTCAATGTTCT GA | 1220 |
| 22 | H45_39-60 | CAGAACATTGAATGCAACTGGG | 525 | CCCAGTTGCATTCAATGTTC TG | 1221 |
| 22 | H45_40-61 | AGAACATTGAATGCAACTGGGG | 526 | CCCCAGTTGCATTCAATGTT CT | 1222 |
| 23 | H45_(-4)-19 | ACAGGAACTCCAGGATGGCATT G | 527 | CAATGCCATCCTGGAGTTCC TGT | 1223 |
| 23 | H45_(-3)-20 | CAGGAACTCCAGGATGGCATTG G | 528 | CCAATGCCATCCTGGAGTTC CTG | 1224 |
| 23 | H45_(-2)-21 | AGGAACTCCAGGATGGCATTGG G | 529 | CCCAATGCCATCCTGGAGTT CCT | 1225 |
| 23 | H45_(-1)-22 | GGAACTCCAGGATGGCATTGGG C | 530 | GCCCAATGCCATCCTGGAGT TCC | 1226 |
| 23 | H45_1-23 | GAACTCCAGGATGGCATTGGGC A | 531 | TGCCCAATGCCATCCTGGAG TTC | 1227 |
| 23 | H45_2-24 | AACTCCAGGATGGCATTGGGCA G | 532 | CTGCCCAATGCCATCCTGGA GTT | 1228 |
| 23 | H45_3-25 | ACTCCAGGATGGCATTGGGCAG C | 533 | GCTGCCCAATGCCATCCTGG AGT | 1229 |
| 23 | H45_4-26 | CTCCAGGATGGCATTGGGCAGC G | 534 | CGCTGCCCAATGCCATCCTG GAG | 1230 |
| 23 | H45_5-27 | TCCAGGATGGCATTGGGCAGCG G | 535 | CCGCTGCCCAATGCCATCCT GGA | 1231 |
| 23 | H45_6-28 | CCAGGATGGCATTGGGCAGCGG C | 536 | GCCGCTGCCCAATGCCATCC TGG | 1232 |

**[Table 4-19]**

| | | | | | |
|---|---|---|---|---|---|
| 23 | H45_7-29 | CAGGATGGCATTGGGCAGCGGC A | 537 | TGCCGCTGCCCAATGCCATC CTG | 1233 |
| 23 | H45_8-30 | AGGATGGCATTGGGCAGCGGCA A | 538 | TTGCCGCTGCCCAATGCCAT CCT | 1234 |
| 23 | H45_9-31 | GGATGGCATTGGGCAGCGGCAA A | 539 | TTTGCCGCTGCCCAATGCCA TCC | 1235 |
| 23 | H45_10-32 | GATGGCATTGGGCAGCGGCAAA C | 540 | GTTTGCCGCTGCCCAATGCC ATC | 1236 |
| 23 | H45_11-33 | ATGGCATTGGGCAGCGGCAAAC T | 541 | AGTTTGCCGCTGCCCAATGC CAT | 1237 |
| 23 | H45_12-34 | TGGCATTGGGCAGCGGCAAACT G | 542 | CAGTTTGCCGCTGCCCAATG CCA | 1238 |
| 23 | H45_13-35 | GGCATTGGGCAGCGGCAAACTG T | 543 | ACAGTTTGCCGCTGCCCAAT GCC | 1239 |
| 23 | H45_14-36 | GCATTGGGCAGCGGCAAACTGT T | 544 | AACAGTTTGCCGCTGCCCAA TGC | 1240 |
| 23 | H45_15-37 | CATTGGGCAGCGGCAAACTGTT G | 545 | CAACAGTTTGCCGCTGCCCA ATG | 1241 |
| 23 | H45_16-38 | ATTGGGCAGCGGCAAACTGTTG T | 546 | ACAACAGTTTGCCGCTGCCC AAT | 1242 |
| 23 | H45_17-39 | TTGGGCAGCGGCAAACTGTTGT C | 547 | GACAACAGTTTGCCGCTGCC CAA | 1243 |
| 23 | H45_18-40 | TGGGCAGCGGCAAACTGTTGTC A | 548 | TGACAACAGTTTGCCGCTGC CCA | 1244 |
| 23 | H45_19-41 | GGGCAGCGGCAAACTGTTGTCA G | 549 | CTGACAACAGTTTGCCGCTG CCC | 1245 |
| 23 | H45_20-42 | GGCAGCGGCAAACTGTTGTCAG A | 550 | TCTGACAACAGTTTGCCGCT GCC | 1246 |
| 23 | H45_21-43 | GCAGCGGCAAACTGTTGTCAGA A | 551 | TTCTGACAACAGTTTGCCGC TGC | 1247 |
| 23 | H45_22-44 | CAGCGGCAAACTGTTGTCAGAA C | 552 | GTTCTGACAACAGTTTGCCG CTG | 1248 |
| 23 | H45_23-45 | AGCGGCAAACTGTTGTCAGAAC A | 553 | TGTTCTGACAACAGTTTGCC GCT | 1249 |
| 23 | H45_24-46 | GCGGCAAACTGTTGTCAGAACA T | 554 | ATGTTCTGACAACAGTTTGC CGC | 1250 |

**[Table 4-20]**

| | | | | | |
|---|---|---|---|---|---|
| 23 | H45_25-47 | CGGCAAACTGTTGTCAGAACAT T | 555 | AATGTTCTGACAACAGTTTG CCG | 1251 |
| 23 | H45_26-48 | GGCAAACTGTTGTCAGAACATT G | 556 | CAATGTTCTGACAACAGTTT GCC | 1252 |
| 23 | H45_27-49 | GCAAACTGTTGTCAGAACATTG A | 557 | TCAATGTTCTGACAACAGTT TGC | 1253 |
| 23 | H45_28-50 | CAAACTGTTGTCAGAACATTGA A | 558 | TTCAATGTTCTGACAACAGT TTG | 1254 |
| 23 | H45_29-51 | AAACTGTTGTCAGAACATTGAA T | 559 | ATTCAATGTTCTGACAACAG TTT | 1255 |
| 23 | H45_30-52 | AACTGTTGTCAGAACATTGAAT G | 560 | CATTCAATGTTCTGACAACA GTT | 1256 |
| 23 | H45_31-53 | ACTGTTGTCAGAACATTGAATG C | 561 | GCATTCAATGTTCTGACAAC AGT | 1257 |
| 23 | H45_32-54 | CTGTTGTCAGAACATTGAATGC A | 562 | TGCATTCAATGTTCTGACAA CAG | 1258 |
| 23 | H45_33-55 | TGTTGTCAGAACATTGAATGCA A | 563 | TTGCATTCAATGTTCTGACA ACA | 1259 |
| 23 | H45_34-56 | GTTGTCAGAACATTGAATGCAA C | 564 | GTTGCATTCAATGTTCTGAC AAC | 1260 |
| 23 | H45_35-57 | TTGTCAGAACATTGAATGCAAC T | 565 | AGTTGCATTCAATGTTCTGA CAA | 1261 |
| 23 | H45_36-58 | TGTCAGAACATTGAATGCAACT G | 566 | CAGTTGCATTCAATGTTCTG ACA | 1262 |
| 23 | H45_37-59 | GTCAGAACATTGAATGCAACTG G | 567 | CCAGTTGCATTCAATGTTCT GAC | 1263 |
| 23 | H45_38-60 | TCAGAACATTGAATGCAACTGG G | 568 | CCCAGTTGCATTCAATGTTC TGA | 1264 |
| 23 | H45_39-61 | CAGAACATTGAATGCAACTGGG G | 569 | CCCCAGTTGCATTCAATGTT CTG | 1265 |
| 23 | H45_40-62 | AGAACATTGAATGCAACTGGGG A | 570 | TCCCCAGTTGCATTCAATGT TCT | 1266 |
| 24 | H45_(-5)-19 | TACAGGAACTCCAGGATGGCAT TG | 571 | CAATGCCATCCTGGAGTTCC TGTA | 1267 |
| 24 | H45_(-4)-20 | ACAGGAACTCCAGGATGGCATT GG | 572 | CCAATGCCATCCTGGAGTTC CTGT | 1268 |

**[Table 4-21]**

| | | | | | |
|---|---|---|---|---|---|
| 24 | H45_(-3)-21 | CAGGAACTCCAGGATGGCATTG GG | 573 | CCCAATGCCATCCTGGAGTT CCTG | 1269 |
| 24 | H45_(-2)-22 | AGGAACTCCAGGATGGCATTGG GC | 574 | GCCCAATGCCATCCTGGAGT TCCT | 1270 |
| 24 | H45_(-1)-23 | GGAACTCCAGGATGGCATTGGG CA | 575 | TGCCCAATGCCATCCTGGAG TTCC | 1271 |
| 24 | H45_1-24 | GAACTCCAGGATGGCATTGGGC AG | 576 | CTGCCCAATGCCATCCTGGA GTTC | 1272 |
| 24 | H45_2-25 | AACTCCAGGATGGCATTGGGCA GC | 577 | GCTGCCCAATGCCATCCTGG AGTT | 1273 |
| 24 | H45_3-26 | ACTCCAGGATGGCATTGGGCAG CG | 578 | CGCTGCCCAATGCCATCCTG GAGT | 1274 |
| 24 | H45_4-27 | CTCCAGGATGGCATTGGGCAGC GG | 579 | CCGCTGCCCAATGCCATCCT GGAG | 1275 |
| 24 | H45_5-28 | TCCAGGATGGCATTGGGCAGCG GC | 580 | GCCGCTGCCCAATGCCATCC TGGA | 1276 |
| 24 | H45_6-29 | CCAGGATGGCATTGGGCAGCGG CA | 581 | TGCCGCTGCCCAATGCCATC CTGG | 1277 |
| 24 | H45_7-30 | CAGGATGGCATTGGGCAGCGGC AA | 582 | TTGCCGCTGCCCAATGCCAT CCTG | 1278 |
| 24 | H45_8-31 | AGGATGGCATTGGGCAGCGGCA AA | 583 | TTTGCCGCTGCCCAATGCCA TCCT | 1279 |
| 24 | H45_9-32 | GGATGGCATTGGGCAGCGGCAA AC | 584 | GTTTGCCGCTGCCCAATGCC ATCC | 1280 |
| 24 | H45_10-33 | GATGGCATTGGGCAGCGGCAAA CT | 585 | AGTTTGCCGCTGCCCAATGC CATC | 1281 |
| 24 | H45_11-34 | ATGGCATTGGGCAGCGGCAAAC TG | 586 | CAGTTTGCCGCTGCCCAATG CCAT | 1282 |
| 24 | H45_12-35 | TGGCATTGGGCAGCGGCAAACT GT | 587 | ACAGTTTGCCGCTGCCCAAT GCCA | 1283 |
| 24 | H45_13-36 | GGCATTGGGCAGCGGCAAACTG TT | 588 | AACAGTTTGCCGCTGCCCAA TGCC | 1284 |
| 24 | H45_14-37 | GCATTGGGCAGCGGCAAACTGT TG | 589 | CAACAGTTTGCCGCTGCCCA ATGC | 1285 |
| 24 | H45_15-38 | CATTGGGCAGCGGCAAACTGTT GT | 590 | ACAACAGTTTGCCGCTGCCC AATG | 1286 |

**[Table 4-22]**

| | | | | | |
|---|---|---|---|---|---|
| 24 | H45_16-39 | ATTGGGCAGCGGCAAACTGTTG TC | 591 | GACAACAGTTTGCCGCTGCC CAAT | 1287 |
| 24 | H45_17-40 | TTGGGCAGCGGCAAACTGTTGT CA | 592 | TGACAACAGTTTGCCGCTGC CCAA | 1288 |
| 24 | H45_18-41 | TGGGCAGCGGCAAACTGTTGTC AG | 593 | CTGACAACAGTTTGCCGCTG CCCA | 1289 |
| 24 | H45_19-42 | GGGCAGCGGCAAACTGTTGTCA GA | 594 | TCTGACAACAGTTTGCCGCT GCCC | 1290 |
| 24 | H45_20-43 | GGCAGCGGCAAACTGTTGTCAG AA | 595 | TTCTGACAACAGTTTGCCGC TGCC | 1291 |
| 24 | H45_21-44 | GCAGCGGCAAACTGTTGTCAGA AC | 596 | GTTCTGACAACAGTTTGCCG CTGC | 1292 |
| 24 | H45_22-45 | CAGCGGCAAACTGTTGTCAGAA CA | 597 | TGTTCTGACAACAGTTTGCC GCTG | 1293 |
| 24 | H45_23-46 | AGCGGCAAACTGTTGTCAGAAC AT | 598 | ATGTTCTGACAACAGTTTGC CGCT | 1294 |
| 24 | H45_24-47 | GCGGCAAACTGTTGTCAGAACA TT | 599 | AATGTTCTGACAACAGTTTG CCGC | 1295 |
| 24 | H45_25-48 | CGGCAAACTGTTGTCAGAACAT TG | 600 | CAATGTTCTGACAACAGTTT GCCG | 1296 |
| 24 | H45_26-49 | GGCAAACTGTTGTCAGAACATT GA | 601 | TCAATGTTCTGACAACAGTT TGCC | 1297 |
| 24 | H45_27-50 | GCAAACTGTTGTCAGAACATTG AA | 602 | TTCAATGTTCTGACAACAGT TTGC | 1298 |
| 24 | H45_28-51 | CAAACTGTTGTCAGAACATTGA AT | 603 | ATTCAATGTTCTGACAACAG TTTG | 1299 |
| 24 | H45_29-52 | AAACTGTTGTCAGAACATTGAA TG | 604 | CATTCAATGTTCTGACAACA GTTT | 1300 |
| 24 | H45_30-53 | AACTGTTGTCAGAACATTGAAT GC | 605 | GCATTCAATGTTCTGACAAC AGTT | 1301 |
| 24 | H45_31-54 | ACTGTTGTCAGAACATTGAATG CA | 606 | TGCATTCAATGTTCTGACAA CAGT | 1302 |
| 24 | H45_32-55 | CTGTTGTCAGAACATTGAATGC AA | 607 | TTGCATTCAATGTTCTGACA ACAG | 1303 |
| 24 | H45_33-56 | TGTTGTCAGAACATTGAATGCA AC | 608 | GTTGCATTCAATGTTCTGAC AACA | 1304 |

**[Table 4-23]**

| | | | | | |
|---|---|---|---|---|---|
| 24 | H45_34-57 | GTTGTCAGAACATTGAATGCAA CT | 609 | AGTTGCATTCAATGTTCTGA CAAC | 1305 |
| 24 | H45_35-58 | TTGTCAGAACATTGAATGCAAC TG | 610 | CAGTTGCATTCAATGTTCTG ACAA | 1306 |
| 24 | H45_36-59 | TGTCAGAACATTGAATGCAACT GG | 611 | CCAGTTGCATTCAATGTTCT GACA | 1307 |
| 24 | H45_37-60 | GTCAGAACATTGAATGCAACTG GG | 612 | CCCAGTTGCATTCAATGTTC TGAC | 1308 |
| 24 | H45_38-61 | TCAGAACATTGAATGCAACTGG GG | 613 | CCCCAGTTGCATTCAATGTT CTGA | 1309 |
| 24 | H45_39-62 | CAGAACATTGAATGCAACTGGG GA | 614 | TCCCCAGTTGCATTCAATGT TCTG | 1310 |
| 24 | H45_40-63 | AGAACATTGAATGCAACTGGGG AA | 615 | TTCCCCAGTTGCATTCAATG TTCT | 1311 |
| 25 | H45_(-6)-19 | TTACAGGAACTCCAGGATGGCA TTG | 616 | CAATGCCATCCTGGAGTTCC TGTAA | 1312 |
| 25 | H45_(-5)-20 | TACAGGAACTCCAGGATGGCAT TGG | 617 | CCAATGCCATCCTGGAGTTC CTGTA | 1313 |
| 25 | H45_(-4)-21 | ACAGGAACTCCAGGATGGCATT GGG | 618 | CCCAATGCCATCCTGGAGTT CCTGT | 1314 |
| 25 | H45_(-3)-22 | CAGGAACTCCAGGATGGCATTG GGC | 619 | GCCCAATGCCATCCTGGAGT TCCTG | 1315 |
| 25 | H45_(-2)-23 | AGGAACTCCAGGATGGCATTGG GCA | 620 | TGCCCAATGCCATCCTGGAG TTCCT | 1316 |
| 25 | H45_(-1)-24 | GGAACTCCAGGATGGCATTGGG CAG | 621 | CTGCCCAATGCCATCCTGGA GTTCC | 1317 |
| 25 | H45_1-25 | GAACTCCAGGATGGCATTGGGC AGC | 622 | GCTGCCCAATGCCATCCTGG AGTTC | 1318 |
| 25 | H45_2-26 | AACTCCAGGATGGCATTGGGCA GCG | 623 | CGCTGCCCAATGCCATCCTG GAGTT | 1319 |
| 25 | H45_3-27 | ACTCCAGGATGGCATTGGGCAG CGG | 624 | CCGCTGCCCAATGCCATCCT GGAGT | 1320 |
| 25 | H45_4-28 | CTCCAGGATGGCATTGGGCAGC GGC | 625 | GCCGCTGCCCAATGCCATCC TGGAG | 1321 |
| 25 | H45_5-29 | TCCAGGATGGCATTGGGCAGCG GCA | 626 | TGCCGCTGCCCAATGCCATC CTGGA | 1322 |

**[Table 4-24]**

| | | | | | |
|---|---|---|---|---|---|
| 25 | H45_6-30 | CCAGGATGGCATTGGGCAGCGG CAA | 627 | TTGCCGCTGCCCAATGCCAT CCTGG | 1323 |
| 25 | H45_7-31 | CAGGATGGCATTGGGCAGCGGC AAA | 628 | TTTGCCGCTGCCCAATGCCA TCCTG | 1324 |
| 25 | H45_8-32 | AGGATGGCATTGGGCAGCGGCA AAC | 629 | GTTTGCCGCTGCCCAATGCC ATCCT | 1325 |
| 25 | H45_9-33 | GGATGGCATTGGGCAGCGGCAA ACT | 630 | AGTTTGCCGCTGCCCAATGC CATCC | 1326 |
| 25 | H45_10-34 | GATGGCATTGGGCAGCGGCAAA CTG | 631 | CAGTTTGCCGCTGCCCAATG CCATC | 1327 |
| 25 | H45_11-35 | ATGGCATTGGGCAGCGGCAAAC TGT | 632 | ACAGTTTGCCGCTGCCCAAT GCCAT | 1328 |
| 25 | H45_12-36 | TGGCATTGGGCAGCGGCAAACT GTT | 633 | AACAGTTTGCCGCTGCCCAA TGCCA | 1329 |
| 25 | H45_13-37 | GGCATTGGGCAGCGGCAAACTG TTG | 634 | CAACAGTTTGCCGCTGCCCA ATGCC | 1330 |
| 25 | H45_14-38 | GCATTGGGCAGCGGCAAACTGT TGT | 635 | ACAACAGTTTGCCGCTGCCC AATGC | 1331 |
| 25 | H45_15-39 | CATTGGGCAGCGGCAAACTGTT GTC | 636 | GACAACAGTTTGCCGCTGCC CAATG | 1332 |
| 25 | H45_16-40 | ATTGGGCAGCGGCAAACTGTTG TCA | 637 | TGACAACAGTTTGCCGCTGC CCAAT | 1333 |
| 25 | H45_17-41 | TTGGGCAGCGGCAAACTGTTGT CAG | 638 | CTGACAACAGTTTGCCGCTG CCCAA | 1334 |
| 25 | H45_18-42 | TGGGCAGCGGCAAACTGTTGTC AGA | 639 | TCTGACAACAGTTTGCCGCT GCCCA | 1335 |
| 25 | H45_19-43 | GGGCAGCGGCAAACTGTTGTCA GAA | 640 | TTCTGACAACAGTTTGCCGC TGCCC | 1336 |
| 25 | H45_20-44 | GGCAGCGGCAAACTGTTGTCAG AAC | 641 | GTTCTGACAACAGTTTGCCG CTGCC | 1337 |
| 25 | H45_21-45 | GCAGCGGCAAACTGTTGTCAGA ACA | 642 | TGTTCTGACAACAGTTTGCC GCTGC | 1338 |
| 25 | H45_22-46 | CAGCGGCAAACTGTTGTCAGAA CAT | 643 | ATGTTCTGACAACAGTTTGC CGCTG | 1339 |
| 25 | H45_23-47 | AGCGGCAAACTGTTGTCAGAAC ATT | 644 | AATGTTCTGACAACAGTTTG CCGCT | 1340 |

**[Table 4-25]**

| | | | | | |
|---|---|---|---|---|---|
| 25 | H45_24-48 | GCGGCAAACTGTTGTCAGAACA TTG | 645 | CAATGTTCTGACAACAGTTT GCCGC | 1341 |
| 25 | H45_25-49 | CGGCAAACTGTTGTCAGAACAT TGA | 646 | TCAATGTTCTGACAACAGTT TGCCG | 1342 |
| 25 | H45_26-50 | GGCAAACTGTTGTCAGAACATT GAA | 647 | TTCAATGTTCTGACAACAGT TTGCC | 1343 |
| 25 | H45_27-51 | GCAAACTGTTGTCAGAACATTG AAT | 648 | ATTCAATGTTCTGACAACAG TTTGC | 1344 |
| 25 | H45_28-52 | CAAACTGTTGTCAGAACATTGA ATG | 649 | CATTCAATGTTCTGACAACA GTTTG | 1345 |
| 25 | H45_29-53 | AAACTGTTGTCAGAACATTGAA TGC | 650 | GCATTCAATGTTCTGACAAC AGTTT | 1346 |
| 25 | H45_30-54 | AACTGTTGTCAGAACATTGAAT GCA | 651 | TGCATTCAATGTTCTGACAA CAGTT | 1347 |
| 25 | H45_31-55 | ACTGTTGTCAGAACATTGAATG CAA | 652 | TTGCATTCAATGTTCTGACA ACAGT | 1348 |
| 25 | H45_32-56 | CTGTTGTCAGAACATTGAATGC AAC | 653 | GTTGCATTCAATGTTCTGAC AACAG | 1349 |
| 25 | H45_33-57 | TGTTGTCAGAACATTGAATGCA ACT | 654 | AGTTGCATTCAATGTTCTGA CAACA | 1350 |
| 25 | H45_34-58 | GTTGTCAGAACATTGAATGCAA CTG | 655 | CAGTTGCATTCAATGTTCTG ACAAC | 1351 |
| 25 | H45_35-59 | TTGTCAGAACATTGAATGCAAC TGG | 656 | CCAGTTGCATTCAATGTTCT GACAA | 1352 |
| 25 | H45_36-60 | TGTCAGAACATTGAATGCAACT GGG | 657 | CCCAGTTGCATTCAATGTTC TGACA | 1353 |
| 25 | H45_37-61 | GTCAGAACATTGAATGCAACTG GGG | 658 | CCCCAGTTGCATTCAATGTT CTGAC | 1354 |
| 25 | H45_38-62 | TCAGAACATTGAATGCAACTGG GGA | 659 | TCCCCAGTTGCATTCAATGT TCTGA | 1355 |
| 25 | H45_39-63 | CAGAACATTGAATGCAACTGGG GAA | 660 | TTCCCCAGTTGCATTCAATG TTCTG | 1356 |
| 25 | H45_40-64 | AGAACATTGAATGCAACTGGGG AAG | 661 | CTTCCCCAGTTGCATTCAAT GTTCT | 1357 |
| 26 | H45_(-7)-19 | CTTACAGGAACTCCAGGATGGC ATTG | 662 | CAATGCCATCCTGGAGTTCC TGTAAG | 1358 |

**[Table 4-26]**

| | | | | | |
|---|---|---|---|---|---|
| 26 | H45_(-6)-20 | TTACAGGAACTCCAGGATGGCA TTGG | 663 | CCAATGCCATCCTGGAGTTC CTGTAA | 1359 |
| 26 | H45_(-5)-21 | TACAGGAACTCCAGGATGGCAT TGGG | 664 | CCCAATGCCATCCTGGAGTT CCTGTA | 1360 |
| 26 | H45_(-4)-22 | ACAGGAACTCCAGGATGGCATT GGGC | 665 | GCCCAATGCCATCCTGGAGT TCCTGT | 1361 |
| 26 | H45_(-3)-23 | CAGGAACTCCAGGATGGCATTG GGCA | 666 | TGCCCAATGCCATCCTGGAG TTCCTG | 1362 |
| 26 | H45_(-2)-24 | AGGAACTCCAGGATGGCATTGG GCAG | 667 | CTGCCCAATGCCATCCTGGA GTTCCT | 1363 |
| 26 | H45_(-1)-25 | GGAACTCCAGGATGGCATTGGG CAGC | 668 | GCTGCCCAATGCCATCCTGG AGTTCC | 1364 |
| 26 | H45_1-26 | GAACTCCAGGATGGCATTGGGC AGCG | 669 | CGCTGCCCAATGCCATCCTG GAGTTC | 1365 |
| 26 | H45_2-27 | AACTCCAGGATGGCATTGGGCA GCGG | 670 | CCGCTGCCCAATGCCATCCT GGAGTT | 1366 |
| 26 | H45_3-28 | ACTCCAGGATGGCATTGGGCAG CGGC | 671 | GCCGCTGCCCAATGCCATCC TGGAGT | 1367 |
| 26 | H45_4-29 | CTCCAGGATGGCATTGGGCAGC GGCA | 672 | TGCCGCTGCCCAATGCCATC CTGGAG | 1368 |
| 26 | H45_5-30 | TCCAGGATGGCATTGGGCAGCG GCAA | 673 | TTGCCGCTGCCCAATGCCAT CCTGGA | 1369 |
| 26 | H45_6-31 | CCAGGATGGCATTGGGCAGCGG CAAA | 674 | TTTGCCGCTGCCCAATGCCA TCCTGG | 1370 |
| 26 | H45_7-32 | CAGGATGGCATTGGGCAGCGGC AAAC | 675 | GTTTGCCGCTGCCCAATGCC ATCCTG | 1371 |
| 26 | H45_8-33 | AGGATGGCATTGGGCAGCGGCA AACT | 676 | AGTTTGCCGCTGCCCAATGC CATCCT | 1372 |
| 26 | H45_9-34 | GGATGGCATTGGGCAGCGGCAA ACTG | 677 | CAGTTTGCCGCTGCCCAATG CCATCC | 1373 |
| 26 | H45_10-35 | GATGGCATTGGGCAGCGGCAAA CTGT | 678 | ACAGTTTGCCGCTGCCCAAT GCCATC | 1374 |
| 26 | H45_11-36 | ATGGCATTGGGCAGCGGCAAAC TGTT | 679 | AACAGTTTGCCGCTGCCCAA TGCCAT | 1375 |
| 26 | H45_12-37 | TGGCATTGGGCAGCGGCAAACT GTTG | 680 | CAACAGTTTGCCGCTGCCCA ATGCCA | 1376 |

**[Table 4-27]**

| | | | | | |
|---|---|---|---|---|---|
| 26 | H45_13-38 | GGCATTGGGCAGCGGCAAACTG TTGT | 681 | ACAACAGTTTGCCGCTGCCC AATGCC | 1377 |
| 26 | H45_14-39 | GCATTGGGCAGCGGCAAACTGT TGTC | 682 | GACAACAGTTTGCCGCTGCC CAATGC | 1378 |
| 26 | H45_15-40 | CATTGGGCAGCGGCAAACTGTT GTCA | 683 | TGACAACAGTTTGCCGCTGC CCAATG | 1379 |
| 26 | H45_16-41 | ATTGGGCAGCGGCAAACTGTTG TCAG | 684 | CTGACAACAGTTTGCCGCTG CCCAAT | 1380 |
| 26 | H45_17-42 | TTGGGCAGCGGCAAACTGTTGT CAGA | 685 | TCTGACAACAGTTTGCCGCT GCCCAA | 1381 |
| 26 | H45_18-43 | TGGGCAGCGGCAAACTGTTGTC AGAA | 686 | TTCTGACAACAGTTTGCCGC TGCCCA | 1382 |
| 26 | H45_19-44 | GGGCAGCGGCAAACTGTTGTCA GAAC | 687 | GTTCTGACAACAGTTTGCCG CTGCCC | 1383 |
| 26 | H45_20-45 | GGCAGCGGCAAACTGTTGTCAG AACA | 688 | TGTTCTGACAACAGTTTGCC GCTGCC | 1384 |
| 26 | H45_21-46 | GCAGCGGCAAACTGTTGTCAGA ACAT | 689 | ATGTTCTGACAACAGTTTGC CGCTGC | 1385 |
| 26 | H45_22-47 | CAGCGGCAAACTGTTGTCAGAA CATT | 690 | AATGTTCTGACAACAGTTTG CCGCTG | 1386 |
| 26 | H45_23-48 | AGCGGCAAACTGTTGTCAGAAC ATTG | 691 | CAATGTTCTGACAACAGTTT GCCGCT | 1387 |
| 26 | H45_24-49 | GCGGCAAACTGTTGTCAGAACA TTGA | 692 | TCAATGTTCTGACAACAGTT TGCCGC | 1388 |
| 26 | H45_25-50 | CGGCAAACTGTTGTCAGAACAT TGAA | 693 | TTCAATGTTCTGACAACAGT TTGCCG | 1389 |
| 26 | H45_26-51 | GGCAAACTGTTGTCAGAACATT GAAT | 694 | ATTCAATGTTCTGACAACAG TTTGCC | 1390 |
| 26 | H45_27-52 | GCAAACTGTTGTCAGAACATTG AATG | 695 | CATTCAATGTTCTGACAACA GTTTGC | 1391 |
| 26 | H45_28-53 | CAAACTGTTGTCAGAACATTGA ATGC | 696 | GCATTCAATGTTCTGACAAC AGTTTG | 1392 |
| 26 | H45_29-54 | AAACTGTTGTCAGAACATTGAA TGCA | 697 | TGCATTCAATGTTCTGACAA CAGTTT | 1393 |
| 26 | H45_30-55 | AACTGTTGTCAGAACATTGAAT GCAA | 698 | TTGCATTCAATGTTCTGACA ACAGTT | 1394 |

**[Table 4-28]**

| | | | | | |
|---|---|---|---|---|---|
| 26 | H45_31-56 | ACTGTTGTCAGAACATTGAATG CAAC | 699 | GTTGCATTCAATGTTCTGAC AACAGT | 1395 |
| 26 | H45_32-57 | CTGTTGTCAGAACATTGAATGC AACT | 700 | AGTTGCATTCAATGTTCTGA CAACAG | 1396 |
| 26 | H45_33-58 | TGTTGTCAGAACATTGAATGCA ACTG | 701 | CAGTTGCATTCAATGTTCTG ACAACA | 1397 |
| 26 | H45_34-59 | GTTGTCAGAACATTGAATGCAA CTGG | 702 | CCAGTTGCATTCAATGTTCT GACAAC | 1398 |
| 26 | H45_35-60 | TTGTCAGAACATTGAATGCAAC TGGG | 703 | CCCAGTTGCATTCAATGTTC TGACAA | 1399 |
| 26 | H45_36-61 | TGTCAGAACATTGAATGCAACT GGGG | 704 | CCCCAGTTGCATTCAATGTT CTGACA | 1400 |
| 26 | H45_37-62 | GTCAGAACATTGAATGCAACTG GGGA | 705 | TCCCCAGTTGCATTCAATGT TCTGAC | 1401 |
| 26 | H45_38-63 | TCAGAACATTGAATGCAACTGG GGAA | 706 | TTCCCCAGTTGCATTCAATG TTCTGA | 1402 |
| 26 | H45_39-64 | CAGAACATTGAATGCAACTGGG GAAG | 707 | CTTCCCCAGTTGCATTCAAT GTTCTG | 1403 |
| 26 | H45_40-65 | AGAACATTGAATGCAACTGGGG AAGA | 708 | TCTTCCCCAGTTGCATTCAA TGTTCT | 1404 |
| 27 | H45_(-8)-19 | TCTTACAGGAACTCCAGGATGG CATTG | 709 | CAATGCCATCCTGGAGTTCC TGTAAGA | 1405 |
| 27 | H45_(-7)-20 | CTTACAGGAACTCCAGGATGGC ATTGG | 710 | CCAATGCCATCCTGGAGTTC CTGTAAG | 1406 |
| 27 | H45_(-6)-21 | TTACAGGAACTCCAGGATGGCA TTGGG | 711 | CCCAATGCCATCCTGGAGTT CCTGTAA | 1407 |
| 27 | H45_(-5)-22 | TACAGGAACTCCAGGATGGCAT TGGGC | 712 | GCCCAATGCCATCCTGGAGT TCCTGTA | 1408 |
| 27 | H45_(-4)-23 | ACAGGAACTCCAGGATGGCATT GGGCA | 713 | TGCCCAATGCCATCCTGGAG TTCCTGT | 1409 |
| 27 | H45_(-3)-24 | CAGGAACTCCAGGATGGCATTG GGCAG | 714 | CTGCCCAATGCCATCCTGGA GTTCCTG | 1410 |
| 27 | H45_(-2)-25 | AGGAACTCCAGGATGGCATTGG GCAGC | 715 | GCTGCCCAATGCCATCCTGG AGTTCCT | 1411 |
| 27 | H45_(-1)-26 | GGAACTCCAGGATGGCATTGGG CAGCG | 716 | CGCTGCCCAATGCCATCCTG GAGTTCC | 1412 |

**[Table 4-29]**

| | | | | | |
|---|---|---|---|---|---|
| 27 | H45_1-27 | GAACTCCAGGATGGCATTGGGC AGCGG | 717 | CCGCTGCCCAATGCCATCCT GGAGTTC | 1413 |
| 27 | H45_2-28 | AACTCCAGGATGGCATTGGGCA GCGGC | 718 | GCCGCTGCCCAATGCCATCC TGGAGTT | 1414 |
| 27 | H45_3-29 | ACTCCAGGATGGCATTGGGCAG CGGCA | 719 | TGCCGCTGCCCAATGCCATC CTGGAGT | 1415 |
| 27 | H45_4-30 | CTCCAGGATGGCATTGGGCAGC GGCAA | 720 | TTGCCGCTGCCCAATGCCAT CCTGGAG | 1416 |
| 27 | H45_5-31 | TCCAGGATGGCATTGGGCAGCG GCAAA | 721 | TTTGCCGCTGCCCAATGCCA TCCTGGA | 1417 |
| 27 | H45_6-32 | CCAGGATGGCATTGGGCAGCGG CAAAC | 722 | GTTTGCCGCTGCCCAATGCC ATCCTGG | 1418 |
| 27 | H45_7-33 | CAGGATGGCATTGGGCAGCGGC AAACT | 723 | AGTTTGCCGCTGCCCAATGC CATCCTG | 1419 |
| 27 | H45_8-34 | AGGATGGCATTGGGCAGCGGCA AACTG | 724 | CAGTTTGCCGCTGCCCAATG CCATCCT | 1420 |
| 27 | H45_9-35 | GGATGGCATTGGGCAGCGGCAA ACTGT | 725 | ACAGTTTGCCGCTGCCCAAT GCCATCC | 1421 |
| 27 | H45_10-36 | GATGGCATTGGGCAGCGGCAAA CTGTT | 726 | AACAGTTTGCCGCTGCCCAA TGCCATC | 1422 |
| 27 | H45_11-37 | ATGGCATTGGGCAGCGGCAAAC TGTTG | 727 | CAACAGTTTGCCGCTGCCCA ATGCCAT | 1423 |
| 27 | H45_12-38 | TGGCATTGGGCAGCGGCAAACT GTTGT | 728 | ACAACAGTTTGCCGCTGCCC AATGCCA | 1424 |
| 27 | H45_13-39 | GGCATTGGGCAGCGGCAAACTG TTGTC | 729 | GACAACAGTTTGCCGCTGCC CAATGCC | 1425 |
| 27 | H45_14-40 | GCATTGGGCAGCGGCAAACTGT TGTCA | 730 | TGACAACAGTTTGCCGCTGC CCAATGC | 1426 |
| 27 | H45_15-41 | CATTGGGCAGCGGCAAACTGTT GTCAG | 731 | CTGACAACAGTTTGCCGCTG CCCAATG | 1427 |
| 27 | H45_16-42 | ATTGGGCAGCGGCAAACTGTTG TCAGA | 732 | TCTGACAACAGTTTGCCGCT GCCCAAT | 1428 |
| 27 | H45_17-43 | TTGGGCAGCGGCAAACTGTTGT CAGAA | 733 | TTCTGACAACAGTTTGCCGC TGCCCAA | 1429 |
| 27 | H45_18-44 | TGGGCAGCGGCAAACTGTTGTC AGAAC | 734 | GTTCTGACAACAGTTTGCCG CTGCCCA | 1430 |

**[Table 4-30]**

| | | | | | |
|---|---|---|---|---|---|
| 27 | H45_19-45 | GGGCAGCGGCAAACTGTTGTCA GAACA | 735 | TGTTCTGACAACAGTTTGCC GCTGCCC | 1431 |
| 27 | H45_20-46 | GGCAGCGGCAAACTGTTGTCAG AACAT | 736 | ATGTTCTGACAACAGTTTGC CGCTGCC | 1432 |
| 27 | H45_21-47 | GCAGCGGCAAACTGTTGTCAGA ACATT | 737 | AATGTTCTGACAACAGTTTG CCGCTGC | 1433 |
| 27 | H45_22-48 | CAGCGGCAAACTGTTGTCAGAA CATTG | 738 | CAATGTTCTGACAACAGTTT GCCGCTG | 1434 |
| 27 | H45_23-49 | AGCGGCAAACTGTTGTCAGAAC ATTGA | 739 | TCAATGTTCTGACAACAGTT TGCCGCT | 1435 |
| 27 | H45_24-50 | GCGGCAAACTGTTGTCAGAACA TTGAA | 740 | TTCAATGTTCTGACAACAGT TTGCCGC | 1436 |
| 27 | H45_25-51 | CGGCAAACTGTTGTCAGAACAT TGAAT | 741 | ATTCAATGTTCTGACAACAG TTTGCCG | 1437 |
| 27 | H45_26-52 | GGCAAACTGTTGTCAGAACATT GAATG | 742 | CATTCAATGTTCTGACAACA GTTTGCC | 1438 |
| 27 | H45_27-53 | GCAAACTGTTGTCAGAACATTG AATGC | 743 | GCATTCAATGTTCTGACAAC AGTTTGC | 1439 |
| 27 | H45_28-54 | CAAACTGTTGTCAGAACATTGA ATGCA | 744 | TGCATTCAATGTTCTGACAA CAGTTTG | 1440 |
| 27 | H45_29-55 | AAACTGTTGTCAGAACATTGAA TGCAA | 745 | TTGCATTCAATGTTCTGACA ACAGTTT | 1441 |
| 27 | H45_30-56 | AACTGTTGTCAGAACATTGAAT GCAAC | 746 | GTTGCATTCAATGTTCTGAC AACAGTT | 1442 |
| 27 | H45_31-57 | ACTGTTGTCAGAACATTGAATG CAACT | 747 | AGTTGCATTCAATGTTCTGA CAACAGT | 1443 |
| 27 | H45_32-58 | CTGTTGTCAGAACATTGAATGC AACTG | 748 | CAGTTGCATTCAATGTTCTG ACAACAG | 1444 |
| 27 | H45_33-59 | TGTTGTCAGAACATTGAATGCA ACTGG | 749 | CCAGTTGCATTCAATGTTCT GACAACA | 1445 |
| 27 | H45_34-60 | GTTGTCAGAACATTGAATGCAA CTGGG | 750 | CCCAGTTGCATTCAATGTTC TGACAAC | 1446 |
| 27 | H45_35-61 | TTGTCAGAACATTGAATGCAAC TGGGG | 751 | CCCCAGTTGCATTCAATGTT CTGACAA | 1447 |
| 27 | H45_36-62 | TGTCAGAACATTGAATGCAACT GGGGA | 752 | TCCCCAGTTGCATTCAATGT TCTGACA | 1448 |

**[Table 4-31]**

| | | | | | |
|---|---|---|---|---|---|
| 27 | H45_37-63 | GTCAGAACATTGAATGCAACTG GGGAA | 753 | TTCCCCAGTTGCATTCAATG TTCTGAC | 1449 |
| 27 | H45_38-64 | TCAGAACATTGAATGCAACTGG GGAAG | 754 | CTTCCCCAGTTGCATTCAAT GTTCTGA | 1450 |
| 27 | H45_39-65 | CAGAACATTGAATGCAACTGGG GAAGA | 755 | TCTTCCCCAGTTGCATTCAA TGTTCTG | 1451 |
| 27 | H45_40-66 | AGAACATTGAATGCAACTGGGG AAGAA | 756 | TTCTTCCCCAGTTGCATTCA ATGTTCT | 1452 |
| 28 | H45_(-9)-19 | ATCTTACAGGAACTCCAGGATG GCATTG | 757 | CAATGCCATCCTGGAGTTCC TGTAAGAT | 1453 |
| 28 | H45_(-8)-20 | TCTTACAGGAACTCCAGGATGG CATTGG | 758 | CCAATGCCATCCTGGAGTTC CTGTAAGA | 1454 |
| 28 | H45_(-7) -21 | CTTACAGGAACTCCAGGATGGC ATTGGG | 759 | CCCAATGCCATCCTGGAGTT CCTGTAAG | 1455 |
| 28 | H45_(-6)-22 | TTACAGGAACTCCAGGATGGCA TTGGGC | 760 | GCCCAATGCCATCCTGGAGT TCCTGTAA | 1456 |
| 28 | H45_(-5)-23 | TACAGGAACTCCAGGATGGCAT TGGGCA | 761 | TGCCCAATGCCATCCTGGAG TTCCTGTA | 1457 |
| 28 | H45_(-4)-24 | ACAGGAACTCCAGGATGGCATT GGGCAG | 762 | CTGCCCAATGCCATCCTGGA GTTCCTGT | 1458 |
| 28 | H45_(-3) -25 | CAGGAACTCCAGGATGGCATTG GGCAGC | 763 | GCTGCCCAATGCCATCCTGG AGTTCCTG | 1459 |
| 28 | H45_(-2)-26 | AGGAACTCCAGGATGGCATTGG GCAGCG | 764 | CGCTGCCCAATGCCATCCTG GAGTTCCT | 1460 |
| 28 | H45_(-1)-27 | GGAACTCCAGGATGGCATTGGG CAGCGG | 765 | CCGCTGCCCAATGCCATCCT GGAGTTCC | 1461 |
| 28 | H45_1-28 | GAACTCCAGGATGGCATTGGGC AGCGGC | 766 | GCCGCTGCCCAATGCCATCC TGGAGTTC | 1462 |
| 28 | H45_2-29 | AACTCCAGGATGGCATTGGGCA GCGGCA | 767 | TGCCGCTGCCCAATGCCATC CTGGAGTT | 1463 |
| 28 | H45_3-30 | ACTCCAGGATGGCATTGGGCAG CGGCAA | 768 | TTGCCGCTGCCCAATGCCAT CCTGGAGT | 1464 |
| 28 | H45_4-31 | CTCCAGGATGGCATTGGGCAGC GGCAAA | 769 | TTTGCCGCTGCCCAATGCCA TCCTGGAG | 1465 |
| 28 | H45_5-32 | TCCAGGATGGCATTGGGCAGCG GCAAAC | 770 | GTTTGCCGCTGCCCAATGCC ATCCTGGA | 1466 |

**[Table 4-32]**

| | | | | | |
|---|---|---|---|---|---|
| 28 | H45_6-33 | CCAGGATGGCATTGGGCAGCGG CAAACT | 771 | AGTTTGCCGCTGCCCAATGC CATCCTGG | 1467 |
| 28 | H45_7-34 | CAGGATGGCATTGGGCAGCGGC AAACTG | 772 | CAGTTTGCCGCTGCCCAATG CCATCCTG | 1468 |
| 28 | H45_8-35 | AGGATGGCATTGGGCAGCGGCA AACTGT | 773 | ACAGTTTGCCGCTGCCCAAT GCCATCCT | 1469 |
| 28 | H45_9-36 | GGATGGCATTGGGCAGCGGCAA ACTGTT | 774 | AACAGTTTGCCGCTGCCCAA TGCCATCC | 1470 |
| 28 | H45_10-37 | GATGGCATTGGGCAGCGGCAAA CTGTTG | 775 | CAACAGTTTGCCGCTGCCCA ATGCCATC | 1471 |
| 28 | H45_11-38 | ATGGCATTGGGCAGCGGCAAAC TGTTGT | 776 | ACAACAGTTTGCCGCTGCCC AATGCCAT | 1472 |
| 28 | H45_12-39 | TGGCATTGGGCAGCGGCAAACT GTTGTC | 777 | GACAACAGTTTGCCGCTGCC CAATGCCA | 1473 |
| 28 | H45_13-40 | GGCATTGGGCAGCGGCAAACTG TTGTCA | 778 | TGACAACAGTTTGCCGCTGC CCAATGCC | 1474 |
| 28 | H45_14-41 | GCATTGGGCAGCGGCAAACTGT TGTCAG | 779 | CTGACAACAGTTTGCCGCTG CCCAATGC | 1475 |
| 28 | H45_15-42 | CATTGGGCAGCGGCAAACTGTT GTCAGA | 780 | TCTGACAACAGTTTGCCGCT GCCCAATG | 1476 |
| 28 | H45_16-43 | ATTGGGCAGCGGCAAACTGTTG TCAGAA | 781 | TTCTGACAACAGTTTGCCGC TGCCCAAT | 1477 |
| 28 | H45_17-44 | TTGGGCAGCGGCAAACTGTTGT CAGAAC | 782 | GTTCTGACAACAGTTTGCCG CTGCCCAA | 1478 |
| 28 | H45_18-45 | TGGGCAGCGGCAAACTGTTGTC AGAACA | 783 | TGTTCTGACAACAGTTTGCC GCTGCCCA | 1479 |
| 28 | H45_19-46 | GGGCAGCGGCAAACTGTTGTCA GAACAT | 784 | ATGTTCTGACAACAGTTTGC CGCTGCCC | 1480 |
| 28 | H45_20-47 | GGCAGCGGCAAACTGTTGTCAG AACATT | 785 | AATGTTCTGACAACAGTTTG CCGCTGCC | 1481 |
| 28 | H45_21-48 | GCAGCGGCAAACTGTTGTCAGA ACATTG | 786 | CAATGTTCTGACAACAGTTT GCCGCTGC | 1482 |
| 28 | H45_22-49 | CAGCGGCAAACTGTTGTCAGAA CATTGA | 787 | TCAATGTTCTGACAACAGTT TGCCGCTG | 1483 |
| 28 | H45_23-50 | AGCGGCAAACTGTTGTCAGAAC ATTGAA | 788 | TTCAATGTTCTGACAACAGT TTGCCGCT | 1484 |

**[Table 4-33]**

| | | | | | |
|---|---|---|---|---|---|
| 28 | H45_24-51 | GCGGCAAACTGTTGTCAGAACA TTGAAT | 789 | ATTCAATGTTCTGACAACAG TTTGCCGC | 1485 |
| 28 | H45_25-52 | CGGCAAACTGTTGTCAGAACAT TGAATG | 790 | CATTCAATGTTCTGACAACA GTTTGCCG | 1486 |
| 28 | H45_26-53 | GGCAAACTGTTGTCAGAACATT GAATGC | 791 | GCATTCAATGTTCTGACAAC AGTTTGCC | 1487 |
| 28 | H45_27-54 | GCAAACTGTTGTCAGAACATTG AATGCA | 792 | TGCATTCAATGTTCTGACAA CAGTTTGC | 1488 |
| 28 | H45_28-55 | CAAACTGTTGTCAGAACATTGA ATGCAA | 793 | TTGCATTCAATGTTCTGACA ACAGTTTG | 1489 |
| 28 | H45_29-56 | AAACTGTTGTCAGAACATTGAA TGCAAC | 794 | GTTGCATTCAATGTTCTGAC AACAGTTT | 1490 |
| 28 | H45_30-57 | AACTGTTGTCAGAACATTGAAT GCAACT | 795 | AGTTGCATTCAATGTTCTGA CAACAGTT | 1491 |
| 28 | H45_31-58 | ACTGTTGTCAGAACATTGAATG CAACTG | 796 | CAGTTGCATTCAATGTTCTG ACAACAGT | 1492 |
| 28 | H45_32-59 | CTGTTGTCAGAACATTGAATGC AACTGG | 797 | CCAGTTGCATTCAATGTTCT GACAACAG | 1493 |
| 28 | H45_33-60 | TGTTGTCAGAACATTGAATGCA ACTGGG | 798 | CCCAGTTGCATTCAATGTTC TGACAACA | 1494 |
| 28 | H45_34-61 | GTTGTCAGAACATTGAATGCAA CTGGGG | 799 | CCCCAGTTGCATTCAATGTT CTGACAAC | 1495 |
| 28 | H45_35-62 | TTGTCAGAACATTGAATGCAAC TGGGGA | 800 | TCCCCAGTTGCATTCAATGT TCTGACAA | 1496 |
| 28 | H45_36-63 | TGTCAGAACATTGAATGCAACT GGGGAA | 801 | TTCCCCAGTTGCATTCAATG TTCTGACA | 1497 |
| 28 | H45_37-64 | GTCAGAACATTGAATGCAACTG GGGAAG | 802 | CTTCCCCAGTTGCATTCAAT GTTCTGAC | 1498 |
| 28 | H45_38-65 | TCAGAACATTGAATGCAACTGG GGAAGA | 803 | TCTTCCCCAGTTGCATTCAA TGTTCTGA | 1499 |
| 28 | H45_39-66 | CAGAACATTGAATGCAACTGGG GAAGAA | 804 | TTCTTCCCCAGTTGCATTCA ATGTTCTG | 1500 |
| 28 | H45_40-67 | AGAACATTGAATGCAACTGGGG AAGAAA | 805 | TTTCTTCCCCAGTTGCATTC AATGTTCT | 1501 |
| 29 | H45_(-10)-19 | TATCTTACAGGAACTCCAGGAT GGCATTG | 806 | CAATGCCATCCTGGAGTTCC TGTAAGATA | 1502 |

**[Table 4-34]**

| | | | | | |
|---|---|---|---|---|---|
| 29 | H45_(-9)-20 | ATCTTACAGGAACTCCAGGATG GCATTGG | 807 | CCAATGCCATCCTGGAGTTC CTGTAAGAT | 1503 |
| 29 | H45_(-8)-21 | TCTTACAGGAACTCCAGGATGG CATTGGG | 808 | CCCAATGCCATCCTGGAGTT CCTGTAAGA | 1504 |
| 29 | H45_(-7)-22 | CTTACAGGAACTCCAGGATGGC ATTGGGC | 809 | GCCCAATGCCATCCTGGAGT TCCTGTAAG | 1505 |
| 29 | H45_(-6)-23 | TTACAGGAACTCCAGGATGGCA TTGGGCA | 810 | TGCCCAATGCCATCCTGGAG TTCCTGTAA | 1506 |
| 29 | H45_(-5)-24 | TACAGGAACTCCAGGATGGCAT TGGGCAG | 811 | CTGCCCAATGCCATCCTGGA GTTCCTGTA | 1507 |
| 29 | H45_(-4)-25 | ACAGGAACTCCAGGATGGCATT GGGCAGC | 812 | GCTGCCCAATGCCATCCTGG AGTTCCTGT | 1508 |
| 29 | H45_(-3)-26 | CAGGAACTCCAGGATGGCATTG GGCAGCG | 813 | CGCTGCCCAATGCCATCCTG GAGTTCCTG | 1509 |
| 29 | H45_(-2)-27 | AGGAACTCCAGGATGGCATTGG GCAGCGG | 814 | CCGCTGCCCAATGCCATCCT GGAGTTCCT | 1510 |
| 29 | H45_(-1)-28 | GGAACTCCAGGATGGCATTGGG CAGCGGC | 815 | GCCGCTGCCCAATGCCATCC TGGAGTTCC | 1511 |
| 29 | H45_1-29 | GAACTCCAGGATGGCATTGGGC AGCGGCA | 816 | TGCCGCTGCCCAATGCCATC CTGGAGTTC | 1512 |
| 29 | H45_2-30 | AACTCCAGGATGGCATTGGGCA GCGGCAA | 817 | TTGCCGCTGCCCAATGCCAT CCTGGAGTT | 1513 |
| 29 | H45_3-31 | ACTCCAGGATGGCATTGGGCAG CGGCAAA | 818 | TTTGCCGCTGCCCAATGCCA TCCTGGAGT | 1514 |
| 29 | H45_4-32 | CTCCAGGATGGCATTGGGCAGC GGCAAAC | 819 | GTTTGCCGCTGCCCAATGCC ATCCTGGAG | 1515 |
| 29 | H45_5-33 | TCCAGGATGGCATTGGGCAGCG GCAAACT | 820 | AGTTTGCCGCTGCCCAATGC CATCCTGGA | 1516 |
| 29 | H45_6-34 | CCAGGATGGCATTGGGCAGCGG CAAACTG | 821 | CAGTTTGCCGCTGCCCAATG CCATCCTGG | 1517 |
| 29 | H45_7-35 | CAGGATGGCATTGGGCAGCGGC AAACTGT | 822 | ACAGTTTGCCGCTGCCCAAT GCCATCCTG | 1518 |
| 29 | H45_8-36 | AGGATGGCATTGGGCAGCGGCA AACTGTT | 823 | AACAGTTTGCCGCTGCCCAA TGCCATCCT | 1519 |
| 29 | H45_9-37 | GGATGGCATTGGGCAGCGGCAA ACTGTTG | 824 | CAACAGTTTGCCGCTGCCCA ATGCCATCC | 1520 |

**[Table 4-35]**

| | | | | | |
|---|---|---|---|---|---|
| 29 | H45_10-38 | GATGGCATTGGGCAGCGGCAAA CTGTTGT | 825 | ACAACAGTTTGCCGCTGCCC AATGCCATC | 1521 |
| 29 | H45_11-39 | ATGGCATTGGGCAGCGGCAAAC TGTTGTC | 826 | GACAACAGTTTGCCGCTGCC CAATGCCAT | 1522 |
| 29 | H45_12-40 | TGGCATTGGGCAGCGGCAAACT GTTGTCA | 827 | TGACAACAGTTTGCCGCTGC CCAATGCCA | 1523 |
| 29 | H45_13-41 | GGCATTGGGCAGCGGCAAACTG TTGTCAG | 828 | CTGACAACAGTTTGCCGCTG CCCAATGCC | 1524 |
| 29 | H45_14-42 | GCATTGGGCAGCGGCAAACTGT TGTCAGA | 829 | TCTGACAACAGTTTGCCGCT GCCCAATGC | 1525 |
| 29 | H45_15-43 | CATTGGGCAGCGGCAAACTGTT GTCAGAA | 830 | TTCTGACAACAGTTTGCCGC TGCCCAATG | 1526 |
| 29 | H45_16-44 | ATTGGGCAGCGGCAAACTGTTG TCAGAAC | 831 | GTTCTGACAACAGTTTGCCG CTGCCCAAT | 1527 |
| 29 | H45_17-45 | TTGGGCAGCGGCAAACTGTTGT CAGAACA | 832 | TGTTCTGACAACAGTTTGCC GCTGCCCAA | 1528 |
| 29 | H45_18-46 | TGGGCAGCGGCAAACTGTTGTC AGAACAT | 833 | ATGTTCTGACAACAGTTTGC CGCTGCCCA | 1529 |
| 29 | H45_19-47 | GGGCAGCGGCAAACTGTTGTCA GAACATT | 834 | AATGTTCTGACAACAGTTTG CCGCTGCCC | 1530 |
| 29 | H45_20-48 | GGCAGCGGCAAACTGTTGTCAG AACATTG | 835 | CAATGTTCTGACAACAGTTT GCCGCTGCC | 1531 |
| 29 | H45_21-49 | GCAGCGGCAAACTGTTGTCAGA ACATTGA | 836 | TCAATGTTCTGACAACAGTT TGCCGCTGC | 1532 |
| 29 | H45_22-50 | CAGCGGCAAACTGTTGTCAGAA CATTGAA | 837 | TTCAATGTTCTGACAACAGT TTGCCGCTG | 1533 |
| 29 | H45_23-51 | AGCGGCAAACTGTTGTCAGAAC ATTGAAT | 838 | ATTCAATGTTCTGACAACAG TTTGCCGCT | 1534 |
| 29 | H45_24-52 | GCGGCAAACTGTTGTCAGAACA TTGAATG | 839 | CATTCAATGTTCTGACAACA GTTTGCCGC | 1535 |
| 29 | H45_25-53 | CGGCAAACTGTTGTCAGAACAT TGAATGC | 840 | GCATTCAATGTTCTGACAAC AGTTTGCCG | 1536 |
| 29 | H45_26-54 | GGCAAACTGTTGTCAGAACATT GAATGCA | 841 | TGCATTCAATGTTCTGACAA CAGTTTGCC | 1537 |
| 29 | H45_27-55 | GCAAACTGTTGTCAGAACATTG AATGCAA | 842 | TTGCATTCAATGTTCTGACA ACAGTTTGC | 1538 |

**[Table 4-36]**

| | | | | | |
|---|---|---|---|---|---|
| 29 | H45_28-56 | CAAACTGTTGTCAGAACATTGA ATGCAAC | 843 | GTTGCATTCAATGTTCTGAC AACAGTTTG | 1539 |
| 29 | H45_29-57 | AAACTGTTGTCAGAACATTGAA TGCAACT | 844 | AGTTGCATTCAATGTTCTGA CAACAGTTT | 1540 |
| 29 | H45_30-58 | AACTGTTGTCAGAACATTGAAT GCAACTG | 845 | CAGTTGCATTCAATGTTCTG ACAACAGTT | 1541 |
| 29 | H45_31-59 | ACTGTTGTCAGAACATTGAATG CAACTGG | 846 | CCAGTTGCATTCAATGTTCT GACAACAGT | 1542 |
| 29 | H45_32-60 | CTGTTGTCAGAACATTGAATGC AACTGGG | 847 | CCCAGTTGCATTCAATGTTC TGACAACAG | 1543 |
| 29 | H45_33-61 | TGTTGTCAGAACATTGAATGCA ACTGGGG | 848 | CCCCAGTTGCATTCAATGTT CTGACAACA | 1544 |
| 29 | H45_34-62 | GTTGTCAGAACATTGAATGCAA CTGGGGA | 849 | TCCCCAGTTGCATTCAATGT TCTGACAAC | 1545 |
| 29 | H45_35-63 | TTGTCAGAACATTGAATGCAAC TGGGGAA | 850 | TTCCCCAGTTGCATTCAATG TTCTGACAA | 1546 |
| 29 | H45_36-64 | TGTCAGAACATTGAATGCAACT GGGGAAG | 851 | CTTCCCCAGTTGCATTCAAT GTTCTGACA | 1547 |
| 29 | H45_37-65 | GTCAGAACATTGAATGCAACTG GGGAAGA | 852 | TCTTCCCCAGTTGCATTCAA TGTTCTGAC | 1548 |
| 29 | H45_38-66 | TCAGAACATTGAATGCAACTGG GGAAGAA | 853 | TTCTTCCCCAGTTGCATTCA ATGTTCTGA | 1549 |
| 29 | H45_39-67 | CAGAACATTGAATGCAACTGGG GAAGAAA | 854 | TTTCTTCCCCAGTTGCATTC AATGTTCTG | 1550 |
| 29 | H45_40-68 | AGAACATTGAATGCAACTGGGG AAGAAAT | 855 | ATTTCTTCCCCAGTTGCATT CAATGTTCT | 1551 |
| 30 | H45_(-11)-19 | GTATCTTACAGGAACTCCAGGA TGGCATTG | 856 | CAATGCCATCCTGGAGTTCC TGTAAGATAC | 1552 |
| 30 | H45_(-10)-20 | TATCTTACAGGAACTCCAGGAT GGCATTGG | 857 | CCAATGCCATCCTGGAGTTC CTGTAAGATA | 1553 |
| 30 | H45_(-9) -21 | ATCTTACAGGAACTCCAGGATG GCATTGGG | 858 | CCCAATGCCATCCTGGAGTT CCTGTAAGAT | 1554 |
| 30 | H45_(-8)-22 | TCTTACAGGAACTCCAGGATGG CATTGGGC | 859 | GCCCAATGCCATCCTGGAGT TCCTGTAAGA | 1555 |
| 30 | H45_(-7)-23 | CTTACAGGAACTCCAGGATGGC ATTGGGCA | 860 | TGCCCAATGCCATCCTGGAG TTCCTGTAAG | 1556 |

**[Table 4-37]**

| | | | | | |
|---|---|---|---|---|---|
| 30 | H45_(-6)-24 | TTACAGGAACTCCAGGATGGCA TTGGGCAG | 861 | CTGCCCAATGCCATCCTGGA GTTCCTGTAA | 1557 |
| 30 | H45_(-5)-25 | TACAGGAACTCCAGGATGGCAT TGGGCAGC | 862 | GCTGCCCAATGCCATCCTGG AGTTCCTGTA | 1558 |
| 30 | H15_(-4)-26 | ACAGGAACTCCAGGATGGCATT GGGCAGCG | 863 | CGCTGCCCAATGCCATCCTG GAGTTCCTGT | 1559 |
| 30 | H45_(-3)-27 | CAGGAACTCCAGGATGGCATTG GGCAGCGG | 864 | CCGCTGCCCAATGCCATCCT GGAGTTCCTG | 1560 |
| 30 | H45_(-2)-28 | AGGAACTCCAGGATGGCATTGG GCAGCGGC | 865 | GCCGCTGCCCAATGCCATCC TGGAGTTCCT | 1561 |
| 30 | H45_(-1)-29 | GGAACTCCAGGATGGCATTGGG CAGCGGCA | 866 | TGCCGCTGCCCAATGCCATC CTGGAGTTCC | 1562 |
| 30 | H45_1-30 | GAACTCCAGGATGGCATTGGGC AGCGGCAA | 867 | TTGCCGCTGCCCAATGCCAT CCTGGAGTTC | 1563 |
| 30 | H45_2-31 | AACTCCAGGATGGCATTGGGCA GCGGCAAA | 868 | TTTGCCGCTGCCCAATGCCA TCCTGGAGTT | 1564 |
| 30 | H45_3-32 | ACTCCAGGATGGCATTGGGCAG CGGCAAAC | 869 | GTTTGCCGCTGCCCAATGCC ATCCTGGAGT | 1565 |
| 30 | H45_4-33 | CTCCAGGATGGCATTGGGCAGC GGCAAACT | 870 | AGTTTGCCGCTGCCCAATGC CATCCTGGAG | 1566 |
| 30 | H45_5-34 | TCCAGGATGGCATTGGGCAGCG GCAAACTG | 871 | CAGTTTGCCGCTGCCCAATG CCATCCTGGA | 1567 |
| 30 | H45_6-35 | CCAGGATGGCATTGGGCAGCGG CAAACTGT | 872 | ACAGTTTGCCGCTGCCCAAT GCCATCCTGG | 1568 |
| 30 | H45_7-36 | CAGGATGGCATTGGGCAGCGGC AAACTGTT | 873 | AACAGTTTGCCGCTGCCCAA TGCCATCCTG | 1569 |
| 30 | H45_8-37 | AGGATGGCATTGGGCAGCGGCA AACTGTTG | 874 | CAACAGTTTGCCGCTGCCCA ATGCCATCCT | 1570 |
| 30 | H45_9-38 | GGATGGCATTGGGCAGCGGCAA ACTGTTGT | 875 | ACAACAGTTTGCCGCTGCCC AATGCCATCC | 1571 |
| 30 | H45_10-39 | GATGGCATTGGGCAGCGGCAAA CTGTTGTC | 876 | GACAACAGTTTGCCGCTGCC CAATGCCATC | 1572 |
| 30 | H45_11-40 | ATGGCATTGGGCAGCGGCAAAC TGTTGTCA | 877 | TGACAACAGTTTGCCGCTGC CCAATGCCAT | 1573 |
| 30 | H45_12-41 | TGGCATTGGGCAGCGGCAAACT GTTGTCAG | 878 | CTGACAACAGTTTGCCGCTG CCCAATGCCA | 1574 |

**[Table 4-38]**

| | | | | | |
|---|---|---|---|---|---|
| 30 | H45_13-42 | GGCATTGGGCAGCGGCAAACTG TTGTCAGA | 879 | TCTGACAACAGTTTGCCGCT GCCCAATGCC | 1575 |
| 30 | H45_14-43 | GCATTGGGCAGCGGCAAACTGT TGTCAGAA | 880 | TTCTGACAACAGTTTGCCGC TGCCCAATGC | 1576 |
| 30 | H45_15-44 | CATTGGGCAGCGGCAAACTGTT GTCAGAAC | 881 | GTTCTGACAACAGTTTGCCG CTGCCCAATG | 1577 |
| 30 | H45_16-45 | ATTGGGCAGCGGCAAACTGTTG TCAGAACA | 882 | TGTTCTGACAACAGTTTGCC GCTGCCCAAT | 1578 |
| 30 | H45_17-46 | TTGGGCAGCGGCAAACTGTTGT CAGAACAT | 883 | ATGTTCTGACAACAGTTTGC CGCTGCCCAA | 1579 |
| 30 | H45_18-47 | TGGGCAGCGGCAAACTGTTGTC AGAACATT | 884 | AATGTTCTGACAACAGTTTG CCGCTGCCCA | 1580 |
| 30 | H45_19-48 | GGGCAGCGGCAAACTGTTGTCA GAACATTG | 885 | CAATGTTCTGACAACAGTTT GCCGCTGCCC | 1581 |
| 30 | H45_20-49 | GGCAGCGGCAAACTGTTGTCAG AACATTGA | 886 | TCAATGTTCTGACAACAGTT TGCCGCTGCC | 1582 |
| 30 | H45_21-50 | GCAGCGGCAAACTGTTGTCAGA ACATTGAA | 887 | TTCAATGTTCTGACAACAGT TTGCCGCTGC | 1583 |
| 30 | H45_22-51 | CAGCGGCAAACTGTTGTCAGAA CATTGAAT | 888 | ATTCAATGTTCTGACAACAG TTTGCCGCTG | 1584 |
| 30 | H45_23-52 | AGCGGCAAACTGTTGTCAGAAC ATTGAATG | 889 | CATTCAATGTTCTGACAACA GTTTGCCGCT | 1585 |
| 30 | H45_24-53 | GCGGCAAACTGTTGTCAGAACA TTGAATGC | 890 | GCATTCAATGTTCTGACAAC AGTTTGCCGC | 1586 |
| 30 | H45_25-54 | CGGCAAACTGTTGTCAGAACAT TGAATGCA | 891 | TGCATTCAATGTTCTGACAA CAGTTTGCCG | 1587 |
| 30 | H45_26-55 | GGCAAACTGTTGTCAGAACATT GAATGCAA | 892 | TTGCATTCAATGTTCTGACA ACAGTTTGCC | 1588 |
| 30 | H45_27-56 | GCAAACTGTTGTCAGAACATTG AATGCAAC | 893 | GTTGCATTCAATGTTCTGAC AACAGTTTGC | 1589 |
| 30 | H45_28-57 | CAAACTGTTGTCAGAACATTGA ATGCAACT | 894 | AGTTGCATTCAATGTTCTGA CAACAGTTTG | 1590 |
| 30 | H45_29-58 | AAACTGTTGTCAGAACATTGAA TGCAACTG | 895 | CAGTTGCATTCAATGTTCTG ACAACAGTTT | 1591 |
| 30 | H45_30-59 | AACTGTTGTCAGAACATTGAAT GCAACTGG | 896 | CCAGTTGCATTCAATGTTCT GACAACAGTT | 1592 |

**[Table 4-39]**

| | | | | | |
|---|---|---|---|---|---|
| 30 | H45_31-60 | ACTGTTGTCAGAACATTGAATG CAACTGGG | 897 | CCCAGTTGCATTCAATGTTC TGACAACAGT | 1593 |
| 30 | H45_32-61 | CTGTTGTCAGAACATTGAATGC AACTGGGG | 898 | CCCCAGTTGCATTCAATGTT CTGACAACAG | 1594 |
| 30 | H45_33-62 | TGTTGTCAGAACATTGAATGCA ACTGGGGA | 899 | TCCCCAGTTGCATTCAATGT TCTGACAACA | 1595 |
| 30 | H45_34-63 | GTTGTCAGAACATTGAATGCAA CTGGGGAA | 900 | TTCCCCAGTTGCATTCAATG TTCTGACAAC | 1596 |
| 30 | H45_35-64 | TTGTCAGAACATTGAATGCAAC TGGGGAAG | 901 | CTTCCCCAGTTGCATTCAAT GTTCTGACAA | 1597 |
| 30 | H45_36-65 | TGTCAGAACATTGAATGCAACT GGGGAAGA | 902 | TCTTCCCCAGTTGCATTCAA TGTTCTGACA | 1598 |
| 30 | H45_37-66 | GTCAGAACATTGAATGCAACTG GGGAAGAA | 903 | TTCTTCCCCAGTTGCATTCA ATGTTCTGAC | 1599 |
| 30 | H45_38-67 | TCAGAACATTGAATGCAACTGG GGAAGAAA | 904 | TTTCTTCCCCAGTTGCATTC AATGTTCTGA | 1600 |
| 30 | H45_39-68 | CAGAACATTGAATGCAACTGGG GAAGAAAT | 905 | ATTTCTTCCCCAGTTGCATT CAATGTTCTG | 1601 |
| 30 | H45_40-69 | AGAACATTGAATGCAACTGGGG AAGAAATA | 906 | TATTTCTTCCCCAGTTGCAT TCAATGTTCT | 1602 |

In one embodiment, the first unit oligomer comprises a base sequence complementary to:
(a) any one base sequence selected from the group consisting of SEQ ID NOs: 211 to 906;
(b) a base sequence that hybridizes under stringent conditions to a base sequence complementary to any one base sequence selected from the group consisting of SEQ ID NOs: 211 to 906;
(c) a base sequence that has at least 85% identity with any one base sequence selected from the group consisting of SEQ ID NOs: 211 to 906, and has a length within ±15% of the length of the any one base sequence selected; or
(d) a partial base sequence of any one base sequence selected from the group consisting of the base sequences (a), (b), and (c) .

Herein, the base sequence (c) is a mutant type of the base sequence (a), and examples of such a mutant type also include
(c-1) a base sequence that has at least 85% identity with any one base sequence selected from the group consisting of SEQ ID Nos: 211 to 906, and has a length within ±15% of the length of the any one base sequence selected,
(c-2) a base sequence that has at least 86% identity with any one base sequence selected from the group consisting of SEQ ID NOs: 211 to 906, and has a length within ±14% of the length of the any one base sequence selected,
(c-3) a base sequence that has at least 87% identity with any one base sequence selected from the group consisting of SEQ ID NOs: 211 to 906, and has a length within ±13% of the length of the any one base sequence selected,
(c-4) a base sequence that has at least 88% identity with any one base sequence selected from the group consisting of SEQ ID NOs: 211 to 906, and has a length within ±12% of the length of the any one base sequence selected,
(c-5) a base sequence that has at least 89% identity with any one base sequence selected from the group consisting of SEQ ID NOs: 211 to 906, and has a length within ±11% of the length of the any one base sequence selected,
(c-6) a base sequence that has at least 90% identity with any one base sequence selected from the group consisting of SEQ ID NOs: 211 to 906, and has a length within ±10% of the length of the any one base sequence selected,
(c-7) a base sequence that has at least 91% identity with any one base sequence selected from the group consisting of SEQ ID NOs: 211 to 906, and has a length within ±9% of the length of the any one base sequence selected,
(c-8) a base sequence that has at least 92% identity with any one base sequence selected from the group consisting of SEQ ID NOs: 211 to 906, and has a length within ±8% of the length of the any one base sequence selected,
(c-9) a base sequence that has at least 93% identity with any one base sequence selected from the group consisting of SEQ ID NOs: 211 to 906, and has a length within ±7% of the length of the any one base sequence selected,
(c-10) a base sequence that has at least 94% identity with any one base sequence selected from the group consisting of SEQ ID NOs: 211 to 906, and has a length within ±6% of the length of the any one base sequence selected,
(c-11) a base sequence that has at least 95% identity with any one base sequence selected from the group consisting of SEQ ID NOs: 211 to 906, and has a length within ±5% of the length of the any one base sequence selected,
(c-12) a base sequence that has at least 96% identity with any one base sequence selected from the group consisting of SEQ ID NOs: 211 to 906, and has a length within ±4% of the length of the any one base sequence selected,
(c-13) a base sequence that has at least 97% identity with any one base sequence selected from the group consisting of SEQ ID NOs: 211 to 906, and has a length within ±3% of the length of the any one base sequence selected,
(c-14) a base sequence that has at least 98% identity with any one base sequence selected from the group consisting of SEQ ID NOs: 211 to 906, and has a length within ±2% of the length of the any one base sequence selected,
(c-15) a base sequence that has at least 99% identity with any one base sequence selected from the group consisting of SEQ ID NOs: 211 to 906, and has a length within ±1% of the length of the any one base sequence selected, and
(c-16) a base sequence that has at least 99.5% identity with any one base sequence selected from the group consisting of SEQ ID NOs: 211 to 906, and has a length within ±0.5% of the length of the any one base sequence selected.

In one embodiment, the first unit oligomer comprises or consists of:
(a) any one base sequence selected from the group consisting of SEQ ID NOs: 907 to 1602; or
(b) a base sequence that has at least 85% identity with any one base sequence selected from the group consisting of SEQ ID NOs: 907 to 1602, and has a length within ±15% of the length of the any one base sequence selected.

Herein, the base sequence (b) is a mutant type of the base sequence (a), and examples of such a mutant type also include:
(b-1) a base sequence that has at least 85% identity with any one base sequence selected from the group consisting of SEQ ID NOs: 907 to 1602, and has a length within ±15% of the length of the any one base sequence selected,
(b-2) a base sequence that has at least 86% identity with any one base sequence selected from the group consisting of SEQ ID NOs: 907 to 1602, and has a length within ±14% of the length of the any one base sequence selected,
(b-3) a base sequence that has at least 87% identity with any one base sequence selected from the group consisting of SEQ ID NOs: 907 to 1602, and has a length within ±13% of the length of the any one base sequence selected,
(b-4) a base sequence that has at least 88% identity with any one base sequence selected from the group consisting of SEQ ID NOs: 907 to 1602, and has a length within ±12% of the length of the any one base sequence selected,
(b-5) a base sequence that has at least 89% identity with any one base sequence selected from the group consisting of SEQ ID NOs: 907 to 1602, and has a length within ±11% of the length of the any one base sequence selected,
(b-6) a base sequence that has at least 90% identity with any one base sequence selected from the group consisting of SEQ ID NOs: 907 to 1602, and has a length within ±10% of the length of the any one base sequence selected,
(b-7) a base sequence that has at least 91% identity with any one base sequence selected from the group consisting of SEQ ID NOs: 907 to 1602, and has a length within ±9% of the length of the any one base sequence selected,
(b-8) a base sequence that has at least 92% identity with any one base sequence selected from the group consisting of SEQ ID NOs: 907 to 1602, and has a length within ±8% of the length of the any one base sequence selected,
(b-9) a base sequence that has at least 93% identity with any one base sequence selected from the group consisting of SEQ ID NOs: 907 to 1602, and has a length within ±7% of the length of the any one base sequence selected,
(b-10) a base sequence that has at least 94% identity with any one base sequence selected from the group consisting of SEQ ID NOs: 907 to 1602, and has a length within ±6% of the length of the any one base sequence selected,
(b-11) a base sequence that has at least 95% identity with any one base sequence selected from the group consisting of SEQ ID NOs: 907 to 1602, and has a length within ±5% of the length of the any one base sequence selected,
(b-12) a base sequence that has at least 96% identity with any one base sequence selected from the group consisting of SEQ ID NOs: 907 to 1602, and has a length within ±4% of the length of the any one base sequence selected,
(b-13) a base sequence that has at least 97% identity with any one base sequence selected from the group consisting of SEQ ID NOs: 907 to 1602, and has a length within ±3% of the length of the any one base sequence selected,
(b-14) a base sequence that has at least 98% identity with any one base sequence selected from the group consisting of SEQ ID NOs: 907 to 1602, and has a length within ±2% of the length of the any one base sequence selected,
(b-15) a base sequence that has at least 99% identity with any one base sequence selected from the group consisting of SEQ ID NOs: 907 to 1602, and has a length within ±1% of the length of the any one base sequence selected, and
(b-16) a base sequence that has at least 99.5% identity with any one base sequence selected from the group consisting of SEQ ID NOs: 907 to 1602, and has a length within ±0.5% of the length of the any one base sequence selected.

In one embodiment, the first unit oligomer comprises or consists of any one base sequence selected from the group consisting of SEQ ID NOs: 907 to 1602.

In one embodiment, the first unit oligomer comprises or consists of any one base sequence selected from the group consisting of SEQ ID NOs: 1180, 1190, 1201, 1212, 1222, 1224, and 1239.

Table 5 below shows examples of the target sequence of the second unit oligomer, and a complementary sequence (antisense sequence) thereof.

**[Table 5-1]**

| **Table 5** | | | | | |
|---|---|---|---|---|---|
| **Length mer** | **Target site** | **Targe sequence** | **SEQ ID NO:** | **Antisense sequence (5' to 3')** | **SEQ ID NO:** |
| 1 | H45_(-61) | C | 1 | G | 106 |
| 1 | H45_(-62) | T | 2 | A | 107 |
| 1 | H45_(-63) | A | 3 | T | 108 |
| 1 | H45_(-64) | A | 4 | T | 109 |
| 1 | H45_(-65) | T | 5 | A | 110 |
| 1 | H45_(-66) | T | 6 | A | 111 |
| 2 | H45_(-61)-(-60) | CT | 7 | AG | 112 |
| 2 | H45_(-62)-(-61) | TC | 8 | GA | 113 |
| 2 | H45_(-63)-(-62) | AT | 9 | AT | 114 |
| 2 | H45_(-64)-(-63) | AA | 10 | TT | 115 |
| 2 | H45_(-65)-(-64) | TA | 11 | TA | 116 |
| 2 | H45_(-66)-(-65) | TT | 12 | AA | 117 |
| 2 | H45_(-67)-(-66) | TT | 13 | AA | 118 |
| 3 | H45_(-61)-(-59) | CTT | 14 | AAG | 119 |
| 3 | H45_(-62)-(-60) | TCT | 15 | AGA | 120 |
| 3 | H45_(-63)-(-61) | ATC | 16 | GAT | 121 |
| 3 | H45_(-64)-(-62) | AAT | 17 | ATT | 122 |
| 3 | H45_(-65)-(-63) | TAA | 18 | TTA | 123 |
| 3 | H45_(-66)-(-64) | TTA | 19 | TAA | 124 |
| 3 | H45_(-67)-(-65) | TTT | 20 | AAA | 125 |
| 3 | H45_(-68)-(-66) | GTT | 21 | AAC | 126 |
| 4 | H45_(-61)-(-58) | CTTT | 22 | AAAG | 127 |
| 4 | H45_(-62)-(-59) | TCTT | 23 | AAGA | 128 |
| 4 | H45_(-63)-(-60) | ATCT | 24 | AGAT | 129 |
| 4 | H45_(-64)-(-61) | AATC | 25 | GATT | 130 |

**[Table 5-2]**

| | | | | | |
|---|---|---|---|---|---|
| 4 | H45_(-65)-(-62) | TAAT | 26 | ATTA | 131 |
| 4 | H45_(-66)-(-63) | TTAA | 27 | TTAA | 132 |
| 4 | H45_(-67)-(-64) | TTTA | 28 | TAAA | 133 |
| 4 | H45_(-68)-(-65) | GTTT | 29 | AAAC | 134 |
| 4 | H45_(-69)-(-66) | TGTT | 30 | AACA | 135 |
| 5 | H45_(-61)-(-57) | CTTTT | 31 | AAAAG | 136 |
| 5 | H45_(-62)-(-58) | TCTTT | 32 | AAAGA | 137 |
| 5 | H45_(-63)-(-59) | ATCTT | 33 | AAGAT | 138 |
| 5 | H45_(-64)-(-60) | AATCT | 34 | AGATT | 139 |
| 5 | H45_(-65)-(-61) | TAATC | 35 | GATTA | 140 |
| 5 | H45_(-66)-(-62) | TTAAT | 36 | ATTAA | 141 |
| 5 | H45_(-67)-(-63) | TTTAA | 37 | TTAAA | 142 |
| 5 | H45_(-68)-(-64) | GTTTA | 38 | TAAAC | 143 |
| 5 | H45_(-69)-(-65) | TGTTT | 39 | AAACA | 144 |
| 5 | H45_(-70)-(-66) | CTGTT | 40 | AACAG | 145 |
| 6 | H45_(-61)-(-56) | CTTTTC | 41 | GAAAAG | 146 |
| 6 | H45_(-62)-(-57) | TCTTTT | 42 | AAAAGA | 147 |
| 6 | H45_(-63)-(-58) | ATCTTT | 43 | AAAGAT | 148 |
| 6 | H45_(-64)-(-59) | AATCTT | 44 | AAGATT | 149 |
| 6 | H45_(-65)-(-60) | TAATCT | 45 | AGATTA | 150 |
| 6 | H45_(-66)-(-61) | TTAATC | 46 | GATTAA | 151 |
| 6 | H45_(-67)-(-62) | TTTAAT | 47 | ATTAAA | 152 |
| 6 | H45_(-68)-(-63) | GTTTAA | 48 | TTAAAC | 153 |
| 6 | H45_(-69)-(-64) | TGTTTA | 49 | TAAACA | 154 |
| 6 | H45_(-70)-(-65) | CTGTTT | 50 | AAACAG | 150 |
| 6 | H45_(-71)-(-66) | ACTGTT | 51 | AACAGT | 156 |
| 7 | H45_(-61)-(-55) | CTTTTCT | 52 | AGAAAAG | 157 |
| 7 | H45_(-62)-(-56) | TCTTTTC | 53 | GAAAAGA | 158 |
| 7 | H45_(-63)-(-57) | ATCTTTT | 54 | AAAAGAT | 159 |
| 7 | H45_(-64)-(-58) | AATCTTT | 55 | AAAGATT | 160 |
| 7 | H45_(-65)-(-59) | TAATCTT | 56 | AAGATTA | 161 |
| 7 | H45_(-66)-(-60) | TTAATCT | 57 | AGATTAA | 162 |
| 7 | H45_(-67)-(-61) | TTTAATC | 58 | GATTAAA | 163 |
| 7 | H45_(-68)-(-62) | GTTTAAT | 59 | ATTAAAC | 164 |
| 7 | H45_(-69)-(-63) | TGTTTAA | 60 | TTAAACA | 165 |

**[Table 5-3]**

| | | | | | |
|---|---|---|---|---|---|
| 7 | H45_(-70)-(-64) | CTGTTTA | 61 | TAAACAG | 166 |
| 7 | H45_(-71)-(-65) | ACTGTTT | 62 | AAACAGT | 167 |
| 7 | H45_(-72)-(-66) | CACTGTT | 63 | AACAGTG | 168 |
| 8 | H45_(-61)-(-54) | CTTTTCTC | 64 | GAGAAAAG | 169 |
| 8 | H45_(-62)-(-55) | TCTTTTCT | 65 | AGAAAAGA | 170 |
| 8 | H45_(-63)-(-56) | ATCTTTTC | 66 | GAAAAGAT | 171 |
| 8 | H45_(-64)-(-57) | AATCTTTT | 67 | AAAAGATT | 172 |
| 8 | H45_(-65)-(-58) | TAATCTTT | 68 | AAAGATTA | 173 |
| 8 | H45_(-66)-(-59) | TTAATCTT | 69 | AAGATTAA | 174 |
| 8 | H45_(-67)-(-60) | TTTAATCT | 70 | AGATTAAA | 175 |
| 8 | H45_(-68)-(-61) | GTTTAATC | 71 | GATTAAAC | 176 |
| 8 | H45_(-69)-(-62) | TGTTTAAT | 72 | ATTAAACA | 177 |
| 8 | H45_(-70)-(-63) | CTGTTTAA | 73 | TTAAACAG | 178 |
| 8 | H45_(-71)-(-64) | ACTGTTTA | 74 | TAAACAGT | 179 |
| 8 | H45_(-72)-(-65) | CACTGTTT | 75 | AAACAGTG | 180 |
| 8 | H45_(-73)-(-66) | ACACTGTT | 76 | AACAGTGT | 181 |
| 9 | H45_(-61)-(-53) | CTTTTCTCA | 77 | TGAGAAAAG | 182 |
| 9 | H45_(-62)-(-54) | TCTTTTCTC | 78 | GAGAAAAGA | 183 |
| 9 | H45_(-63)-(-55) | ATCTTTTCT | 79 | AGAAAAGAT | 184 |
| 9 | H45_(-64)-(-56) | AATCTTTTC | 80 | GAAAAGATT | 185 |
| 9 | H45_(-65)-(-57) | TAATCTTTT | 81 | AAAAGATTA | 186 |
| 9 | H45_(-66)-(-58) | TTAATCTTT | 82 | AAAGATTAA | 187 |
| 9 | H45_(-67)-(-59) | TTTAATCTT | 83 | AAGATTAAA | 188 |
| 9 | H45_(-68)-(-60) | GTTTAATCT | 84 | AGATTAAAC | 189 |
| 9 | H45_(-69)-(-61) | TGTTTAATC | 85 | GATTAAACA | 190 |
| 9 | H45_(-70)-(-62) | CTGTTTAAT | 86 | ATTAAACAG | 191 |
| 9 | H45_(-71)-(-63) | ACTGTTTAA | 87 | TTAAACAGT | 192 |
| 9 | H45_(-72)-(-64) | CACTGTTTA | 88 | TAAACAGTG | 193 |
| 9 | H45_(-73)-(-65) | ACACTGTTT | 89 | AAACAGTGT | 194 |
| 9 | H45_(-74)-(-66) | CACACTGTT | 90 | AACAGTGTG | 195 |
| 10 | H45_(-61)-(-52) | CTTTTCTCAA | 91 | TTGAGAAAAG | 196 |
| 10 | H45_(-62)-(-53) | TCTTTTCTCA | 92 | TGAGAAAAGA | 197 |
| 10 | H45_(-63)-(-54) | ATCTTTTCTC | 93 | GAGAAAAGAT | 198 |
| 10 | H45_(-64)-(-55) | AATCTTTTCT | 94 | AGAAAAGATT | 199 |
| 10 | H45_(-65)-(-56) | TAATCTTTTC | 95 | GAAAAGATTA | 200 |

**[Table 5-4]**

| | | | | | |
|---|---|---|---|---|---|
| 10 | H45_(-66)-(-57) | TTAATCTTTT | 96 | AAAAGATTAA | 201 |
| 10 | H45_(-67)-(-58) | TTTAATCTTT | 97 | AAAGATTAAA | 202 |
| 10 | H45_(-68)-(-59) | GTTTAATCTT | 98 | AAGATTAAAC | 203 |
| 10 | H45_(-69)-(-60) | TGTTTAATCT | 99 | AGATTAAACA | 204 |
| 10 | H45_(-70)-(-61) | CTGTTTAATC | 100 | GATTAAACAG | 205 |
| 10 | H45_(-71)-(-62) | ACTGTTTAAT | 101 | ATTAAACAGT | 206 |
| 10 | H45_(-72)-(-63) | CACTGTTTAA | 102 | TTAAACAGTG | 207 |
| 10 | H45_(-73)-(-64) | ACACTGTTTA | 103 | TAAACAGTGT | 208 |
| 10 | H45_(-74)-(-65) | CACACTGTTT | 104 | AAACAGTGTG | 209 |
| 10 | H45_(-75)-(-66) | GCACACTGTT | 105 | AACAGTGTGC | 210 |

In one embodiment, the second unit oligomer comprises a base sequence complementary to:
(a) any one base sequence selected from the group consisting of SEQ ID NOs: 1 to 105;
(b) a base sequence that hybridizes under stringent conditions to a base sequence complementary to any one base sequence selected from the group consisting of SEQ ID NOs: 1 to 105;
(c) a base sequence that has at least 85% identity with any one base sequence selected from the group consisting of SEQ ID NOs: 1 to 105, and has a length within ±15% of the length of the any one base sequence selected; or
(d) a partial base sequence of any one base sequence selected from the group consisting of the base sequences (a), (b), and (c) .

Herein, the base sequence (c) is a mutant type of the base sequence (a), and examples of such a mutant type also include:
(c-1) a base sequence that has at least 85% identity with any one base sequence selected from the group consisting of SEQ ID NOs: 1 to 105, and has a length within ±15% of the length of the any one base sequence selected,
(c-2) a base sequence that has at least 86% identity with any one base sequence selected from the group consisting of SEQ ID NOs: 1 to 105, and has a length within ±14% of the length of the any one base sequence selected,
(c-3) a base sequence that has at least 87% identity with any one base sequence selected from the group consisting of SEQ ID NOs: 1 to 105, and has a length within ±13% of the length of the any one base sequence selected,
(c-4) a base sequence that has at least 88% identity with any one base sequence selected from the group consisting of SEQ ID NOs: 1 to 105, and has a length within ±12% of the length of the any one base sequence selected,
(c-5) a base sequence that has at least 89% identity with any one base sequence selected from the group consisting of SEQ ID NOs: 1 to 105, and has a length within ±11% of the length of the any one base sequence selected,
(c-6) a base sequence that has at least 90% identity with any one base sequence selected from the group consisting of SEQ ID NOs: 1 to 105, and has a length within ±10% of the length of the any one base sequence selected,
(c-7) a base sequence that has at least 91% identity with any one base sequence selected from the group consisting of SEQ ID NOs: 1 to 105, and has a length within ±9% of the length of the any one base sequence selected,
(c-8) a base sequence that has at least 92% identity with any one base sequence selected from the group consisting of SEQ ID NOs: 1 to 105, and has a length within ±8% of the length of the any one base sequence selected,
(c-9) a base sequence that has at least 93% identity with any one base sequence selected from the group consisting of SEQ ID NOs: 1 to 105, and has a length within ±7% of the length of the any one base sequence selected,
(c-10) a base sequence that has at least 94% identity with any one base sequence selected from the group consisting of SEQ ID NOs: 1 to 105, and has a length within ±6% of the length of the any one base sequence selected,
(c-11) a base sequence that has at least 95% identity with any one base sequence selected from the group consisting of SEQ ID NOs: 1 to 105, and has a length within ±5% of the length of the any one base sequence selected,
(c-12) a base sequence that has at least 96% identity with any one base sequence selected from the group consisting of SEQ ID NOs: 1 to 105, and has a length within ±4% of the length of the any one base sequence selected,
(c-13) a base sequence that has at least 97% identity with any one base sequence selected from the group consisting of SEQ ID NOs: 1 to 105, and has a length within ±3% of the length of the any one base sequence selected,
(c-14) a base sequence that has at least 98% identity with any one base sequence selected from the group consisting of SEQ ID NOs: 1 to 105, and has a length within ±2% of the length of the any one base sequence selected,
(c-15) a base sequence that has at least 99% identity with any one base sequence selected from the group consisting of SEQ ID NOs: 1 to 105, and has a length within ±1% of the length of the any one base sequence selected, and
(c-16) a base sequence that has at least 99.5% identity with any one base sequence selected from the group consisting of SEQ ID NOs: 1 to 105, and has a length within ±0.5% of the length of the any one base sequence selected.

In one embodiment, the second unit oligomer comprises or consists of:
(a) any one base sequence selected from the group consisting of SEQ ID NOs: 106 to 210; or
(b) a base sequence that has at least 85% identity with any one base sequence selected from the group consisting of SEQ ID NOs: 106 to 210, and has a length within ±15% of the length of the any one base sequence selected.

Herein, the base sequence (b) is a mutant type of the base sequence (a), and examples of such a mutant type also include
(b-1) a base sequence that has at least 85% identity with any one base sequence selected from the group consisting of SEQ ID NOs: 106 to 210, and has a length within ±15% of the length of the any one base sequence selected,
(b-2) a base sequence that has at least 86% identity with any one base sequence selected from the group consisting of SEQ ID NOs: 106 to 210, and has a length within ±14% of the length of the any one base sequence selected,
(b-3) a base sequence that has at least 87% identity with any one base sequence selected from the group consisting of SEQ ID NOs: 106 to 210, and has a length within ±13% of the length of the any one base sequence selected,
(b-4) a base sequence that has at least 88% identity with any one base sequence selected from the group consisting of SEQ ID NOs: 106 to 210, and has a length within ±12% of the length of the any one base sequence selected,
(b-5) a base sequence that has at least 89% identity with any one base sequence selected from the group consisting of SEQ ID NOs: 106 to 210, and has a length within ±11% of the length of the any one base sequence selected,
(b-6) a base sequence that has at least 90% identity with any one base sequence selected from the group consisting of SEQ ID NOs: 106 to 210, and has a length within ±10% of the length of the any one base sequence selected,
(b-7) a base sequence that has at least 91% identity with any one base sequence selected from the group consisting of SEQ ID NOs: 106 to 210, and has a length within ±9% of the length of the any one base sequence selected,
(b-8) a base sequence that has at least 92% identity with any one base sequence selected from the group consisting of SEQ ID NOs: 106 to 210, and has a length within ±8% of the length of the any one base sequence selected,
(b-9) a base sequence that has at least 93% identity with any one base sequence selected from the group consisting of SEQ ID NOs: 106 to 210, and has a length within ±7% of the length of the any one base sequence selected,
(b-10) a base sequence that has at least 94% identity with any one base sequence selected from the group consisting of SEQ ID NOs: 106 to 210, and has a length within ±6% of the length of the any one base sequence selected,
(b-11) a base sequence that has at least 95% identity with any one base sequence selected from the group consisting of SEQ ID NOs: 106 to 210, and has a length within ±5% of the length of the any one base sequence selected,
(b-12) a base sequence that has at least 96% identity with any one base sequence selected from the group consisting of SEQ ID NOs: 106 to 210, and has a length within ±4% of the length of the any one base sequence selected,
(b-13) a base sequence that has at least 97% identity with any one base sequence selected from the group consisting of SEQ ID NOs: 106 to 210, and has a length within ±3% of the length of the any one base sequence selected,
(b-14) a base sequence that has at least 98% identity with any one base sequence selected from the group consisting of SEQ ID NOs: 106 to 210, and has a length within ±2% of the length of the any one base sequence selected,
(b-15) a base sequence that has at least 99% identity with any one base sequence selected from the group consisting of SEQ ID NOs: 106 to 210, and has a length within ±1% of the length of the any one base sequence selected, and
(b-16) a base sequence that has at least 99.5% identity with any one base sequence selected from the group consisting of SEQ ID NOs: 106 to 210, and has a length within ±0.5% of the length of the any one base sequence selected.

In one embodiment, the second unit oligomer comprises or consists of any one base sequence selected from the group consisting of SEQ ID NOs: 106 to 210.

In one embodiment, the second unit oligomer comprises or consists of any one base sequence selected from the group consisting of SEQ ID NOs: 114, 124, 151, 201, 203, and 205.

In one embodiment, the first unit oligomer comprises or consists of any one base sequence selected from the group consisting of SEQ ID NOs: 907 to 1602, the second unit oligomer comprises or consists of any one base sequence selected from the group consisting of SEQ ID NOs: 106 to 210, and the first antisense oligomer comprises the first unit oligomer and the second unit oligomer from the 5' ends in this order.

In one embodiment, the first antisense oligomer comprises the first unit oligomer and the second unit oligomer from the 5' ends in this order, and
the first unit oligomer comprises or consists of a base sequence of SEQ ID NO: 1201, and the second unit oligomer comprises or consists of a base sequence of SEQ ID NO: 151,
the first unit oligomer comprises or consists of a base sequence of SEQ ID NO: 1201, and the second unit oligomer comprises or consists of a base sequence of SEQ ID NO: 201,
the first unit oligomer comprises or consists of a base sequence of SEQ ID NO: 1201, and the second unit oligomer comprises or consists of a base sequence of SEQ ID NO: 203,
the first unit oligomer comprises or consists of a base sequence of SEQ ID NO: 1201, and the second unit oligomer comprises or consists of a base sequence of SEQ ID NO: 205,
the first unit oligomer comprises or consists of a base sequence of SEQ ID NO: 1239, and the second unit oligomer comprises or consists of a base sequence of SEQ ID NO: 114,
the first unit oligomer comprises or consists of a base sequence of SEQ ID NO: 1224, and the second unit oligomer comprises or consists of a base sequence of SEQ ID NO: 124,
the first unit oligomer comprises or consists of a base sequence of SEQ ID NO: 1180, and the second unit oligomer comprises or consists of a base sequence of SEQ ID NO: 151,
the first unit oligomer comprises or consists of a base sequence of SEQ ID NO: 1190, and the second unit oligomer comprises or consists of a base sequence of SEQ ID NO: 151,
the first unit oligomer comprises or consists of a base sequence of SEQ ID NO: 1212, and the second unit oligomer comprises or consists of a base sequence of SEQ ID NO: 151, or
the first unit oligomer comprises or consists of a base sequence of SEQ ID NO: 1222, and the second unit oligomer comprises or consists of a base sequence of SEQ ID NO: 151.

Table 6 below shows examples of the target sequence of the second antisense oligomer of the present invention, and a complementary sequence (antisense sequence) thereof.

**[Table 6-1]**

| **Table 6** | | | | | |
|---|---|---|---|---|---|
| **Length mer** | **Target site** | **Targe sequence** | **SEQ ID NO:** | **Antisense sequence (5' to 3')** | **SEQ ID NO:** |
| 15 | H55_(-18)-(-4) | ACATTTGGTCCTTTG | 3507 | CAAAGGACCAAATGT | 4299 |
| 15 | H55_(-17)-(-3) | CATTTGGTCCTTTGC | 3508 | GCAAAGGACCAAATG | 4300 |
| 15 | H55_(-16)-(-2) | ATTTGGTCCTTTGCA | 3509 | TGCAAAGGACCAAAT | 4301 |
| 15 | H55_(-15)-(-1) | TTTGGTCCTTTGCAG | 3510 | CTGCAAAGGACCAAA | 4302 |
| 15 | H55_(-14)-1 | TTGGTCCTTTGCAGG | 3511 | CCTGCAAAGGACCAA | 4303 |
| 15 | H55_(-13)-2 | TGGTCCTTTGCAGGG | 3512 | CCCTGCAAAGGACCA | 4304 |
| 15 | H55_(-12)-3 | GGTCCTTTGCAGGGT | 3513 | ACCCTGCAAAGGACC | 4305 |
| 15 | H55_(-11)-4 | GTCCTTTGCAGGGTG | 3514 | CACCCTGCAAAGGAC | 4306 |
| 15 | H55_(-10)-5 | TCCTTTGCAGGGTGA | 3515 | TCACCCTGCAAAGGA | 4307 |
| 15 | H55_(-9)-6 | CCTTTGCAGGGTGAG | 3516 | CTCACCCTGCAAAGG | 4308 |
| 15 | H55_(-8)-7 | CTTTGCAGGGTGAGT | 3517 | ACTCACCCTGCAAAG | 4309 |
| 15 | H55_(-7)-8 | TTTGCAGGGTGAGTG | 3518 | CACTCACCCTGCAAA | 4310 |
| 15 | H55_(-6)-9 | TTGCAGGGTGAGTGA | 3519 | TCACTCACCCTGCAA | 4311 |
| 15 | H55_(-5)-10 | TGCAGGGTGAGTGAG | 3520 | CTCACTCACCCTGCA | 4312 |
| 15 | H55_(-4)-11 | GCAGGGTGAGTGAGC | 3521 | GCTCACTCACCCTGC | 4313 |
| 15 | H55_(-3)-12 | CAGGGTGAGTGAGCG | 3522 | CGCTCACTCACCCTG | 4314 |
| 15 | H55_(-2)- | AGGGTGAGTGAGCGA | 3523 | TCGCTCACTCACCCT | 4315 |

**[Table 6-2]**

| | | | | | |
|---|---|---|---|---|---|
| | 13 | | | | |
| 15 | H55_(-1)- 14 | GGGTGAGTGAGCGAG | 3524 | CTCGCTCACTCACCC | 4316 |
| 15 | H55_1-15 | GGTGAGTGAGCGAGA | 3525 | TCTCGCTCACTCACC | 4317 |
| 15 | H55_2-16 | GTGAGTGAGCGAGAG | 3526 | CTCTCGCTCACTCAC | 4318 |
| 15 | H55_3-17 | TGAGTGAGCGAGAGG | 3527 | CCTCTCGCTCACTCA | 4319 |
| 15 | H55_4-18 | GAGTGAGCGAGAGGC | 3528 | GCCTCTCGCTCACTC | 4320 |
| 15 | H55_5-19 | AGTGAGCGAGAGGCT | 3529 | AGCCTCTCGCTCACT | 4321 |
| 15 | H55_6-20 | GTGAGCGAGAGGCTG | 3530 | CAGCCTCTCGCTCAC | 4322 |
| 15 | H55_7-21 | TGAGCGAGAGGCTGC | 3531 | GCAGCCTCTCGCTCA | 4323 |
| 15 | H55_8-22 | GAGCGAGAGGCTGCT | 3532 | AGCAGCCTCTCGCTC | 4324 |
| 15 | H55_9-23 | AGCGAGAGGCTGCTT | 3533 | AAGCAGCCTCTCGCT | 4325 |
| 15 | H55_10-24 | GCGAGAGGCTGCTTT | 3534 | AAAGCAGCCTCTCGC | 4326 |
| 15 | H55_11-25 | CGAGAGGCTGCTTTG | 3535 | CAAAGCAGCCTCTCG | 4327 |
| 15 | H55_12-26 | GAGAGGCTGCTTTGG | 3536 | CCAAAGCAGCCTCTC | 4328 |
| 15 | H55_13-27 | AGAGGCTGCTTTGGA | 3537 | TCCAAAGCAGCCTCT | 4329 |
| 15 | H55_14-28 | GAGGCTGCTTTGGAA | 3538 | TTCCAAAGCAGCCTC | 4330 |
| 15 | H55_15-29 | AGGCTGCTTTGGAAG | 3539 | CTTCCAAAGCAGCCT | 4331 |
| 15 | H55_16-30 | GGCTGCTTTGGAAGA | 3540 | TCTTCCAAAGCAGCC | 4332 |
| 15 | H55_17-31 | GCTGCTTTGGAAGAA | 3541 | TTCTTCCAAAGCAGC | 4333 |
| 15 | H55_18-32 | CTGCTTTGGAAGAAA | 3542 | TTTCTTCCAAAGCAG | 4334 |
| 15 | H55_19-33 | TGCTTTGGAAGAAAC | 3543 | GTTTCTTCCAAAGCA | 4335 |
| 15 | H55_20-34 | GCTTTGGAAGAAACT | 3544 | AGTTTCTTCCAAAGC | 4336 |
| 15 | H55_21-35 | CTTTGGAAGAAACTC | 3545 | GAGTTTCTTCCAAAG | 4337 |
| 15 | H55_22-36 | TTTGGAAGAAACTCA | 3546 | TGAGTTTCTTCCAAA | 4338 |
| 15 | H55_23-37 | TTGGAAGAAACTCAT | 3547 | ATGAGTTTCTTCCAA | 4339 |
| 15 | H55_24-38 | TGGAAGAAACTCATA | 3548 | TATGAGTTTCTTCCA | 4340 |
| 16 | H55_(-19)-(-4) | AACATTTGGTCCTTTG | 3549 | CAAAGGACCAAATGTT | 4341 |
| 16 | H55_(-18)-(-3) | ACATTTGGTCCTTTGC | 3550 | GCAAAGGACCAAATGT | 4342 |
| 16 | H55_(-17)-(-2) | CATTTGGTCCTTTGCA | 3551 | TGCAAAGGACCAAATG | 4343 |
| 16 | H55_(-16)-(-1) | ATTTGGTCCTTTGCAG | 3552 | CTGCAAAGGACCAAAT | 4344 |

**[Table 6-3]**

| | | | | | |
|---|---|---|---|---|---|
| 16 | H55_(-15)-1 | TTTGGTCCTTTGCAGG | 3553 | CCTGCAAAGGACCAAA | 4345 |
| 16 | H55_(-14)-2 | TTGGTCCTTTGCAGGG | 3554 | CCCTGCAAAGGACCAA | 4346 |
| 16 | H55_(-13)-3 | TGGTCCTTTGCAGGGT | 3555 | ACCCTGCAAAGGACCA | 4347 |
| 16 | H55_(-12)-4 | GGTCCTTTGCAGGGTG | 3556 | CACCCTGCAAAGGACC | 4348 |
| 16 | H55_(-11)-5 | GTCCTTTGCAGGGTGA | 3557 | TCACCCTGCAAAGGAC | 4349 |
| 16 | H55_(-10)-6 | TCCTTTGCAGGGTGAG | 3558 | CTCACCCTGCAAAGGA | 4350 |
| 16 | H55_(-9)-7 | CCTTTGCAGGGTGAGT | 3559 | ACTCACCCTGCAAAGG | 4351 |
| 16 | H55_(-8)-8 | CTTTGCAGGGTGAGTG | 3560 | CACTCACCCTGCAAAG | 4352 |
| 16 | H55_(-7)-9 | TTTGCAGGGTGAGTGA | 3561 | TCACTCACCCTGCAAA | 4353 |
| 16 | H55_(-6)-10 | TTGCAGGGTGAGTGAG | 3562 | CTCACTCACCCTGCAA | 4354 |
| 16 | H55_(-5)-11 | TGCAGGGTGAGTGAGC | 3563 | GCTCACTCACCCTGCA | 4355 |
| 16 | H55_(-4)-12 | GCAGGGTGAGTGAGCG | 3564 | CGCTCACTCACCCTGC | 4356 |
| 16 | H55_(-3)-13 | CAGGGTGAGTGAGCGA | 3565 | TCGCTCACTCACCCTG | 4357 |
| 16 | H55_(-2)-14 | AGGGTGAGTGAGCGAG | 3566 | CTCGCTCACTCACCCT | 4358 |
| 16 | N55_(-1)-15 | GGGTGAGTGAGCGAGA | 3567 | TCTCGCTCACTCACCC | 4359 |
| 16 | H55_1-16 | GGTGAGTGAGCGAGAG | 3568 | CTCTCGCTCACTCACC | 4360 |
| 16 | H55_2-17 | GTGAGTGAGCGAGAGG | 3569 | CCTCTCGCTCACTCAC | 4361 |
| 16 | H55_3-18 | TGAGTGAGCGAGAGGC | 3570 | GCCTCTCGCTCACTCA | 4362 |
| 16 | H55_4-19 | GAGTGAGCGAGAGGCT | 3571 | AGCCTCTCGCTCACTC | 4363 |
| 16 | H55_5-20 | AGTGAGCGAGAGGCTG | 3572 | CAGCCTCTCGCTCACT | 4364 |

**[Table 6-4]**

| | | | | | |
|---|---|---|---|---|---|
| 16 | H55_6-21 | GTGAGCGAGAGGCTGC | 3573 | GCAGCCTCTCGCTCAC | 4365 |
| 16 | H55_7-22 | TGAGCGAGAGGCTGCT | 3574 | AGCAGCCTCTCGCTCA | 4366 |
| 16 | H55_8-23 | GAGCGAGAGGCTGCTT | 3575 | AAGCAGCCTCTCGCTC | 4367 |
| 16 | H55_9-24 | AGCGAGAGGCTGCTTT | 3576 | AAAGCAGCCTCTCGCT | 4368 |
| 16 | H55_10-25 | GCGAGAGGCTGCTTTG | 3577 | CAAAGCAGCCTCTCGC | 4369 |
| 16 | H55_11-26 | CGAGAGGCTGCTTTGG | 3578 | CCAAAGCAGCCTCTCG | 4370 |
| 16 | H55_12-27 | GAGAGGCTGCTTTGGA | 3579 | TCCAAAGCAGCCTCTC | 4371 |
| 16 | H55_13-28 | AGAGGCTGCTTTGGAA | 3580 | TTCCAAAGCAGCCTCT | 4372 |
| 16 | H55_14-29 | GAGGCTGCTTTGGAAG | 3581 | CTTCCAAAGCAGCCTC | 4373 |
| 16 | H55_15-30 | AGGCTGCTTTGGAAGA | 3582 | TCTTCCAAAGCAGCCT | 4374 |
| 16 | H55_16-31 | GGCTGCTTTGGAAGAA | 3583 | TTCTTCCAAAGCAGCC | 4375 |
| 16 | H55_17-32 | GCTGCTTTGGAAGAAA | 3584 | TTTCTTCCAAAGCAGC | 4376 |
| 16 | H55_18-33 | CTGCTTTGGAAGAAAC | 3585 | GTTTCTTCCAAAGCAG | 4377 |
| 16 | H55_19-34 | TGCTTTGGAAGAAACT | 3586 | AGTTTCTTCCAAAGCA | 4378 |
| 16 | H55_20-35 | GCTTTGGAAGAAACTC | 3587 | GAGTTTCTTCCAAAGC | 4379 |
| 16 | H55_21-36 | CTTTGGAAGAAACTCA | 3588 | TGAGTTTCTTCCAAAG | 4380 |
| 16 | H55_22-37 | TTTGGAAGAAACTCAT | 3589 | ATGAGTTTCTTCCAAA | 4381 |
| 16 | H55_23-38 | TTGGAAGAAACTCATA | 3590 | TATGAGTTTCTTCCAA | 4382 |
| 16 | H55_24-39 | TGGAAGAAACTCATAG | 3591 | CTATGAGTTTCTTCCA | 4383 |
| 17 | H55_(-20)-(-4) | GAACATTTGGTCCTTTG | 3592 | CAAAGGACCAAATGTTC | 4384 |
| 17 | H55_(-19)-(-3) | AACATTTGGTCCTTTGC | 3593 | GCAAAGGACCAAATGTT | 4385 |
| 17 | H55_(-18)-(-2) | ACATTTGGTCCTTTGCA | 3594 | TGCAAAGGACCAAATGT | 4386 |
| 17 | H55_(-17)-(-1) | CATTTGGTCCTTTGCAG | 3595 | CTGCAAAGGACCAAATG | 4387 |
| 17 | H55_(-16)-1 | ATTTGGTCCTTTGCAGG | 3596 | CCTGCAAAGGACCAAAT | 4388 |
| 17 | H55_(-15)-2 | TTTGGTCCTTTGCAGGG | 3597 | CCCTGCAAAGGACCAAA | 4389 |
| 17 | H55_(-14)-3 | TTGGTCCTTTGCAGGGT | 3598 | ACCCTGCAAAGGACCAA | 4390 |
| 17 | H55_(-13)-4 | TGGTCCTTTGCAGGGTG | 3599 | CACCCTGCAAAGGACCA | 4391 |

**[Table 6-5]**

| | | | | | |
|---|---|---|---|---|---|
| 17 | H55_(-12)-5 | GGTCCTTTGCAGGGTGA | 3600 | TCACCCTGCAAAGGACC | 4392 |
| 17 | H55_(-11)-6 | GTCCTTTGCAGGGTGAG | 3601 | CTCACCCTGCAAAGGAC | 4393 |
| 17 | H55_(-10)-7 | TCCTTTGCAGGGTGAGT | 3602 | ACTCACCCTGCAAAGGA | 4394 |
| 17 | H55_(-9)-8 | CCTTTGCAGGGTGAGTG | 3603 | CACTCACCCTGCAAAGG | 4395 |
| 17 | H55_(-8)-9 | CTTTGCAGGGTGAGTGA | 3604 | TCACTCACCCTGCAAAG | 4396 |
| 17 | H55_(-7)-10 | TTTGCAGGGTGAGTGAG | 3605 | CTCACTCACCCTGCAAA | 4397 |
| 17 | H55_(-6)-11 | TTGCAGGGTGAGTGAGC | 3606 | GCTCACTCACCCTGCAA | 4398 |
| 17 | H55_(-5)-12 | TGCAGGGTGAGTGAGCG | 3607 | CGCTCACTCACCCTGCA | 4399 |
| 17 | H55_(-4)-13 | GCAGGGTGAGTGAGCGA | 3608 | TCGCTCACTCACCCTGC | 4400 |
| 17 | H55_(-3)-14 | CAGGGTGAGTGAGCGAG | 3609 | CTCGCTCACTCACCCTG | 4401 |
| 17 | H55_(-2)-15 | AGGGTGAGTGAGCGAGA | 3610 | TCTCGCTCACTCACCCT | 4402 |
| 17 | H55_(-1)-16 | GGGTGAGTGAGCGAGAG | 3611 | CTCTCGCTCACTCACCC | 4403 |
| 17 | H55_1-17 | GGTGAGTGAGCGAGAGG | 3612 | CCTCTCGCTCACTCACC | 4404 |
| 17 | H55_2-18 | GTGAGTGAGCGAGAGGC | 3613 | GCCTCTCGCTCACTCAC | 4405 |
| 17 | H55_3-19 | TGAGTGAGCGAGAGGCT | 3614 | AGCCTCTCGCTCACTCA | 4406 |
| 17 | H55_4-20 | GAGTGAGCGAGAGGCTG | 3615 | CAGCCTCTCGCTCACTC | 4407 |
| 17 | H55_5-21 | AGTGAGCGAGAGGCTGC | 3616 | GCAGCCTCTCGCTCACT | 4408 |
| 17 | H55_6-22 | GTGAGCGAGAGGCTGCT | 3617 | AGCAGCCTCTCGCTCAC | 4409 |
| 17 | H55_7-23 | TGAGCGAGAGGCTGCTT | 3618 | AAGCAGCCTCTCGCTCA | 4410 |
| 17 | H55_8-24 | GAGCGAGAGGCTGCTTT | 3619 | AAAGCAGCCTCTCGCTC | 4411 |
| 17 | H55_9-25 | AGCGAGAGGCTGCTTTG | 3620 | CAAAGCAGCCTCTCGCT | 4412 |
| 17 | H55_10-26 | GCGAGAGGCTGCTTTGG | 3621 | CCAAAGCAGCCTCTCGC | 4413 |
| 17 | H55_11-27 | CGAGAGGCTGCTTTGGA | 3622 | TCCAAAGCAGCCTCTCG | 4414 |

**[Table 6-6]**

| | | | | | |
|---|---|---|---|---|---|
| 17 | H55_12-28 | GAGAGGCTGCTTTGGAA | 3623 | TTCCAAAGCAGCCTCTC | 4415 |
| 17 | H55_13-29 | AGAGGCTGCTTTGGAAG | 3624 | CTTCCAAAGCAGCCTCT | 4416 |
| 17 | H55_14-30 | GAGGCTGCTTTGGAAGA | 3625 | TCTTCCAAAGCAGCCTC | 4417 |
| 17 | H55_15-31 | AGGCTGCTTTGGAAGAA | 3626 | TTCTTCCAAAGCAGCCT | 4418 |
| 17 | H55_16-32 | GGCTGCTTTGGAAGAAA | 3627 | TTTCTTCCAAAGCAGCC | 4419 |
| 17 | H55_17-33 | GCTGCTTTGGAAGAAAC | 3628 | GTTTCTTCCAAAGCAGC | 4420 |
| 17 | H55_18-34 | CTGCTTTGGAAGAAACT | 3629 | AGTTTCTTCCAAAGCAG | 4421 |
| 17 | H55_19-35 | TGCTTTGGAAGAAACTC | 3630 | GAGTTTCTTCCAAAGCA | 4422 |
| 17 | H55_20-36 | GCTTTGGAAGAAACTCA | 3631 | TGAGTTTCTTCCAAAGC | 4423 |
| 17 | H55_21-37 | CTTTGGAAGAAACTCAT | 3632 | ATGAGTTTCTTCCAAAG | 4424 |
| 17 | H55_22-38 | TTTGGAAGAAACTCATA | 3633 | TATGAGTTTCTTCCAAA | 4425 |
| 17 | H55_23-39 | TTGGAAGAAACTCATAG | 3634 | CTATGAGTTTCTTCCAA | 4426 |
| 17 | H55_24-40 | TGGAAGAAACTCATAGA | 3635 | TCTATGAGTTTCTTCCA | 4427 |
| 18 | H55_(-21)-(-4) | TGAACATTTGGTCCTTTG | 3636 | CAAAGGACCAAATGTTCA | 4428 |
| 18 | H55_(-20) - (-3) | GAACATTTGGTCCTTTGC | 3637 | GCAAAGGACCAAATGTTC | 4429 |
| 18 | H55_(-19)-(-2) | AACATTTGGTCCTTTGCA | 3638 | TGCAAAGGACCAAATGTT | 4430 |
| 18 | H55_(-18)-(-1) | ACATTTGGTCCTTTGCAG | 3639 | CTGCAAAGGACCAAATGT | 4431 |
| 18 | H55_(-17)-1 | CATTTGGTCCTTTGCAGG | 3640 | CCTGCAAAGGACCAAATG | 4432 |
| 18 | H55_(-16)-2 | ATTTGGTCCTTTGCAGGG | 3641 | CCCTGCAAAGGACCAAAT | 4433 |
| 18 | H55_(-15) -3 | TTTGGTCCTTTGCAGGGT | 3642 | ACCCTGCAAAGGACCAAA | 4434 |
| 18 | H55_(-14) -4 | TTGGTCCTTTGCAGGGTG | 3643 | CACCCTGCAAAGGACCAA | 4435 |
| 18 | H55_(-13)-5 | TGGTCCTTTGCAGGGTGA | 3644 | TCACCCTGCAAAGGACCA | 4436 |
| 18 | H55_(-12)-6 | GGTCCTTTGCAGGGTGAG | 3645 | CTCACCCTGCAAAGGACC | 4437 |
| 18 | H55_(-11)-7 | GTCCTTTGCAGGGTGAGT | 3646 | ACTCACCCTGCAAAGGAC | 4438 |

**[Table 6-7]**

| | | | | | |
|---|---|---|---|---|---|
| 18 | H55_(-10)-8 | TCCTTTGCAGGGTGAGTG | 3647 | CACTCACCCTGCAAAGGA | 4439 |
| 18 | H55_(-9)-9 | CCTTTGCAGGGTGAGTGA | 3648 | TCACTCACCCTGCAAAGG | 4440 |
| 18 | H55_(-8)-10 | CTTTGCAGGGTGAGTGAG | 3649 | CTCACTCACCCTGCAAAG | 4441 |
| 18 | H55_(-7)-11 | TTTGCAGGGTGAGTGAGC | 3650 | GCTCACTCACCCTGCAAA | 4442 |
| 18 | H55_(-6)-12 | TTGCAGGGTGAGTGAGCG | 3651 | CGCTCACTCACCCTGCAA | 4443 |
| 18 | H55_(-5)-13 | TGCAGGGTGAGTGAGCGA | 3652 | TCGCTCACTCACCCTGCA | 4444 |
| 18 | H55_(-4)-14 | GCAGGGTGAGTGAGCGAG | 3653 | CTCGCTCACTCACCCTGC | 4445 |
| 18 | H55_(-3)-15 | CAGGGTGAGTGAGCGAGA | 3654 | TCTCGCTCACTCACCCTG | 4446 |
| 18 | H55_(-2)-16 | AGGGTGAGTGAGCGAGAG | 3655 | CTCTCGCTCACTCACCCT | 4447 |
| 18 | H55_(-1)- 17 | GGGTGAGTGAGCGAGAGG | 3656 | CCTCTCGCTCACTCACCC | 4448 |
| 18 | H55_1-18 | GGTGAGTGAGCGAGAGGC | 3657 | GCCTCTCGCTCACTCACC | 4449 |
| 18 | H55_2-19 | GTGAGTGAGCGAGAGGCT | 3658 | AGCCTCTCGCTCACTCAC | 4450 |
| 18 | H55_3-20 | TGAGTGAGCGAGAGGCTG | 3659 | CAGCCTCTCGCTCACTCA | 4451 |
| 18 | H55_4-21 | GAGTGAGCGAGAGGCTGC | 3660 | GCAGCCTCTCGCTCACTC | 4452 |
| 18 | H55_5-22 | AGTGAGCGAGAGGCTGCT | 3661 | AGCAGCCTCTCGCTCACT | 4453 |
| 18 | H55_6-23 | GTGAGCGAGAGGCTGCTT | 3662 | AAGCAGCCTCTCGCTCAC | 4454 |
| 18 | H55_7-24 | TGAGCGAGAGGCTGCTTT | 3663 | AAAGCAGCCTCTCGCTCA | 4455 |
| 18 | H55_8-25 | GAGCGAGAGGCTGCTTTG | 3664 | CAAAGCAGCCTCTCGCTC | 4456 |
| 18 | H55_9-26 | AGCGAGAGGCTGCTTTGG | 3665 | CCAAAGCAGCCTCTCGCT | 4457 |
| 18 | H55_10-27 | GCGAGAGGCTGCTTTGGA | 3666 | TCCAAAGCAGCCTCTCGC | 4458 |
| 18 | H55_11-28 | CGAGAGGCTGCTTTGGAA | 3667 | TTCCAAAGCAGCCTCTCG | 4459 |
| 18 | H55_12-29 | GAGAGGCTGCTTTGGAAG | 3668 | CTTCCAAAGCAGCCTCTC | 4460 |
| 18 | H55_13-30 | AGAGGCTGCTTTGGAAGA | 3669 | TCTTCCAAAGCAGCCTCT | 4461 |
| 18 | H55_14-31 | GAGGCTGCTTTGGAAGAA | 3670 | TTCTTCCAAAGCAGCCTC | 4462 |
| 18 | H55_15-32 | AGGCTGCTTTGGAAGAAA | 3671 | TTTCTTCCAAAGCAGCCT | 4463 |

**[Table 6-8]**

| | | | | | |
|---|---|---|---|---|---|
| 18 | H55_16-33 | GGCTGCTTTGGAAGAAAC | 3672 | GTTTCTTCCAAAGCAGCC | 4464 |
| 18 | H55_17-34 | GCTGCTTTGGAAGAAACT | 3673 | AGTTTCTTCCAAAGCAGC | 4465 |
| 18 | H55_18-35 | CTGCTTTGGAAGAAACTC | 3674 | GAGTTTCTTCCAAAGCAG | 4466 |
| 18 | H55_19-36 | TGCTTTGGAAGAAACTCA | 3675 | TGAGTTTCTTCCAAAGCA | 4467 |
| 18 | H55_20-37 | GCTTTGGAAGAAACTCAT | 3676 | ATGAGTTTCTTCCAAAGC | 4468 |
| 18 | H55_21-38 | CTTTGGAAGAAACTCATA | 3677 | TATGAGTTTCTTCCAAAG | 4469 |
| 18 | H55_22-39 | TTTGGAAGAAACTCATAG | 3678 | CTATGAGTTTCTTCCAAA | 4470 |
| 18 | H55_23-40 | TTGGAAGAAACTCATAGA | 3679 | TCTATGAGTTTCTTCCAA | 4471 |
| 18 | H55_24-41 | TGGAAGAAACTCATAGAT | 3680 | ATCTATGAGTTTCTTCCA | 4472 |
| 19 | H55_(-22)-(-4) | CTGAACATTTGGTCCTTTG | 3681 | CAAAGGACCAAATGTTCAG | 4473 |
| 19 | H55_(-21)-(-3) | TGAACATTTGGTCCTTTGC | 3682 | GCAAAGGACCAAATGTTCA | 4474 |
| 19 | H55_(-20)-(-2) | GAACATTTGGTCCTTTGCA | 3683 | TGCAAAGGACCAAATGTTC | 4475 |
| 19 | H55_(-19)-(-1) | AACATTTGGTCCTTTGCAG | 3684 | CTGCAAAGGACCAAATGTT | 4476 |
| 19 | H55_(-18)-1 | ACATTTGGTCCTTTGCAGG | 3685 | CCTGCAAAGGACCAAATGT | 4477 |
| 19 | H55_(-17)-2 | CATTTGGTCCTTTGCAGGG | 3686 | CCCTGCAAAGGACCAAATG | 4478 |
| 19 | H55_(-16)-3 | ATTTGGTCCTTTGCAGGGT | 3687 | ACCCTGCAAAGGACCAAAT | 4479 |
| 19 | H55_(-15)-4 | TTTGGTCCTTTGCAGGGTG | 3688 | CACCCTGCAAAGGACCAAA | 4480 |
| 19 | H55_(-14)-5 | TTGGTCCTTTGCAGGGTGA | 3689 | TCACCCTGCAAAGGACCAA | 4481 |
| 19 | H55_(-13)-6 | TGGTCCTTTGCAGGGTGAG | 3690 | CTCACCCTGCAAAGGACCA | 4482 |
| 19 | H55_(-12)-7 | GGTCCTTTGCAGGGTGAGT | 3691 | ACTCACCCTGCAAAGGACC | 4483 |
| 19 | H55_(-11)-8 | GTCCTTTGCAGGGTGAGTG | 3692 | CACTCACCCTGCAAAGGAC | 4484 |
| 19 | H55_(-10)-9 | TCCTTTGCAGGGTGAGTGA | 3693 | TCACTCACCCTGCAAAGGA | 4485 |

**[Table 6-9]**

| | | | | | |
|---|---|---|---|---|---|
| 19 | H55_(-9)-10 | CCTTTGCAGGGTGAGTGAG | 3694 | CTCACTCACCCTGCAAAGG | 4486 |
| 19 | H55_(-8)-11 | CTTTGCAGGGTGAGTGAGC | 3695 | GCTCACTCACCCTGCAAAG | 4487 |
| 19 | H55_(-7)-12 | TTTGCAGGGTGAGTGAGCG | 3696 | CGCTCACTCACCCTGCAAA | 4488 |
| 19 | H55_(-6)-13 | TTGCAGGGTGAGTGAGCGA | 3697 | TCGCTCACTCACCCTGCAA | 4489 |
| 19 | H55_(-5)-14 | TGCAGGGTGAGTGAGCGAG | 3698 | CTCGCTCACTCACCCTGCA | 4490 |
| 19 | H55_(-4)-15 | GCAGGGTGAGTGAGCGAGA | 3699 | TCTCGCTCACTCACCCTGC | 4491 |
| 19 | H55_(-3)-16 | CAGGGTGAGTGAGCGAGAG | 3700 | CTCTCGCTCACTCACCCTG | 4492 |
| 19 | H55_(-2)-17 | AGGGTGAGTGAGCGAGAGG | 3701 | CCTCTCGCTCACTCACCCT | 4493 |
| 19 | H55_(-1)- 18 | GGGTGAGTGAGCGAGAGGC | 3702 | GCCTCTCGCTCACTCACCC | 4494 |
| 19 | H55_1-19 | GGTGAGTGAGCGAGAGGCT | 3703 | AGCCTCTCGCTCACTCACC | 4495 |
| 19 | H55_2-20 | GTGAGTGAGCGAGAGGCTG | 3704 | CAGCCTCTCGCTCACTCAC | 4496 |
| 19 | H55_3-21 | TGAGTGAGCGAGAGGCTGC | 3705 | GCAGCCTCTCGCTCACTCA | 4497 |
| 19 | H55_4-22 | GAGTGAGCGAGAGGCTGCT | 3706 | AGCAGCCTCTCGCTCACTC | 4498 |
| 19 | H55_5-23 | AGTGAGCGAGAGGCTGCTT | 3707 | AAGCAGCCTCTCGCTCACT | 4499 |
| 19 | H55_6-24 | GTGAGCGAGAGGCTGCTTT | 3708 | AAAGCAGCCTCTCGCTCAC | 4500 |
| 19 | H55_7-25 | TGAGCGAGAGGCTGCTTTG | 3709 | CAAAGCAGCCTCTCGCTCA | 4501 |
| 19 | H55_8-26 | GAGCGAGAGGCTGCTTTGG | 3710 | CCAAAGCAGCCTCTCGCTC | 4502 |
| 19 | H55_9-27 | AGCGAGAGGCTGCTTTGGA | 3711 | TCCAAAGCAGCCTCTCGCT | 4503 |
| 19 | H55_10-28 | GCGAGAGGCTGCTTTGGAA | 3712 | TTCCAAAGCAGCCTCTCGC | 4504 |
| 19 | H55_11-29 | CGAGAGGCTGCTTTGGAAG | 3713 | CTTCCAAAGCAGCCTCTCG | 4505 |
| 19 | H55_12-30 | GAGAGGCTGCTTTGGAAGA | 3714 | TCTTCCAAAGCAGCCTCTC | 4506 |
| 19 | H55_13-31 | AGAGGCTGCTTTGGAAGAA | 3715 | TTCTTCCAAAGCAGCCTCT | 4507 |
| 19 | H55_14-32 | GAGGCTGCTTTGGAAGAAA | 3716 | TTTCTTCCAAAGCAGCCTC | 4508 |
| 19 | H55_15-33 | AGGCTGCTTTGGAAGAAAC | 3717 | GTTTCTTCCAAAGCAGCCT | 4509 |
| 19 | H55_16-34 | GGCTGCTTTGGAAGAAACT | 3718 | AGTTTCTTCCAAAGCAGCC | 4510 |
| 19 | H55_17-35 | GCTGCTTTGGAAGAAACTC | 3719 | GAGTTTCTTCCAAAGCAGC | 4511 |

**[Table 6-10]**

| | | | | | |
|---|---|---|---|---|---|
| 19 | H55_18-36 | CTGCTTTGGAAGAAACTCA | 3720 | TGAGTTTCTTCCAAAGCAG | 4512 |
| 19 | H55_19-37 | TGCTTTGGAAGAAACTCAT | 3721 | ATGAGTTTCTTCCAAAGCA | 4513 |
| 19 | H55_20-38 | GCTTTGGAAGAAACTCATA | 3722 | TATGAGTTTCTTCCAAAGC | 4514 |
| 19 | H55_21-39 | CTTTGGAAGAAACTCATAG | 3723 | CTATGAGTTTCTTCCAAAG | 4515 |
| 19 | H55_22-40 | TTTGGAAGAAACTCATAGA | 3724 | TCTATGAGTTTCTTCCAAA | 4516 |
| 19 | H55_23-41 | TTGGAAGAAACTCATAGAT | 3725 | ATCTATGAGTTTCTTCCAA | 4517 |
| 19 | H55_24-42 | TGGAAGAAACTCATAGATT | 3726 | AATCTATGAGTTTCTTCCA | 4518 |
| 20 | H55_(-23)-(-4) | TCTGAACATTTGGTCCTTTG | 3727 | CAAAGGACCAAATGTTCAGA | 4519 |
| 20 | H55_(-22)-(-3) | CTGAACATTTGGTCCTTTGC | 3728 | GCAAAGGACCAAATGTTCAG | 4520 |
| 20 | H55_(-21)-(-2) | TGAACATTTGGTCCTTTGCA | 3729 | TGCAAAGGACCAAATGTTCA | 4521 |
| 20 | H55_(-20)-(-1) | GAACATTTGGTCCTTTGCAG | 3730 | CTGCAAAGGACCAAATGTTC | 4522 |
| 20 | H55_(-19)-1 | AACATTTGGTCCTTTGCAGG | 3731 | CCTGCAAAGGACCAAATGTT | 4523 |
| 20 | H55_(-18)-2 | ACATTTGGTCCTTTGCAGGG | 3732 | CCCTGCAAAGGACCAAATGT | 4524 |
| 20 | H55_(-17)-3 | CATTTGGTCCTTTGCAGGGT | 3733 | ACCCTGCAAAGGACCAAATG | 4525 |
| 20 | H55_(-16)-4 | ATTTGGTCCTTTGCAGGGTG | 3734 | CACCCTGCAAAGGACCAAAT | 4526 |
| 20 | H55_(-15)-5 | TTTGGTCCTTTGCAGGGTGA | 3735 | TCACCCTGCAAAGGACCAAA | 4527 |
| 20 | H55_(-14)-6 | TTGGTCCTTTGCAGGGTGAG | 3736 | CTCACCCTGCAAAGGACCAA | 4528 |
| 20 | H55_(-13)-7 | TGGTCCTTTGCAGGGTGAGT | 3737 | ACTCACCCTGCAAAGGACCA | 4529 |
| 20 | H55_(-12)-8 | GGTCCTTTGCAGGGTGAGTG | 3738 | CACTCACCCTGCAAAGGACC | 4530 |
| 20 | H55_(-11)-9 | GTCCTTTGCAGGGTGAGTGA | 3739 | TCACTCACCCTGCAAAGGAC | 4531 |
| 20 | H55_(-10)-10 | TCCTTTGCAGGGTGAGTGAG | 3740 | CTCACTCACCCTGCAAAGGA | 4532 |

**[Table 6-11]**

| | | | | | |
|---|---|---|---|---|---|
| 20 | H55_(-9)-11 | CCTTTGCAGGGTGAGTGAGC | 3741 | GCTCACTCACCCTGCAAAGG | 4533 |
| 20 | H55_(-8)-12 | CTTTGCAGGGTGAGTGAGCG | 3742 | CGCTCACTCACCCTGCAAAG | 4534 |
| 20 | H55_(-7)-13 | TTTGCAGGGTGAGTGAGCGA | 3743 | TCGCTCACTCACCCTGCAAA | 4535 |
| 20 | 1155_(-6)-14 | TTGCAGGGTGAGTGAGCGAG | 3744 | CTCGCTCACTCACCCTGCAA | 4536 |
| 20 | H55_(-5)- 15 | TGCAGGGTGAGTGAGCGAGA | 3745 | TCTCGCTCACTCACCCTGCA | 4537 |
| 20 | H55_(-4)- 16 | GCAGGGTGAGTGAGCGAGAG | 3746 | CTCTCGCTCACTCACCCTGC | 4538 |
| 20 | H55_(-3)- 17 | CAGGGTGAGTGAGCGAGAGG | 3747 | CCTCTCGCTCACTCACCCTG | 4539 |
| 20 | H55_(-2)-18 | AGGGTGAGTGAGCGAGAGGC | 3748 | GCCTCTCGCTCACTCACCCT | 4540 |
| 20 | H55_(-1)-19 | GGGTGAGTGAGCGAGAGGCT | 3749 | AGCCTCTCGCTCACTCACCC | 4541 |
| 20 | H55_1-20 | GGTGAGTGAGCGAGAGGCTG | 3750 | CAGCCTCTCGCTCACTCACC | 4542 |
| 20 | H55_2-21 | GTGAGTGAGCGAGAGGCTGC | 3751 | GCAGCCTCTCGCTCACTCAC | 4543 |
| 20 | H55_3-22 | TGAGTGAGCGAGAGGCTGCT | 3752 | AGCAGCCTCTCGCTCACTCA | 4544 |
| 20 | H55_4-23 | GAGTGAGCGAGAGGCTGCTT | 3753 | AAGCAGCCTCTCGCTCACTC | 4545 |
| 20 | H55_5-24 | AGTGAGCGAGAGGCTGCTTT | 3754 | AAAGCAGCCTCTCGCTCACT | 4546 |
| 20 | H55_6-25 | GTGAGCGAGAGGCTGCTTTG | 3755 | CAAAGCAGCCTCTCGCTCAC | 4517 |
| 20 | H55_7-26 | TGAGCGAGAGGCTGCTTTGG | 3756 | CCAAAGCAGCCTCTCGCTCA | 4548 |
| 20 | H55_8-27 | GAGCGAGAGGCTGCTTTGGA | 3757 | TCCAAAGCAGCCTCTCGCTC | 4549 |
| 20 | H55_9-28 | AGCGAGAGGCTGCTTTGGAA | 3758 | TTCCAAAGCAGCCTCTCGCT | 4550 |
| 20 | H55_10-29 | GCGAGAGGCTGCTTTGGAAG | 3759 | CTTCCAAAGCAGCCTCTCGC | 4551 |
| 20 | H55_11-30 | CGAGAGGCTGCTTTGGAAGA | 3760 | TCTTCCAAAGCAGCCTCTCG | 4552 |
| 20 | H55_12-31 | GAGAGGCTGCTTTGGAAGAA | 3761 | TTCTTCCAAAGCAGCCTCTC | 4553 |
| 20 | H55_13-32 | AGAGGCTGCTTTGGAAGAAA | 3762 | TTTCTTCCAAAGCAGCCTCT | 4554 |
| 20 | H55_14-33 | GAGGCTGCTTTGGAAGAAAC | 3763 | GTTTCTTCCAAAGCAGCCTC | 4555 |
| 20 | H55_15-34 | AGGCTGCTTTGGAAGAAACT | 3764 | AGTTTCTTCCAAAGCAGCCT | 4556 |
| 20 | H55_16-35 | GGCTGCTTTGGAAGAAACTC | 3765 | GAGTTTCTTCCAAAGCAGCC | 4557 |
| 20 | H55_17-36 | GCTGCTTTGGAAGAAACTCA | 3766 | TGAGTTTCTTCCAAAGCAGC | 4558 |

**[Table 6-12]**

| | | | | | |
|---|---|---|---|---|---|
| 20 | H55_18-37 | CTGCTTTGGAAGAAACTCAT | 3767 | ATGAGTTTCTTCCAAAGCAG | 4559 |
| 20 | H55_19-38 | TGCTTTGGAAGAAACTCATA | 3768 | TATGAGTTTCTTCCAAAGCA | 4560 |
| 20 | H55_20-39 | GCTTTGGAAGAAACTCATAG | 3769 | CTATGAGTTTCTTCCAAAGC | 4561 |
| 20 | H55_21-40 | CTTTGGAAGAAACTCATAGA | 3770 | TCTATGAGTTTCTTCCAAAG | 4562 |
| 20 | H55_22-41 | TTTGGAAGAAACTCATAGAT | 3771 | ATCTATGAGTTTCTTCCAAA | 4563 |
| 20 | H55_23-42 | TTGGAAGAAACTCATAGATT | 3772 | AATCTATGAGTTTCTTCCAA | 4564 |
| 20 | H55_24-43 | TGGAAGAAACTCATAGATTA | 3773 | TAATCTATGAGTTTCTTCCA | 4565 |
| 21 | H55_(-24)-(-4) | ATCTGAACATTTGGTCCTTTG | 3774 | CAAAGGACCAAATGTTCAGAT | 4566 |
| 21 | H55_(-23)-(-3) | TCTGAACATTTGGTCCTTTGC | 3775 | GCAAAGGACCAAATGTTCAGA | 4567 |
| 21 | H55_(-22)-(-2) | CTGAACATTTGGTCCTTTGCA | 3776 | TGCAAAGGACCAAATGTTCAG | 4568 |
| 21 | H55_(-21)-(-1) | TGAACATTTGGTCCTTTGCAG | 3777 | CTGCAAAGGACCAAATGTTCA | 4569 |
| 21 | H55_(-20)-1 | GAACATTTGGTCCTTTGCAGG | 3778 | CCTGCAAAGGACCAAATGTTC | 4570 |
| 21 | H55_(-19)-2 | AACATTTGGTCCTTTGCAGGG | 3779 | CCCTGCAAAGGACCAAATGTT | 4571 |
| 21 | H55_(-18)-3 | ACATTTGGTCCTTTGCAGGGT | 3780 | ACCCTGCAAAGGACCAAATGT | 4572 |
| 21 | H55_(-17)-4 | CATTTGGTCCTTTGCAGGGTG | 3781 | CACCCTGCAAAGGACCAAATG | 4573 |
| 21 | H55_(-16)-5 | ATTTGGTCCTTTGCAGGGTGA | 3782 | TCACCCTGCAAAGGACCAAAT | 4574 |
| 21 | H55_(-15)-6 | TTTGGTCCTTTGCAGGGTGAG | 3783 | CTCACCCTGCAAAGGACCAAA | 4575 |
| 21 | H55_(-14)-7 | TTGGTCCTTTGCAGGGTGAGT | 3784 | ACTCACCCTGCAAAGGACCAA | 4576 |
| 21 | H55_(-13)-8 | TGGTCCTTTGCAGGGTGAGTG | 3785 | CACTCACCCTGCAAAGGACCA | 4577 |
| 21 | H55_(-12)-9 | GGTCCTTTGCAGGGTGAGTGA | 3786 | TCACTCACCCTGCAAAGGACC | 4578 |
| 21 | H55_(-11)-10 | GTCCTTTGCAGGGTGAGTGAG | 3787 | CTCACTCACCCTGCAAAGGAC | 4579 |

**[Table 6-13]**

| | | | | | |
|---|---|---|---|---|---|
| 21 | H55_(-10)-11 | TCCTTTGCAGGGTGAGTGAGC | 3788 | GCTCACTCACCCTGCAAAGGA | 4580 |
| 21 | H55_(-9)-12 | CCTTTGCAGGGTGAGTGAGCG | 3789 | CGCTCACTCACCCTGCAAAGG | 4581 |
| 21 | H55_(-8)-13 | CTTTGCAGGGTGAGTGAGCGA | 3790 | TCGCTCACTCACCCTGCAAAG | 4582 |
| 21 | H55_(-7)-14 | TTTGCAGGGTGAGTGAGCGAG | 3791 | CTCGCTCACTCACCCTGCAAA | 4583 |
| 21 | H55_(-6)-15 | TTGCAGGGTGAGTGAGCGAGA | 3792 | TCTCGCTCACTCACCCTGCAA | 4584 |
| 21 | H55_(-5)-16 | TGCAGGGTGAGTGAGCGAGAG | 3793 | CTCTCGCTCACTCACCCTGCA | 4585 |
| 21 | H55_(-4)-17 | GCAGGGTGAGTGAGCGAGAGG | 3794 | CCTCTCGCTCACTCACCCTGC | 4586 |
| 21 | H55_(-3)-18 | CAGGGTGAGTGAGCGAGAGGC | 3795 | GCCTCTCGCTCACTCACCCTG | 4587 |
| 21 | H55_(-2)-19 | AGGGTGAGTGAGCGAGAGGCT | 3796 | AGCCTCTCGCTCACTCACCCT | 4588 |
| 21 | H55_(-1)-20 | GGGTGAGTGAGCGAGAGGCTG | 3797 | CAGCCTCTCGCTCACTCACCC | 4589 |
| 21 | H55_1-21 | GGTGAGTGAGCGAGAGGCTGC | 3798 | GCAGCCTCTCGCTCACTCACC | 4590 |
| 21 | H55_2-22 | GTGAGTGAGCGAGAGGCTGCT | 3799 | AGCAGCCTCTCGCTCACTCAC | 4591 |
| 21 | H55_3-23 | TGAGTGAGCGAGAGGCTGCTT | 3800 | AAGCAGCCTCTCGCTCACTCA | 4592 |
| 21 | H55_4-24 | GAGTGAGCGAGAGGCTGCTTT | 3801 | AAAGCAGCCTCTCGCTCACTC | 4593 |
| 21 | H55_5-25 | AGTGAGCGAGAGGCTGCTTTG | 3802 | CAAAGCAGCCTCTCGCTCACT | 4594 |
| 21 | H55_6-26 | GTGAGCGAGAGGCTGCTTTGG | 3803 | CCAAAGCAGCCTCTCGCTCAC | 4595 |
| 21 | H55_7-27 | TGAGCGAGAGGCTGCTTTGGA | 3804 | TCCAAAGCAGCCTCTCGCTCA | 4596 |
| 21 | H55_8-28 | GAGCGAGAGGCTGCTTTGGAA | 3805 | TTCCAAAGCAGCCTCTCGCTC | 4597 |
| 21 | H55_9-29 | AGCGAGAGGCTGCTTTGGAAG | 3806 | CTTCCAAAGCAGCCTCTCGCT | 4598 |
| 21 | H55_10-30 | GCGAGAGGCTGCTTTGGAAGA | 3807 | TCTTCCAAAGCAGCCTCTCGC | 4599 |
| 21 | H55_11-31 | CGAGAGGCTGCTTTGGAAGAA | 3808 | TTCTTCCAAAGCAGCCTCTCG | 4600 |
| 21 | H55_12-32 | GAGAGGCTGCTTTGGAAGAAA | 3809 | TTTCTTCCAAAGCAGCCTCTC | 4601 |
| 21 | H55_13-33 | AGAGGCTGCTTTGGAAGAAAC | 3810 | GTTTCTTCCAAAGCAGCCTCT | 4602 |
| 21 | H55_14-34 | GAGGCTGCTTTGGAAGAAACT | 3811 | AGTTTCTTCCAAAGCAGCCTC | 4603 |
| 21 | H55_15-35 | AGGCTGCTTTGGAAGAAACTC | 3812 | GAGTTTCTTCCAAAGCAGCCT | 4604 |

**[Table 6-14]**

| | | | | | |
|---|---|---|---|---|---|
| 21 | H55_16-36 | GGCTGCTTTGGAAGAAACTCA | 3813 | TGAGTTTCTTCCAAAGCAGCC | 4605 |
| 21 | H55_17-37 | GCTGCTTTGGAAGAAACTCAT | 3814 | ATGAGTTTCTTCCAAAGCAGC | 4606 |
| 21 | H55_18-38 | CTGCTTTGGAAGAAACTCATA | 3816 | TATGAGTTTCTTCCAAAGCAG | 4607 |
| 21 | H55_19-39 | TGCTTTGGAAGAAACTCATAG | 3816 | CTATGAGTTTCTTCCAAAGCA | 4608 |
| 21 | H55_20-40 | GCTTTGGAAGAAACTCATAGA | 3817 | TCTATGAGTTTCTTCCAAAGC | 4609 |
| 21 | H55_21-41 | CTTTGGAAGAAACTCATAGAT | 3818 | ATCTATGAGTTTCTTCCAAAG | 4610 |
| 21 | H55_22-42 | TTTGGAAGAAACTCATAGATT | 3819 | AATCTATGAGTTTCTTCCAAA | 4611 |
| 21 | H55_23-43 | TTGGAAGAAACTCATAGATTA | 3820 | TAATCTATGAGTTTCTTCCAA | 4612 |
| 21 | H55_24-44 | TGGAAGAAACTCATAGATTAC | 3821 | GTAATCTATGAGTTTCTTCCA | 4613 |
| 22 | H55_(-25)-(-4) | CATCTGAACATTTGGTCCTTT G | 3822 | CAAAGGACCAAATGTTCAGAT G | 4614 |
| 22 | H55_(-24)-(-3) | ATCTGAACATTTGGTCCTTTG C | 3823 | GCAAAGGACCAAATGTTCAGA T | 4615 |
| 22 | H55_(-23)-(-2) | TCTGAACATTTGGTCCTTTGC A | 3824 | TGCAAAGGACCAAATGTTCAG A | 4616 |
| 22 | H55_(-22)-(-1) | CTGAACATTTGGTCCTTTGCA G | 3825 | CTGCAAAGGACCAAATGTTCA G | 4617 |
| 22 | H55_(-21)-1 | TGAACATTTGGTCCTTTGCAG G | 3826 | CCTGCAAAGGACCAAATGTTC A | 4618 |
| 22 | H55_(-20)-2 | GAACATTTGGTCCTTTGCAGG G | 3827 | CCCTGCAAAGGACCAAATGTT C | 4619 |
| 22 | H55_(-19)-3 | AACATTTGGTCCTTTGCAGGG T | 3828 | ACCCTGCAAAGGACCAAATGT T | 4620 |
| 22 | H55_(-18)-4 | ACATTTGGTCCTTTGCAGGGT G | 3829 | CACCCTGCAAAGGACCAAATG T | 4621 |
| 22 | H55_(-17)-5 | CATTTGGTCCTTTGCAGGGTG A | 3830 | TCACCCTGCAAAGGACCAAAT G | 4622 |
| 22 | H55_(-16)-6 | ATTTGGTCCTTTGCAGGGTGA G | 3831 | CTCACCCTGCAAAGGACCAAA T | 4623 |
| 22 | H55_(-15)-7 | TTTGGTCCTTTGCAGGGTGAG T | 3832 | ACTCACCCTGCAAAGGACCAA A | 4624 |
| 22 | H55_(-14)-8 | TTGGTCCTTTGCAGGGTGAGT G | 3833 | CACTCACCCTGCAAAGGACCA A | 4625 |
| 22 | H55_(-13)-9 | TGGTCCTTTGCAGGGTGAGTG A | 3834 | TCACTCACCCTGCAAAGGACC A | 4626 |

**[Table 6-15]**

| | | | | | |
|---|---|---|---|---|---|
| 22 | H55_(-12)-10 | GGTCCTTTGCAGGGTGAGTGA G | 3835 | CTCACTCACCCTGCAAAGGAC C | 4627 |
| 22 | H55_(-11)-11 | GTCCTTTGCAGGGTGAGTGAG C | 3836 | GCTCACTCACCCTGCAAAGGA C | 4628 |
| 22 | H55_(-10)-12 | TCCTTTGCAGGGTGAGTGAGC G | 3837 | CGCTCACTCACCCTGCAAAGG A | 4629 |
| 22 | H55_(-9)-13 | CCTTTGCAGGGTGAGTGAGCG A | 3838 | TCGCTCACTCACCCTGCAAAG G | 4630 |
| 22 | H55_(-8)-14 | CTTTGCAGGGTGAGTGAGCGA G | 3839 | CTCGCTCACTCACCCTGCAAA G | 4631 |
| 22 | H55_(-7)-15 | TTTGCAGGGTGAGTGAGCGAG A | 3840 | TCTCGCTCACTCACCCTGCAA A | 4632 |
| 22 | H55_(-6)- 16 | TTGCAGGGTGAGTGAGCGAGA G | 3841 | CTCTCGCTCACTCACCCTGCA A | 4633 |
| 22 | H55_(-5)-17 | TGCAGGGTGAGTGAGCGAGAG G | 3842 | CCTCTCGCTCACTCACCCTGC A | 4634 |
| 22 | H55_(-4)-18 | GCAGGGTGAGTGAGCGAGAGG C | 3843 | GCCTCTCGCTCACTCACCCTG C | 4635 |
| 22 | H55_(-3)-19 | CAGGGTGAGTGAGCGAGAGGC T | 3844 | AGCCTCTCGCTCACTCACCCT G | 4636 |
| 22 | H55_(-2)-20 | AGGGTGAGTGAGCGAGAGGCT G | 3845 | CAGCCTCTCGCTCACTCACCC T | 4637 |
| 22 | H55_(-1)-21 | GGGTGAGTGAGCGAGAGGCTG C | 3846 | GCAGCCTCTCGCTCACTCACC C | 4638 |
| 22 | H55_1-22 | GGTGAGTGAGCGAGAGGCTGC T | 3847 | AGCAGCCTCTCGCTCACTCAC C | 4639 |
| 22 | H55_2-23 | GTGAGTGAGCGAGAGGCTGCT T | 3848 | AAGCAGCCTCTCGCTCACTCA C | 4640 |
| 22 | H55_3-24 | TGAGTGAGCGAGAGGCTGCTT T | 3849 | AAAGCAGCCTCTCGCTCACTC A | 4641 |
| 22 | H55_4-25 | GAGTGAGCGAGAGGCTGCTTT G | 3850 | CAAAGCAGCCTCTCGCTCACT C | 4642 |
| 22 | H55_5-26 | AGTGAGCGAGAGGCTGCTTTG G | 3851 | CCAAAGCAGCCTCTCGCTCAC T | 4643 |
| 22 | II55_6-27 | GTGAGCGAGAGGCTGCTTTGG A | 3852 | TCCAAAGCAGCCTCTCGCTCA C | 4644 |

**[Table 6-16]**

| | | | | | |
|---|---|---|---|---|---|
| 22 | H55_7-28 | TGAGCGAGAGGCTGCTTTGGA A | 3853 | TTCCAAAGCAGCCTCTCGCTC A | 4645 |
| 22 | H55_8-29 | GAGCGAGAGGCTGCTTTGGAA G | 3854 | CTTCCAAAGCAGCCTCTCGCT C | 4646 |
| 22 | H55_9-30 | AGCGAGAGGCTGCTTTGGAAG A | 3855 | TCTTCCAAAGCAGCCTCTCGC T | 4647 |
| 22 | H55_10-31 | GCGAGAGGCTGCTTTGGAAGA A | 3856 | TTCTTCCAAAGCAGCCTCTCG C | 4648 |
| 22 | H55_11-32 | CGAGAGGCTGCTTTGGAAGAA A | 3857 | TTTCTTCCAAAGCAGCCTCTC G | 4649 |
| 22 | H55_12-33 | GAGAGGCTGCTTTGGAAGAAA C | 3858 | GTTTCTTCCAAAGCAGCCTCT C | 4650 |
| 22 | H55_13-34 | AGAGGCTGCTTTGGAAGAAAC T | 3859 | AGTTTCTTCCAAAGCAGCCTC T | 4651 |
| 22 | H55_14-35 | GAGGCTGCTTTGGAAGAAACT C | 3860 | GAGTTTCTTCCAAAGCAGCCT C | 4652 |
| 22 | H55_15-36 | AGGCTGCTTTGGAAGAAACTC A | 3861 | TGAGTTTCTTCCAAAGCAGCC T | 4653 |
| 22 | H55_16-37 | GGCTGCTTTGGAAGAAACTCA T | 3862 | ATGAGTTTCTTCCAAAGCAGC C | 4654 |
| 22 | H55_17-38 | GCTGCTTTGGAAGAAACTCAT A | 3863 | TATGAGTTTCTTCCAAAGCAG C | 4655 |
| 22 | H55_18-39 | CTGCTTTGGAAGAAACTCATA G | 3864 | CTATGAGTTTCTTCCAAAGCA G | 4656 |
| 22 | H55_19-40 | TGCTTTGGAAGAAACTCATAG A | 3865 | TCTATGAGTTTCTTCCAAAGC A | 4657 |
| 22 | H55_20-41 | GCTTTGGAAGAAACTCATAGA T | 3866 | ATCTATGAGTTTCTTCCAAAG C | 4658 |
| 22 | H55_21-42 | CTTTGGAAGAAACTCATAGAT T | 3867 | AATCTATGAGTTTCTTCCAAA G | 4659 |
| 22 | H55_22-43 | TTTGGAAGAAACTCATAGATT A | 3868 | TAATCTATGAGTTTCTTCCAA A | 4660 |
| 22 | H55_23-44 | TTGGAAGAAACTCATAGATTA C | 3869 | GTAATCTATGAGTTTCTTCCA A | 4661 |
| 22 | H55_24-45 | TGGAAGAAACTCATAGATTAC T | 3870 | AGTAATCTATGAGTTTCTTCC A | 4662 |

**[Table 6-17]**

| | | | | | |
|---|---|---|---|---|---|
| 23 | H55_(-26)-(-4) | GCATCTGAACATTTGGTCCTT TG | 3871 | CAAAGGACCAAATGTTCAGAT GC | 4663 |
| 23 | H55_(-25)-(-3) | CATCTGAACATTTGGTCCTTT GC | 3872 | GCAAAGGACCAAATGTTCAGA TG | 4664 |
| 23 | H55_(-24)-(-2) | ATCTGAACATTTGGTCCTTTG CA | 3873 | TGCAAAGGACCAAATGTTCAG AT | 4665 |
| 23 | H55_(-23)-(-1) | TCTGAACATTTGGTCCTTTGC AG | 3874 | CTGCAAAGGACCAAATGTTCA GA | 4666 |
| 23 | H55_(-22)-1 | CTGAACATTTGGTCCTTTGCA GG | 3875 | CCTGCAAAGGACCAAATGTTC AG | 4667 |
| 23 | H55_(-21)-2 | TGAACATTTGGTCCTTTGCAG GG | 3876 | CCCTGCAAAGGACCAAATGTT CA | 4668 |
| 23 | H55_(-20)-3 | GAACATTTGGTCCTTTGCAGG GT | 3877 | ACCCTGCAAAGGACCAAATGT TC | 4669 |
| 23 | H55_(-19)-4 | AACATTTGGTCCTTTGCAGGG TG | 3878 | CACCCTGCAAAGGACCAAATG TT | 4670 |
| 23 | H55_(-18)-5 | ACATTTGGTCCTTTGCAGGGT GA | 3879 | TCACCCTGCAAAGGACCAAAT GT | 4671 |
| 23 | H55_(-17)-6 | CATTTGGTCCTTTGCAGGGTG AG | 3880 | CTCACCCTGCAAAGGACCAAA TG | 4672 |
| 23 | H55_(-16) -7 | ATTTGGTCCTTTGCAGGGTGA GT | 3881 | ACTCACCCTGCAAAGGACCAA AT | 4673 |
| 23 | H55_(-15)-8 | TTTGGTCCTTTGCAGGGTGAG TG | 3882 | CACTCACCCTGCAAAGGACCA AA | 4674 |
| 23 | H55_(-14)-9 | TTGGTCCTTTGCAGGGTGAGT GA | 3883 | TCACTCACCCTGCAAAGGACC AA | 4675 |
| 23 | H55_(-13)-10 | TGGTCCTTTGCAGGGTGAGTG AG | 3884 | CTCACTCACCCTGCAAAGGAC CA | 4676 |
| 23 | H55_(-12)-11 | GGTCCTTTGCAGGGTGAGTGA GC | 3885 | GCTCACTCACCCTGCAAAGGA CC | 4677 |
| 23 | H55_(-11)-12 | GTCCTTTGCAGGGTGAGTGAG CG | 3886 | CGCTCACTCACCCTGCAAAGG AC | 4678 |
| 23 | H55_(-10)-13 | TCCTTTGCAGGGTGAGTGAGC GA | 3887 | TCGCTCACTCACCCTGCAAAG GA | 4679 |
| 23 | H55_(-9)-14 | CCTTTGCAGGGTGAGTGAGCG AG | 3888 | CTCGCTCACTCACCCTGCAAA GG | 4680 |

**[Table 6-18]**

| | | | | | |
|---|---|---|---|---|---|
| 23 | H55_(-8)-15 | CTTTGCAGGGTGAGTGAGCGA GA | 3889 | TCTCGCTCACTCACCCTGCAA AG | 4681 |
| 23 | H55_(-7)-16 | TTTGCAGGGTGAGTGAGCGAG AG | 3890 | CTCTCGCTCACTCACCCTGCA AA | 4682 |
| 23 | H55_(-6)-17 | TTGCAGGGTGAGTGAGCGAGA GG | 3891 | CCTCTCGCTCACTCACCCTGC AA | 4683 |
| 23 | H55_(-5)-18 | TGCAGGGTGAGTGAGCGAGAG GC | 3892 | GCCTCTCGCTCACTCACCCTG CA | 4684 |
| 23 | H55_(-4)-19 | GCAGGGTGAGTGAGCGAGAGG CT | 3893 | AGCCTCTCGCTCACTCACCCT GC | 4685 |
| 23 | H55_(-3)-20 | CAGGGTGAGTGAGCGAGAGGC TG | 3894 | CAGCCTCTCGCTCACTCACCC TG | 4686 |
| 23 | H55_(-2)-21 | AGGGTGAGTGAGCGAGAGGCT GC | 3895 | GCAGCCTCTCGCTCACTCACC CT | 4687 |
| 23 | H55_(-1)-22 | GGGTGAGTGAGCGAGAGGCTG CT | 3896 | AGCAGCCTCTCGCTCACTCAC CC | 4688 |
| 23 | H55_1-23 | GGTGAGTGAGCGAGAGGCTGC TT | 3897 | AAGCAGCCTCTCGCTCACTCA CC | 4689 |
| 23 | H55_2-24 | GTGAGTGAGCGAGAGGCTGCT TT | 3898 | AAAGCAGCCTCTCGCTCACTC AC | 4690 |
| 23 | H55_3-25 | TGAGTGAGCGAGAGGCTGCTT TG | 3899 | CAAAGCAGCCTCTCGCTCACT CA | 4691 |
| 23 | H55_4-26 | GAGTGAGCGAGAGGCTGCTTT GG | 3900 | CCAAAGCAGCCTCTCGCTCAC TC | 4692 |
| 23 | H55_5-27 | AGTGAGCGAGAGGCTGCTTTG GA | 3901 | TCCAAAGCAGCCTCTCGCTCA CT | 4693 |
| 23 | H55_6-28 | GTGAGCGAGAGGCTGCTTTGG AA | 3902 | TTCCAAAGCAGCCTCTCGCTC AC | 4694 |
| 23 | H55_7-29 | TGAGCGAGAGGCTGCTTTGGA AG | 3903 | CTTCCAAAGCAGCCTCTCGCT CA | 4695 |
| 23 | H55_8-30 | GAGCGAGAGGCTGCTTTGGAA GA | 3904 | TCTTCCAAAGCAGCCTCTCGC TC | 4696 |
| 23 | H55_9-31 | AGCGAGAGGCTGCTTTGGAAG AA | 3905 | TTCTTCCAAAGCAGCCTCTCG CT | 4697 |
| 23 | H55_10-32 | GCGAGAGGCTGCTTTGGAAGA AA | 3906 | TTTCTTCCAAAGCAGCCTCTC GC | 4698 |

**[Table 6-19]**

| | | | | | |
|---|---|---|---|---|---|
| 23 | H55_11-33 | CGAGAGGCTGCTTTGGAAGAA AC | 3907 | GTTTCTTCCAAAGCAGCCTCT CG | 1699 |
| 23 | H55_12-34 | GAGAGGCTGCTTTGGAAGAAA CT | 3908 | AGTTTCTTCCAAAGCAGCCTC TC | 4700 |
| 23 | H55_13-35 | AGAGGCTGCTTTGGAAGAAAC TC | 3909 | GAGTTTCTTCCAAAGCAGCCT CT | 4701 |
| 23 | H55_14-36 | GAGGCTGCTTTGGAAGAAACT CA | 3910 | TGAGTTTCTTCCAAAGCAGCC TC | 4702 |
| 23 | H55_15-37 | AGGCTGCTTTGGAAGAAACTC AT | 3911 | ATGAGTTTCTTCCAAAGCAGC CT | 4703 |
| 23 | H55_16-38 | GGCTGCTTTGGAAGAAACTCA TA | 3912 | TATGAGTTTCTTCCAAAGCAG CC | 4704 |
| 23 | H55_17-39 | GCTGCTTTGGAAGAAACTCAT AG | 3913 | CTATGAGTTTCTTCCAAAGCA GC | 4705 |
| 23 | H55_18-40 | CTGCTTTGGAAGAAACTCATA GA | 3914 | TCTATGAGTTTCTTCCAAAGC AG | 4706 |
| 23 | H55_19-41 | TGCTTTGGAAGAAACTCATAG AT | 3915 | ATCTATGAGTTTCTTCCAAAG CA | 4707 |
| 23 | H55_20-42 | GCTTTGGAAGAAACTCATAGA TT | 3916 | AATCTATGAGTTTCTTCCAAA GC | 4708 |
| 23 | H55_21-43 | CTTTGGAAGAAACTCATAGAT TA | 3917 | | 4709 |
| 23 | H55_22-44 | TTTGGAAGAAACTCATAGATT AC | 3918 | GTAATCTATGAGTTTCTTCCA AA | 4710 |
| 23 | H55_23-45 | TTGGAAGAAACTCATAGATTA CT | 3919 | AGTAATCTATGAGTTTCTTCC AA | 4711 |
| 23 | H55_24-46 | TGGAAGAAACTCATAGATTAC TG | 3920 | CAGTAATCTATGAGTTTCTTC CA | 4712 |
| 24 | H55_(-27)-(-4) | TGCATCTGAACATTTGGTCCT TTG | 3921 | CAAAGGACCAAATGTTCAGAT GCA | 4713 |
| 24 | H55_(-26)-(-3) | GCATCTGAACATTTGGTCCTT TGC | 3922 | GCAAAGGACCAAATGTTCAGA TGC | 4714 |
| 24 | H55_(-25)-(-2) | CATCTGAACATTTGGTCCTTT GCA | 3923 | TGCAAAGGACCAAATGTTCAG ATG | 4715 |
| 24 | H55_(-24)-(-1) | ATCTGAACATTTGGTCCTTTG CAG | 3924 | CTGCAAAGGACCAAATGTTCA GAT | 4716 |

**[Table 6-20]**

| | | | | | |
|---|---|---|---|---|---|
| 24 | H55_(-23)-1 | TCTGAACATTTGGTCCTTTGC AGG | 3925 | CCTGCAAAGGACCAAATGTTC AGA | 4717 |
| 24 | H55_(-22)-2 | CTGAACATTTGGTCCTTTGCA GGG | 3926 | CCCTGCAAAGGACCAAATGTT CAG | 4718 |
| 24 | H55_(-21)-3 | TGAACATTTGGTCCTTTGCAG GGT | 3927 | ACCCTGCAAAGGACCAAATGT TCA | 4719 |
| 24 | H55_(-20)-4 | GAACATTTGGTCCTTTGCAGG GTG | 3928 | CACCCTGCAAAGGACCAAATG TTC | 4720 |
| 24 | H55_(-19)-5 | AACATTTGGTCCTTTGCAGGG TGA | 3929 | TCACCCTGCAAAGGACCAAAT GTT | 4721 |
| 24 | H55_(-18)-6 | ACATTTGGTCCTTTGCAGGGT GAG | 3930 | CTCACCCTGCAAAGGACCAAA TGT | 4722 |
| 24 | H55_(-17)-7 | CATTTGGTCCTTTGCAGGGTG AGT | 3931 | ACTCACCCTGCAAAGGACCAA ATG | 4723 |
| 24 | H55_(-16)-8 | ATTTGGTCCTTTGCAGGGTGA GTG | 3932 | CACTCACCCTGCAAAGGACCA AAT | 4724 |
| 24 | H55_(-15)-9 | TTTGGTCCTTTGCAGGGTGAG TGA | 3933 | TCACTCACCCTGCAAAGGACC AAA | 4725 |
| 24 | H55_(-14)-10 | TTGGTCCTTTGCAGGGTGAGT GAG | 3934 | CTCACTCACCCTGCAAAGGAC CAA | 4726 |
| 24 | H55_(-13)-11 | TGGTCCTTTGCAGGGTGAGTG AGC | 3935 | GCTCACTCACCCTGCAAAGGA CCA | 4727 |
| 24 | H55_(-12)-12 | GGTCCTTTGCAGGGTGAGTGA GCG | 3936 | CGCTCACTCACCCTGCAAAGG ACC | 4728 |
| 24 | H55_(-11)-13 | GTCCTTTGCAGGGTGAGTGAG CGA | 3937 | TCGCTCACTCACCCTGCAAAG GAC | 4729 |
| 24 | H55_(-10)-14 | TCCTTTGCAGGGTGAGTGAGC GAG | 3938 | CTCGCTCACTCACCCTGCAAA GGA | 4730 |
| 24 | H55_(-9)-15 | CCTTTGCAGGGTGAGTGAGCG AGA | 3939 | TCTCGCTCACTCACCCTGCAA AGG | 4731 |
| 24 | H55_(-8)- 16 | CTTTGCAGGGTGAGTGAGCGA GAG | 3940 | CTCTCGCTCACTCACCCTGCA AAG | 4732 |
| 24 | H55_(-7)-17 | TTTGCAGGGTGAGTGAGCGAG AGG | 3941 | CCTCTCGCTCACTCACCCTGC AAA | 4733 |
| 24 | H55_(-6)- 18 | TTGCAGGGTGAGTGAGCGAGA GGC | 3942 | GCCTCTCGCTCACTCACCCTG CAA | 4734 |

**[Table 6-21]**

| | | | | | |
|---|---|---|---|---|---|
| 24 | H55_(-5)-19 | TGCAGGGTGAGTGAGCGAGAG GCT | 3943 | AGCCTCTCGCTCACTCACCCT GCA | 4735 |
| 24 | H55_(-4)-20 | GCAGGGTGAGTGAGCGAGAGG CTG | 3944 | CAGCCTCTCGCTCACTCACCC TGC | 4736 |
| 24 | H55_(-3)-21 | CAGGGTGAGTGAGCGAGAGGC TGC | 3945 | GCAGCCTCTCGCTCACTCACC CTG | 4737 |
| 24 | H55_(-2)-22 | AGGGTGAGTGAGCGAGAGGCT GCT | 3946 | AGCAGCCTCTCGCTCACTCAC CCT | 4738 |
| 24 | H55_(-1)-23 | GGGTGAGTGAGCGAGAGGCTG CTT | 3947 | AAGCAGCCTCTCGCTCACTCA CCC | 4739 |
| 24 | H55_1-24 | GGTGAGTGAGCGAGAGGCTGC TTT | 3948 | AAAGCAGCCTCTCGCTCACTC ACC | 4740 |
| 24 | H55_2-25 | GTGAGTGAGCGAGAGGCTGCT TTG | 3949 | CAAAGCAGCCTCTCGCTCACT CAC | 4741 |
| 24 | H55_3-26 | TGAGTGAGCGAGAGGCTGCTT TGG | 3950 | CCAAAGCAGCCTCTCGCTCAC TCA | 4742 |
| 24 | H55_4-27 | GAGTGAGCGAGAGGCTGCTTT GGA | 3951 | TCCAAAGCAGCCTCTCGCTCA CTC | 4743 |
| 24 | H55_5-28 | AGTGAGCGAGAGGCTGCTTTG GAA | 3952 | TTCCAAAGCAGCCTCTCGCTC ACT | 4744 |
| 24 | H55_6-29 | GTGAGCGAGAGGCTGCTTTGG AAG | 3953 | CTTCCAAAGCAGCCTCTCGCT CAC | 4745 |
| 24 | H55_7-30 | TGAGCGAGAGGCTGCTTTGGA AGA | 3954 | TCTTCCAAAGCAGCCTCTCGC TCA | 4746 |
| 24 | H55_8-31 | GAGCGAGAGGCTGCTTTGGAA GAA | 3955 | TTCTTCCAAAGCAGCCTCTCG CTC | 4747 |
| 24 | H55_9-32 | AGCGAGAGGCTGCTTTGGAAG AAA | 3956 | TTTCTTCCAAAGCAGCCTCTC GCT | 4748 |
| 24 | H55_10-33 | GCGAGAGGCTGCTTTGGAAGA AAC | 3957 | GTTTCTTCCAAAGCAGCCTCT CGC | 4749 |
| 24 | H55_11-34 | CGAGAGGCTGCTTTGGAAGAA ACT | 3958 | AGTTTCTTCCAAAGCAGCCTC TCG | 4750 |
| 24 | H55_12-35 | GAGAGGCTGCTTTGGAAGAAA CTC | 3959 | GAGTTTCTTCCAAAGCAGCCT CTC | 4751 |
| 24 | H55_13-36 | AGAGGCTGCTTTGGAAGAAAC TCA | 3960 | TGAGTTTCTTCCAAAGCAGCC TCT | 4752 |

**[Table 6-22]**

| | | | | | |
|---|---|---|---|---|---|
| 24 | H55_14-37 | GAGGCTGCTTTGGAAGAAACT CAT | 3961 | ATGAGTTTCTTCCAAAGCAGC CTC | 4753 |
| 24 | H55_15-38 | AGGCTGCTTTGGAAGAAACTC ATA | 3962 | TATGAGTTTCTTCCAAAGCAG CCT | 4754 |
| 24 | H55_16-39 | GGCTGCTTTGGAAGAAACTCA TAG | 3963 | CTATGAGTTTCTTCCAAAGCA GCC | 4755 |
| 24 | H55_17-40 | GCTGCTTTGGAAGAAACTCAT AGA | 3964 | TCTATGAGTTTCTTCCAAAGC AGC | 4756 |
| 24 | H55_18-41 | CTGCTTTGGAAGAAACTCATA GAT | 3965 | ATCTATGAGTTTCTTCCAAAG CAG | 4757 |
| 24 | H55_19-42 | TGCTTTGGAAGAAACTCATAG ATT | 3966 | AATCTATGAGTTTCTTCCAAA GCA | 4758 |
| 24 | H55_20-43 | GCTTTGGAAGAAACTCATAGA TTA | 3967 | TAATCTATGAGTTTCTTCCAA AGC | 4759 |
| 24 | H55_21-44 | CTTTGGAAGAAACTCATAGAT TAC | 3968 | GTAATCTATGAGTTTCTTCCA AAG | 4760 |
| 24 | H55_22-45 | TTTGGAAGAAACTCATAGATT ACT | 3969 | AGTAATCTATGAGTTTCTTCC AAA | 4761 |
| 24 | H55_23-46 | TTGGAAGAAACTCATAGATTA CTG | 3970 | CAGTAATCTATGAGTTTCTTC CAA | 4762 |
| 24 | H55_24-47 | TGGAAGAAACTCATAGATTAC TGC | 3971 | GCAGTAATCTATGAGTTTCTT CCA | 4763 |
| 25 | H55_(-28)-(-4) | TTGCATCTGAACATTTGGTCC TTTG | 3972 | CAAAGGACCAAATGTTCAGAT GCAA | 4764 |
| 25 | H55_(-27)-(-3) | TGCATCTGAACATTTGGTCCT TTGC | 3973 | GCAAAGGACCAAATGTTCAGA TGCA | 4765 |
| 25 | H55_(-26)-(-2) | GCATCTGAACATTTGGTCCTT TGCA | 3974 | TGCAAAGGACCAAATGTTCAG ATGC | 4766 |
| 25 | H55_(-25)-(-1) | CATCTGAACATTTGGTCCTTT GCAG | 3975 | CTGCAAAGGACCAAATGTTCA GATG | 4767 |
| 25 | H55_(-24)-1 | ATCTGAACATTTGGTCCTTTG CAGG | 3976 | CCTGCAAAGGACCAAATGTTC AGAT | 4768 |
| 25 | H55_(-23)-2 | TCTGAACATTTGGTCCTTTGC AGGG | 3977 | CCCTGCAAAGGACCAAATGTT CAGA | 4769 |
| 25 | H55_(-22)-3 | CTGAACATTTGGTCCTTTGCA GGGT | 3978 | ACCCTGCAAAGGACCAAATGT TCAG | 4770 |

**[Table 6-23]**

| | | | | | |
|---|---|---|---|---|---|
| 25 | H55_(-21)-4 | TGAACATTTGGTCCTTTGCAG GGTG | 3979 | CACCCTGCAAAGGACCAAATG TTCA | 4771 |
| 25 | H55_(-20)-5 | GAACATTTGGTCCTTTGCAGG GTGA | 3980 | TCACCCTGCAAAGGACCAAAT GTTC | 4772 |
| 25 | H55_(-19)-6 | AACATTTGGTCCTTTGCAGGG TGAG | 3981 | CTCACCCTGCAAAGGACCAAA TGTT | 4773 |
| 25 | H55_(-18)-7 | ACATTTGGTCCTTTGCAGGGT GAGT | 3982 | ACTCACCCTGCAAAGGACCAA ATGT | 4774 |
| 25 | H55_(-17)-8 | CATTTGGTCCTTTGCAGGGTG AGTG | 3983 | CACTCACCCTGCAAAGGACCA AATG | 4775 |
| 25 | H55_(-16) -9 | ATTTGGTCCTTTGCAGGGTGA GTGA | 3984 | TCACTCACCCTGCAAAGGACC AAAT | 4776 |
| 25 | H55_(-15)-10 | TTTGGTCCTTTGCAGGGTGAG TGAG | 3985 | CTCACTCACCCTGCAAAGGAC CAAA | 4777 |
| 25 | H55_(-14)-11 | TTGGTCCTTTGCAGGGTGAGT GAGC | 3986 | GCTCACTCACCCTGCAAAGGA CCAA | 4778 |
| 25 | H55_(-13)-12 | TGGTCCTTTGCAGGGTGAGTG AGCG | 3987 | CGCTCACTCACCCTGCAAAGG ACCA | 4779 |
| 25 | H55_(-12)-13 | GGTCCTTTGCAGGGTGAGTGA GCGA | 3988 | TCGCTCACTCACCCTGCAAAG GACC | 4780 |
| 25 | H55_(-11)-14 | GTCCTTTGCAGGGTGAGTGAG CGAG | 3989 | CTCGCTCACTCACCCTGCAAA GGAC | 4781 |
| 25 | H55_(-10)-15 | TCCTTTGCAGGGTGAGTGAGC GAGA | 3990 | TCTCGCTCACTCACCCTGCAA AGGA | 4782 |
| 25 | H55_(-9)-16 | CCTTTGCAGGGTGAGTGAGCG AGAG | 3991 | CTCTCGCTCACTCACCCTGCA AAGG | 4783 |
| 25 | H55_(-8)-17 | CTTTGCAGGGTGAGTGAGCGA GAGG | 3992 | CCTCTCGCTCACTCACCCTGC AAAG | 4784 |
| 25 | H55_(-7)-18 | TTTGCAGGGTGAGTGAGCGAG AGGC | 3993 | GCCTCTCGCTCACTCACCCTG CAAA | 4785 |
| 25 | H55_(-6)-19 | TTGCAGGGTGAGTGAGCGAGA GGCT | 3994 | AGCCTCTCGCTCACTCACCCT GCAA | 4786 |
| 25 | H55_(-5)-20 | TGCAGGGTGAGTGAGCGAGAG GCTG | 3995 | CAGCCTCTCGCTCACTCACCC TGCA | 4787 |
| 25 | H55_(-4)-21 | GCAGGGTGAGTGAGCGAGAGG CTGC | 3996 | GCAGCCTCTCGCTCACTCACC CTGC | 4788 |

**[Table 6-24]**

| | | | | | |
|---|---|---|---|---|---|
| 25 | H55_(-3)-22 | CAGGGTGAGTGAGCGAGAGGC TGCT | 3997 | AGCAGCCTCTCGCTCACTCAC CCTG | 4789 |
| 25 | H55_(-2)-23 | AGGGTGAGTGAGCGAGAGGCT GCTT | 3998 | AAGCAGCCTCTCGCTCACTCA CCCT | 4790 |
| 25 | H55_(-1)-24 | GGGTGAGTGAGCGAGAGGCTG CTTT | 3999 | AAAGCAGCCTCTCGCTCACTC ACCC | 4791 |
| 25 | H55_1-25 | GGTGAGTGAGCGAGAGGCTGC TTTG | 4000 | CAAAGCAGCCTCTCGCTCACT CACC | 4792 |
| 25 | H55_2-26 | GTGAGTGAGCGAGAGGCTGCT TTGG | 4001 | CCAAAGCAGCCTCTCGCTCAC TCAC | 4793 |
| 25 | H55_3-27 | TGAGTGAGCGAGAGGCTGCTT TGGA | 4002 | TCCAAAGCAGCCTCTCGCTCA CTCA | 4794 |
| 25 | H55_4-28 | GAGTGAGCGAGAGGCTGCTTT GGAA | 4003 | TTCCAAAGCAGCCTCTCGCTC ACTC | 4795 |
| 25 | H55_5-29 | AGTGAGCGAGAGGCTGCTTTG GAAG | 4004 | CTTCCAAAGCAGCCTCTCGCT CACT | 4796 |
| 25 | H55_6-30 | GTGAGCGAGAGGCTGCTTTGG AAGA | 4005 | TCTTCCAAAGCAGCCTCTCGC TCAC | 4797 |
| 25 | H55_7-31 | TGAGCGAGAGGCTGCTTTGGA AGAA | 4006 | TTCTTCCAAAGCAGCCTCTCG CTCA | 4798 |
| 25 | H55_8-32 | GAGCGAGAGGCTGCTTTGGAA GAAA | 4007 | TTTCTTCCAAAGCAGCCTCTC GCTC | 4799 |
| 25 | H55_9-33 | AGCGAGAGGCTGCTTTGGAAG AAAC | 4008 | GTTTCTTCCAAAGCAGCCTCT CGCT | 4800 |
| 25 | H55_10-34 | GCGAGAGGCTGCTTTGGAAGA AACT | 4009 | AGTTTCTTCCAAAGCAGCCTC TCGC | 4801 |
| 25 | H55_11-35 | CGAGAGGCTGCTTTGGAAGAA ACTC | 4010 | GAGTTTCTTCCAAAGCAGCCT CTCG | 4802 |
| 25 | H55_12-36 | GAGAGGCTGCTTTGGAAGAAA CTCA | 4011 | TGAGTTTCTTCCAAAGCAGCC TCTC | 4803 |
| 25 | H55_13-37 | AGAGGCTGCTTTGGAAGAAAC TCAT | 4012 | ATGAGTTTCTTCCAAAGCAGC CTCT | 4804 |
| 25 | H55_14-38 | GAGGCTGCTTTGGAAGAAACT CATA | 4013 | TATGAGTTTCTTCCAAAGCAG CCTC | 4805 |
| 25 | H55_15-39 | AGGCTGCTTTGGAAGAAACTC ATAG | 4014 | CTATGAGTTTCTTCCAAAGCA GCCT | 4806 |

**[Table 6-25]**

| | | | | | |
|---|---|---|---|---|---|
| 25 | H55_16-40 | GGCTGCTTTGGAAGAAACTCA TAGA | 4015 | TCTATGAGTTTCTTCCAAAGC AGCC | 4807 |
| 25 | H55_17-41 | GCTGCTTTGGAAGAAACTCAT AGAT | 4016 | ATCTATGAGTTTCTTCCAAAG CAGC | 4808 |
| 25 | H55_18-42 | CTGCTTTGGAAGAAACTCATA GATT | 4017 | AATCTATGAGTTTCTTCCAAA GCAG | 4809 |
| 25 | H55_19-43 | TGCTTTGGAAGAAACTCATAG ATTA | 4018 | TAATCTATGAGTTTCTTCCAA AGCA | 4810 |
| 25 | H55_20-44 | GCTTTGGAAGAAACTCATAGA TTAC | 4019 | GTAATCTATGAGTTTCTTCCA AAGC | 4811 |
| 25 | H55_21-45 | CTTTGGAAGAAACTCATAGAT TACT | 4020 | AGTAATCTATGAGTTTCTTCC AAAG | 4812 |
| 25 | H55_22-46 | TTTGGAAGAAACTCATAGATT ACTG | 4021 | CAGTAATCTATGAGTTTCTTC CAAA | 4813 |
| 25 | H55_23-47 | TTGGAAGAAACTCATAGATTA CTGC | 4022 | GCAGTAATCTATGAGTTTCTT CCAA | 4814 |
| 25 | H55_24-48 | TGGAAGAAACTCATAGATTAC TGCA | 4023 | TGCAGTAATCTATGAGTTTCT TCCA | 4815 |
| 26 | H55_(-29)-(-4) | ATTGCATCTGAACATTTGGTC CTTTG | 4024 | CAAAGGACCAAATGTTCAGAT GCAAT | 4816 |
| 26 | H55_(-28)-(-3) | TTGCATCTGAACATTTGGTCC TTTGC | 4025 | GCAAAGGACCAAATGTTCAGA TGCAA | 4817 |
| 26 | H55_(-27)-(-2) | TGCATCTGAACATTTGGTCCT TTGCA | 4026 | TGCAAAGGACCAAATGTTCAG ATGCA | 4818 |
| 26 | H55_(-26)-(-1) | GCATCTGAACATTTGGTCCTT TGCAG | 4027 | CTGCAAAGGACCAAATGTTCA GATGC | 4819 |
| 26 | H55_(-25)-1 | CATCTGAACATTTGGTCCTTT GCAGG | 4028 | CCTGCAAAGGACCAAATGTTC AGATG | 4820 |
| 26 | H55_(-24) -2 | ATCTGAACATTTGGTCCTTTG CAGGG | 4029 | CCCTGCAAAGGACCAAATGTT CAGAT | 4821 |
| 26 | H55_(-23)-3 | TCTGAACATTTGGTCCTTTGC AGGGT | 4030 | ACCCTGCAAAGGACCAAATGT TCAGA | 4822 |
| 26 | H55_(-22)-4 | CTGAACATTTGGTCCTTTGCA GGGTG | 4031 | CACCCTGCAAAGGACCAAATG TTCAG | 4823 |
| 26 | H55_(-21)-5 | TGAACATTTGGTCCTTTGCAG GGTGA | 4032 | TCACCCTGCAAAGGACCAAAT GTTCA | 4824 |

**[Table 6-26]**

| | | | | | |
|---|---|---|---|---|---|
| 26 | H55_(-20)-6 | GAACATTTGGTCCTTTGCAGG GTGAG | 4033 | CTCACCCTGCAAAGGACCAAA TGTTC | 4825 |
| 26 | H55_(-19)-7 | AACATTTGGTCCTTTGCAGGG TGAGT | 4034 | ACTCACCCTGCAAAGGACCAA ATGTT | 4826 |
| 26 | H55_(-18)-8 | ACATTTGGTCCTTTGCAGGGT GAGTG | 4035 | CACTCACCCTGCAAAGGACCA AATGT | 4827 |
| 26 | H55_(-17)-9 | CATTTGGTCCTTTGCAGGGTG AGTGA | 4036 | TCACTCACCCTGCAAAGGACC AAATG | 4828 |
| 26 | II55_(-16)-10 | ATTTGGTCCTTTGCAGGGTGA GTGAG | 4037 | CTCACTCACCCTGCAAAGGAC CAAAT | 4829 |
| 26 | H55_(-15)-11 | TTTGGTCCTTTGCAGGGTGAG TGAGC | 4038 | GCTCACTCACCCTGCAAAGGA CCAAA | 4830 |
| 26 | H55_(-14)-12 | TTGGTCCTTTGCAGGGTGAGT GAGCG | 4039 | CGCTCACTCACCCTGCAAAGG ACCAA | 4831 |
| 26 | H55_(-13)-13 | TGGTCCTTTGCAGGGTGAGTG AGCGA | 4040 | TCGCTCACTCACCCTGCAAAG GACCA | 4832 |
| 26 | H55_(-12)-14 | GGTCCTTTGCAGGGTGAGTGA GCGAG | 4041 | CTCGCTCACTCACCCTGCAAA GGACC | 4833 |
| 26 | H55_(-11)-15 | GTCCTTTGCAGGGTGAGTGAG CGAGA | 4042 | TCTCGCTCACTCACCCTGCAA AGGAC | 4834 |
| 26 | H55_(-10)-16 | TCCTTTGCAGGGTGAGTGAGC GAGAG | 4043 | CTCTCGCTCACTCACCCTGCA AAGGA | 4835 |
| 26 | H55_(-9)- 17 | CCTTTGCAGGGTGAGTGAGCG AGAGG | 4044 | CCTCTCGCTCACTCACCCTGC AAAGG | 4836 |
| 26 | H55_(-8)-18 | CTTTGCAGGGTGAGTGAGCGA GAGGC | 4045 | GCCTCTCGCTCACTCACCCTG CAAAG | 4837 |
| 26 | H55_(-7)-19 | TTTGCAGGGTGAGTGAGCGAG AGGCT | 4046 | AGCCTCTCGCTCACTCACCCT GCAAA | 4838 |
| 26 | H55_(-6)-20 | TTGCAGGGTGAGTGAGCGAGA GGCTG | 4047 | CAGCCTCTCGCTCACTCACCC TGCAA | 4839 |
| 26 | H55_(-5)-21 | TGCAGGGTGAGTGAGCGAGAG GCTGC | 4048 | GCAGCCTCTCGCTCACTCACC CTGCA | 4840 |
| 26 | H55_(-4)-22 | GCAGGGTGAGTGAGCGAGAGG CTGCT | 4049 | AGCAGCCTCTCGCTCACTCAC CCTGC | 4841 |
| 26 | H55_(-3)-23 | CAGGGTGAGTGAGCGAGAGGC TGCTT | 4050 | AAGCAGCCTCTCGCTCACTCA CCCTG | 4842 |

**[Table 6-27]**

| | | | | | |
|---|---|---|---|---|---|
| 26 | H55_(-2)-24 | AGGGTGAGTGAGCGAGAGGCT GCTTT | 4051 | AAAGCAGCCTCTCGCTCACTC ACCCT | 4843 |
| 26 | H55_(-1)-25 | GGGTGAGTGAGCGAGAGGCTG CTTTG | 4052 | CAAAGCAGCCTCTCGCTCACT CACCC | 4844 |
| 26 | H55_1-26 | GGTGAGTGAGCGAGAGGCTGC TTTGG | 4053 | CCAAAGCAGCCTCTCGCTCAC TCACC | 4845 |
| 26 | H55_2-27 | GTGAGTGAGCGAGAGGCTGCT TTGGA | 4054 | TCCAAAGCAGCCTCTCGCTCA CTCAC | 4846 |
| 26 | H55_3-28 | TGAGTGAGCGAGAGGCTGCTT TGGAA | 4055 | TTCCAAAGCAGCCTCTCGCTC ACTCA | 4847 |
| 26 | H55_4-29 | GAGTGAGCGAGAGGCTGCTTT GGAAG | 4056 | CTTCCAAAGCAGCCTCTCGCT CACTC | 4848 |
| 26 | H55_5-30 | AGTGAGCGAGAGGCTGCTTTG GAAGA | 4057 | TCTTCCAAAGCAGCCTCTCGC TCACT | 4849 |
| 26 | H55_6-31 | GTGAGCGAGAGGCTGCTTTGG AAGAA | 4058 | TTCTTCCAAAGCAGCCTCTCG CTCAC | 4850 |
| 26 | H55_7-32 | TGAGCGAGAGGCTGCTTTGGA AGAAA | 4059 | TTTCTTCCAAAGCAGCCTCTC GCTCA | 4851 |
| 26 | H55_8-33 | GAGCGAGAGGCTGCTTTGGAA GAAAC | 4060 | GTTTCTTCCAAAGCAGCCTCT CGCTC | 4852 |
| 26 | H55_9-34 | AGCGAGAGGCTGCTTTGGAAG AAACT | 4061 | AGTTTCTTCCAAAGCAGCCTC TCGCT | 4853 |
| 26 | H55_10-35 | GCGAGAGGCTGCTTTGGAAGA AACTC | 4062 | GAGTTTCTTCCAAAGCAGCCT CTCGC | 4854 |
| 26 | H55_11-36 | CGAGAGGCTGCTTTGGAAGAA ACTCA | 4063 | TGAGTTTCTTCCAAAGCAGCC TCTCG | 4855 |
| 26 | H55_12-37 | GAGAGGCTGCTTTGGAAGAAA CTCAT | 4064 | ATGAGTTTCTTCCAAAGCAGC CTCTC | 4856 |
| 26 | H55_13-38 | AGAGGCTGCTTTGGAAGAAAC TCATA | 4065 | TATGAGTTTCTTCCAAAGCAG CCTCT | 4857 |
| 26 | H55_14-39 | GAGGCTGCTTTGGAAGAAACT CATAG | 4066 | CTATGAGTTTCTTCCAAAGCA GCCTC | 4858 |
| 26 | H55_15-40 | AGGCTGCTTTGGAAGAAACTC ATAGA | 4067 | TCTATGAGTTTCTTCCAAAGC AGCCT | 4859 |
| 26 | H55_16-41 | GGCTGCTTTGGAAGAAACTCA TAGAT | 4068 | ATCTATGAGTTTCTTCCAAAG CAGCC | 4860 |

**[Table 6-28]**

| | | | | | |
|---|---|---|---|---|---|
| 26 | H55_17-42 | GCTGCTTTGGAAGAAACTCAT AGATT | 4069 | AATCTATGAGTTTCTTCCAAA GCAGC | 4861 |
| 26 | H55_18-43 | CTGCTTTGGAAGAAACTCATA GATTA | 4070 | TAATCTATGAGTTTCTTCCAA AGCAG | 4862 |
| 26 | H55_19-44 | TGCTTTGGAAGAAACTCATAG ATTAC | 4071 | GTAATCTATGAGTTTCTTCCA AAGCA | 4863 |
| 26 | H55_20-45 | GCTTTGGAAGAAACTCATAGA TTACT | 4072 | AGTAATCTATGAGTTTCTTCC AAAGC | 4864 |
| 26 | H55_21-46 | CTTTGGAAGAAACTCATAGAT TACTG | 4073 | CAGTAATCTATGAGTTTCTTC CAAAG | 4865 |
| 26 | H55_22-47 | TTTGGAAGAAACTCATAGATT ACTGC | 4074 | GCAGTAATCTATGAGTTTCTT CCAAA | 4866 |
| 26 | H55_23-48 | TTGGAAGAAACTCATAGATTA CTGCA | 4075 | TGCAGTAATCTATGAGTTTCT TCCAA | 4867 |
| 26 | H55_24-49 | TGGAAGAAACTCATAGATTAC TGCAA | 4076 | TTGCAGTAATCTATGAGTTTC TTCCA | 4868 |
| 27 | H55_(-30)-(-4) | AATTGCATCTGAACATTTGGT CCTTTG | 4077 | CAAAGGACCAAATGTTCAGAT GCAATT | 4869 |
| 27 | H55_(-29)-(-3) | ATTGCATCTGAACATTTGGTC CTTTGC | 4078 | GCAAAGGACCAAATGTTCAGA TGCAAT | 4870 |
| 27 | H55_(-28)-(-2) | TTGCATCTGAACATTTGGTCC TTTGCA | 4079 | TGCAAAGGACCAAATGTTCAG ATGCAA | 4871 |
| 27 | H55_(-27)-(-1) | TGCATCTGAACATTTGGTCCT TTGCAG | 4080 | CTGCAAAGGACCAAATGTTCA GATGCA | 4872 |
| 27 | H55_(-26)-1 | GCATCTGAACATTTGGTCCTT TGCAGG | 4081 | CCTGCAAAGGACCAAATGTTC AGATGC | 4873 |
| 27 | H55_(-25)-2 | CATCTGAACATTTGGTCCTTT GCAGGG | 4082 | CCCTGCAAAGGACCAAATGTT CAGATG | 4874 |
| 27 | H55_(-24)-3 | ATCTGAACATTTGGTCCTTTG CAGGGT | 4083 | ACCCTGCAAAGGACCAAATGT TCAGAT | 4875 |
| 27 | H55_(-23)-4 | TCTGAACATTTGGTCCTTTGC AGGGTG | 4084 | CACCCTGCAAAGGACCAAATG TTCAGA | 4876 |
| 27 | H55_(-22)-5 | CTGAACATTTGGTCCTTTGCA GGGTGA | 4085 | TCACCCTGCAAAGGACCAAAT GTTCAG | 4877 |
| 27 | H55_(-21)-6 | TGAACATTTGGTCCTTTGCAG GGTGAG | 4086 | CTCACCCTGCAAAGGACCAAA TGTTCA | 4878 |

**[Table 6-29]**

| | | | | | |
|---|---|---|---|---|---|
| 27 | H55_(-20)-7 | GAACATTTGGTCCTTTGCAGG GTGAGT | 4087 | ACTCACCCTGCAAAGGACCAA ATGTTC | 4879 |
| 27 | H55_(-19)-8 | AACATTTGGTCCTTTGCAGGG TGAGTG | 4088 | CACTCACCCTGCAAAGGACCA AATGTT | 4880 |
| 27 | H55_(-18)-9 | ACATTTGGTCCTTTGCAGGGT GAGTGA | 4089 | TCACTCACCCTGCAAAGGACC AAATGT | 4881 |
| 27 | H55_(-17)-10 | CATTTGGTCCTTTGCAGGGTG AGTGAG | 4090 | CTCACTCACCCTGCAAAGGAC CAAATG | 4882 |
| 27 | H55_(-16)-11 | ATTTGGTCCTTTGCAGGGTGA GTGAGC | 4091 | GCTCACTCACCCTGCAAAGGA CCAAAT | 4883 |
| 27 | H55_(-15)-12 | TTTGGTCCTTTGCAGGGTGAG TGAGCG | 4092 | CGCTCACTCACCCTGCAAAGG ACCAAA | 4884 |
| 27 | H55_(-14)-13 | TTGGTCCTTTGCAGGGTGAGT GAGCGA | 4093 | TCGCTCACTCACCCTGCAAAG GACCAA | 4885 |
| 27 | H55_(-13)-14 | TGGTCCTTTGCAGGGTGAGTG AGCGAG | 4094 | CTCGCTCACTCACCCTGCAAA GGACCA | 4886 |
| 27 | H55_(-12)-15 | GGTCCTTTGCAGGGTGAGTGA GCGAGA | 4095 | TCTCGCTCACTCACCCTGCAA AGGACC | 4887 |
| 27 | H55_(-11)-16 | GTCCTTTGCAGGGTGAGTGAG CGAGAG | 4096 | CTCTCGCTCACTCACCCTGCA AAGGAC | 4888 |
| 27 | H55_(-10)-17 | TCCTTTGCAGGGTGAGTGAGC GAGAGG | 4097 | CCTCTCGCTCACTCACCCTGC AAAGGA | 4889 |
| 27 | H55_(-9)-18 | CCTTTGCAGGGTGAGTGAGCG AGAGGC | 4098 | GCCTCTCGCTCACTCACCCTG CAAAGG | 4890 |
| 27 | H55_(-8)-19 | CTTTGCAGGGTGAGTGAGCGA GAGGCT | 4099 | AGCCTCTCGCTCACTCACCCT GCAAAG | 4891 |
| 27 | H55_(-7)-20 | TTTGCAGGGTGAGTGAGCGAG AGGCTG | 4100 | CAGCCTCTCGCTCACTCACCC TGCAAA | 4892 |
| 27 | H55_(-6)-21 | TTGCAGGGTGAGTGAGCGAGA GGCTGC | 4101 | GCAGCCTCTCGCTCACTCACC CTGCAA | 4893 |
| 27 | H55_(-5)-22 | TGCAGGGTGAGTGAGCGAGAG GCTGCT | 4102 | AGCAGCCTCTCGCTCACTCAC CCTGCA | 4894 |
| 27 | H55_(-4)-23 | GCAGGGTGAGTGAGCGAGAGG CTGCTT | 4103 | AAGCAGCCTCTCGCTCACTCA CCCTGC | 4895 |
| 27 | H55_(-3)-24 | CAGGGTGAGTGAGCGAGAGGC TGCTTT | 4104 | AAAGCAGCCTCTCGCTCACTC ACCCTG | 4896 |

**[Table 6-30]**

| | | | | | |
|---|---|---|---|---|---|
| 27 | H55_(-2)-25 | AGGGTGAGTGAGCGAGAGGCT GCTTTG | 4105 | CAAAGCAGCCTCTCGCTCACT CACCCT | 4897 |
| 27 | H55_(-1)- 26 | GGGTGAGTGAGCGAGAGGCTG CTTTGG | 4106 | CCAAAGCAGCCTCTCGCTCAC TCACCC | 4898 |
| 27 | H55_1-27 | GGTGAGTGAGCGAGAGGCTGC TTTGGA | 4107 | TCCAAAGCAGCCTCTCGCTCA CTCACC | 4899 |
| 27 | H55_2-28 | GTGAGTGAGCGAGAGGCTGCT TTGGAA | 4108 | TTCCAAAGCAGCCTCTCGCTC ACTCAC | 4900 |
| 27 | H55_3-29 | TGAGTGAGCGAGAGGCTGCTT TGGAAG | 4109 | CTTCCAAAGCAGCCTCTCGCT CACTCA | 4901 |
| 27 | H55_4-30 | GAGTGAGCGAGAGGCTGCTTT GGAAGA | 4110 | TCTTCCAAAGCAGCCTCTCGC TCACTC | 4902 |
| 27 | H55_5-31 | AGTGAGCGAGAGGCTGCTTTG GAAGAA | 4111 | TTCTTCCAAAGCAGCCTCTCG CTCACT | 4903 |
| 27 | H55_6-32 | GTGAGCGAGAGGCTGCTTTGG AAGAAA | 4112 | TTTCTTCCAAAGCAGCCTCTC GCTCAC | 4904 |
| 27 | H55_7-33 | TGAGCGAGAGGCTGCTTTGGA AGAAAC | 4113 | GTTTCTTCCAAAGCAGCCTCT CGCTCA | 4905 |
| 27 | H55_8-34 | GAGCGAGAGGCTGCTTTGGAA GAAACT | 4114 | AGTTTCTTCCAAAGCAGCCTC TCGCTC | 4906 |
| 27 | H55_9-35 | AGCGAGAGGCTGCTTTGGAAG AAACTC | 4115 | GAGTTTCTTCCAAAGCAGCCT CTCGCT | 4907 |
| 27 | H55_10-36 | GCGAGAGGCTGCTTTGGAAGA AACTCA | 4116 | TGAGTTTCTTCCAAAGCAGCC TCTCGC | 4908 |
| 27 | H55_11-37 | CGAGAGGCTGCTTTGGAAGAA ACTCAT | 4117 | ATGAGTTTCTTCCAAAGCAGC CTCTCG | 4909 |
| 27 | H55_12-38 | GAGAGGCTGCTTTGGAAGAAA CTCATA | 4118 | TATGAGTTTCTTCCAAAGCAG CCTCTC | 4910 |
| 27 | H55_13-39 | AGAGGCTGCTTTGGAAGAAAC TCATAG | 4119 | CTATGAGTTTCTTCCAAAGCA GCCTCT | 4911 |
| 27 | H55_14-40 | GAGGCTGCTTTGGAAGAAACT CATAGA | 4120 | TCTATGAGTTTCTTCCAAAGC AGCCTC | 4912 |
| 27 | H55_15-41 | AGGCTGCTTTGGAAGAAACTC ATAGAT | 4121 | ATCTATGAGTTTCTTCCAAAG CAGCCT | 4913 |
| 27 | H55_16-42 | GGCTGCTTTGGAAGAAACTCA TAGATT | 4122 | AATCTATGAGTTTCTTCCAAA GCAGCC | 4914 |

**[Table 6-31]**

| | | | | | |
|---|---|---|---|---|---|
| 27 | H55_17-43 | GCTGCTTTGGAAGAAACTCAT AGATTA | 4123 | TAATCTATGAGTTTCTTCCAA AGCAGC | 4915 |
| 27 | H55_18-44 | CTGCTTTGGAAGAAACTCATA GATTAC | 4124 | GTAATCTATGAGTTTCTTCCA AAGCAG | 4916 |
| 27 | H55_19-45 | TGCTTTGGAAGAAACTCATAG ATTACT | 4125 | AGTAATCTATGAGTTTCTTCC AAAGCA | 4917 |
| 27 | H55_20-46 | GCTTTGGAAGAAACTCATAGA TTACTG | 4126 | CAGTAATCTATGAGTTTCTTC CAAAGC | 4918 |
| 27 | H55_21-47 | CTTTGGAAGAAACTCATAGAT TACTGC | 4127 | GCAGTAATCTATGAGTTTCTT CCAAAG | 4919 |
| 27 | H55_22-48 | TTTGGAAGAAACTCATAGATT ACTGCA | 4128 | TGCAGTAATCTATGAGTTTCT TCCAAA | 4920 |
| 27 | H55_23-49 | TTGGAAGAAACTCATAGATTA CTGCAA | 4129 | TTGCAGTAATCTATGAGTTTC TTCCAA | 4921 |
| 27 | H55_24-50 | TGGAAGAAACTCATAGATTAC TGCAAC | 4130 | GTTGCAGTAATCTATGAGTTT CTTCCA | 4922 |
| 28 | H55_(-31)-(-4) | TAATTGCATCTGAACATTTGG TCCTTTG | 4131 | CAAAGGACCAAATGTTCAGAT GCAATTA | 4923 |
| 28 | H55_ (-30)-(-3) | AATTGCATCTGAACATTTGGT CCTTTGC | 4132 | GCAAAGGACCAAATGTTCAGA TGCAATT | 4924 |
| 28 | H55_(-29)-(-2) | ATTGCATCTGAACATTTGGTC CTTTGCA | 4133 | TGCAAAGGACCAAATGTTCAG ATGCAAT | 4925 |
| 28 | H55_(-28)-(-1) | TTGCATCTGAACATTTGGTCC TTTGCAG | 4134 | CTGCAAAGGACCAAATGTTCA GATGCAA | 4926 |
| 28 | H55_(-27)-1 | TGCATCTGAACATTTGGTCCT TTGCAGG | 4135 | CCTGCAAAGGACCAAATGTTC AGATGCA | 4927 |
| 28 | H55_(-26)-2 | GCATCTGAACATTTGGTCCTT TGCAGGG | 4136 | CCCTGCAAAGGACCAAATGTT CAGATGC | 4928 |
| 28 | H55_(-25)-3 | CATCTGAACATTTGGTCCTTT GCAGGGT | 4137 | ACCCTGCAAAGGACCAAATGT TCAGATG | 4929 |
| 28 | H55_(-24)-4 | ATCTGAACATTTGGTCCTTTG CAGGGTG | 4138 | CACCCTGCAAAGGACCAAATG TTCAGAT | 4930 |
| 28 | H55_(-23)-5 | TCTGAACATTTGGTCCTTTGC AGGGTGA | 4139 | TCACCCTGCAAAGGACCAAAT GTTCAGA | 4931 |
| 28 | H55_(-22)-6 | CTGAACATTTGGTCCTTTGCA GGGTGAG | 4140 | CTCACCCTGCAAAGGACCAAA TGTTCAG | 4932 |

**[Table 6-32]**

| | | | | | |
|---|---|---|---|---|---|
| 28 | H55_(-21)-7 | TGAACATTTGGTCCTTTGCAG GGTGAGT | 4141 | ACTCACCCTGCAAAGGACCAA ATGTTCA | 4933 |
| 28 | H55_(-20)-8 | GAACATTTGGTCCTTTGCAGG GTGAGTG | 4142 | CACTCACCCTGCAAAGGACCA AATGTTC | 4934 |
| 28 | H55_(-19)-9 | AACATTTGGTCCTTTGCAGGG TGAGTGA | 4143 | TCACTCACCCTGCAAAGGACC AAATGTT | 4935 |
| 28 | H55_(-18)-10 | ACATTTGGTCCTTTGCAGGGT GAGTGAG | 4144 | CTCACTCACCCTGCAAAGGAC CAAATGT | 4936 |
| 28 | H55_(-17)-11 | CATTTGGTCCTTTGCAGGGTG AGTGAGC | 4145 | GCTCACTCACCCTGCAAAGGA CCAAATG | 4937 |
| 28 | H55_(-16)-12 | ATTTGGTCCTTTGCAGGGTGA GTGAGCG | 4146 | CGCTCACTCACCCTGCAAAGG ACCAAAT | 4938 |
| 28 | H55_(-15)-13 | TTTGGTCCTTTGCAGGGTGAG TGAGCGA | 4147 | TCGCTCACTCACCCTGCAAAG GACCAAA | 4939 |
| 28 | H55_(-14)-14 | TTGGTCCTTTGCAGGGTGAGT GAGCGAG | 4148 | CTCGCTCACTCACCCTGCAAA GGACCAA | 4940 |
| 28 | H55_(-13)-15 | TGGTCCTTTGCAGGGTGAGTG AGCGAGA | 4149 | TCTCGCTCACTCACCCTGCAA AGGACCA | 4941 |
| 28 | H55_(-12)-16 | GGTCCTTTGCAGGGTGAGTGA GCGAGAG | 4150 | CTCTCGCTCACTCACCCTGCA AAGGACC | 4942 |
| 28 | H55_(-11)-17 | GTCCTTTGCAGGGTGAGTGAG CGAGAGG | 4151 | CCTCTCGCTCACTCACCCTGC AAAGGAC | 4943 |
| 28 | H55_(-10)-18 | TCCTTTGCAGGGTGAGTGAGC GAGAGGC | 4152 | GCCTCTCGCTCACTCACCCTG CAAAGGA | 4944 |
| 28 | H55_(-9)-19 | CCTTTGCAGGGTGAGTGAGCG AGAGGCT | 4153 | AGCCTCTCGCTCACTCACCCT GCAAAGG | 4945 |
| 28 | H55_(-8)-20 | CTTTGCAGGGTGAGTGAGCGA GAGGCTG | 4154 | CAGCCTCTCGCTCACTCACCC TGCAAAG | 4946 |
| 28 | H55_(-7)-21 | TTTGCAGGGTGAGTGAGCGAG AGGCTGC | 4155 | GCAGCCTCTCGCTCACTCACC CTGCAAA | 4947 |
| 28 | H55_(-6)-22 | TTGCAGGGTGAGTGAGCGAGA GGCTGCT | 4156 | AGCAGCCTCTCGCTCACTCAC CCTGCAA | 4948 |
| 28 | H55_(-5)-23 | TGCAGGGTGAGTGAGCGAGAG GCTGCTT | 4157 | AAGCAGCCTCTCGCTCACTCA CCCTGCA | 4949 |
| 28 | H55_(-4)-24 | GCAGGGTGAGTGAGCGAGAGG CTGCTTT | 4158 | AAAGCAGCCTCTCGCTCACTC ACCCTGC | 4950 |

**[Table 6-33]**

| | | | | | |
|---|---|---|---|---|---|
| 28 | H55_(-3)-25 | CAGGGTGAGTGAGCGAGAGGC TGCTTTG | 4159 | CAAAGCAGCCTCTCGCTCACT CACCCTG | 4951 |
| 28 | H55_(-2)-26 | AGGGTGAGTGAGCGAGAGGCT GCTTTGG | 4160 | CCAAAGCAGCCTCTCGCTCAC TCACCCT | 4952 |
| 28 | H55_(-1)-27 | GGGTGAGTGAGCGAGAGGCTG CTTTGGA | 4161 | TCCAAAGCAGCCTCTCGCTCA CTCACCC | 4953 |
| 28 | H55_1-28 | GGTGAGTGAGCGAGAGGCTGC TTTGGAA | 4162 | TTCCAAAGCAGCCTCTCGCTC ACTCACC | 4954 |
| 28 | H55_2-29 | GTGAGTGAGCGAGAGGCTGCT TTGGAAG | 4163 | CTTCCAAAGCAGCCTCTCGCT CACTCAC | 4955 |
| 28 | H55_3-30 | TGAGTGAGCGAGAGGCTGCTT TGGAAGA | 4164 | TCTTCCAAAGCAGCCTCTCGC TCACTCA | 4956 |
| 28 | H55_4-31 | GAGTGAGCGAGAGGCTGCTTT GGAAGAA | 4165 | TTCTTCCAAAGCAGCCTCTCG CTCACTC | 4957 |
| 28 | H55_5-32 | AGTGAGCGAGAGGCTGCTTTG GAAGAAA | 4166 | TTTCTTCCAAAGCAGCCTCTC GCTCACT | 4958 |
| 28 | H55_6-33 | GTGAGCGAGAGGCTGCTTTGG AAGAAAC | 4167 | GTTTCTTCCAAAGCAGCCTCT CGCTCAC | 4959 |
| 28 | H55_7-34 | TGAGCGAGAGGCTGCTTTGGA AGAAACT | 4168 | AGTTTCTTCCAAAGCAGCCTC TCGCTCA | 4960 |
| 28 | H55_8-35 | GAGCGAGAGGCTGCTTTGGAA GAAACTC | 4169 | GAGTTTCTTCCAAAGCAGCCT CTCGCTC | 4961 |
| 28 | H55_9-36 | AGCGAGAGGCTGCTTTGGAAG AAACTCA | 4170 | TGAGTTTCTTCCAAAGCAGCC TCTCGCT | 4962 |
| 28 | H55_10-37 | GCGAGAGGCTGCTTTGGAAGA AACTCAT | 4171 | ATGAGTTTCTTCCAAAGCAGC CTCTCGC | 4963 |
| 28 | H55_11-38 | CGAGAGGCTGCTTTGGAAGAA ACTCATA | 4172 | TATGAGTTTCTTCCAAAGCAG CCTCTCG | 4964 |
| 28 | H55_12-39 | GAGAGGCTGCTTTGGAAGAAA CTCATAG | 4173 | CTATGAGTTTCTTCCAAAGCA GCCTCTC | 4965 |
| 28 | H55_13-40 | AGAGGCTGCTTTGGAAGAAAC TCATAGA | 4174 | TCTATGAGTTTCTTCCAAAGC AGCCTCT | 4966 |
| 28 | H55_14-41 | GAGGCTGCTTTGGAAGAAACT CATAGAT | 4175 | ATCTATGAGTTTCTTCCAAAG CAGCCTC | 4967 |
| 28 | H55_15-42 | AGGCTGCTTTGGAAGAAACTC ATAGATT | 4176 | AATCTATGAGTTTCTTCCAAA GCAGCCT | 4968 |

**[Table 6-34]**

| | | | | | |
|---|---|---|---|---|---|
| 28 | H55_16-43 | GGCTGCTTTGGAAGAAACTCA TAGATTA | 4177 | TAATCTATGAGTTTCTTCCAA AGCAGCC | 4969 |
| 28 | H55_17-44 | GCTGCTTTGGAAGAAACTCAT AGATTAC | 4178 | GTAATCTATGAGTTTCTTCCA AAGCAGC | 4970 |
| 28 | H55_18-45 | CTGCTTTGGAAGAAACTCATA GATTACT | 4179 | AGTAATCTATGAGTTTCTTCC AAAGCAG | 4971 |
| 28 | H55_19-46 | TGCTTTGGAAGAAACTCATAG ATTACTG | 4180 | CAGTAATCTATGAGTTTCTTC CAAAGCA | 4972 |
| 28 | H55_20-47 | GCTTTGGAAGAAACTCATAGA TTACTGC | 4181 | GCAGTAATCTATGAGTTTCTT CCAAAGC | 4973 |
| 28 | H55_21-48 | CTTTGGAAGAAACTCATAGAT TACTGCA | 4182 | TGCAGTAATCTATGAGTTTCT TCCAAAG | 4974 |
| 28 | H55_22-49 | TTTGGAAGAAACTCATAGATT ACTGCAA | 4183 | TTGCAGTAATCTATGAGTTTC TTCCAAA | 4975 |
| 28 | H55_23-50 | TTGGAAGAAACTCATAGATTA CTGCAAC | 4184 | GTTGCAGTAATCTATGAGTTT CTTCCAA | 4976 |
| 28 | H55_24-51 | TGGAAGAAACTCATAGATTAC TGCAACA | 4185 | TGTTGCAGTAATCTATGAGTT TCTTCCA | 4977 |
| 29 | H55_(-32)-(-4) | ATAATTGCATCTGAACATTTG GTCCTTTG | 4186 | CAAAGGACCAAATGTTCAGAT GCAATTAT | 4978 |
| 29 | H55_(-31)-(-3) | TAATTGCATCTGAACATTTGG TCCTTTGC | 4187 | GCAAAGGACCAAATGTTCAGA TGCAATTA | 4979 |
| 29 | H55_(-30)-(-2) | AATTGCATCTGAACATTTGGT CCTTTGCA | 4188 | TGCAAAGGACCAAATGTTCAG ATGCAATT | 4980 |
| 29 | H55_(-29)-(-1) | ATTGCATCTGAACATTTGGTC CTTTGCAG | 4189 | CTGCAAAGGACCAAATGTTCA GATGCAAT | 4981 |
| 29 | H55_(-28)-1 | TTGCATCTGAACATTTGGTCC TTTGCAGG | 4190 | CCTGCAAAGGACCAAATGTTC AGATGCAA | 4982 |
| 29 | H55_(-27)-2 | TGCATCTGAACATTTGGTCCT TTGCAGGG | 4191 | CCCTGCAAAGGACCAAATGTT CAGATGCA | 4983 |
| 29 | H55_(-26)-3 | GCATCTGAACATTTGGTCCTT TGCAGGGT | 4192 | ACCCTGCAAAGGACCAAATGT TCAGATGC | 4984 |
| 29 | H55_(-25)-4 | CATCTGAACATTTGGTCCTTT GCAGGGTG | 4193 | CACCCTGCAAAGGACCAAATG TTCAGATG | 4985 |
| 29 | H55_(-24)-5 | ATCTGAACATTTGGTCCTTTG CAGGGTGA | 4194 | TCACCCTGCAAAGGACCAAAT GTTCAGAT | 4986 |

**[Table 6-35]**

| | | | | | |
|---|---|---|---|---|---|
| 29 | H55_(-23)-6 | TCTGAACATTTGGTCCTTTGC AGGGTGAG | 4195 | CTCACCCTGCAAAGGACCAAA TGTTCAGA | 4987 |
| 29 | H55_(-22)-7 | CTGAACATTTGGTCCTTTGCA GGGTGAGT | 4196 | ACTCACCCTGCAAAGGACCAA ATGTTCAG | 4988 |
| 29 | H55_(-21) -8 | TGAACATTTGGTCCTTTGCAG GGTGAGTG | 4197 | CACTCACCCTGCAAAGGACCA AATGTTCA | 4989 |
| 29 | H55_(-20)-9 | GAACATTTGGTCCTTTGCAGG GTGAGTGA | 4198 | TCACTCACCCTGCAAAGGACC AAATGTTC | 4990 |
| 29 | H55_(-19)-10 | AACATTTGGTCCTTTGCAGGG TGAGTGAG | 4199 | CTCACTCACCCTGCAAAGGAC CAAATGTT | 4991 |
| 29 | H55_(-18)-11 | ACATTTGGTCCTTTGCAGGGT GAGTGAGC | 4200 | GCTCACTCACCCTGCAAAGGA CCAAATGT | 4992 |
| 29 | H55_(-17)-12 | CATTTGGTCCTTTGCAGGGTG AGTGAGCG | 4201 | CGCTCACTCACCCTGCAAAGG ACCAAATG | 4993 |
| 29 | H55_(-16)-13 | ATTTGGTCCTTTGCAGGGTGA GTGAGCGA | 4202 | TCGCTCACTCACCCTGCAAAG GACCAAAT | 4994 |
| 29 | H55_(-15)-14 | TTTGGTCCTTTGCAGGGTGAG TGAGCGAG | 4203 | CTCGCTCACTCACCCTGCAAA GGACCAAA | 4995 |
| 29 | H55_(-14)-15 | TTGGTCCTTTGCAGGGTGAGT GAGCGAGA | 4204 | TCTCGCTCACTCACCCTGCAA AGGACCAA | 4996 |
| 29 | H55_(-13)-16 | TGGTCCTTTGCAGGGTGAGTG AGCGAGAG | 4205 | CTCTCGCTCACTCACCCTGCA AAGGACCA | 4997 |
| 29 | H55_(-12)-17 | GGTCCTTTGCAGGGTGAGTGA GCGAGAGG | 4206 | CCTCTCGCTCACTCACCCTGC AAAGGACC | 4998 |
| 29 | H55_(-11)-18 | GTCCTTTGCAGGGTGAGTGAG CGAGAGGC | 4207 | GCCTCTCGCTCACTCACCCTG CAAAGGAC | 4999 |
| 29 | H55_(-10)-19 | TCCTTTGCAGGGTGAGTGAGC GAGAGGCT | 4208 | AGCCTCTCGCTCACTCACCCT GCAAAGGA | 5000 |
| 29 | H55_(-9)-20 | CCTTTGCAGGGTGAGTGAGCG AGAGGCTG | 4209 | CAGCCTCTCGCTCACTCACCC TGCAAAGG | 5001 |
| 29 | H55_(-8)-21 | CTTTGCAGGGTGAGTGAGCGA GAGGCTGC | 4210 | GCAGCCTCTCGCTCACTCACC CTGCAAAG | 5002 |
| 29 | H55_(-7)-22 | TTTGCAGGGTGAGTGAGCGAG AGGCTGCT | 4211 | AGCAGCCTCTCGCTCACTCAC CCTGCAAA | 5003 |
| 29 | H55_(-6)-23 | TTGCAGGGTGAGTGAGCGAGA GGCTGCTT | 4212 | AAGCAGCCTCTCGCTCACTCA CCCTGCAA | 5004 |

**[Table 6-36]**

| | | | | | |
|---|---|---|---|---|---|
| 29 | H55_(-5)-24 | TGCAGGGTGAGTGAGCGAGAG GCTGCTTT | 4213 | AAAGCAGCCTCTCGCTCACTC ACCCTGCA | 5005 |
| 29 | H55_(-4)-25 | GCAGGGTGAGTGAGCGAGAGG CTGCTTTG | 4214 | CAAAGCAGCCTCTCGCTCACT CACCCTGC | 5006 |
| 29 | H55_(-3)-26 | CAGGGTGAGTGAGCGAGAGGC TGCTTTGG | 4215 | CCAAAGCAGCCTCTCGCTCAC TCACCCTG | 5007 |
| 29 | H55_(-2)-27 | AGGGTGAGTGAGCGAGAGGCT GCTTTGGA | 4216 | TCCAAAGCAGCCTCTCGCTCA CTCACCCT | 5008 |
| 29 | H55_(-1)-28 | GGGTGAGTGAGCGAGAGGCTG CTTTGGAA | 4217 | TTCCAAAGCAGCCTCTCGCTC ACTCACCC | 5009 |
| 29 | H55_1-29 | GGTGAGTGAGCGAGAGGCTGC TTTGGAAG | 4218 | CTTCCAAAGCAGCCTCTCGCT CACTCACC | 5010 |
| 29 | H55_2-30 | GTGAGTGAGCGAGAGGCTGCT TTGGAAGA | 4219 | TCTTCCAAAGCAGCCTCTCGC TCACTCAC | 5011 |
| 29 | H55_3-31 | TGAGTGAGCGAGAGGCTGCTT TGGAAGAA | 4220 | TTCTTCCAAAGCAGCCTCTCG CTCACTCA | 5012 |
| 29 | H55_4-32 | GAGTGAGCGAGAGGCTGCTTT GGAAGAAA | 4221 | TTTCTTCCAAAGCAGCCTCTC GCTCACTC | 5013 |
| 29 | H55_5-33 | AGTGAGCGAGAGGCTGCTTTG GAAGAAAC | 4222 | GTTTCTTCCAAAGCAGCCTCT CGCTCACT | 5014 |
| 29 | H55_6-34 | GTGAGCGAGAGGCTGCTTTGG AAGAAACT | 4223 | AGTTTCTTCCAAAGCAGCCTC TCGCTCAC | 5015 |
| 29 | H55_7-35 | TGAGCGAGAGGCTGCTTTGGA AGAAACTC | 4224 | GAGTTTCTTCCAAAGCAGCCT CTCGCTCA | 5016 |
| 29 | H55_8-36 | GAGCGAGAGGCTGCTTTGGAA GAAACTCA | 4225 | TGAGTTTCTTCCAAAGCAGCC TCTCGCTC | 5017 |
| 29 | H55_9-37 | AGCGAGAGGCTGCTTTGGAAG AAACTCAT | 4226 | ATGAGTTTCTTCCAAAGCAGC CTCTCGCT | 5018 |
| 29 | H55_10-38 | GCGAGAGGCTGCTTTGGAAGA AACTCATA | 4227 | TATGAGTTTCTTCCAAAGCAG CCTCTCGC | 5019 |
| 29 | H55_11-39 | CGAGAGGCTGCTTTGGAAGAA ACTCATAG | 4228 | CTATGAGTTTCTTCCAAAGCA GCCTCTCG | 5020 |
| 29 | H55_12-40 | GAGAGGCTGCTTTGGAAGAAA CTCATAGA | 4229 | TCTATGAGTTTCTTCCAAAGC AGCCTCTC | 5021 |
| 29 | H55_13-41 | AGAGGCTGCTTTGGAAGAAAC TCATAGAT | 4230 | ATCTATGAGTTTCTTCCAAAG CAGCCTCT | 5022 |

**[Table 6-37]**

| | | | | | |
|---|---|---|---|---|---|
| 29 | H55_14-42 | GAGGCTGCTTTGGAAGAAACT CATAGATT | 4231 | AATCTATGAGTTTCTTCCAAA GCAGCCTC | 5023 |
| 29 | H55_15-43 | AGGCTGCTTTGGAAGAAACTC ATAGATTA | 4232 | TAATCTATGAGTTTCTTCCAA AGCAGCCT | 5024 |
| 29 | H55_16-44 | GGCTGCTTTGGAAGAAACTCA TAGATTAC | 4233 | GTAATCTATGAGTTTCTTCCA AAGCAGCC | 5025 |
| 29 | H55_17-45 | GCTGCTTTGGAAGAAACTCAT AGATTACT | 4234 | AGTAATCTATGAGTTTCTTCC AAAGCAGC | 5026 |
| 29 | H55_18-46 | CTGCTTTGGAAGAAACTCATA GATTACTG | 4235 | CAGTAATCTATGAGTTTCTTC CAAAGCAG | 5027 |
| 29 | H55_19-47 | TGCTTTGGAAGAAACTCATAG ATTACTGC | 4236 | GCAGTAATCTATGAGTTTCTT CCAAAGCA | 5028 |
| 29 | H55_20-48 | GCTTTGGAAGAAACTCATAGA TTACTGCA | 4237 | TGCAGTAATCTATGAGTTTCT TCCAAAGC | 5029 |
| 29 | H55_21-49 | CTTTGGAAGAAACTCATAGAT TACTGCAA | 4238 | TTGCAGTAATCTATGAGTTTC TTCCAAAG | 5030 |
| 29 | H55_22-50 | TTTGGAAGAAACTCATAGATT ACTGCAAC | 4239 | GTTGCAGTAATCTATGAGTTT CTTCCAAA | 5031 |
| 29 | H55_23-51 | TTGGAAGAAACTCATAGATTA CTGCAACA | 4240 | TGTTGCAGTAATCTATGAGTT TCTTCCAA | 5032 |
| 29 | H55_24-52 | TGGAAGAAACTCATAGATTAC TGCAACAG | 4241 | CTGTTGCAGTAATCTATGAGT TTCTTCCA | 5033 |
| 30 | H55_(-33)-(-4) | AATAATTGCATCTGAACATTT GGTCCTTTG | 4242 | CAAAGGACCAAATGTTCAGAT GCAATTATT | 5034 |
| 30 | H55_ (-32)-(-3) | ATAATTGCATCTGAACATTTG GTCCTTTGC | 4243 | GCAAAGGACCAAATGTTCAGA TGCAATTAT | 5035 |
| 30 | H55_ (-31)-(-2) | TAATTGCATCTGAACATTTGG TCCTTTGCA | 4244 | TGCAAAGGACCAAATGTTCAG ATGCAATTA | 5036 |
| 30 | H55_(-30)-(-1) | AATTGCATCTGAACATTTGGT CCTTTGCAG | 4245 | CTGCAAAGGACCAAATGTTCA GATGCAATT | 5037 |
| 30 | H55_(-29)-1 | ATTGCATCTGAACATTTGGTC CTTTGCAGG | 4246 | CCTGCAAAGGACCAAATGTTC AGATGCAAT | 5038 |
| 30 | H55_(-28) -2 | TTGCATCTGAACATTTGGTCC TTTGCAGGG | 4247 | CCCTGCAAAGGACCAAATGTT CAGATGCAA | 5039 |
| 30 | H55_(-27) -3 | TGCATCTGAACATTTGGTCCT TTGCAGGGT | 4248 | ACCCTGCAAAGGACCAAATGT TCAGATGCA | 5040 |

**[Table 6-38]**

| | | | | | |
|---|---|---|---|---|---|
| 30 | H55_(-26)-4 | GCATCTGAACATTTGGTCCTT TGCAGGGTG | 4249 | CACCCTGCAAAGGACCAAATG TTCAGATGC | 5041 |
| 30 | H55_(-25)-5 | CATCTGAACATTTGGTCCTTT GCAGGGTGA | 4250 | TCACCCTGCAAAGGACCAAAT GTTCAGATG | 5042 |
| 30 | H55_(-24)-6 | ATCTGAACATTTGGTCCTTTG CAGGGTGAG | 4251 | CTCACCCTGCAAAGGACCAAA TGTTCAGAT | 5043 |
| 30 | H55_(-23)-7 | TCTGAACATTTGGTCCTTTGC AGGGTGAGT | 4252 | ACTCACCCTGCAAAGGACCAA ATGTTCAGA | 5044 |
| 30 | H55_(-22)-8 | CTGAACATTTGGTCCTTTGCA GGGTGAGTG | 4253 | CACTCACCCTGCAAAGGACCA AATGTTCAG | 5045 |
| 30 | H55_(-21) -9 | TGAACATTTGGTCCTTTGCAG GGTGAGTGA | 4254 | TCACTCACCCTGCAAAGGACC AAATGTTCA | 5046 |
| 30 | H55_(-20)-10 | GAACATTTGGTCCTTTGCAGG GTGAGTGAG | 4255 | CTCACTCACCCTGCAAAGGAC CAAATGTTC | 5047 |
| 30 | H55_(-19)-11 | AACATTTGGTCCTTTGCAGGG TGAGTGAGC | 4256 | GCTCACTCACCCTGCAAAGGA CCAAATGTT | 5048 |
| 30 | H55_(-18)-12 | ACATTTGGTCCTTTGCAGGGT GAGTGAGCG | 4257 | CGCTCACTCACCCTGCAAAGG ACCAAATGT | 5049 |
| 30 | H55_(-17)-13 | CATTTGGTCCTTTGCAGGGTG AGTGAGCGA | 4258 | TCGCTCACTCACCCTGCAAAG GACCAAATG | 5050 |
| 30 | H55_(-16)-14 | ATTTGGTCCTTTGCAGGGTGA GTGAGCGAG | 4259 | CTCGCTCACTCACCCTGCAAA GGACCAAAT | 5051 |
| 30 | H55_(-15)-15 | TTTGGTCCTTTGCAGGGTGAG TGAGCGAGA | 4260 | TCTCGCTCACTCACCCTGCAA AGGACCAAA | 5052 |
| 30 | H55_(-14) -16 | TTGGTCCTTTGCAGGGTGAGT GAGCGAGAG | 4261 | CTCTCGCTCACTCACCCTGCA AAGGACCAA | 5053 |
| 30 | H55_(-13)-17 | TGGTCCTTTGCAGGGTGAGTG AGCGAGAGG | 4262 | CCTCTCGCTCACTCACCCTGC AAAGGACCA | 5054 |
| 30 | H55_(-12) -18 | GGTCCTTTGCAGGGTGAGTGA GCGAGAGGC | 4263 | GCCTCTCGCTCACTCACCCTG CAAAGGACC | 5055 |
| 30 | H55_(-11)-19 | GTCCTTTGCAGGGTGAGTGAG CGAGAGGCT | 4264 | AGCCTCTCGCTCACTCACCCT GCAAAGGAC | 5066 |
| 30 | H55_(-10)-20 | TCCTTTGCAGGGTGAGTGAGC GAGAGGCTG | 4265 | CAGCCTCTCGCTCACTCACCC TGCAAAGGA | 5057 |
| 30 | H55_(-9)-21 | CCTTTGCAGGGTGAGTGAGCG AGAGGCTGC | 4266 | GCAGCCTCTCGCTCACTCACC CTGCAAAGG | 5058 |

**[Table 6-39]**

| | | | | | |
|---|---|---|---|---|---|
| 30 | H55_(-8)-22 | CTTTGCAGGGTGAGTGAGCGA GAGGCTGCT | 4267 | AGCAGCCTCTCGCTCACTCAC CCTGCAAAG | 5059 |
| 30 | H55_(-7)-23 | TTTGCAGGGTGAGTGAGCGAG AGGCTGCTT | 4268 | AAGCAGCCTCTCGCTCACTCA CCCTGCAAA | 5060 |
| 30 | H55_(-6)-24 | TTGCAGGGTGAGTGAGCGAGA GGCTGCTTT | 4269 | AAAGCAGCCTCTCGCTCACTC ACCCTGCAA | 5061 |
| 30 | H55_(-5)- 25 | TGCAGGGTGAGTGAGCGAGAG GCTGCTTTG | 4270 | CAAAGCAGCCTCTCGCTCACT CACCCTGCA | 5062 |
| 30 | H55_(-4)-26 | GCAGGGTGAGTGAGCGAGAGG CTGCTTTGG | 4271 | CCAAAGCAGCCTCTCGCTCAC TCACCCTGC | 5063 |
| 30 | H55_(-3)-27 | CAGGGTGAGTGAGCGAGAGGC TGCTTTGGA | 4272 | TCCAAAGCAGCCTCTCGCTCA CTCACCCTG | 5064 |
| 30 | H55_(-2)-28 | AGGGTGAGTGAGCGAGAGGCT GCTTTGGAA | 4273 | TTCCAAAGCAGCCTCTCGCTC ACTCACCCT | 5065 |
| 30 | H55_(-1)-29 | GGGTGAGTGAGCGAGAGGCTG CTTTGGAAG | 4274 | CTTCCAAAGCAGCCTCTCGCT CACTCACCC | 5066 |
| 30 | H55_1-30 | GGTGAGTGAGCGAGAGGCTGC TTTGGAAGA | 4275 | TCTTCCAAAGCAGCCTCTCGC TCACTCACC | 5067 |
| 30 | H55_2-31 | GTGAGTGAGCGAGAGGCTGCT TTGGAAGAA | 4276 | TTCTTCCAAAGCAGCCTCTCG CTCACTCAC | 5068 |
| 30 | H55_3-32 | TGAGTGAGCGAGAGGCTGCTT TGGAAGAAA | 4277 | TTTCTTCCAAAGCAGCCTCTC GCTCACTCA | 5069 |
| 30 | H55_4-33 | GAGTGAGCGAGAGGCTGCTTT GGAAGAAAC | 4278 | GTTTCTTCCAAAGCAGCCTCT CGCTCACTC | 5070 |
| 30 | H55_5-34 | AGTGAGCGAGAGGCTGCTTTG GAAGAAACT | 4279 | AGTTTCTTCCAAAGCAGCCTC TCGCTCACT | 5071 |
| 30 | H55_6-35 | GTGAGCGAGAGGCTGCTTTGG AAGAAACTC | 4280 | GAGTTTCTTCCAAAGCAGCCT CTCGCTCAC | 5072 |
| 30 | H55_7-36 | TGAGCGAGAGGCTGCTTTGGA AGAAACTCA | 4281 | TGAGTTTCTTCCAAAGCAGCC TCTCGCTCA | 5073 |
| 30 | H55_8-37 | GAGCGAGAGGCTGCTTTGGAA GAAACTCAT | 4282 | ATGAGTTTCTTCCAAAGCAGC CTCTCGCTC | 5074 |
| 30 | H55_9-38 | AGCGAGAGGCTGCTTTGGAAG AAACTCATA | 4283 | TATGAGTTTCTTCCAAAGCAG CCTCTCGCT | 5075 |
| 30 | H55_10-39 | GCGAGAGGCTGCTTTGGAAGA AACTCATAG | 4284 | CTATGAGTTTCTTCCAAAGCA GCCTCTCGC | 5076 |

**[Table 6-40]**

| | | | | | |
|---|---|---|---|---|---|
| 30 | H55_11-40 | CGAGAGGCTGCTTTGGAAGAA ACTCATAGA | 4285 | TCTATGAGTTTCTTCCAAAGC AGCCTCTCG | 5077 |
| 30 | H55_12-41 | GAGAGGCTGCTTTGGAAGAAA CTCATAGAT | 4286 | ATCTATGAGTTTCTTCCAAAG CAGCCTCTC | 5078 |
| 30 | H55_13-42 | AGAGGCTGCTTTGGAAGAAAC TCATAGATT | 4287 | AATCTATGAGTTTCTTCCAAA GCAGCCTCT | 5079 |
| 30 | H55_14-43 | GAGGCTGCTTTGGAAGAAACT CATAGATTA | 4288 | TAATCTATGAGTTTCTTCCAA AGCAGCCTC | 5080 |
| 30 | H55_15-44 | AGGCTGCTTTGGAAGAAACTC ATAGATTAC | 4289 | GTAATCTATGAGTTTCTTCCA AAGCAGCCT | 5081 |
| 30 | H55_16-45 | GGCTGCTTTGGAAGAAACTCA TAGATTACT | 4290 | AGTAATCTATGAGTTTCTTCC AAAGCAGCC | 5082 |
| 30 | H55_17-46 | GCTGCTTTGGAAGAAACTCAT AGATTACTG | 4291 | CAGTAATCTATGAGTTTCTTC CAAAGCAGC | 5083 |
| 30 | H55_18-47 | CTGCTTTGGAAGAAACTCATA GATTACTGC | 4292 | GCAGTAATCTATGAGTTTCTT CCAAAGCAG | 5084 |
| 30 | H55_19-48 | TGCTTTGGAAGAAACTCATAG ATTACTGCA | 4293 | TGCAGTAATCTATGAGTTTCT TCCAAAGCA | 5085 |
| 30 | H55_20-49 | GCTTTGGAAGAAACTCATAGA TTACTGCAA | 4294 | TTGCAGTAATCTATGAGTTTC TTCCAAAGC | 5086 |
| 30 | H55_21-50 | CTTTGGAAGAAACTCATAGAT TACTGCAAC | 4295 | GTTGCAGTAATCTATGAGTTT CTTCCAAAG | 5087 |
| 30 | H55_22-51 | TTTGGAAGAAACTCATAGATT ACTGCAACA | 4296 | TGTTGCAGTAATCTATGAGTT TCTTCCAAA | 5088 |
| 30 | H55_23-52 | TTGGAAGAAACTCATAGATTA CTGCAACAG | 4297 | CTGTTGCAGTAATCTATGAGT TTCTTCCAA | 5089 |
| 30 | H55_24-53 | TGGAAGAAACTCATAGATTAC TGCAACAGT | 4298 | ACTGTTGCAGTAATCTATGAG TTTCTTCCA | 5090 |

In one embodiment, the second antisense oligomer of the present invention comprises a base sequence complementary to:
(a) any one base sequence selected from the group consisting of SEQ ID NOs: 3507 to 4298;
(b) a base sequence that hybridizes under stringent conditions to a base sequence complementary to any one base sequence selected from the group consisting of SEQ ID NOs: 3507 to 4298;
(c) a base sequence that has at least 85% identity with any one base sequence selected from the group consisting of SEQ ID NOs: 3507 to 4298, and has a length within ±15% of the length of the any one base sequence selected; or
(d) a partial base sequence of any one base sequence selected from the group consisting of the base sequences (a), (b), and (c) .

Herein, the base sequence (c) is a mutant type of the base sequence (a), and examples of such a mutant type also include:
(c-1) a base sequence that has at least 85% identity with any one base sequence selected from the group consisting of SEQ ID NOs: 3507 to 4298, and has a length within ±15% of the length of the any one base sequence selected,
(c-2) a base sequence that has at least 86% identity with any one base sequence selected from the group consisting of SEQ ID NOs: 3507 to 4298, and has a length within ±14% of the length of the any one base sequence selected,
(c-3) a base sequence that has at least 87% identity with any one base sequence selected from the group consisting of SEQ ID NOs: 3507 to 4298, and has a length within ±13% of the length of the any one base sequence selected,
(c-4) a base sequence that has at least 88% identity with any one base sequence selected from the group consisting of SEQ ID NOs: 3507 to 4298, and has a length within ±12% of the length of the any one base sequence selected,
(c-5) a base sequence that has at least 89% identity with any one base sequence selected from the group consisting of SEQ ID NOs: 3507 to 4298, and has a length within ±11% of the length of the any one base sequence selected,
(c-6) a base sequence that has at least 90% identity with any one base sequence selected from the group consisting of SEQ ID NOs: 3507 to 4298, and has a length within ±10% of the length of the any one base sequence selected,
(c-7) a base sequence that has at least 91% identity with any one base sequence selected from the group consisting of SEQ ID NOs: 3507 to 4298, and has a length within ±9% of the length of the any one base sequence selected,
(c-8) a base sequence that has at least 92% identity with any one base sequence selected from the group consisting of SEQ ID NOs: 3507 to 4298, and has a length within ±8% of the length of the any one base sequence selected,
(c-9) a base sequence that has at least 93% identity with any one base sequence selected from the group consisting of SEQ ID NOs: 3507 to 4298, and has a length within ±7% of the length of the any one base sequence selected,
(c-10) a base sequence that has at least 94% identity with any one base sequence selected from the group consisting of SEQ ID NOs: 3507 to 4298, and has a length within ±6% of the length of the any one base sequence selected,
(c-11) a base sequence that has at least 95% identity with any one base sequence selected from the group consisting of SEQ ID NOs: 3507 to 4298, and has a length within ±5% of the length of the any one base sequence selected,
(c-12) a base sequence that has at least 96% identity with any one base sequence selected from the group consisting of SEQ ID NOs: 3507 to 4298, and has a length within ±4% of the length of the any one base sequence selected,
(c-13) a base sequence that has at least 97% identity with any one base sequence selected from the group consisting of SEQ ID NOs: 3507 to 4298, and has a length within ±3% of the length of the any one base sequence selected,
(c-14) a base sequence that has at least 98% identity with any one base sequence selected from the group consisting of SEQ ID NOs: 3507 to 4298, and has a length within ±2% of the length of the any one base sequence selected,
(c-15) a base sequence that has at least 99% identity with any one base sequence selected from the group consisting of SEQ ID NOs: 3507 to 4298, and has a length within ±1% of the length of the any one base sequence selected, and
(c-16) a base sequence that has at least 99.5% identity with any one base sequence selected from the group consisting of SEQ ID NOs: 3507 to 4298, and has a length within ±0.5% of the length of the any one base sequence selected.

In one embodiment, the second antisense oligomer of the present invention comprises or consists of:
(a) any one base sequence selected from the group consisting of SEQ ID NOs: 4299 to 5090; or
(b) a base sequence that has at least 85% identity with any one base sequence selected from the group consisting of SEQ ID NOs: 4299 to 5090, and has a length within ±15% of the length of the any one base sequence selected.

0 10 3 1 Herein, the base sequence (b) is a mutant type of the base sequence (a), and examples of such a mutant type also include:
(b-1) a base sequence that has at least 85% identity with any one base sequence selected from the group consisting of SEQ ID NOs: 4299 to 5090, and has a length within ±15% of the length of the any one base sequence selected,
(b-2) a base sequence that has at least 86% identity with any one base sequence selected from the group consisting of SEQ ID NOs: 4299 to 5090, and has a length within ±14% of the length of the any one base sequence selected,
(b-3) a base sequence that has at least 87% identity with any one base sequence selected from the group consisting of SEQ ID NOs: 4299 to 5090, and has a length within ±13% of the length of the any one base sequence selected,
(b-4) a base sequence that has at least 88% identity with any one base sequence selected from the group consisting of SEQ ID NOs: 4299 to 5090, and has a length within ±12% of the length of the any one base sequence selected,
(b-5) a base sequence that has at least 89% identity with any one base sequence selected from the group consisting of SEQ ID NOs: 4299 to 5090, and has a length within ±11% of the length of the any one base sequence selected,
(b-6) a base sequence that has at least 90% identity with any one base sequence selected from the group consisting of SEQ ID NOs: 4299 to 5090, and has a length within ±10% of the length of the any one base sequence selected,
(b-7) a base sequence that has at least 91% identity with any one base sequence selected from the group consisting of SEQ ID NOs: 4299 to 5090, and has a length within ±9% of the length of the any one base sequence selected,
(b-8) a base sequence that has at least 92% identity with any one base sequence selected from the group consisting of SEQ ID NOs: 4299 to 5090, and has a length within ±8% of the length of the any one base sequence selected,
(b-9) a base sequence that has at least 93% identity with any one base sequence selected from the group consisting of SEQ ID NOs: 4299 to 5090, and has a length within ±7% of the length of the any one base sequence selected,
(b-10) a base sequence that has at least 94% identity with any one base sequence selected from the group consisting of SEQ ID NOs: 4299 to 5090, and has a length within ±6% of the length of the any one base sequence selected,
(b-11) a base sequence that has at least 95% identity with any one base sequence selected from the group consisting of SEQ ID NOs: 4299 to 5090, and has a length within ±5% of the length of the any one base sequence selected,
(b-12) a base sequence that has at least 96% identity with any one base sequence selected from the group consisting of SEQ ID NOs: 4299 to 5090, and has a length within ±4% of the length of the any one base sequence selected,
(b-13) a base sequence that has at least 97% identity with any one base sequence selected from the group consisting of SEQ ID NOs: 4299 to 5090, and has a length within ±3% of the length of the any one base sequence selected,
(b-14) a base sequence that has at least 98% identity with any one base sequence selected from the group consisting of SEQ ID NOs: 4299 to 5090, and has a length within ±2% of the length of the any one base sequence selected,
(b-15) a base sequence that has at least 99% identity with any one base sequence selected from the group consisting of SEQ ID NOs: 4299 to 5090, and has a length within ±1% of the length of the any one base sequence selected, and
(b-16) a base sequence that has at least 99.5% identity with any one base sequence selected from the group consisting of SEQ ID NOs: 4299 to 5090, and has a length within ±0.5% of the length of the any one base sequence selected.

In one embodiment, the second antisense oligomer of the present invention comprises or consists of any one base sequence selected from the group consisting of SEQ ID Nos: 4299 to 5090.

In one embodiment, the second antisense oligomer comprises or consists of any one base sequence selected from the group consisting of SEQ ID NOs: 4698, 4702, 4752, 4923, 4926, 4936, 4950, and 4977.

Table 7 below shows examples of the target sequence of the third antisense oligomer of the present invention, and a complementary sequence (antisense sequence) thereof.

**[Table 7-1]**

| **Table 7** | | | | | |
|---|---|---|---|---|---|
| **Length mer** | **Target site** | **Targe sequence** | **SEQ ID NO:** | **Antisense sequence (5' to 3')** | **SEQ ID NO:** |
| 15 | H45_16 9-183 | AAAAAGAGGTAGGGC | 1603 | GCCCTACCTCTTTTT | 255 5 |
| 15 | H45_17 0-184 | AAAAGAGGTAGGGCG | 1604 | CGCCCTACCTCTTTT | 255 6 |
| 15 | H45_17 1-185 | AAAGAGGTAGGGCGA | 1605 | TCGCCCTACCTCTTT | 255 7 |
| 15 | H45_17 2-186 | AAGAGGTAGGGCGAC | 1606 | GTCGCCCTACCTCTT | 255 8 |
| 15 | H45_17 3-187 | AGAGGTAGGGCGACA | 1607 | TGTCGCCCTACCTCT | 255 9 |
| 15 | H45_17 4-188 | GAGGTAGGGCGACAG | 1608 | CTGTCGCCCTACCTC | 256 0 |

**[Table 7-2]**

| | | | | | |
|---|---|---|---|---|---|
| 15 | H45_17 5-189 | AGGTAGGGCGACAGA | 1609 | TCTGTCGCCCTACCT | 256 1 |
| 15 | H45_17 6-190 | GGTAGGGCGACAGAT | 1610 | ATCTGTCGCCCTACC | 256 2 |
| 15 | H45_17 7-191 | GTAGGGCGACAGATC | 1611 | GATCTGTCGCCCTAC | 256 3 |
| 15 | H45_17 8-192 | TAGGGCGACAGATCT | 1612 | AGATCTGTCGCCCTA | 256 4 |
| 15 | H45_17 9-193 | AGGGCGACAGATCTA | 1613 | TAGATCTGTCGCCCT | 256 5 |
| 15 | H45_18 0-194 | GGGCGACAGATCTAA | 1614 | TTAGATCTGTCGCCC | 256 6 |
| 15 | H45_18 1-195 | GGCGACAGATCTAAT | 1615 | ATTAGATCTGTCGCC | 256 7 |
| 15 | H45_18 2-196 | GCGACAGATCTAATA | 1616 | TATTAGATCTGTCGC | 256 8 |
| 15 | H45_18 3-197 | CGACAGATCTAATAG | 1617 | CTATTAGATCTGTCG | 256 9 |
| 15 | H45_18 4-198 | GACAGATCTAATAGG | 1618 | CCTATTAGATCTGTC | 257 0 |
| 15 | H45_18 5-199 | ACAGATCTAATAGGA | 1619 | TCCTATTAGATCTGT | 257 1 |
| 15 | H45_18 6-200 | CAGATCTAATAGGAA | 1620 | TTCCTATTAGATCTG | 257 2 |
| 15 | H45_18 7-201 | AGATCTAATAGGAAT | 1621 | ATTCCTATTAGATCT | 257 3 |
| 15 | H45_18 8-202 | GATCTAATAGGAATG | 1622 | CATTCCTATTAGATC | 257 4 |
| 15 | H45_18 9-203 | ATCTAATAGGAATGA | 1623 | TCATTCCTATTAGAT | 257 5 |
| 15 | H45_19 0-204 | TCTAATAGGAATGAA | 1624 | TTCATTCCTATTAGA | 257 6 |
| 15 | H45_19 1-205 | CTAATAGGAATGAAA | 1625 | TTTCATTCCTATTAG | 257 7 |
| 15 | H45_19 2-206 | TAATAGGAATGAAAA | 1626 | TTTTCATTCCTATTA | 257 8 |

**[Table 7-3]**

| | | | | | |
|---|---|---|---|---|---|
| 15 | H45_19 3-207 | AATAGGAATGAAAAC | 1627 | GTTTTCATTCCTATT | 257 9 |
| 15 | H45_19 4-208 | ATAGGAATGAAAACA | 1628 | TGTTTTCATTCCTAT | 258 0 |
| 15 | H45_19 5-209 | TAGGAATGAAAACAT | 1629 | ATGTTTTCATTCCTA | 258 1 |
| 15 | H45_19 6-210 | AGGAATGAAAACATT | 1630 | AATGTTTTCATTCCT | 258 2 |
| 15 | H45_19 7-211 | GGAATGAAAACATTT | 1631 | AAATGTTTTCATTCC | 258 3 |
| 15 | H45_19 8-212 | GAATGAAAACATTTT | 1632 | AAAATGTTTTCATTC | 258 4 |
| 15 | H45_19 9-213 | AATGAAAACATTTTA | 1633 | TAAAATGTTTTCATT | 258 5 |
| 15 | H45_20 0-214 | ATGAAAACATTTTAG | 1634 | CTAAAATGTTTTCAT | 258 6 |
| 15 | H45_20 1-215 | TGAAAACATTTTAGC | 1635 | GCTAAAATGTTTTCA | 258 7 |
| 15 | H45_20 2-216 | GAAAACATTTTAGCA | 1636 | TGCTAAAATGTTTTC | 258 8 |
| 15 | H45_20 3-217 | AAAACATTTTAGCAG | 1637 | CTGCTAAAATGTTTT | 258 9 |
| 15 | H45_20 4-218 | AAACATTTTAGCAGA | 1638 | TCTGCTAAAATGTTT | 259 0 |
| 15 | H45_20 5-219 | AACATTTTAGCAGAC | 1639 | GTCTGCTAAAATGTT | 259 1 |
| 15 | H45_20 6-220 | ACATTTTAGCAGACT | 1640 | AGTCTGCTAAAATGT | 259 2 |
| 15 | H45_20 7-221 | CATTTTAGCAGACTT | 1641 | AAGTCTGCTAAAATG | 259 3 |
| 15 | H45_20 8-222 | ATTTTAGCAGACTTT | 1642 | AAAGTCTGCTAAAAT | 259 4 |
| 15 | H45_20 9-223 | TTTTAGCAGACTTTT | 1643 | AAAAGTCTGCTAAAA | 259 5 |
| 15 | H45_21 0-224 | TTTAGCAGACTTTTT | 1644 | AAAAAGTCTGCTAAA | 259 6 |

**[Table 7-4]**

| | | | | | |
|---|---|---|---|---|---|
| 15 | H45_21 1-225 | TTAGCAGACTTTTTA | 1645 | TAAAAAGTCTGCTAA | 259 7 |
| 15 | H45_21 2-226 | TAGCAGACTTTTTAA | 1646 | TTAAAAAGTCTGCTA | 259 8 |
| 15 | H45_21 3-227 | AGCAGACTTTTTAAG | 1647 | CTTAAAAAGTCTGCT | 259 9 |
| 15 | H45_21 4-228 | GCAGACTTTTTAAGC | 1648 | GCTTAAAAAGTCTGC | 260 0 |
| 15 | H45_21 5-229 | CAGACTTTTTAAGCT | 1649 | AGCTTAAAAAGTCTG | 260 1 |
| 15 | H45_21 6-230 | AGACTTTTTAAGCTT | 1650 | AAGCTTAAAAAGTCT | 260 2 |
| 15 | H45_21 7-231 | GACTTTTTAAGCTTT | 1651 | AAAGCTTAAAAAGTC | 260 3 |
| 15 | H45_21 8-232 | ACTTTTTAAGCTTTC | 1652 | GAAAGCTTAAAAAGT | 260 4 |
| 15 | H45_21 9-233 | CTTTTTAAGCTTTCT | 1653 | AGAAAGCTTAAAAAG | 260 5 |
| 15 | H45_22 0-234 | TTTTTAAGCTTTCTT | 1654 | AAGAAAGCTTAAAAA | 260 6 |
| 16 | H45_16 8-183 | AAAAAAGAGGTAGGGC | 1655 | GCCCTACCTCTTTTTT | 260 7 |
| 16 | H45_16 9-184 | AAAAAGAGGTAGGGCG | 1656 | CGCCCTACCTCTTTTT | 260 8 |
| 16 | H45_17 0-185 | AAAAGAGGTAGGGCGA | 1657 | TCGCCCTACCTCTTTT | 260 9 |
| 16 | H45_17 1-186 | AAAGAGGTAGGGCGAC | 1658 | GTCGCCCTACCTCTTT | 261 0 |
| 16 | H45_17 2-187 | AAGAGGTAGGGCGACA | 1659 | TGTCGCCCTACCTCTT | 261 1 |
| 16 | H45_17 3-188 | AGAGGTAGGGCGACAG | 1660 | CTGTCGCCCTACCTCT | 261 2 |
| 16 | H45_17 4-189 | GAGGTAGGGCGACAGA | 1661 | TCTGTCGCCCTACCTC | 261 3 |
| 16 | H45_17 5-190 | AGGTAGGGCGACAGAT | 1662 | ATCTGTCGCCCTACCT | 261 4 |

**[Table 7-5]**

| | | | | | |
|---|---|---|---|---|---|
| 16 | H45_17 6-191 | GGTAGGGCGACAGATC | 1663 | GATCTGTCGCCCTACC | 261 5 |
| 16 | H45_17 7-192 | GTAGGGCGACAGATCT | 1664 | AGATCTGTCGCCCTAC | 261 6 |
| 16 | H45_17 8-193 | TAGGGCGACAGATCTA | 1665 | TAGATCTGTCGCCCTA | 261 7 |
| 16 | H45_17 9-194 | AGGGCGACAGATCTAA | 1666 | TTAGATCTGTCGCCCT | 261 8 |
| 16 | H45_18 0-195 | GGGCGACAGATCTAAT | 1667 | ATTAGATCTGTCGCCC | 261 9 |
| 16 | H45_18 1-196 | GGCGACAGATCTAATA | 1668 | TATTAGATCTGTCGCC | 262 0 |
| 16 | H45_18 2-197 | GCGACAGATCTAATAG | 1669 | CTATTAGATCTGTCGC | 262 1 |
| 16 | H45_18 3-198 | CGACAGATCTAATAGG | 1670 | CCTATTAGATCTGTCG | 262 2 |
| 16 | H45_18 4-199 | GACAGATCTAATAGGA | 1671 | TCCTATTAGATCTGTC | 262 3 |
| 16 | H45_18 5-200 | ACAGATCTAATAGGAA | 1672 | TTCCTATTAGATCTGT | 262 4 |
| 16 | H45_18 6-201 | CAGATCTAATAGGAAT | 1673 | ATTCCTATTAGATCTG | 262 5 |
| 16 | H45_18 7-202 | AGATCTAATAGGAATG | 1674 | CATTCCTATTAGATCT | 262 6 |
| 16 | H45_18 8-203 | GATCTAATAGGAATGA | 1675 | TCATTCCTATTAGATC | 262 7 |
| 16 | H45_18 9-204 | ATCTAATAGGAATGAA | 1676 | TTCATTCCTATTAGAT | 262 8 |
| 16 | H45_19 0-205 | TCTAATAGGAATGAAA | 1677 | TTTCATTCCTATTAGA | 262 9 |
| 16 | H45_19 1-206 | CTAATAGGAATGAAAA | 1678 | TTTTCATTCCTATTAG | 263 0 |
| 16 | H45_19 2-207 | TAATAGGAATGAAAAC | 1679 | GTTTTCATTCCTATTA | 263 1 |
| 16 | H45_19 3-208 | AATAGGAATGAAAACA | 1680 | TGTTTTCATTCCTATT | 263 2 |

**[Table 7-6]**

| | | | | | |
|---|---|---|---|---|---|
| 16 | H45_19 4-209 | ATAGGAATGAAAACAT | 1681 | ATGTTTTCATTCCTAT | 263 3 |
| 16 | H45_19 5-210 | TAGGAATGAAAACATT | 1682 | AATGTTTTCATTCCTA | 263 4 |
| 16 | H45_19 6-211 | AGGAATGAAAACATTT | 1683 | AAATGTTTTCATTCCT | 263 5 |
| 16 | H45_19 7-212 | GGAATGAAAACATTTT | 1684 | AAAATGTTTTCATTCC | 263 6 |
| 16 | H45_19 8-213 | GAATGAAAACATTTTA | 1685 | TAAAATGTTTTCATTC | 263 7 |
| 16 | H45_19 9-214 | AATGAAAACATTTTAG | 1686 | CTAAAATGTTTTCATT | 263 8 |
| 16 | H45_20 0-215 | ATGAAAACATTTTAGC | 1687 | GCTAAAATGTTTTCAT | 263 9 |
| 16 | H45_20 1-216 | TGAAAACATTTTAGCA | 1688 | TGCTAAAATGTTTTCA | 264 0 |
| 16 | H45_20 2-217 | GAAAACATTTTAGCAG | 1689 | CTGCTAAAATGTTTTC | 264 1 |
| 16 | H45_20 3-218 | AAAACATTTTAGCAGA | 1690 | TCTGCTAAAATGTTTT | 264 2 |
| 16 | H45_20 4-219 | AAACATTTTAGCAGAC | 1691 | GTCTGCTAAAATGTTT | 264 3 |
| 16 | H45_20 5-220 | AACATTTTAGCAGACT | 1692 | AGTCTGCTAAAATGTT | 264 4 |
| 16 | H45_20 6-221 | ACATTTTAGCAGACTT | 1693 | AAGTCTGCTAAAATGT | 264 5 |
| 16 | H45_20 7-222 | CATTTTAGCAGACTTT | 1694 | AAAGTCTGCTAAAATG | 264 6 |
| 16 | H45_20 8-223 | ATTTTAGCAGACTTTT | 1695 | AAAAGTCTGCTAAAAT | 264 7 |
| 16 | H45_20 9-224 | TTTTAGCAGACTTTTT | 1696 | AAAAAGTCTGCTAAAA | 264 8 |
| 16 | H45_21 0-225 | TTTAGCAGACTTTTTA | 1697 | TAAAAAGTCTGCTAAA | 264 9 |
| 16 | H45_21 1-226 | TTAGCAGACTTTTTAA | 1698 | TTAAAAAGTCTGCTAA | 265 0 |

**[Table 7-7]**

| | | | | | |
|---|---|---|---|---|---|
| 16 | H45_21 2-227 | TAGCAGACTTTTTAAG | 1699 | CTTAAAAAGTCTGCTA | 265 1 |
| 16 | H45_21 3-228 | AGCAGACTTTTTAAGC | 1700 | GCTTAAAAAGTCTGCT | 265 2 |
| 16 | H45_21 4-229 | GCAGACTTTTTAAGCT | 1701 | AGCTTAAAAAGTCTGC | 265 3 |
| 16 | H45_21 5-230 | CAGACTTTTTAAGCTT | 1702 | AAGCTTAAAAAGTCTG | 265 4 |
| 16 | H45_21 6-231 | AGACTTTTTAAGCTTT | 1703 | AAAGCTTAAAAAGTCT | 265 5 |
| 16 | H45_21 7-232 | GACTTTTTAAGCTTTC | 1704 | GAAAGCTTAAAAAGTC | 265 6 |
| 16 | H45_21 8-233 | ACTTTTTAAGCTTTCT | 1705 | AGAAAGCTTAAAAAGT | 265 7 |
| 16 | H45_21 9-234 | CTTTTTAAGCTTTCTT | 1706 | AAGAAAGCTTAAAAAG | 265 8 |
| 16 | H45_22 0-235 | TTTTTAAGCTTTCTTT | 1707 | AAAGAAAGCTTAAAAA | 265 9 |
| 17 | H45_16 7-183 | GAAAAAAGAGGTAGGGC | 1708 | GCCCTACCTCTTTTTTC | 266 0 |
| 17 | H45_16 8-184 | AAAAAAGAGGTAGGGCG | 1709 | CGCCCTACCTCTTTTTT | 266 1 |
| 17 | H45_16 9-185 | AAAAAGAGGTAGGGCGA | 1710 | TCGCCCTACCTCTTTTT | 266 2 |
| 17 | H45_17 0-186 | AAAAGAGGTAGGGCGAC | 1711 | GTCGCCCTACCTCTTTT | 266 3 |
| 17 | H45_17 1-187 | AAAGAGGTAGGGCGACA | 1712 | TGTCGCCCTACCTCTTT | 266 4 |
| 17 | H45_17 2-188 | AAGAGGTAGGGCGACAG | 1713 | CTGTCGCCCTACCTCTT | 266 5 |
| 17 | H45_17 3-189 | AGAGGTAGGGCGACAGA | 1714 | TCTGTCGCCCTACCTCT | 266 6 |
| 17 | H45_17 4-190 | GAGGTAGGGCGACAGAT | 1715 | ATCTGTCGCCCTACCTC | 266 7 |
| 17 | H45_17 5-191 | AGGTAGGGCGACAGATC | 1716 | GATCTGTCGCCCTACCT | 266 8 |

**[Table 7-8]**

| | | | | | |
|---|---|---|---|---|---|
| 17 | H45_17 6-192 | GGTAGGGCGACAGATCT | 1717 | AGATCTGTCGCCCTACC | 266 9 |
| 17 | H45_17 7-193 | GTAGGGCGACAGATCTA | 1718 | TAGATCTGTCGCCCTAC | 267 0 |
| 17 | H45_17 8-194 | TAGGGCGACAGATCTAA | 1719 | TTAGATCTGTCGCCCTA | 267 1 |
| 17 | H45_17 9-195 | AGGGCGACAGATCTAAT | 1720 | ATTAGATCTGTCGCCCT | 267 2 |
| 17 | H45_18 0-196 | GGGCGACAGATCTAATA | 1721 | TATTAGATCTGTCGCCC | 267 3 |
| 17 | H45_18 1-197 | GGCGACAGATCTAATAG | 1722 | CTATTAGATCTGTCGCC | 267 4 |
| 17 | H45_18 2-198 | GCGACAGATCTAATAGG | 1723 | CCTATTAGATCTGTCGC | 267 5 |
| 17 | H45_18 3-199 | CGACAGATCTAATAGGA | 1724 | TCCTATTAGATCTGTCG | 267 6 |
| 17 | H45_18 4-200 | GACAGATCTAATAGGAA | 1725 | TTCCTATTAGATCTGTC | 267 7 |
| 17 | H45_18 5-201 | ACAGATCTAATAGGAAT | 1726 | ATTCCTATTAGATCTGT | 267 8 |
| 17 | H45_18 6-202 | CAGATCTAATAGGAATG | 1727 | CATTCCTATTAGATCTG | 267 9 |
| 17 | H45_18 7-203 | AGATCTAATAGGAATGA | 1728 | TCATTCCTATTAGATCT | 268 0 |
| 17 | H45_18 8-204 | GATCTAATAGGAATGAA | 1729 | TTCATTCCTATTAGATC | 268 1 |
| 17 | H45_18 9-205 | ATCTAATAGGAATGAAA | 1730 | TTTCATTCCTATTAGAT | 268 2 |
| 17 | H45_19 0-206 | TCTAATAGGAATGAAAA | 1731 | TTTTCATTCCTATTAGA | 268 3 |
| 17 | H45_19 1-207 | CTAATAGGAATGAAAAC | 1732 | GTTTTCATTCCTATTAG | 268 4 |
| 17 | H45_19 2-208 | TAATAGGAATGAAAACA | 1733 | TGTTTTCATTCCTATTA | 268 5 |
| 17 | H45_19 3-209 | AATAGGAATGAAAACAT | 1734 | ATGTTTTCATTCCTATT | 268 6 |

**[Table 7-9]**

| | | | | | |
|---|---|---|---|---|---|
| 17 | H45_19 4-210 | ATAGGAATGAAAACATT | 1735 | AATGTTTTCATTCCTAT | 268 7 |
| 17 | H45_19 5-211 | TAGGAATGAAAACATTT | 1736 | AAATGTTTTCATTCCTA | 268 8 |
| 17 | H45_19 6-212 | AGGAATGAAAACATTTT | 1737 | AAAATGTTTTCATTCCT | 268 9 |
| 17 | H45_19 7-213 | GGAATGAAAACATTTTA | 1738 | TAAAATGTTTTCATTCC | 269 0 |
| 17 | H45_19 8-214 | GAATG.AAAACATTTTAG | 1739 | CTAAAATGTTTTCATTC | 269 1 |
| 17 | H45_19 9-215 | AATGAAAACATTTTAGC | 1740 | GCTAAAATGTTTTCATT | 269 2 |
| 17 | H45_20 0-216 | ATGAAAACATTTTAGCA | 1741 | TGCTAAAATGTTTTCAT | 269 3 |
| 17 | H45_20 1-217 | TGAAAACATTTTAGCAG | 1742 | CTGCTAAAATGTTTTCA | 269 4 |
| 17 | H45_20 2-218 | GAAAACATTTTAGCAGA | 1743 | TCTGCTAAAATGTTTTC | 269 5 |
| 17 | H45_20 3-219 | AAAACATTTTAGCAGAC | 1744 | GTCTGCTAAAATGTTTT | 269 6 |
| 17 | H45_20 4-220 | AAACATTTTAGCAGACT | 1745 | AGTCTGCTAAAATGTTT | 269 7 |
| 17 | H45_20 5-221 | AACATTTTAGCAGACTT | 1746 | AAGTCTGCTAAAATGTT | 269 8 |
| 17 | H45_20 6-222 | ACATTTTAGCAGACTTT | 1747 | AAAGTCTGCTAAAATGT | 269 9 |
| 17 | H45_20 7-223 | CATTTTAGCAGACTTTT | 1748 | AAAAGTCTGCTAAAATG | 270 0 |
| 17 | H45_20 8-224 | ATTTTAGCAGACTTTTT | 1749 | AAAAAGTCTGCTAAAAT | 270 1 |
| 17 | H45_20 9-225 | TTTTAGCAGACTTTTTA | 1750 | TAAAAAGTCTGCTAAAA | 270 2 |
| 17 | H45_21 0-226 | TTTAGCAGACTTTTTAA | 1751 | TTAAAAAGTCTGCTAAA | 270 3 |
| 17 | H45_21 1-227 | TTAGCAGACTTTTTAAG | 1752 | CTTAAAAAGTCTGCTAA | 270 4 |

**[Table 7-10]**

| | | | | | |
|---|---|---|---|---|---|
| 17 | H45_21 2-228 | TAGCAGACTTTTTAAGC | 1753 | GCTTAAAAAGTCTGCTA | 270 5 |
| 17 | H45_21 3-229 | AGCAGACTTTTTAAGCT | 1754 | AGCTTAAAAAGTCTGCT | 270 6 |
| 17 | H45_21 4-230 | GCAGACTTTTTAAGCTT | 1755 | AAGCTTAAAAAGTCTGC | 270 7 |
| 17 | H45_21 5-231 | CAGACTTTTTAAGCTTT | 1756 | AAAGCTTAAAAAGTCTG | 270 8 |
| 17 | H45_21 6-232 | AGACTTTTTAAGCTTTC | 1757 | GAAAGCTTAAAAAGTCT | 270 9 |
| 17 | H45_21 7-233 | GACTTTTTAAGCTTTCT | 1758 | AGAAAGCTTAAAAAGTC | 271 0 |
| 17 | H45_21 8-234 | ACTTTTTAAGCTTTCTT | 1759 | AAGAAAGCTTAAAAAGT | 271 1 |
| 17 | H45_21 9-235 | CTTTTTAAGCTTTCTTT | 1760 | AAAGAAAGCTTAAAAAG | 271 2 |
| 17 | H45_22 0-236 | TTTTTAAGCTTTCTTTA | 1761 | TAAAGAAAGCTTAAAAA | 271 3 |
| 18 | H45_16 6-183 | AGAAAAAAGAGGTAGGGC | 1762 | GCCCTACCTCTTTTTTCT | 271 4 |
| 18 | H45_16 7-184 | GAAAAAAGAGGTAGGGCG | 1763 | CGCCCTACCTCTTTTTTC | 271 5 |
| 18 | H45_16 8-185 | AAAAAAGAGGTAGGGCGA | 1764 | TCGCCCTACCTCTTTTTT | 271 6 |
| 18 | H45_16 9-186 | AAAAAGAGGTAGGGCGAC | 1765 | GTCGCCCTACCTCTTTTT | 271 7 |
| 18 | H45_17 0-187 | AAAAGAGGTAGGGCGACA | 1766 | TGTCGCCCTACCTCTTTT | 271 8 |
| 18 | H45_17 1-188 | AAAGAGGTAGGGCGACAG | 1767 | CTGTCGCCCTACCTCTTT | 271 9 |
| 18 | H45_17 2-189 | AAGAGGTAGGGCGACAGA | 1768 | TCTGTCGCCCTACCTCTT | 272 0 |
| 18 | H45_17 3-190 | AGAGGTAGGGCGACAGAT | 1769 | ATCTGTCGCCCTACCTCT | 272 1 |
| 18 | H45_17 4-191 | GAGGTAGGGCGACAGATC | 1770 | GATCTGTCGCCCTACCTC | 272 2 |

**[Table 7-11]**

| | | | | | |
|---|---|---|---|---|---|
| 18 | H45_17 5-192 | AGGTAGGGCGACAGATCT | 1771 | AGATCTGTCGCCCTACCT | 272 3 |
| 18 | H45_17 6-193 | GGTAGGGCGACAGATCTA | 1772 | TAGATCTGTCGCCCTACC | 272 4 |
| 18 | H45_17 7-194 | GTAGGGCGACAGATCTAA | 1773 | TTAGATCTGTCGCCCTAC | 272 5 |
| 18 | H45_17 8-195 | TAGGGCGACAGATCTAAT | 1774 | ATTAGATCTGTCGCCCTA | 272 6 |
| 18 | H45_17 9-196 | AGGGCGACAGATCTAATA | 1775 | TATTAGATCTGTCGCCCT | 272 7 |
| 18 | H45_18 0-197 | GGGCGACAGATCTAATAG | 1776 | CTATTAGATCTGTCGCCC | 272 8 |
| 18 | H45_18 1-198 | GGCGACAGATCTAATAGG | 1777 | CCTATTAGATCTGTCGCC | 272 9 |
| 18 | H45_18 2-199 | GCGACAGATCTAATAGGA | 1778 | TCCTATTAGATCTGTCGC | 273 0 |
| 18 | H45_18 3-200 | CGACAGATCTAATAGGAA | 1779 | TTCCTATTAGATCTGTCG | 273 1 |
| 18 | H45_18 4-201 | GACAGATCTAATAGGAAT | 1780 | ATTCCTATTAGATCTGTC | 273 2 |
| 18 | H45_18 5-202 | ACAGATCTAATAGGAATG | 1781 | CATTCCTATTAGATCTGT | 273 3 |
| 18 | H45_18 6-203 | CAGATCTAATAGGAATGA | 1782 | TCATTCCTATTAGATCTG | 273 4 |
| 18 | H45_18 7-204 | AGATCTAATAGGAATGAA | 1783 | TTCATTCCTATTAGATCT | 273 5 |
| 18 | H45_18 8-205 | GATCTAATAGGAATGAAA | 1784 | TTTCATTCCTATTAGATC | 273 6 |
| 18 | H45_18 9-206 | ATCTAATAGGAATGAAAA | 1785 | TTTTCATTCCTATTAGAT | 273 7 |
| 18 | H45_19 0-207 | TCTAATAGGAATGAAAAC | 1786 | GTTTTCATTCCTATTAGA | 273 8 |
| 18 | H45_19 1-208 | CTAATAGGAATGAAAACA | 1787 | TGTTTTCATTCCTATTAG | 273 9 |
| 18 | H45_19 2-209 | TAATAGGAATGAAAACAT | 1788 | ATGTTTTCATTCCTATTA | 274 0 |

**[Table 7-12]**

| | | | | | |
|---|---|---|---|---|---|
| 18 | H45_19 3-210 | AATAGGAATGAAAACATT | 1789 | AATGTTTTCATTCCTATT | 274 1 |
| 18 | H45_19 4-211 | ATAGGAATGAAAACATTT | 1790 | AAATGTTTTCATTCCTAT | 274 2 |
| 18 | H45_19 5-212 | TAGGAATGAAAACATTTT | 1791 | AAAATGTTTTCATTCCTA | 274 3 |
| 18 | H45_19 6-213 | AGGAATGAAAACATTTTA | 1792 | TAAAATGTTTTCATTCCT | 274 4 |
| 18 | H45_19 7-214 | GGAATGAAAACATTTTAG | 1793 | CTAAAATGTTTTCATTCC | 274 5 |
| 18 | H45_19 8-215 | GAATGAAAACATTTTAGC | 1794 | GCTAAAATGTTTTCATTC | 274 6 |
| 18 | H45_19 9-216 | AATGAAAACATTTTAGCA | 1795 | TGCTAAAATGTTTTCATT | 274 7 |
| 18 | H45_20 0-217 | ATGAAAACATTTTAGCAG | 1796 | CTGCTAAAATGTTTTCAT | 274 8 |
| 18 | H45_20 1-218 | TGAAAACATTTTAGCAGA | 1797 | TCTGCTAAAATGTTTTCA | 274 9 |
| 18 | H45_20 2-219 | GAAAACATTTTAGCAGAC | 1798 | GTCTGCTAAAATGTTTTC | 275 0 |
| 18 | H45_20 3-220 | AAAACATTTTAGCAGACT | 1799 | AGTCTGCTAAAATGTTTT | 275 1 |
| 18 | H45_20 4-221 | AAACATTTTAGCAGACTT | 1800 | AAGTCTGCTAAAATGTTT | 275 2 |
| 18 | H45_20 5-222 | AACATTTTAGCAGACTTT | 1801 | AAAGTCTGCTAAAATGTT | 275 3 |
| 18 | H45_20 6-223 | ACATTTTAGCAGACTTTT | 1802 | AAAAGTCTGCTAAAATGT | 275 4 |
| 18 | H45_20 7-224 | CATTTTAGCAGACTTTTT | 1803 | AAAAAGTCTGCTAAAATG | 275 5 |
| 18 | H45_20 8-225 | ATTTTAGCAGACTTTTTA | 1804 | TAAAAAGTCTGCTAAAAT | 275 6 |
| 18 | H45_20 9-226 | TTTTAGCAGACTTTTTAA | 1805 | TTAAAAAGTCTGCTAAAA | 275 7 |
| 18 | H45_21 0-227 | TTTAGCAGACTTTTTAAG | 1806 | CTTAAAAAGTCTGCTAAA | 275 8 |

**[Table 7-13]**

| | | | | | |
|---|---|---|---|---|---|
| 18 | H45_21 1-228 | TTAGCAGACTTTTTAAGC | 1807 | GCTTAAAAAGTCTGCTAA | 275 9 |
| 18 | H45_21 2-229 | TAGCAGACTTTTTAAGCT | 1808 | AGCTTAAAAAGTCTGCTA | 276 0 |
| 18 | H45_21 3-230 | AGCAGACTTTTTAAGCTT | 1809 | AAGCTTAAAAAGTCTGCT | 276 1 |
| 18 | H45_21 4-231 | GCAGACTTTTTAAGCTTT | 1810 | AAAGCTTAAAAAGTCTGC | 276 2 |
| 18 | H45_21 5-232 | CAGACTTTTTAAGCTTTC | 1811 | GAAAGCTTAAAAAGTCTG | 276 3 |
| 18 | H45_21 6-233 | AGACTTTTTAAGCTTTCT | 1812 | AGAAAGCTTAAAAAGTCT | 276 4 |
| 18 | H45_21 7-234 | GACTTTTTAAGCTTTCTT | 1813 | AAGAAAGCTTAAAAAGTC | 276 5 |
| 18 | H45_21 8-235 | ACTTTTTAAGCTTTCTTT | 1814 | AAAGAAAGCTTAAAAAGT | 276 6 |
| 18 | H45_21 9-236 | CTTTTTAAGCTTTCTTTA | 1815 | TAAAGAAAGCTTAAAAAG | 276 7 |
| 18 | H45_22 0-237 | TTTTTAAGCTTTCTTTAG | 1816 | CTAAAGAAAGCTTAAAAA | 276 8 |
| 19 | H45_16 5-183 | CAGAAAAAAGAGGTAGGGC | 1817 | GCCCTACCTCTTTTTTCTG | 276 9 |
| 19 | H45_16 6-184 | AGAAAAAAGAGGTAGGGCG | 1818 | CGCCCTACCTCTTTTTTCT | 277 0 |
| 19 | H45_16 7-185 | GAAAAAAGAGGTAGGGCGA | 1819 | TCGCCCTACCTCTTTTTTC | 277 1 |
| 19 | H45_16 8-186 | AAAAAAGAGGTAGGGCGAC | 1820 | GTCGCCCTACCTCTTTTTT | 277 2 |
| 19 | H45_16 9-187 | AAAAAGAGGTAGGGCGACA | 1821 | TGTCGCCCTACCTCTTTTT | 277 3 |
| 19 | H45_17 0-188 | AAAAGAGGTAGGGCGACAG | 1822 | CTGTCGCCCTACCTCTTTT | 277 4 |
| 19 | H45_17 1-189 | AAAGAGGTAGGGCGACAGA | 1823 | TCTGTCGCCCTACCTCTTT | 277 5 |
| 19 | H45_17 2-190 | AAGAGGTAGGGCGACAGAT | 1824 | ATCTGTCGCCCTACCTCTT | 277 6 |

**[Table 7-14]**

| | | | | | |
|---|---|---|---|---|---|
| 19 | H45_17 3-191 | AGAGGTAGGGCGACAGATC | 1825 | GATCTGTCGCCCTACCTCT | 277 7 |
| 19 | H45_17 4-192 | GAGGTAGGGCGACAGATCT | 1826 | AGATCTGTCGCCCTACCTC | 277 8 |
| 19 | H45_17 5-193 | AGGTAGGGCGACAGATCTA | 1827 | TAGATCTGTCGCCCTACCT | 277 9 |
| 19 | H45_17 6-194 | GGTAGGGCGACAGATCTAA | 1828 | TTAGATCTGTCGCCCTACC | 278 0 |
| 19 | H45_17 7-195 | GTAGGGCGACAGATCTAAT | 1829 | ATTAGATCTGTCGCCCTAC | 278 1 |
| 19 | H45_17 8-196 | TAGGGCGACAGATCTAATA | 1830 | TATTAGATCTGTCGCCCTA | 278 2 |
| 19 | H45_17 9-197 | AGGGCGACAGATCTAATAG | 1831 | CTATTAGATCTGTCGCCCT | 278 3 |
| 19 | H45_18 0-198 | GGGCGACAGATCTAATAGG | 1832 | CCTATTAGATCTGTCGCCC | 278 4 |
| 19 | H45_18 1-199 | GGCGACAGATCTAATAGGA | 1833 | TCCTATTAGATCTGTCGCC | 278 5 |
| 19 | H45_18 2-200 | GCGACAGATCTAATAGGAA | 1834 | TTCCTATTAGATCTGTCGC | 278 6 |
| 19 | H45_18 3-201 | CGACAGATCTAATAGGAAT | 1835 | ATTCCTATTAGATCTGTCG | 278 7 |
| 19 | H45_18 4-202 | GACAGATCTAATAGGAATG | 1836 | CATTCCTATTAGATCTGTC | 278 8 |
| 19 | H45_18 5-203 | ACAGATCTAATAGGAATGA | 1837 | TCATTCCTATTAGATCTGT | 278 9 |
| 19 | H45_18 6-204 | CAGATCTAATAGGAATGAA | 1838 | TTCATTCCTATTAGATCTG | 279 0 |
| 19 | H45_18 7-205 | AGATCTAATAGGAATGAAA | 1839 | TTTCATTCCTATTAGATCT | 279 1 |
| 19 | H45_18 8-206 | GATCTAATAGGAATGAAAA | 1840 | TTTTCATTCCTATTAGATC | 279 2 |
| 19 | H45_18 9-207 | ATCTAATAGGAATGAAAAC | 1841 | GTTTTCATTCCTATTAGAT | 279 3 |
| 19 | H45_19 0-208 | TCTAATAGGAATGAAAACA | 1842 | TGTTTTCATTCCTATTAGA | 279 4 |

**[Table 7-15]**

| | | | | | |
|---|---|---|---|---|---|
| 19 | H45_19 1-209 | CTAATAGGAATGAAAACAT | 1843 | ATGTTTTCATTCCTATTAG | 279 5 |
| 19 | H45_19 2-210 | TAATAGGAATGAAAACATT | 1844 | AATGTTTTCATTCCTATTA | 279 6 |
| 19 | H45_19 3-211 | AATAGGAATGAAAACATTT | 1845 | AAATGTTTTCATTCCTATT | 279 7 |
| 19 | H45_19 4-212 | ATAGGAATGAAAACATTTT | 1846 | AAAATGTTTTCATTCCTAT | 279 8 |
| 19 | H45_19 5-213 | TAGGAATGAAAACATTTTA | 1847 | TAAAATGTTTTCATTCCTA | 279 9 |
| 19 | H45_19 6-214 | AGGAATGAAAACATTTTAG | 1848 | CTAAAATGTTTTCATTCCT | 280 0 |
| 19 | H45_19 7-215 | GGAATGAAAACATTTTAGC | 1849 | GCTAAAATGTTTTCATTCC | 280 1 |
| 19 | H45_19 8-216 | GAATGAAAACATTTTAGCA | 1850 | TGCTAAAATGTTTTCATTC | 280 2 |
| 19 | H45_19 9-217 | AATGAAAACATTTTAGCAG | 1851 | CTGCTAAAATGTTTTCATT | 280 3 |
| 19 | H45_20 0-218 | ATGAAAACATTTTAGCAGA | 1852 | TCTGCTAAAATGTTTTCAT | 280 4 |
| 19 | H45_20 1-219 | TGAAAACATTTTAGCAGAC | 1853 | GTCTGCTAAAATGTTTTCA | 280 5 |
| 19 | H45_20 2-220 | GAA.AACATTTTAGCAGACT | 1854 | AGTCTGCTAAAATGTTTTC | 280 6 |
| 19 | H45_20 3-221 | AAAACATTTTAGCAGACTT | 1855 | AAGTCTGCTAAAATGTTTT | 280 7 |
| 19 | H45_20 4-222 | AAACATTTTAGCAGACTTT | 1856 | AAAGTCTGCTAAAATGTTT | 280 8 |
| 19 | H45_20 5-223 | AACATTTTAGCAGACTTTT | 1857 | AAAAGTCTGCTAAAATGTT | 280 9 |
| 19 | H45_20 6-224 | ACATTTTAGCAGACTTTTT | 1858 | AAAAAGTCTGCTAAAATGT | 281 0 |
| 19 | H45_20 7-225 | CATTTTAGCAGACTTTTTA | 1859 | TAAAE1AGTCTGCTAAAATG | 281 1 |
| 19 | H45_20 8-226 | ATTTTAGCAGACTTTTTAA | 1860 | TTAAAAAGTCTGCTAAAAT | 281 2 |

**[Table 7-16]**

| | | | | | |
|---|---|---|---|---|---|
| 19 | H45_20 9-227 | TTTTAGCAGACTTTTTAAG | 1861 | CTTAAAAAGTCTGCTAAAA | 281 3 |
| 19 | H45_21 0-228 | TTTAGCAGACTTTTTAAGC | 1862 | GCTTAAAAAGTCTGCTAAA | 281 4 |
| 19 | H45_21 1-229 | TTAGCAGACTTTTTAAGCT | 1863 | AGCTTAAAAAGTCTGCTAA | 281 5 |
| 19 | H45_21 2-230 | TAGCAGACTTTTTAAGCTT | 1864 | AAGCTTAAAAAGTCTGCTA | 281 6 |
| 19 | H45_21 3-231 | AGCAGACTTTTTAAGCTTT | 1865 | AAAGCTTAAAAAGTCTGCT | 281 7 |
| 19 | H45_21 4-232 | GCAGACTTTTTAAGCTTTC | 1866 | GAAAGCTTAAAAAGTCTGC | 281 8 |
| 19 | H45_21 5-233 | CAGACTTTTTAAGCTTTCT | 1867 | AGAAAGCTTAAAAAGTCTG | 281 9 |
| 19 | H45_21 6-234 | AGACTTTTTAAGCTTTCTT | 1868 | AAGAAAGCTTAAAAAGTCT | 282 0 |
| 19 | H45_21 7-235 | GACTTTTTAAGCTTTCTTT | 1869 | AAAGAAAGCTTAAAAAGTC | 282 1 |
| 19 | H45_21 8-236 | ACTTTTTAAGCTTTCTTTA | 1870 | TAAAGAAAGCTTAAAAAGT | 282 2 |
| 19 | H45_21 9-237 | CTTTTTAAGCTTTCTTTAG | 1871 | CTAAAGAAAGCTTAAAAAG | 282 3 |
| 19 | H45_22 0-238 | TTTTTAAGCTTTCTTTAGA | 1872 | TCTAAAGAAAGCTTAAAAA | 282 4 |
| 20 | H45_16 4-183 | ACAGAAAAAAGAGGTAGGGC | 1873 | GCCCTACCTCTTTTTTCTGT | 282 5 |
| 20 | H45_16 5-184 | CAGAAAAAAGAGGTAGGGCG | 1874 | CGCCCTACCTCTTTTTTCTG | 282 6 |
| 20 | H45_16 6-185 | AGAAAAAAGAGGTAGGGCGA | 1875 | TCGCCCTACCTCTTTTTTCT | 282 7 |
| 20 | H45_16 7-186 | GAAAAAAGAGGTAGGGCGAC | 1876 | GTCGCCCTACCTCTTTTTTC | 282 8 |
| 20 | H45_16 8-187 | AA.AAAAGAGGTAGGGCGACA | 1877 | TGTCGCCCTACCTCTTTTTT | 282 9 |
| 20 | H45_16 9-188 | AAAAAGAGGTAGGGCGACAG | 1878 | CTGTCGCCCTACCTCTTTTT | 283 0 |

**[Table 7-17]**

| | | | | | |
|---|---|---|---|---|---|
| 20 | H45_17 0-189 | AAAAGAGGTAGGGCGACAGA | 1879 | TCTGTCGCCCTACCTCTTTT | 283 1 |
| 20 | H45_17 1-190 | AAAGAGGTAGGGCGACAGAT | 1880 | ATCTGTCGCCCTACCTCTTT | 283 2 |
| 20 | H45_17 2-191 | AAGAGGTAGGGCGACAGATC | 1881 | GATCTGTCGCCCTACCTCTT | 283 3 |
| 20 | H45_17 3-192 | AGAGGTAGGGCGACAGATCT | 1882 | AGATCTGTCGCCCTACCTCT | 283 4 |
| 20 | H45_17 4-193 | GAGGTAGGGCGACAGATCTA | 1883 | TAGATCTGTCGCCCTACCTC | 283 5 |
| 20 | H45_17 5-194 | AGGTAGGGCGACAGATCTAA | 1884 | TTAGATCTGTCGCCCTACCT | 283 6 |
| 20 | H45_17 6-195 | GGTAGGGCGACAGATCTAAT | 1885 | ATTAGATCTGTCGCCCTACC | 283 7 |
| 20 | H45_17 7-196 | GTAGGGCGACAGATCTAATA | 1886 | TATTAGATCTGTCGCCCTAC | 283 8 |
| 20 | H45_17 8-197 | TAGGGCGACAGATCTAATAG | 1887 | CTATTAGATCTGTCGCCCTA | 283 9 |
| 20 | H45_17 9-198 | AGGGCGACAGATCTAATAGG | 1888 | CCTATTAGATCTGTCGCCCT | 284 0 |
| 20 | H45_18 0-199 | GGGCGACAGATCTAATAGGA | 1889 | TCCTATTAGATCTGTCGCCC | 284 1 |
| 20 | H45_18 1-200 | GGCGACAGATCTAATAGGAA | 1890 | TTCCTATTAGATCTGTCGCC | 284 2 |
| 20 | H45_18 2-201 | GCGACAGATCTAATAGGAAT | 1891 | ATTCCTATTAGATCTGTCGC | 284 3 |
| 20 | H45_18 3-202 | CGACAGATCTAATAGGAATG | 1892 | CATTCCTATTAGATCTGTCG | 284 4 |
| 20 | H45_18 4-203 | GACAGATCTAATAGGAATGA | 1893 | TCATTCCTATTAGATCTGTC | 284 5 |
| 20 | H45_18 5-204 | ACAGATCTAATAGGAATGAA | 1894 | TTCATTCCTATTAGATCTGT | 284 6 |
| 20 | H45_18 6-205 | CAGATCTAATAGGAATGAAA | 1895 | TTTCATTCCTATTAGATCTG | 284 7 |
| 20 | H45_18 7-206 | AGATCTAATAGGAATGAAAA | 1896 | TTTTCATTCCTATTAGATCT | 284 8 |

**[Table 7-18]**

| | | | | | |
|---|---|---|---|---|---|
| 20 | H45_18 8-207 | GATCTAATAGGAATGAAAAC | 1897 | GTTTTCATTCCTATTAGATC | 284 9 |
| 20 | H45_18 9-208 | ATCTAATAGGAATGAAAACA | 1898 | TGTTTTCATTCCTATTAGAT | 285 0 |
| 20 | H45_19 0-209 | TCTAATAGGAATGAAAACAT | 1899 | ATGTTTTCATTCCTATTAGA | 285 1 |
| 20 | H45_19 1-210 | CT.AATAGGAATGAAAACATT | 1900 | AATGTTTTCATTCCTATTAG | 285 2 |
| 20 | H45_19 2-211 | TAATAGGAATGAAAACATTT | 1901 | AAATGTTTTCATTCCTATTA | 285 3 |
| 20 | H45_19 3-212 | AATAGGAATGAAAACATTTT | 1902 | AAAATGTTTTCATTCCTATT | 285 4 |
| 20 | H45_19 4-213 | ATAGGAATGAAAACATTTTA | 1903 | TAAAATGTTTTCATTCCTAT | 285 5 |
| 20 | H45_19 5-214 | TAGGAATGAAAACATTTTAG | 1904 | CTAAAATGTTTTCATTCCTA | 285 6 |
| 20 | H45_19 6-215 | AGGAATGAAAACATTTTAGC | 1905 | GCTAAAATGTTTTCATTCCT | 285 7 |
| 20 | H45_19 7-216 | GGAATGAAAACATTTTAGCA | 1906 | TGCTAAAATGTTTTCATTCC | 285 8 |
| 20 | H45_19 8-217 | GAATGAAAACATTTTAGCAG | 1907 | CTGCTAAAATGTTTTCATTC | 285 9 |
| 20 | H45_19 9-218 | AATGAAAACATTTTAGCAGA | 1908 | TCTGCTAAAATGTTTTCATT | 286 0 |
| 20 | H45_20 0-219 | ATGAAAACATTTTAGCAGAC | 1909 | GTCTGCTAAAATGTTTTCAT | 286 1 |
| 20 | H45_20 1-220 | TGAAAACATTTTAGCAGACT | 1910 | AGTCTGCTAAAATGTTTTCA | 286 2 |
| 20 | H45_20 2-221 | GAAAACATTTTAGCAGACTT | 1911 | AAGTCTGCTAAAATGTTTTC | 286 3 |
| 20 | H45_20 3-222 | AAAACATTTTAGCAGACTTT | 1912 | AAAGTCTGCTAAAATGTTTT | 286 4 |
| 20 | H45_20 4-223 | AAACATTTTAGCAGACTTTT | 1913 | AAAAGTCTGCTAAAATGTTT | 286 5 |
| 20 | H45_20 5-224 | AACATTTTAGCAGACTTTTT | 1914 | AAAAAGTCTGCTAAAATGTT | 286 6 |

**[Table 7-19]**

| | | | | | |
|---|---|---|---|---|---|
| 20 | H45_20 6-225 | ACATTTTAGCAGACTTTTTA | 1915 | TAAAAAGTCTGCTAAAATGT | 286 7 |
| 20 | H45_20 7-226 | CATTTTAGCAGACTTTTTAA | 1916 | TTAAAAAGTCTGCTAAAATG | 286 8 |
| 20 | H45_20 8-227 | ATTTTAGCAGACTTTTTAAG | 1917 | CTTAAAAAGTCTGCTAAAAT | 286 9 |
| 20 | H45_20 9-228 | TTTTAGCAGACTTTTTAAGC | 1918 | GCTTAAAAAGTCTGCTAAAA | 287 0 |
| 20 | H45_21 0-229 | TTTAGCAGACTTTTTAAGCT | 1919 | AGCTTAAAAAGTCTGCTAAA | 287 1 |
| 20 | H45_21 1-230 | TTAGCAGACTTTTTAAGCTT | 1920 | AAGCTTAAAAAGTCTGCTAA | 287 2 |
| 20 | H45_21 2-231 | TAGCAGACTTTTTAAGCTTT | 1921 | AAAGCTTAAAAAGTCTGCTA | 287 3 |
| 20 | H45_21 3-232 | AGCAGACTTTTTAAGCTTTC | 1922 | GAAAGCTTAAAAAGTCTGCT | 287 4 |
| 20 | H45_21 4-233 | GCAGACTTTTTAAGCTTTCT | 1923 | AGAAAGCTTAAAAAGTCTGC | 287 5 |
| 20 | H45_21 5-234 | CAGACTTTTTAAGCTTTCTT | 1924 | AAGAAAGCTTAAAAAGTCTG | 287 6 |
| 20 | H45_21 6-235 | AGACTTTTTAAGCTTTCTTT | 1925 | AAAGAAAGCTTAAAAAGTCT | 287 7 |
| 20 | H45_21 7-236 | GACTTTTTAAGCTTTCTTTA | 1926 | TAAAGAAAGCTTAAAAAGTC | 287 8 |
| 20 | H45_21 8-237 | ACTTTTTAAGCTTTCTTTAG | 1927 | CTAAAGAAAGCTTAAAAAGT | 287 9 |
| 20 | H45_21 9-238 | CTTTTTAAGCTTTCTTTAGA | 1928 | TCTAAAGAAAGCTTAAAAAG | 288 0 |
| 20 | H45_22 0-239 | TTTTTAAGCTTTCTTTAGAA | 1929 | TTCTAAAGAAAGCTTAAAAA | 288 1 |
| 21 | H45_16 3-183 | GACAGAAAAAAGAGGTAGGGC | 1930 | GCCCTACCTCTTTTTTCTGTC | 288 2 |
| 21 | H45_16 4-184 | ACAGAAAAAAGAGGTAGGGCG | 1931 | CGCCCTACCTCTTTTTTCTGT | 288 3 |
| 21 | H45_16 5-185 | CAGAAAAAAGAGGTAGGGCGA | 1932 | TCGCCCTACCTCTTTTTTCTG | 288 4 |

**[Table 7-20]**

| | | | | | |
|---|---|---|---|---|---|
| 21 | H45_16 6-186 | AGAAAAAAGAGGTAGGGCGAC | 1933 | GTCGCCCTACCTCTTTTTTCT | 288 5 |
| 21 | H45_16 7-187 | GAAAAAAGAGGTAGGGCGACA | 1934 | TGTCGCCCTACCTCTTTTTTC | 288 6 |
| 21 | H45_16 8-188 | AAAAAAGAGGTAGGGCGACAG | 1935 | CTGTCGCCCTACCTCTTTTTT | 288 7 |
| 21 | H45_16 9-189 | AAAAAGAGGTAGGGCGACAGA | 1936 | TCTGTCGCCCTACCTCTTTTT | 288 8 |
| 21 | H45_17 0-190 | AAAAGAGGTAGGGCGACAGAT | 1937 | ATCTGTCGCCCTACCTCTTTT | 288 9 |
| 21 | H45_17 1-191 | AAAGAGGTAGGGCGACAGATC | 1938 | GATCTGTCGCCCTACCTCTTT | 289 0 |
| 21 | H45_17 2-192 | AAGAGGTAGGGCGACAGATCT | 1939 | AGATCTGTCGCCCTACCTCTT | 289 1 |
| 21 | H45_17 3-193 | AGAGGTAGGGCGACAGATCTA | 1940 | TAGATCTGTCGCCCTACCTCT | 289 2 |
| 21 | H45_17 4-194 | GAGGTAGGGCGACAGATCTAA | 1941 | TTAGATCTGTCGCCCTACCTC | 289 3 |
| 21 | H45_17 5-195 | AGGTAGGGCGACAGATCTAAT | 1912 | ATTAGATCTGTCGCCCTACCT | 289 4 |
| 21 | H45_17 6-196 | GGTAGGGCGACAGATCTAATA | 1943 | TATTAGATCTGTCGCCCTACC | 289 5 |
| 21 | H45_17 7-197 | GTAGGGCGACAGATCTAATAG | 1944 | CTATTAGATCTGTCGCCCTAC | 289 6 |
| 21 | H45_17 8-198 | TAGGGCGACAGATCTAATAGG | 1945 | CCTATTAGATCTGTCGCCCTA | 289 7 |
| 21 | H45_17 9-199 | AGGGCGACAGATCT.AATAGGA | 1946 | TCCTATTAGATCTGTCGCCCT | 289 8 |
| 21 | H45_18 0-200 | GGGCGACAGATCTAATAGGAA | 1947 | TTCCTATTAGATCTGTCGCCC | 289 9 |
| 21 | H45_18 1-201 | GGCGACAGATCTAATAGGAAT | 1948 | ATTCCTATTAGATCTGTCGCC | 290 0 |
| 21 | H45_18 2-202 | GCGACAGATCTAATAGGAATG | 1949 | CATTCCTATTAGATCTGTCGC | 290 1 |
| 21 | H45_18 3-203 | CGACAGATCTAATAGGAATGA | 1950 | TCATTCCTATTAGATCTGTCG | 290 2 |

**[Table 7-21]**

| | | | | | |
|---|---|---|---|---|---|
| 21 | H45_18 4-204 | GACAGATCTAATAGGAATGAA | 1951 | TTCATTCCTATTAGATCTGTC | 290 3 |
| 21 | H45_18 5-205 | ACAGATCTAATAGGAATGAAA | 1952 | TTTCATTCCTATTAGATCTGT | 290 4 |
| 21 | H45_18 6-206 | CAGATCTAATAGGAATGAAAA | 1953 | TTTTCATTCCTATTAGATCTG | 290 5 |
| 21 | H45_18 7-207 | AGATCTAATAGGAATGAAAAC | 1954 | GTTTTCATTCCTATTAGATCT | 290 6 |
| 21 | H45_18 8-208 | GATCTAATAGGAATGAAAACA | 1955 | TGTTTTCATTCCTATTAGATC | 290 7 |
| 21 | H45_18 9-209 | ATCTAATAGGAATGAAAACAT | 1956 | ATGTTTTCATTCCTATTAGAT | 290 8 |
| 21 | H45_19 0-210 | TCTAATAGGAATGAAAACATT | 1957 | AATGTTTTCATTCCTATTAGA | 290 9 |
| 21 | H45_19 1-211 | CTAATAGGAATGAAAACATTT | 1958 | AAATGTTTTCATTCCTATTAG | 291 0 |
| 21 | H45_19 2-212 | TAATAGGAATGAAAACATTTT | 1959 | AAAATGTTTTCATTCCTATTA | 291 1 |
| 21 | H45_19 3-213 | AATAGGAATGAAAACATTTTA | 1960 | TAAAATGTTTTCATTCCTATT | 291 2 |
| 21 | H45_19 4-214 | ATAGGAATGAAAACATTTTAG | 1961 | CTAAAATGTTTTCATTCCTAT | 291 3 |
| 21 | H45_19 5-215 | TAGGAATGAAAACATTTTAGC | 1962 | GCTAAAATGTTTTCATTCCTA | 291 4 |
| 21 | H45_19 6-216 | AGGAATGAAAACATTTTAGCA | 1963 | TGCTAAAATGTTTTCATTCCT | 291 5 |
| 21 | H45_19 7-217 | GGAATGAAAACATTTTAGCAG | 1964 | CTGCTAAAATGTTTTCATTCC | 291 6 |
| 21 | H45_19 8-218 | GAATGAAAACATTTTAGCAGA | 1965 | TCTGCTAAAATGTTTTCATTC | 291 7 |
| 21 | H45_19 9-219 | tIATGAAAACATTTTAGCAGAC | 1966 | GTCTGCTAAAATGTTTTCATT | 291 8 |
| 21 | H45_20 0-220 | ATGAAAACATTTTAGCAGACT | 1967 | AGTCTGCTAAAATGTTTTCAT | 291 9 |
| 21 | H45_20 1-221 | TGAAAACATTTTAGCAGACTT | 1968 | AAGTCTGCTAAAATGTTTTCA | 292 0 |

**[Table 7-22]**

| | | | | | |
|---|---|---|---|---|---|
| 21 | H45_20 2-222 | GAAAACATTTTAGCAGACTTT | 1969 | AAAGTCTGCTAAAATGTTTTC | 292 1 |
| 21 | H45_20 3-223 | AAAACATTTTAGCAGACTTTT | 1970 | AAAAGTCTGCTAAAATGTTTT | 292 2 |
| 21 | H45_20 4-224 | AAACATTTTAGCAGACTTTTT | 1971 | AAAAAGTCTGCTAAAATGTTT | 292 3 |
| 21 | H45_20 5-225 | AACATTTTAGCAGACTTTTTA | 1972 | TAAAAAGTCTGCTAAAATGTT | 292 4 |
| 21 | H45_20 6-226 | ACATTTTAGCAGACTTTTTAA | 1973 | TTAAAAAGTCTGCTAAAATGT | 292 5 |
| 21 | H45_20 7-227 | CATTTTAGCAGACTTTTTAAG | 1974 | CTTAAAAAGTCTGCTAAAATG | 292 6 |
| 21 | H45_20 8-228 | ATTTTAGCAGACTTTTTAAGC | 1975 | GCTTAAAAAGTCTGCTAAAAT | 292 7 |
| 21 | H45_20 9-229 | TTTTAGCAGACTTTTTAAGCT | 1976 | AGCTTAAAAAGTCTGCTAAAA | 292 8 |
| 21 | H45_21 0-230 | TTTAGCAGACTTTTTAAGCTT | 1977 | AAGCTTAAAAAGTCTGCTAAA | 292 9 |
| 21 | H45_21 1-231 | TTAGCAGACTTTTTAAGCTTT | 1978 | AAAGCTTAAAAAGTCTGCTAA | 293 0 |
| 21 | H45_21 2-232 | TAGCAGACTTTTTAAGCTTTC | 1979 | GAAAGCTTAAAAAGTCTGCTA | 293 1 |
| 21 | H45_21 3-233 | AGCAGACTTTTTAAGCTTTCT | 1980 | AGAAAGCTTAAAAAGTCTGCT | 293 2 |
| 21 | H45_21 4-234 | GCAGACTTTTTAAGCTTTCTT | 1981 | AAGAAAGCTTAAAAAGTCTGC | 293 3 |
| 21 | H45_21 5-235 | CAGACTTTTTAAGCTTTCTTT | 1982 | AAAGAAAGCTTAAAAAGTCTG | 293 4 |
| 21 | H45_21 6-236 | AGACTTTTTAAGCTTTCTTTA | 1983 | TAAAGAAAGCTTAAAAAGTCT | 293 5 |
| 21 | H45_21 7-237 | GACTTTTTAAGCTTTCTTTAG | 1984 | CTAAAGAAAGCTTAAAAAGTC | 293 6 |
| 21 | H45_21 8-238 | ACTTTTTAAGCTTTCTTTAGA | 1985 | TCTAAAGAAAGCTTAAAAAGT | 293 7 |
| 21 | H45_21 9-239 | CTTTTTAAGCTTTCTTTAGAA | 1986 | TTCTAAAGAAAGCTTAAAAAG | 293 8 |

**[Table 7-23]**

| | | | | | |
|---|---|---|---|---|---|
| 21 | H45_22 0-240 | TTTTTAAGCTTTCTTTAGAAG | 1987 | CTTCTAAAGAAAGCTTAAAAA | 293 9 |
| 22 | H45_16 2-183 | AGACAGAAAAAAGAGGTAGGGC | 1988 | GCCCTACCTCTTTTTTCTGTCT | 294 0 |
| 22 | H45_16 3-184 | GACAGAAAAAAGAGGTAGGGCG | 1989 | CGCCCTACCTCTTTTTTCTGTC | 294 1 |
| 22 | H45_16 4-185 | ACAGAAAAAAGAGGTAGGGCGA | 1990 | TCGCCCTACCTCTTTTTTCTGT | 294 2 |
| 22 | H45_16 5-186 | CAGAAAAAAGAGGTAGGGCGAC | 1991 | GTCGCCCTACCTCTTTTTTCTG | 294 3 |
| 22 | H45_16 6-187 | AGAAAAAAGAGGTAGGGCGACA | 1992 | TGTCGCCCTACCTCTTTTTTCT | 294 4 |
| 22 | H45_16 7-188 | GAAAAAAGAGGTAGGGCGACAG | 1993 | CTGTCGCCCTACCTCTTTTTTC | 294 5 |
| 22 | H45_16 8-189 | AAAAAAGAGGTAGGGCGACAGA | 1994 | TCTGTCGCCCTACCTCTTTTTT | 294 6 |
| 22 | H45_16 9-190 | AAAAAGAGGTAGGGCGACAGAT | 1995 | ATCTGTCGCCCTACCTCTTTTT | 294 7 |
| 22 | H45_17 0-191 | AAAAGAGGTAGGGCGACAGATC | 1996 | GATCTGTCGCCCTACCTCTTTT | 294 8 |
| 22 | H45_17 1-192 | AAAGAGGTAGGGCGACAGATCT | 1997 | AGATCTGTCGCCCTACCTCTTT | 294 9 |
| 22 | H45_17 2-193 | AAGAGGTAGGGCGACAGATCTA | 1998 | TAGATCTGTCGCCCTACCTCTT | 295 0 |
| 22 | H45_17 3-194 | AGAGGTAGGGCGACAGATCTAA | 1999 | TTAGATCTGTCGCCCTACCTCT | 295 1 |
| 22 | H45_17 4-195 | GAGGTAGGGCGACAGATCTAAT | 2000 | ATTAGATCTGTCGCCCTACCTC | 295 2 |
| 22 | H45_17 5-196 | AGGTAGGGCGACAGATCTAATA | 2001 | TATTAGATCTGTCGCCCTACCT | 295 3 |
| 22 | H45_17 6-197 | GGTAGGGCGACAGATCTAATAG | 2002 | CTATTAGATCTGTCGCCCTACC | 295 4 |
| 22 | H45_17 7-198 | GTAGGGCGACAGATCTAATAGG | 2003 | CCTATTAGATCTGTCGCCCTAC | 295 5 |
| 22 | H45_17 8-199 | TAGGGCGACAGATCTAATAGGA | 2004 | TCCTATTAGATCTGTCGCCCTA | 295 6 |

**[Table 7-24]**

| | | | | | |
|---|---|---|---|---|---|
| 22 | H45_17 9-200 | AGGGCGACAGATCTAATAGGAA | 2005 | TTCCTATTAGATCTGTCGCCCT | 295 7 |
| 22 | H45_18 0-201 | GGGCGACAGATCTAATAGGAAT | 2006 | ATTCCTATTAGATCTGTCGCCC | 295 8 |
| 22 | H45_18 1-202 | GGCGACAGATCTAATAGGAATG | 2007 | CATTCCTATTAGATCTGTCGCC | 295 9 |
| 22 | H45_18 2-203 | GCGACAGATCTAATAGGAATGA | 2008 | TCATTCCTATTAGATCTGTCGC | 296 0 |
| 22 | H45_18 3-204 | CGACAGATCTAATAGGAATGAA | 2009 | TTCATTCCTATTAGATCTGTCG | 296 1 |
| 22 | H45_18 4-205 | GACAGATCTAATAGGAATGAAA | 2010 | TTTCATTCCTATTAGATCTGTC | 296 2 |
| 22 | H45_18 5-206 | ACAGATCTAATAGGAATGAAAA | 2011 | TTTTCATTCCTATTAGATCTGT | 296 3 |
| 22 | H45_18 6-207 | CAGATCTAATAGGAATGAAAAC | 2012 | GTTTTCATTCCTATTAGATCTG | 296 4 |
| 22 | H45_18 7-208 | AGATCTAATAGGAATGAAAACA | 2013 | TGTTTTCATTCCTATTAGATCT | 296 5 |
| 22 | H45_18 8-209 | GATCTAATAGGAATGAAAACAT | 2014 | ATGTTTTCATTCCTATTAGATC | 296 6 |
| 22 | H45_18 9-210 | ATCTAATAGGAATGAAAACATT | 2015 | AATGTTTTCATTCCTATTAGAT | 296 7 |
| 22 | H45_19 0-211 | TCTAATAGGAATGAAAACATTT | 2016 | AAATGTTTTCATTCCTATTAGA | 296 8 |
| 22 | H45_19 1-212 | CTAATAGGAATGAAAACATTTT | 2017 | AAAATGTTTTCATTCCTATTAG | 296 9 |
| 22 | H45_19 2-213 | TAATAGGAATGAAAACATTTTA | 2018 | TAAAATGTTTTCATTCCTATTA | 297 0 |
| 22 | H45_19 3-214 | AATAGGAATGAAAACATTTTAG | 2019 | CTAAAATGTTTTCATTCCTATT | 297 1 |
| 22 | H45_19 4-215 | ATAGGAATGAAAACATTTTAGC | 2020 | GCTAAAATGTTTTCATTCCTAT | 297 2 |
| 22 | H45_19 5-216 | TAGGAATGAAAACATTTTAGCA | 2021 | TGCTAAAATGTTTTCATTCCTA | 297 3 |
| 22 | H45_19 6-217 | AGGAATGAAAACATTTTAGCAG | 2022 | CTGCTAAAATGTTTTCATTCCT | 297 4 |

**[Table 7-25]**

| | | | | | |
|---|---|---|---|---|---|
| 22 | H45_19 7-218 | GGAATGAAAACATTTTAGCAGA | 2023 | TCTGCTAAAATGTTTTCATTCC | 297 5 |
| 22 | H45_19 8-219 | GAATGAAAACATTTTAGCAGAC | 2024 | GTCTGCTAAAATGTTTTCATTC | 297 6 |
| 22 | H45_19 9-220 | AATGAAAACATTTTAGCAGACT | 2025 | AGTCTGCTAAAATGTTTTCATT | 297 7 |
| 22 | H45_20 0-221 | ATGAAAACATTTTAGCAGACTT | 2026 | AAGTCTGCTAAAATGTTTTCAT | 297 8 |
| 22 | H45_20 1-222 | TGAAAACATTTTAGCAGACTTT | 2027 | AAAGTCTGCTAAAATGTTTTCA | 297 9 |
| 22 | H45_20 2-223 | GAAAACATTTTAGCAGACTTTT | 2028 | AAAAGTCTGCTAAAATGTTTTC | 298 0 |
| 22 | H45_20 3-224 | AAAACATTTTAGCAGACTTTTT | 2029 | AAAAAGTCTGCTAAAATGTTTT | 298 1 |
| 22 | H45_20 4-225 | AAACATTTTAGCAGACTTTTTA | 2030 | TAAAAAGTCTGCTAAAATGTTT | 298 2 |
| 22 | H45_20 5-226 | AACATTTTAGCAGACTTTTTAA | 2031 | TTAAAAAGTCTGCTAAAATGTT | 298 3 |
| 22 | H45_20 6-227 | ACATTTTAGCAGACTTTTTAAG | 2032 | CTTAAAAAGTCTGCTAAAATGT | 298 4 |
| 22 | H45_20 7-228 | CATTTTAGCAGACTTTTTAAGC | 2033 | GCTTAAAAAGTCTGCTAAAATG | 298 5 |
| 22 | H45_20 8-229 | ATTTTAGCAGACTTTTTAAGCT | 2034 | AGCTTAAAAAGTCTGCTAAAAT | 298 6 |
| 22 | H45_20 9-230 | TTTTAGCAGACTTTTTAAGCTT | 2035 | AAGCTTAAAAAGTCTGCTAAAA | 298 7 |
| 22 | H45_21 0-231 | TTTAGCAGACTTTTTAAGCTTT | 2036 | AAAGCTTAAAAAGTCTGCTAAA | 298 8 |
| 22 | H45_21 1-232 | TTAGCAGACTTTTTAAGCTTTC | 2037 | GAAAGCTTAAAAAGTCTGCTAA | 298 9 |
| 22 | H45_21 2-233 | TAGCAGACTTTTTAAGCTTTCT | 2038 | AGAAAGCTTAAAAAGTCTGCTA | 299 0 |
| 22 | H45_21 3-234 | AGCAGACTTTTTAAGCTTTCTT | 2039 | AAGAAAGCTTAAAAAGTCTGCT | 299 1 |
| 22 | H45_21 4-235 | GCAGACTTTTTAAGCTTTCTTT | 2040 | AAAGAAAGCTTAAAAAGTCTGC | 299 2 |

**[Table 7-26]**

| | | | | | |
|---|---|---|---|---|---|
| 22 | H45_21 5-236 | CAGACTTTTTAAGCTTTCTTTA | 2041 | TAAAGAAAGCTTAAAAAGTCTG | 299 3 |
| 22 | H45_21 6-237 | AGACTTTTTAAGCTTTCTTTAG | 2042 | CTAAAGAAAGCTTAAAAAGTCT | 299 4 |
| 22 | H45_21 7-238 | GACTTTTTAAGCTTTCTTTAGA | 2043 | TCTAAAGAAAGCTTAAAAAGTC | 299 5 |
| 22 | H45_21 8-239 | ACTTTTTAAGCTTTCTTTAGAA | 2044 | TTCTAAAGAAAGCTTAAAAAGT | 299 6 |
| 22 | H45_21 9-240 | CTTTTTAAGCTTTCTTTAGAAG | 2045 | CTTCTAAAGAAAGCTTAAAAAG | 299 7 |
| 22 | H45_22 0-241 | TTTTTAAGCTTTCTTTAGAAGA | 2046 | TCTTCTAAAGAAAGCTTAAAAA | 299 8 |
| 23 | H45_16 1-183 | CAGACAGAAAAAAGAGGTAGGGC | 2047 | GCCCTACCTCTTTTTTCTGTCTG | 299 9 |
| 23 | H45_16 2-184 | AGACAGAAAAAAGAGGTAGGGCG | 2048 | CGCCCTACCTCTTTTTTCTGTCT | 300 0 |
| 23 | H45_16 3-185 | GACAGAAAAAAGAGGTAGGGCGA | 2049 | TCGCCCTACCTCTTTTTTCTGTC | 300 1 |
| 23 | H45_16 4-186 | ACAGAAAAAAGAGGTAGGGCGAC | 2050 | GTCGCCCTACCTCTTTTTTCTGT | 300 2 |
| 23 | H45_16 5-187 | CAGAAAAAAGAGGTAGGGCGACA | 2051 | TGTCGCCCTACCTCTTTTTTCTG | 300 3 |
| 23 | H45_16 6-188 | AGAAAAAAGAGGTAGGGCGACAG | 2052 | CTGTCGCCCTACCTCTTTTTTCT | 300 4 |
| 23 | H45_16 7-189 | GAAAAAAGAGGTAGGGCGACAGA | 2053 | TCTGTCGCCCTACCTCTTTTTTC | 300 5 |
| 23 | H45_16 8-190 | AAAAAAGAGGTAGGGCGACAGAT | 2054 | ATCTGTCGCCCTACCTCTTTTTT | 300 6 |
| 23 | H45_16 9-191 | AAAAAGAGGTAGGGCGACAGATC | 2055 | GATCTGTCGCCCTACCTCTTTTT | 300 7 |
| 23 | H45_17 0-192 | AAAAGAGGTAGGGCGACAGATCT | 2056 | AGATCTGTCGCCCTACCTCTTTT | 300 8 |
| 23 | H45_17 1-193 | AAAGAGGTAGGGCGACAGATCTA | 2057 | TAGATCTGTCGCCCTACCTCTTT | 300 9 |
| 23 | H45_17 2-194 | AAGAGGTAGGGCGACAGATCTAA | 2058 | TTAGATCTGTCGCCCTACCTCTT | 301 0 |

**[Table 7-27]**

| | | | | | |
|---|---|---|---|---|---|
| 23 | H45_17 3-195 | AGAGGTAGGGCGACAGATCTAAT | 2059 | ATTAGATCTGTCGCCCTACCTCT | 301 1 |
| 23 | H45_17 4-196 | GAGGTAGGGCGACAGATCTAATA | 2060 | TATTAGATCTGTCGCCCTACCTC | 301 2 |
| 23 | H45_17 5-197 | AGGTAGGGCGACAGATCTAATAG | 2061 | CTATTAGATCTGTCGCCCTACCT | 301 3 |
| 23 | II45_17 6-198 | GGTAGGGCGACAGATCTAATAGG | 2062 | CCTATTAGATCTGTCGCCCTACC | 301 4 |
| 23 | H45_17 7-199 | GTAGGGCGACAGATCTAATAGGA | 2063 | TCCTATTAGATCTGTCGCCCTAC | 301 5 |
| 23 | H45_17 8-200 | TAGGGCGACAGATCTAATAGGAA | 2064 | TTCCTATTAGATCTGTCGCCCTA | 301 6 |
| 23 | H45_17 9-201 | AGGGCGACAGATCTAATAGGAAT | 2065 | ATTCCTATTAGATCTGTCGCCCT | 301 7 |
| 23 | H45_18 0-202 | GGGCGACAGATCTAATAGGAATG | 2066 | CATTCCTATTAGATCTGTCGCCC | 301 8 |
| 23 | H45_18 1-203 | GGCGACAGATCTAATAGGAATGA | 2067 | TCATTCCTATTAGATCTGTCGCC | 301 9 |
| 23 | H45_18 2-204 | GCGACAGATCTAATAGGAATGAA | 2068 | TTCATTCCTATTAGATCTGTCGC | 302 0 |
| 23 | H45_18 3-205 | CGACAGATCTAATAGGAATGAAA | 2069 | TTTCATTCCTATTAGATCTGTCG | 302 1 |
| 23 | H45_18 4-206 | GACAGATCTAATAGGAATGAAAA | 2070 | TTTTCATTCCTATTAGATCTGTC | 302 2 |
| 23 | H45_18 5-207 | ACAGATCTAATAGGAATGAAAAC | 2071 | GTTTTCATTCCTATTAGATCTGT | 302 3 |
| 23 | H45_18 6-208 | CAGATCTAATAGGAATGAAAACA | 2072 | TGTTTTCATTCCTATTAGATCTG | 302 4 |
| 23 | H45_18 7-209 | AGATCTAATAGGAATGAAAACAT | 2073 | ATGTTTTCATTCCTATTAGATCT | 302 5 |
| 23 | H45_18 8-210 | GATCTAATAGGAATGAAAACATT | 2074 | AATGTTTTCATTCCTATTAGATC | 302 6 |
| 23 | H45_18 9-211 | ATCTAATAGGAATGAAAACATTT | 2075 | AAATGTTTTCATTCCTATTAGAT | 302 7 |
| 23 | H45_19 0-212 | TCTAATAGGAATGAAAACATTTT | 2076 | AAAATGTTTTCATTCCTATTAGA | 302 8 |

**[Table 7-28]**

| | | | | | |
|---|---|---|---|---|---|
| 23 | H45_19 1-213 | CTAATAGGAATGAAAACATTTTA | 2077 | TAAAATGTTTTCATTCCTATTAG | 302 9 |
| 23 | H45_19 2-214 | TAATAGGAATGAAAACATTTTAG | 2078 | CTAAAATGTTTTCATTCCTATTA | 303 0 |
| 23 | H45_19 3-215 | AATAGGAATGAAAACATTTTAGC | 2079 | GCTAAAATGTTTTCATTCCTATT | 303 1 |
| 23 | H45_19 4-216 | ATAGGAATGAAAACATTTTAGCA | 2080 | TGCTAAAATGTTTTCATTCCTAT | 303 2 |
| 23 | H45_19 5-217 | TAGGAATGAAAACATTTTAGCAG | 2081 | CTGCTAAAATGTTTTCATTCCTA | 303 3 |
| 23 | H45_19 6-218 | AGGAATGAAAACATTTTAGCAGA | 2082 | TCTGCTAAAATGTTTTCATTCCT | 303 4 |
| 23 | H45_19 7-219 | GGAATGAAAACATTTTAGCAGAC | 2083 | GTCTGCTAAAATGTTTTCATTCC | 303 5 |
| 23 | H45_19 8-220 | GAATGAAAACATTTTAGCAGACT | 2084 | AGTCTGCTAAAATGTTTTCATTC | 303 6 |
| 23 | H45_19 9-221 | AATGAAAACATTTTAGCAGACTT | 2085 | AAGTCTGCTAAAATGTTTTCATT | 303 7 |
| 23 | H45_20 0-222 | ATGAAAACATTTTAGCAGACTTT | 2086 | AAAGTCTGCTAAAATGTTTTCAT | 303 8 |
| 23 | H45_20 1-223 | TGAAAACATTTTAGCAGACTTTT | 2087 | AAAAGTCTGCTAAAATGTTTTCA | 303 9 |
| 23 | H45_20 2-224 | GAAAACATTTTAGCAGACTTTTT | 2088 | AAAAAGTCTGCTAAAATGTTTTC | 304 0 |
| 23 | H45_20 3-225 | AAAACATTTTAGCAGACTTTTTA | 2089 | TAAAAAGTCTGCTAAAATGTTTT | 304 1 |
| 23 | H45_20 4-226 | AAACATTTTAGCAGACTTTTTAA | 2090 | TTAAAAAGTCTGCTAAAATGTTT | 304 2 |
| 23 | H45_20 5-227 | AACATTTTAGCAGACTTTTTAAG | 2091 | CTTAAAAAGTCTGCTAAAATGTT | 304 3 |
| 23 | H45_20 6-228 | ACATTTTAGCAGACTTTTTAAGC | 2092 | GCTTAAAAAGTCTGCTAAAATGT | 304 4 |
| 23 | H45_20 7-229 | CATTTTAGCAGACTTTTTAAGCT | 2093 | AGCTTAAAAAGTCTGCTAAAATG | 304 5 |
| 23 | H45_20 8-230 | ATTTTAGCAGACTTTTTAAGCTT | 2094 | AAGCTTAAAAAGTCTGCTAAAAT | 304 6 |

**[Table 7-29]**

| | | | | | |
|---|---|---|---|---|---|
| 23 | H45_20 9-231 | TTTTAGCAGACTTTTTAAGCTTT | 2095 | AAAGCTTAAAAAGTCTGCTAAAA | 304 7 |
| 23 | H45_21 0-232 | TTTAGCAGACTTTTTAAGCTTTC | 2096 | GAAAGCTTAAAAAGTCTGCTAAA | 304 8 |
| 23 | H45_21 1-233 | TTAGCAGACTTTTTAAGCTTTCT | 2097 | AGAAAGCTTAAAAAGTCTGCTAA | 304 9 |
| 23 | H45_21 2-234 | TAGCAGACTTTTTAAGCTTTCTT | 2098 | AAGAAAGCTTAAAAAGTCTGCTA | 305 0 |
| 23 | H45_21 3-235 | AGCAGACTTTTTAAGCTTTCTTT | 2099 | AAAGAAAGCTTAAAAAGTCTGCT | 305 1 |
| 23 | H45_21 4-236 | GCAGACTTTTTAAGCTTTCTTTA | 2100 | TAAAGAAAGCTTAAAAAGTCTGC | 305 2 |
| 23 | H45_21 5-237 | CAGACTTTTTAAGCTTTCTTTAG | 2101 | CTAAAGAAAGCTTAAAAAGTCTG | 305 3 |
| 23 | H45_21 6-238 | AGACTTTTTAAGCTTTCTTTAGA | 2102 | TCTAAAGAAAGCTTAAAAAGTCT | 305 4 |
| 23 | H45_21 7-239 | GACTTTTTAAGCTTTCTTTAGAA | 2103 | TTCTAAAGAAAGCTTAAAAAGTC | 305 5 |
| 23 | H45_21 8-240 | ACTTTTTAAGCTTTCTTTAGAAG | 2104 | CTTCTAAAGAAAGCTTAAAAAGT | 305 6 |
| 23 | H45_21 9-241 | CTTTTTAAGCTTTCTTTAGAAGA | 2105 | TCTTCTAAAGAAAGCTTAAAAAG | 305 7 |
| 23 | H45_22 0-242 | TTTTTAAGCTTTCTTTAGAAGAA | 2106 | TTCTTCTAAAGAAAGCTTAAAAA | 305 8 |
| 24 | H45_16 0-183 | TCAGACAGAAAAAGAGGTAGGG C | 2107 | GCCCTACCTCTTTTTCTGTCTG A | 305 9 |
| 24 | H45_16 1-184 | CAGACAGAAAAAGAGGTAGGGC G | 2108 | CGCCCTACCTCTTTTTTCTGTCT G | 306 0 |
| 24 | H45_16 2-185 | AGACAGAAAAAAGAGGTAGGGCG A | 2109 | TCGCCCTACCTCTTTTTCTGTC T | 306 1 |
| 24 | H45_16 3-186 | GACAGAAAAAGAGGTAGGGCGA C | 2110 | GTCGCCCTACCTCTTTTTTCTGT C | 306 2 |
| 24 | H45_16 4-187 | ACAGAAAAAAGAGGTAGGGCGAC A | 2111 | TGTCGCCCTACCTCTTTTTTCTG T | 306 3 |
| 24 | H45_16 5-188 | CAGAAAAAAGAGGTAGGGCGACA G | 2112 | CTGTCGCCCTACCTCTTTTTTCT G | 306 4 |

**[Table 7-30]**

| | | | | | |
|---|---|---|---|---|---|
| 24 | H45_16 6-189 | AGAAAAAAGAGGTAGGGCGACAG A | 2113 | TCTGTCGCCCTACCTCTTTTTTC T | 306 5 |
| 24 | H45_16 7-190 | GAAAAAAGAGGTAGGGCGACAGA T | 2114 | ATCTGTCGCCCTACCTCTTTTT C | 306 6 |
| 24 | H45_16 8-191 | AAAAAAGAGGTAGGGCGACAGAT C | 2115 | GATCTGTCGCCCTACCTCTTTTT T | 306 7 |
| 24 | H45_16 9-192 | AAAAAGAGGTAGGGCGACAGATC T | 2116 | AGATCTGTCGCCCTACCTCTTTT T | 306 8 |
| 24 | H45_17 0-193 | AAAAGAGGTAGGGCGACAGATCT A | 2117 | TAGATCTGTCGCCCTACCTCTTT T | 306 9 |
| 24 | H45_17 1-194 | AAAGAGGTAGGGCGACAGATCTA A | 2118 | TTAGATCTGTCGCCCTACCTCTT T | 307 0 |
| 24 | H45_17 2-195 | AAGAGGTAGGGCGACAGATCTAA T | 2119 | ATTAGATCTGTCGCCCTACCTCT T | 307 1 |
| 24 | H45_17 3-196 | AGAGGTAGGGCGACAGATCTAAT A | 2120 | TATTAGATCTGTCGCCCTACCTC T | 307 2 |
| 24 | H45_17 4-197 | GAGGTAGGGCGACAGATCTAATA G | 2121 | CTATTAGATCTGTCGCCCTACCT C | 307 3 |
| 24 | H45_17 5-198 | AGGTAGGGCGACAGATCTAATAG G | 2122 | CCTATTAGATCTGTCGCCCTACC T | 307 4 |
| 24 | H45_17 6-199 | GGTAGGGCGACAGATCTAATAGG A | 2123 | TCCTATTAGATCTGTCGCCCTAC C | 307 5 |
| 24 | H45_17 7-200 | GTAGGGCGACAGATCTAATAGGA A | 2124 | TTCCTATTAGATCTGTCGCCCTA C | 307 6 |
| 24 | H45_17 8-201 | TAGGGCGACAGATCTAATAGGAA T | 2125 | ATTCCTATTAGATCTGTCGCCCT A | 307 7 |
| 24 | H45_17 9-202 | AGGGCGACAGATCTAATAGGAAT G | 2126 | CATTCCTATTAGATCTGTCGCCC T | 307 8 |
| 24 | H45_18 0-203 | GGGCGACAGATCTAATAGGAATG A | 2127 | TCATTCCTATTAGATCTGTCGCC C | 307 9 |
| 24 | H45_18 1-204 | GGCGACAGATCTAATAGGAATGA A | 2128 | TTCATTCCTATTAGATCTGTCGC C | 308 0 |
| 24 | H45_18 2-205 | GCGACAGATCTAATAGGAATGAA A | 2129 | TTTCATTCCTATTAGATCTGTCG C | 308 1 |
| 24 | H45_18 3-206 | CGACAGATCTAATAGGAATGAAA A | 2130 | TTTTCATTCCTATTAGATCTGTC G | 308 2 |

**[Table 7-31]**

| | | | | | |
|---|---|---|---|---|---|
| 24 | H45_18 4-207 | GACAGATCTAATAGGAATGAAAA C | 2131 | GTTTTCATTCCTATTAGATCTGT C | 308 3 |
| 24 | H45_18 5-208 | ACAGATCTAATAGGAATGAAAAC A | 2132 | TGTTTTCATTCCTATTAGATCTG T | 308 4 |
| 24 | H45_18 6-209 | CAGATCTAATAGGAATGAAAACA T | 2133 | ATGTTTTCATTCCTATTAGATCT G | 308 5 |
| 24 | H45_18 7-210 | AGATCTAATAGGAATGAAAACAT T | 2134 | AATGTTTTCATTCCTATTAGATC T | 308 6 |
| 24 | H45_18 8-211 | GATCTAATAGGAATGAAAACATT T | 2135 | AAATGTTTTCATTCCTATTAGAT C | 308 7 |
| 24 | H45_18 9-212 | ATCTAATAGGAATGAAAACATTT T | 2136 | AAAATGTTTTCATTCCTATTAGA T | 308 8 |
| 24 | H45_19 0-213 | TCTAATAGGAATGAAAACATTTT A | 2137 | TAAAATGTTTTCATTCCTATTAG A | 308 9 |
| 24 | H45_19 1-214 | CTAATAGGAATGAAAACATTTTA G | 2138 | CTAAAATGTTTTCATTCCTATTA G | 309 0 |
| 24 | H45_19 2-215 | TAATAGGAATGAAAACATTTTAG C | 2139 | GCTAAAATGTTTTCATTCCTATT A | 309 1 |
| 24 | H45_19 3-216 | AATAGGAATGAAAACATTTTAGC A | 2140 | | 309 2 |
| 24 | H45_19 4-217 | ATAGGAATGAAAACATTTTAGCA G | 2141 | CTGCTAAAATGTTTTCATTCCTA T | 309 3 |
| 24 | H45_19 5-218 | TAGGAATGAAAACATTTTAGCAG A | 2142 | TCTGCTAAAATGTTTTCATTCCT A | 309 4 |
| 24 | H45_19 6-219 | AGGAATGAAAACATTTTAGCAGA C | 2143 | GTCTGCTAAAATGTTTTCATTCC T | 309 5 |
| 24 | H45_19 7-220 | GGAATGAAAACATTTTAGCAGAC T | 2144 | AGTCTGCTAAAATGTTTTCATTC C | 309 6 |
| 24 | H45_19 8-221 | GAATGAAAACATTTTAGCAGACT T | 2145 | AAGTCTGCTAAAATGTTTTCATT C | 309 7 |
| 24 | H45_19 9-222 | AATGAAAACATTTTAGCAGACTT T | 2146 | AAAGTCTGCTAAAATGTTTTCAT T | 309 8 |
| 24 | H45_20 0-223 | ATGAAAACATTTTAGCAGACTTT T | 2147 | AAAAGTCTGCTAAAATGTTTTCA T | 309 9 |
| 24 | H45_20 1-224 | TGAAAACATTTTAGCAGACTTTT T | 2148 | AAAAAGTCTGCTAAAATGTTTTC A | 310 0 |

**[Table 7-32]**

| | | | | | |
|---|---|---|---|---|---|
| 24 | H45_20 2-225 | GAAAACATTTTAGCAGACTTTTT A | 2149 | TAAAAAGTCTGCTAAAATGTTTT C | 310 1 |
| 24 | H45_20 3-226 | AAAACATTTTAGCAGACTTTTTA A | 2150 | TTAAAAAGTCTGCTAAAATGTTT T | 310 2 |
| 24 | H45_20 4-227 | AAACATTTTAGCAGACTTTTTAA G | 2151 | CTTAAAAAGTCTGCTAAAATGTT T | 310 3 |
| 24 | H45_20 5-228 | AACATTTTAGCAGACTTTTTAAG C | 2152 | GCTTAAAAAGTCTGCTAAAATGT T | 310 4 |
| 24 | H45_20 6-229 | ACATTTTAGCAGACTTTTTAAGC T | 2153 | AGCTTAAAAAGTCTGCTAAAATG T | 310 5 |
| 24 | H45_20 7-230 | CATTTTAGCAGACTTTTTAAGCT T | 2154 | AAGCTTAAAAAGTCTGCTAAAAT G | 310 6 |
| 24 | H45_20 8-231 | ATTTTAGCAGACTTTTTAAGCTT T | 2155 | AAAGCTTAAAAAGTCTGCTAAAA T | 310 7 |
| 24 | H45_20 9-232 | TTTTAGCAGACTTTTTAAGCTTT C | 2156 | GAAAGCTTAAAAAGTCTGCTAAA A | 310 8 |
| 24 | H45_21 0-233 | TTTAGCAGACTTTTTAAGCTTTC T | 2157 | AGAAAGCTTAAAAAGTCTGCTAA A | 310 9 |
| 24 | H45_21 1-234 | TTAGCAGACTTTTTAAGCTTTCT T | 2158 | AAGAAAGCTTAAAAAGTCTGCTA A | 311 0 |
| 24 | H45_21 2-235 | TAGCAGACTTTTTAAGCTTTCTT T | 2159 | AAAGAAAGCTTAAAAAGTCTGCT A | 311 1 |
| 24 | H45_21 3-236 | AGCAGACTTTTTAAGCTTTCTTT A | 2160 | TAAAGAAAGCTTAAAAAGTCTGC T | 311 2 |
| 24 | H45_21 4-237 | GCAGACTTTTTAAGCTTTCTTTA G | 2161 | CTAAAGAAAGCTTAAAAAGTCTG C | 311 3 |
| 24 | H45_21 5-238 | CAGACTTTTTAAGCTTTCTTTAG A | 2162 | TCTAAAGAAAGCTTAAAAAGTCT G | 311 4 |
| 24 | H45_21 6-239 | AGACTTTTTAAGCTTTCTTTAGA A | 2163 | TTCTAAAGAAAGCTTAAAAAGTC T | 311 5 |
| 24 | H45_21 7-240 | GACTTTTTAAGCTTTCTTTAGAA G | 2164 | CTTCTAAAGAAAGCTTAAAAAGT C | 311 6 |
| 24 | H45_21 8-241 | ACTTTTTAAGCTTTCTTTAGAAG A | 2165 | TCTTCTAAAGAAAGCTTAAAAAG T | 311 7 |
| 24 | H45_21 9-242 | CTTTTTAAGCTTTCTTTAGAAGA A | 2166 | TTCTTCTAAAGAAAGCTTAAAAA G | 311 8 |

**[Table 7-33]**

| | | | | | |
|---|---|---|---|---|---|
| 24 | H45_22 0-243 | TTTTTAAGCTTTCTTTAGAAGAA T | 2167 | ATTCTTCTAAAGAAAGCTTAAAA A | 311 9 |
| 25 | H45_15 9-183 | GTCAGACAGAAAAAAGAGGTAGG GC | 2168 | GCCCTACCTCTTTTTCTGTCTG AC | 312 0 |
| 25 | H45_16 0-184 | TCAGACAGAAAAAAGAGGTAGGG CG | 2169 | CGCCCTACCTCTTTTTCTGTCT GA | 312 1 |
| 25 | H45_16 1-185 | CAGACAGAAAAAAGAGGTAGGGC GA | 2170 | TCGCCCTACCTCTTTTTCTGTC TG | 312 2 |
| 25 | H45_16 2-186 | AGACAGAAAAAGAGGTAGGGCG AC | 2171 | GTCGCCCTACCTCTTTTTTCTGT CT | 312 3 |
| 25 | H45_16 3-187 | GACAGAAAAAGAGGTAGGGCGA CA | 2172 | TGTCGCCCTACCTCTTTTTCTG TC | 312 4 |
| 25 | H45_16 4-188 | ACAGAAAAAAGAGGTAGGGCGAC AG | 2173 | CTGTCGCCCTACCTCTTTTTTCT GT | 312 5 |
| 25 | H45_16 5-189 | CAGAAAAAAGAGGTAGGGCGACA GA | 2174 | TCTGTCGCCCTACCTCTTTTTTC TG | 312 6 |
| 25 | H45_16 6-190 | AGAAAAAAGAGGTAGGGCGACAG AT | 2175 | ATCTGTCGCCCTACCTCTTTTTT CT | 312 7 |
| 25 | H45_16 7-191 | GAAAAAAGAGGTAGGGCGACAGA TC | 2176 | GATCTGTCGCCCTACCTCTTTTT TC | 312 8 |
| 25 | H45_16 8-192 | AAAAAAGAGGTAGGGCGACAGAT CT | 2177 | AGATCTGTCGCCCTACCTCTTTT TT | 312 9 |
| 25 | H45_16 9-193 | AAAAAGAGGTAGGGCGACAGATC TA | 2178 | TAGATCTGTCGCCCTACCTCTTT TT | 313 0 |
| 25 | H45_17 0-194 | AAAAGAGGTAGGGCGACAGATCT AA | 2179 | TTAGATCTGTCGCCCTACCTCTT TT | 313 1 |
| 25 | H45_17 1-195 | AAAGAGGTAGGGCGACAGATCTA AT | 2180 | ATTAGATCTGTCGCCCTACCTCT TT | 313 2 |
| 25 | H45_17 2-196 | AAGAGGTAGGGCGACAGATCTAA TA | 2181 | TATTAGATCTGTCGCCCTACCTC TT | 313 3 |
| 25 | H45_17 3-197 | AGAGGTAGGGCGACAGATCTAAT AG | 2182 | CTATTAGATCTGTCGCCCTACCT CT | 313 4 |
| 25 | H45_17 4-198 | GAGGTAGGGCGACAGATCTAATA GG | 2183 | CCTATTAGATCTGTCGCCCTACC TC | 313 5 |
| 25 | H45_17 5-199 | AGGTAGGGCGACAGATCTAATAG GA | 2184 | TCCTATTAGATCTGTCGCCCTAC CT | 313 6 |

**[Table 7-34]**

| | | | | | |
|---|---|---|---|---|---|
| 25 | H45_17 6-200 | GGTAGGGCGACAGATCTAATAGG AA | 2185 | TTCCTATTAGATCTGTCGCCCTA CC | 313 7 |
| 25 | H45_17 7-201 | GTAGGGCGACAGATCTAATAGGA AT | 2186 | ATTCCTATTAGATCTGTCGCCCT AC | 313 8 |
| 25 | H45_17 8-202 | TAGGGCGACAGATCTAATAGGAA TG | 2187 | CATTCCTATTAGATCTGTCGCCC TA | 313 9 |
| 25 | H45_17 9-203 | AGGGCGACAGATCTAATAGGAAT GA | 2188 | TCATTCCTATTAGATCTGTCGCC CT | 314 0 |
| 25 | H45_18 0-204 | GGGCGACAGATCTAATAGGAATG AA | 2189 | TTCATTCCTATTAGATCTGTCGC CC | 314 1 |
| 25 | H45_18 1-205 | GGCGACAGATCTAATAGGAATGA AA | 2190 | TTTCATTCCTATTAGATCTGTCG CC | 314 2 |
| 25 | H45_18 2-206 | GCGACAGATCTAATAGGAATGAA AA | 2191 | TTTTCATTCCTATTAGATCTGTC GC | 314 3 |
| 25 | H45_18 3-207 | CGACAGATCTAATAGGAATGAAA AC | 2192 | GTTTTCATTCCTATTAGATCTGT CG | 314 4 |
| 25 | H45_18 4-208 | GACAGATCTAATAGGAATGAAAA CA | 2193 | TGTTTTCATTCCTATTAGATCTG TC | 314 5 |
| 25 | H45_18 5-209 | ACAGATCTAATAGGAATGAAAAC AT | 2194 | ATGTTTTCATTCCTATTAGATCT GT | 314 6 |
| 25 | H45_18 6-210 | CAGATCTAATAGGAATGAAAACA TT | 2195 | AATGTTTTCATTCCTATTAGATC TG | 314 7 |
| 25 | H45_18 7-211 | AGATCTAATAGGAATGAAAACAT TT | 2196 | AAATGTTTTCATTCCTATTAGAT CT | 314 8 |
| 25 | H45_18 8-212 | GATCTAATAGGAATGAAAACATT TT | 2197 | AAAATGTTTTCATTCCTATTAGA TC | 314 9 |
| 25 | H45_18 9-213 | ATCTAATAGGAATGAAAACATTT TA | 2198 | TAAAATGTTTTCATTCCTATTAG AT | 315 0 |
| 25 | H45_19 0-214 | TCTAATAGGAATGAAAACATTTT AG | 2199 | CTAAAATGTTTTCATTCCTATTA GA | 315 1 |
| 25 | H45_19 1-215 | CTAATAGGAATGAAAACATTTTA GC | 2200 | GCTAAAATGTTTTCATTCCTATT AG | 315 2 |
| 25 | H45_19 2-216 | TAATAGGAATGAAAACATTTTAG CA | 2201 | TGCTAAAATGTTTTCATTCCTAT TA | 315 3 |
| 25 | H45_19 3-217 | AATAGGAATGAAAACATTTTAGC AG | 2202 | CTGCTAAAATGTTTTCATTCCTA TT | 315 4 |

**[Table 7-35]**

| | | | | | |
|---|---|---|---|---|---|
| 25 | H45_19 4-218 | ATAGGAATGAAAACATTTTAGCA GA | 2203 | TCTGCTAAAATGTTTTCATTCCT AT | 315 5 |
| 25 | H45_19 5-219 | TAGGAATGAAAACATTTTAGCAG AC | 2204 | GTCTGCTAAAATGTTTTCATTCC TA | 315 6 |
| 25 | H45_19 6-220 | AGGAATGAAAACATTTTAGCAGA CT | 2205 | AGTCTGCTAAAATGTTTTCATTC CT | 315 7 |
| 25 | H45_19 7-221 | GGAATGAAAACATTTTAGCAGAC TT | 2206 | AAGTCTGCTAAAATGTTTTCATT CC | 315 8 |
| 25 | H45_19 8-222 | GAATGAAAACATTTTAGCAGACT TT | 2207 | AAAGTCTGCTAAAATGTTTTCAT TC | 315 9 |
| 25 | H45_19 9-223 | AATGAAAACATTTTAGCAGACTT TT | 2208 | AAAAGTCTGCTAAAATGTTTTCA TT | 316 0 |
| 25 | H45_20 0-224 | ATGAAAACATTTTAGCAGACTTT TT | 2209 | AAAAAGTCTGCTAAAATGTTTTC AT | 316 1 |
| 25 | H45_20 1-225 | TGAAAACATTTTAGCAGACTTTT TA | 2210 | TAAAAAGTCTGCTAAAATGTTTT CA | 316 2 |
| 25 | H45_20 2-226 | GAAAACATTTTAGCAGACTTTTT AA | 2211 | TTAAAAAGTCTGCTAAAATGTTT TC | 316 3 |
| 25 | H45_20 3-227 | AAAACATTTTAGCAGACTTTTTA AG | 2212 | CTTAAAAAGTCTGCTAAAATGTT TT | 316 4 |
| 25 | H45_20 4-228 | AAACATTTTAGCAGACTTTTTAA GC | 2213 | GCTTAAAAAGTCTGCTAAAATGT TT | 316 5 |
| 25 | H45_20 5-229 | AACATTTTAGCAGACTTTTTAAG CT | 2214 | AGCTTAAAAAGTCTGCTAAAATG TT | 316 6 |
| 25 | H45_20 6-230 | ACATTTTAGCAGACTTTTTAAGC TT | 2215 | AAGCTTAAAAAGTCTGCTAAAAT GT | 316 7 |
| 25 | H45_20 7-231 | CATTTTAGCAGACTTTTTAAGCT TT | 2216 | AAAGCTTAAAAAGTCTGCTAAAA TG | 316 8 |
| 25 | H45_20 8-232 | ATTTTAGCAGACTTTTTAAGCTT TC | 2217 | GAAAGCTTAAAAAGTCTGCTAAA AT | 316 9 |
| 25 | H45_20 9-233 | TTTTAGCAGACTTTTTAAGCTTT CT | 2218 | AGAAAGCTTAAAAAGTCTGCTAA AA | 317 0 |
| 25 | H45_21 0-234 | TTTAGCAGACTTTTTAAGCTTTC TT | 2219 | AAGAAAGCTTAAAAAGTCTGCTA AA | 317 1 |
| 25 | H45_21 1-235 | TTAGCAGACTTTTTAAGCTTTCT TT | 2220 | AAAGAAAGCTTAAAAAGTCTGCT AA | 317 2 |

**[Table 7-36]**

| | | | | | |
|---|---|---|---|---|---|
| 25 | H45_21 2-236 | TAGCAGACTTTTTAAGCTTTCTT TA | 2221 | TAAAGAAAGCTTAAAAAGTCTGC TA | 317 3 |
| 25 | H45_21 3-237 | AGCAGACTTTTTAAGCTTTCTTT AG | 2222 | CTAAAGAAAGCTTAAAAAGTCTG CT | 317 4 |
| 25 | H45_21 4-238 | GCAGACTTTTTAAGCTTTCTTTA GA | 2223 | TCTAAAGAAAGCTTAAAAAGTCT GC | 317 5 |
| 25 | H45_21 5-239 | CAGACTTTTTAAGCTTTCTTTAG AA | 2224 | TTCTAAAGAAAGCTTAAAAAGTC TG | 317 6 |
| 25 | H45_21 6-240 | AGACTTTTTAAGCTTTCTTTAGA AG | 2225 | CTTCTAAAGAAAGCTTAAAAAGT CT | 317 7 |
| 25 | H45_21 7-241 | GACTTTTTAAGCTTTCTTTAGAA GA | 2226 | TCTTCTAAAGAAAGCTTAAAAAG TC | 317 8 |
| 25 | H45_21 8-242 | ACTTTTTAAGCTTTCTTTAGAAG AA | 2227 | TTCTTCTAAAGAAAGCTTAAAAA GT | 317 9 |
| 25 | H45_21 9-243 | CTTTTTAAGCTTTCTTTAGAAGA AT | 2228 | ATTCTTCTAAAGAAAGCTTAAAA AG | 318 0 |
| 25 | H45_22 0-244 | TTTTTAAGCTTTCTTTAGAAGAA TA | 2229 | TATTCTTCTAAAGAAAGCTTAAA AA | 318 1 |
| 26 | H45_15 8-183 | TGTCAGACAGAAAAAGAGGTAG GGC | 2230 | GCCCTACCTCTTTTTTCTGTCTG ACA | 318 2 |
| 26 | H45_15 9-184 | GTCAGACAGAAAAAAGAGGTAGG GCG | 2231 | CGCCCTACCTCTTTTTCTGTCT GAC | 318 3 |
| 26 | H45_16 0-185 | TCAGACAGAAAAAAGAGGTAGGG CGA | 2232 | TCGCCCTACCTCTTTTTCTGTC TGA | 318 4 |
| 26 | H45_16 1-186 | CAGACAGAAAAAAGAGGTAGGGC GAC | 2233 | GTCGCCCTACCTCTTTTTCTGT CTG | 318 5 |
| 26 | H45_16 2-187 | AGACAGAAAAAAGAGGTAGGGCG ACA | 2234 | TGTCGCCCTACCTCTTTTTTCTG TCT | 318 6 |
| 26 | H45_16 3-188 | GACAGAAAAAGAGGTAGGGCGA CAG | 2235 | CTGTCGCCCTACCTCTTTTTTCT GTC | 318 7 |
| 26 | H45_16 4-189 | ACAGAAAAAAGAGGTAGGGCGAC AGA | 2236 | TCTGTCGCCCTACCTCTTTTTC TGT | 318 8 |
| 26 | H45_16 5-190 | CAGAAAAAAGAGGTAGGGCGACA GAT | 2237 | ATCTGTCGCCCTACCTCTTTTT CTG | 318 9 |
| 26 | H45_16 6-191 | AGAAAAAAGAGGTAGGGCGACAG ATC | 2238 | GATCTGTCGCCCTACCTCTTTTT TCT | 319 0 |

**[Table 7-37]**

| | | | | | |
|---|---|---|---|---|---|
| 26 | H45_16 7-192 | GAAAAAAGAGGTAGGGCGACAGA TCT | 2239 | AGATCTGTCGCCCTACCTCTTTT TTC | 319 1 |
| 26 | H45_16 8-193 | AAAAAAGAGGTAGGGCGACAGAT CTA | 2240 | TAGATCTGTCGCCCTACCTCTTT TTT | 319 2 |
| 26 | H45_16 9-194 | AAAAAGAGGTAGGGCGACAGATC TAA | 2241 | TTAGATCTGTCGCCCTACCTCTT TTT | 319 3 |
| 26 | H45_17 0-195 | AAAAGAGGTAGGGCGACAGATCT AAT | 2242 | ATTAGATCTGTCGCCCTACCTCT TTT | 319 4 |
| 26 | H45_17 1-196 | AAAGAGGTAGGGCGACAGATCTA ATA | 2243 | TATTAGATCTGTCGCCCTACCTC TTT | 319 5 |
| 26 | H45_17 2-197 | AAGAGGTAGGGCGACAGATCTAA TAG | 2244 | CTATTAGATCTGTCGCCCTACCT CTT | 319 6 |
| 26 | H45_17 3-198 | AGAGGTAGGGCGACAGATCTAAT AGG | 2245 | CCTATTAGATCTGTCGCCCTACC TCT | 319 7 |
| 26 | H45_17 4-199 | GAGGTAGGGCGACAGATCTAATA GGA | 2246 | TCCTATTAGATCTGTCGCCCTAC CTC | 319 8 |
| 26 | H45_17 5-200 | AGGTAGGGCGACAGATCTAATAG GAA | 2247 | TTCCTATTAGATCTGTCGCCCTA CCT | 319 9 |
| 26 | H45_17 6-201 | GGTAGGGCGACAGATCTAATAGG AAT | 2248 | ATTCCTATTAGATCTGTCGCCCT ACC | 320 0 |
| 26 | H45_17 7-202 | GTAGGGCGACAGATCTAATAGGA ATG | 2249 | CATTCCTATTAGATCTGTCGCCC TAC | 320 1 |
| 26 | H45_17 8-203 | TAGGGCGACAGATCTAATAGGAA TGA | 2250 | TCATTCCTATTAGATCTGTCGCC CTA | 320 2 |
| 26 | H45_17 9-204 | AGGGCGACAGATCTAATAGGAAT GAA | 2251 | TTCATTCCTATTAGATCTGTCGC CCT | 320 3 |
| 26 | H45_18 0-205 | GGGCGACAGATCTAATAGGAATG AAA | 2252 | TTTCATTCCTATTAGATCTGTCG CCC | 320 4 |
| 26 | H45_18 1-206 | GGCGACAGATCTAATAGGAATGA AAA | 2253 | TTTTCATTCCTATTAGATCTGTC GCC | 320 5 |
| 26 | H45_18 2-207 | GCGACAGATCTAATAGGAATGAA AAC | 2254 | GTTTTCATTCCTATTAGATCTGT CGC | 320 6 |
| 26 | H45_18 3-208 | CGACAGATCTAATAGGAATGAAA ACA | 2255 | TGTTTTCATTCCTATTAGATCTG TCG | 320 7 |
| 26 | H45_18 4-209 | GACAGATCTAATAGGAATGAAAA CAT | 2256 | ATGTTTTCATTCCTATTAGATCT GTC | 320 8 |

**[Table 7-38]**

| | | | | | |
|---|---|---|---|---|---|
| 26 | H45_18 5-210 | ACAGATCTAATAGGAATGAAAAC ATT | 2257 | AATGTTTTCATTCCTATTAGATC TGT | 320 9 |
| 26 | H45_18 6-211 | CAGATCTAATAGGAATGAAAACA TTT | 2258 | AAATGTTTTCATTCCTATTAGAT CTG | 321 0 |
| 26 | H45_18 7-212 | AGATCTAATAGGAATGAAAACAT TTT | 2259 | AAAATGTTTTCATTCCTATTAGA TCT | 321 1 |
| 26 | H45_18 8-213 | GATCTAATAGGAATGAAAACATT TTA | 2260 | TAAAATGTTTTCATTCCTATTAG ATC | 321 2 |
| 26 | H45_18 9-214 | ATCTAATAGGAATGAAAACATTT TAG | 2261 | CTAAAATGTTTTCATTCCTATTA GAT | 321 3 |
| 26 | H45_19 0-215 | TCTAATAGGAATGAAAACATTTT AGC | 2262 | GCTAAAATGTTTTCATTCCTATT AGA | 321 4 |
| 26 | H45_19 1-216 | CTAATAGGAATGAAAACATTTTA GCA | 2263 | TGCTAAAATGTTTTCATTCCTAT TAG | 321 5 |
| 26 | H45_19 2-217 | TAATAGGAATGAAAACATTTTAG CAG | 2264 | CTGCTAAAATGTTTTCATTCCTA TTA | 321 6 |
| 26 | H45_19 3-218 | AATAGGAATGAAAACATTTTAGC AGA | 2265 | TCTGCTAAAATGTTTTCATTCCT ATT | 321 7 |
| 26 | H45_19 4-219 | ATAGGAATGAAAACATTTTAGCA GAC | 2266 | GTCTGCTAAAATGTTTTCATTCC TAT | 321 8 |
| 26 | H45_19 5-220 | TAGGAATGAAAACATTTTAGCAG ACT | 2267 | AGTCTGCTAAAATGTTTTCATTC CTA | 321 9 |
| 26 | H45_19 6-221 | AGGAATGAAAACATTTTAGCAGA CTT | 2268 | AAGTCTGCTAAAATGTTTTCATT CCT | 322 0 |
| 26 | H45_19 7-222 | GGAATGAAAACATTTTAGCAGAC TTT | 2269 | AAAGTCTGCTAAAATGTTTTCAT TCC | 322 1 |
| 26 | H45_19 8-223 | GAATGAAAACATTTTAGCAGACT TTT | 2270 | AAAAGTCTGCTAAAATGTTTTCA TTC | 322 2 |
| 26 | H45_19 9-224 | AATGAAAACATTTTAGCAGACTT TTT | 2271 | AAAAAGTCTGCTAAAATGTTTTC ATT | 322 3 |
| 26 | H45_20 0-225 | ATGAAAACATTTTAGCAGACTTT TTA | 2272 | TAAAAAGTCTGCTAAAATGTTTT CAT | 322 4 |
| 26 | H45_20 1-226 | TGAAAACATTTTAGCAGACTTTT TAA | 2273 | TTAAAAAGTCTGCTAAAATGTTT TCA | 322 5 |
| 26 | H45_20 2-227 | GAAAACATTTTAGCAGACTTTTT AAG | 2274 | CTTAAAAAGTCTGCTAAAATGTT TTC | 322 6 |

**[Table 7-39]**

| | | | | | |
|---|---|---|---|---|---|
| 26 | H45_20 3-228 | AAAACATTTTAGCAGACTTTTTA AGC | 2275 | GCTTAAAAAGTCTGCTAAAATGT TTT | 322 7 |
| 26 | H45_20 4-229 | AAACATTTTAGCAGACTTTTTAA GCT | 2276 | AGCTTAAAAAGTCTGCTAAAATG TTT | 322 8 |
| 26 | H45_20 5-230 | AACATTTTAGCAGACTTTTTAAG CTT | 2277 | AAGCTTAAAAAGTCTGCTAAAAT GTT | 322 9 |
| 26 | H45_20 6-231 | ACATTTTAGCAGACTTTTTAAGC TTT | 2278 | AAAGCTTAAAAAGTCTGCTAAAA TGT | 323 0 |
| 26 | H45_20 7-232 | CATTTTAGCAGACTTTTTAAGCT TTC | 2279 | GAAAGCTTAAAAAGTCTGCTAAA ATG | 323 1 |
| 26 | H45_20 8-233 | ATTTTAGCAGACTTTTTAAGCTT TCT | 2280 | AGAAAGCTTAAAAAGTCTGCTAA AAT | 323 2 |
| 26 | H45_20 9-234 | TTTTAGCAGACTTTTTAAGCTTT CTT | 2281 | AAGAAAGCTTAAAAAGTCTGCTA AAA | 323 3 |
| 26 | H45_21 0-235 | TTTAGCAGACTTTTTAAGCTTTC TTT | 2282 | AAAGAAAGCTTAAAAAGTCTGCT AAA | 323 4 |
| 26 | H45_21 1-236 | TTAGCAGACTTTTTAAGCTTTCT TTA | 2283 | TAAAGAAAGCTTAAAAAGTCTGC TAA | 323 5 |
| 26 | H45_21 2-237 | TAGCAGACTTTTTAAGCTTTCTT TAG | 2284 | CTAAAGAAAGCTTAAAAAGTCTG CTA | 323 6 |
| 26 | H45_21 3-238 | AGCAGACTTTTTAAGCTTTCTTT AGA | 2285 | TCTAAAGAAAGCTTAAAAAGTCT GCT | 323 7 |
| 26 | H45_21 4-239 | GCAGACTTTTTAAGCTTTCTTTA GAA | 2286 | TTCTAAAGAAAGCTTAAAAAGTC TGC | 323 8 |
| 26 | H45_21 5-240 | CAGACTTTTTAAGCTTTCTTTAG AAG | 2287 | CTTCTAAAGAAAGCTTAAAAAGT CTG | 323 9 |
| 26 | H45_21 6-241 | AGACTTTTTAAGCTTTCTTTAGA AGA | 2288 | TCTTCTAAAGAAAGCTTAAAAAG TCT | 324 0 |
| 26 | H45_21 7-242 | GACTTTTTAAGCTTTCTTTAGAA GAA | 2289 | TTCTTCTAAAGAAAGCTTAAAAA GTC | 324 1 |
| 26 | H45_21 8-243 | ACTTTTTAAGCTTTCTTTAGAAG AAT | 2290 | ATTCTTCTAAAGAAAGCTTAAAA AGT | 324 2 |
| 26 | H45_21 9-244 | CTTTTTAAGCTTTCTTTAGAAGA ATA | 2291 | TATTCTTCTAAAGAAAGCTTAAA AAG | 324 3 |
| 26 | H45_22 0-245 | TTTTTAAGCTTTCTTTAGAAGAA TAT | 2292 | ATATTCTTCTAAAGAAAGCTTAA AAA | 324 4 |

**[Table 7-40]**

| | | | | | |
|---|---|---|---|---|---|
| 27 | H45_15 7-183 | CTGTCAGACAGAAAAAGAGGTA GGGC | 2293 | GCCCTACCTCTTTTTCTGTCTG ACAG | 324 5 |
| 27 | H45_15 8-184 | TGTCAGACAGAAAAAGAGGTAG GGCG | 2294 | CGCCCTACCTCTTTTTCTGTCT GACA | 324 6 |
| 27 | H45_15 9-185 | GTCAGACAGAAAAAGAGGTAGG GCGA | 2295 | TCGCCCTACCTCTTTTTTCTGTC TGAC | 324 7 |
| 27 | H45_16 0-186 | TCAGACAGAAAAAAGAGGTAGGG CGAC | 2296 | GTCGCCCTACCTCTTTTTCTGT CTGA | 324 8 |
| 27 | H45_16 1-187 | CAGACAGAAAAAAGAGGTAGGGC GACA | 2297 | TGTCGCCCTACCTCTTTTTCTG TCTG | 324 9 |
| 27 | H45_16 2-188 | AGACAGAAAAAGAGGTAGGGCG ACAG | 2298 | CTGTCGCCCTACCTCTTTTTTCT GTCT | 325 0 |
| 27 | H45_16 3-189 | GACAGAAAAAGAGGTAGGGCGA CAGA | 2299 | TCTGTCGCCCTACCTCTTTTTC TGTC | 325 1 |
| 27 | H45_16 4-190 | ACAGAAAAAAGAGGTAGGGCGAC AGAT | 2300 | ATCTGTCGCCCTACCTCTTTTTT CTGT | 325 2 |
| 27 | H45_16 5-191 | CAGAAAAAAGAGGTAGGGCGACA GATC | 2301 | GATCTGTCGCCCTACCTCTTTTT TCTG | 325 3 |
| 27 | H45_16 6-192 | AGAAAAAAGAGGTAGGGCGACAG ATCT | 2302 | AGATCTGTCGCCCTACCTCTTTT TTCT | 325 4 |
| 27 | H45_16 7-193 | GAAAAAAGAGGTAGGGCGACAGA TCTA | 2303 | TAGATCTGTCGCCCTACCTCTTT TTTC | 325 5 |
| 27 | H45_16 8-194 | AAAAAAGAGGTAGGGCGACAGAT CTAA | 2304 | TTAGATCTGTCGCCCTACCTCTT TTTT | 325 6 |
| 27 | H45_16 9-195 | AAAAAGAGGTAGGGCGACAGATC TAAT | 2305 | ATTAGATCTGTCGCCCTACCTCT TTTT | 325 7 |
| 27 | H45_17 0-196 | AAAAGAGGTAGGGCGACAGATCT AATA | 2306 | TATTAGATCTGTCGCCCTACCTC TTTT | 325 8 |
| 27 | H45_17 1-197 | AAAGAGGTAGGGCGACAGATCTA ATAG | 2307 | CTATTAGATCTGTCGCCCTACCT CTTT | 325 9 |
| 27 | H45_17 2-198 | AAGAGGTAGGGCGACAGATCTAA TAGG | 2308 | CCTATTAGATCTGTCGCCCTACC TCTT | 326 0 |
| 27 | H45_17 3-199 | AGAGGTAGGGCGACAGATCTAAT AGGA | 2309 | TCCTATTAGATCTGTCGCCCTAC CTCT | 326 1 |
| 27 | H45_17 4-200 | GAGGTAGGGCGACAGATCTAATA GGAA | 2310 | TTCCTATTAGATCTGTCGCCCTA CCTC | 326 2 |

**[Table 7-41]**

| | | | | | |
|---|---|---|---|---|---|
| 27 | H45_17 5-201 | AGGTAGGGCGACAGATCTAATAG GAAT | 2311 | ATTCCTATTAGATCTGTCGCCCT ACCT | 326 3 |
| 27 | H45_17 6-202 | GGTAGGGCGACAGATCTAATAGG AATG | 2312 | CATTCCTATTAGATCTGTCGCCC TACC | 326 4 |
| 27 | H45_17 7-203 | GTAGGGCGACAGATCTAATAGGA ATGA | 2313 | TCATTCCTATTAGATCTGTCGCC CTAC | 326 5 |
| 27 | H45_17 8-204 | TAGGGCGACAGATCTAATAGGAA TGAA | 2314 | TTCATTCCTATTAGATCTGTCGC CCTA | 326 6 |
| 27 | H45_17 9-205 | AGGGCGACAGATCTAATAGGAAT GAAA | 2315 | TTTCATTCCTATTAGATCTGTCG CCCT | 326 7 |
| 27 | H45_18 0-206 | GGGCGACAGATCTAATAGGAATG AAAA | 2316 | TTTTCATTCCTATTAGATCTGTC GCCC | 326 8 |
| 27 | H45_18 1-207 | GGCGACAGATCTAATAGGAATGA AAAC | 2317 | GTTTTCATTCCTATTAGATCTGT CGCC | 326 9 |
| 27 | H45_18 2-208 | GCGACAGATCTAATAGGAATGAA AACA | 2318 | TGTTTTCATTCCTATTAGATCTG TCGC | 327 0 |
| 27 | H45_18 3-209 | CGACAGATCTAATAGGAATGAAA ACAT | 2319 | ATGTTTTCATTCCTATTAGATCT GTCG | 327 1 |
| 27 | H45_18 4-210 | GACAGATCTAATAGGAATGAAAA CATT | 2320 | AATGTTTTCATTCCTATTAGATC TGTC | 327 2 |
| 27 | H45_18 5-211 | ACAGATCTAATAGGAATGAAAAC ATTT | 2321 | AAATGTTTTCATTCCTATTAGAT CTGT | 327 3 |
| 27 | H45_18 6-212 | CAGATCTAATAGGAATGAAAACA TTTT | 2322 | AAAATGTTTTCATTCCTATTAGA TCTG | 327 4 |
| 27 | H45_18 7-213 | AGATCTAATAGGAATGAAAACAT TTTA | 2323 | TAAAATGTTTTCATTCCTATTAG ATCT | 327 5 |
| 27 | H45_18 8-214 | GATCTAATAGGAATGAAAACATT TTAG | 2324 | CTAAAATGTTTTCATTCCTATTA GATC | 327 6 |
| 27 | H45_18 9-215 | ATCTAATAGGAATGAAAACATTT TAGC | 2325 | GCTAAAATGTTTTCATTCCTATT AGAT | 327 7 |
| 27 | H45_19 0-216 | TCTAATAGGAATGAAAACATTTT AGCA | 2326 | TGCTAAAATGTTTTCATTCCTAT TAGA | 327 8 |
| 27 | H45_19 1-217 | CTAATAGGAATGAAAACATTTTA GCAG | 2327 | CTGCTAAAATGTTTTCATTCCTA TTAG | 327 9 |
| 27 | H45_19 2-218 | TAATAGGAATGAAAACATTTTAG CAGA | 2328 | TCTGCTAAAATGTTTTCATTCCT ATTA | 328 0 |

**[Table 7-42]**

| | | | | | |
|---|---|---|---|---|---|
| 27 | H45_19 3-219 | AATAGGAATGAAAACATTTTAGC AGAC | 2329 | GTCTGCTAAAATGTTTTCATTCC TATT | 328 1 |
| 27 | H45_19 4-220 | ATAGGAATGAAAACATTTTAGCA GACT | 2330 | AGTCTGCTAAAATGTTTTCATTC CTAT | 328 2 |
| 27 | H45_19 5-221 | TAGGAATGAAAACATTTTAGCAG ACTT | 2331 | AAGTCTGCTAAAATGTTTTCATT CCTA | 328 3 |
| 27 | H45_19 6-222 | AGGAATGAAAACATTTTAGCAGA CTTT | 2332 | AAAGTCTGCTAAAATGTTTTCAT TCCT | 328 4 |
| 27 | H45_19 7-223 | GGAATGAAAACATTTTAGCAGAC TTTT | 2333 | AAAAGTCTGCTAAAATGTTTTCA TTCC | 328 5 |
| 27 | H45_19 8-224 | GAATGAAAACATTTTAGCAGACT TTTT | 2334 | AAAAAGTCTGCTAAAATGTTTTC ATTC | 328 6 |
| 27 | H45_19 9-225 | AATGAAAACATTTTAGCAGACTT TTTA | 2335 | TAAAAAGTCTGCTAAAATGTTTT CATT | 328 7 |
| 27 | H45_20 0-226 | ATGAAAACATTTTAGCAGACTTT TTAA | 2336 | TTAAAAAGTCTGCTAAAATGTTT TCAT | 328 8 |
| 27 | H45_20 1-227 | TGAAAACATTTTAGCAGACTTTT TAAG | 2337 | CTTAAAAAGTCTGCTAAAATGTT TTCA | 328 9 |
| 27 | H45_20 2-228 | GAAAACATTTTAGCAGACTTTTT AAGC | 2338 | GCTTAAAAAGTCTGCTAAAATGT TTTC | 329 0 |
| 27 | H45_20 3-229 | AAAACATTTTAGCAGACTTTTTA AGCT | 2339 | AGCTTAAAAAGTCTGCTAAAATG TTTT | 329 1 |
| 27 | H45_20 4-230 | AAACATTTTAGCAGACTTTTTAA GCTT | 2340 | AAGCTTAAAAAGTCTGCTAAAAT GTTT | 329 2 |
| 27 | H45_20 5-231 | AACATTTTAGCAGACTTTTTAAG CTTT | 2341 | AAAGCTTAAAAAGTCTGCTAAAA TGTT | 329 3 |
| 27 | H45_20 6-232 | ACATTTTAGCAGACTTTTTAAGC TTTC | 2342 | GAAAGCTTAAAAAGTCTGCTAAA ATGT | 329 4 |
| 27 | H45_20 7-233 | CATTTTAGCAGACTTTTTAAGCT TTCT | 2343 | AGAAAGCTTAAAAAGTCTGCTAA AATG | 329 5 |
| 27 | H45_20 8-234 | ATTTTAGCAGACTTTTTAAGCTT TCTT | 2344 | AAGAAAGCTTAAAAAGTCTGCTA AAAT | 329 6 |
| 27 | H45_20 9-235 | TTTTAGCAGACTTTTTAAGCTTT CTTT | 2345 | AAAGAAAGCTTAAAAAGTCTGCT AAAA | 329 7 |
| 27 | H45_21 0-236 | TTTAGCAGACTTTTTAAGCTTTC TTTA | 2346 | TAAAGAAAGCTTAAAAAGTCTGC TAAA | 329 8 |

**[Table 7-43]**

| | | | | | |
|---|---|---|---|---|---|
| 27 | H45_21 1-237 | TTAGCAGACTTTTTAAGCTTTCT TTAG | 2347 | CTAAAGAAAGCTTAAAAAGTCTG CTAA | 329 9 |
| 27 | H45_21 2-238 | TAGCAGACTTTTTAAGCTTTCTT TAGA | 2348 | TCTAAAGAAAGCTTAAAAAGTCT GCTA | 330 0 |
| 27 | H45_21 3-239 | AGCAGACTTTTTAAGCTTTCTTT AGAA | 2349 | TTCTAAAGAAAGCTTAAAAAGTC TGCT | 330 1 |
| 27 | H45_21 4-240 | GCAGACTTTTTAAGCTTTCTTTA GAAG | 2350 | CTTCTAAAGAAAGCTTAAAAAGT CTGC | 330 2 |
| 27 | H45_21 5-241 | CAGACTTTTTAAGCTTTCTTTAG AAGA | 2351 | TCTTCTAAAGAAAGCTTAAAAAG TCTG | 330 3 |
| 27 | H45_21 6-242 | AGACTTTTTAAGCTTTCTTTAGA AGAA | 2352 | TTCTTCTAAAGAAAGCTTAAAAA GTCT | 330 4 |
| 27 | H45_21 7-243 | GACTTTTTAAGCTTTCTTTAGAA GAAT | 2353 | ATTCTTCTAAAGAAAGCTTAAAA AGTC | 330 5 |
| 27 | H45_21 8-244 | ACTTTTTAAGCTTTCTTTAGAAG AATA | 2354 | TATTCTTCTAAAGAAAGCTTAAA AAGT | 330 6 |
| 27 | H45_21 9-245 | CTTTTTAAGCTTTCTTTAGAAGA ATAT | 2355 | ATATTCTTCTAAAGAAAGCTTAA AAAG | 330 7 |
| 27 | H45_22 0-246 | TTTTTAAGCTTTCTTTAGAAGAA TATT | 2356 | AATATTCTTCTAAAGAAAGCTTA AAAA | 330 8 |
| 28 | H45_15 6-183 | GCTGTCAGACAGAAAAAGAGGT AGGGC | 2357 | GCCCTACCTCTTTTTCTGTCTG ACAGC | 330 9 |
| 28 | H45_15 7-184 | CTGTCAGACAGAAAAAGAGGTA GGGCG | 2358 | CGCCCTACCTCTTTTTCTGTCT GACAG | 331 0 |
| 28 | H45_15 8-185 | TGTCAGACAGAAAAAAGAGGTAG GGCGA | 2359 | TCGCCCTACCTCTTTTTTCTGTC TGACA | 331 1 |
| 28 | H15_15 9-186 | GTCAGACAGAAAAAAGAGGTAGG GCGAC | 2360 | GTCGCCCTACCTCTTTTTTCTGT CTGAC | 331 2 |
| 28 | H45_16 0-187 | TCAGACAGAAAAAAGAGGTAGGG CGACA | 2361 | TGTCGCCCTACCTCTTTTTCTG TCTGA | 331 3 |
| 28 | H45_16 1-188 | CAGACAGAAAAAAGAGGTAGGGC GACAG | 2362 | CTGTCGCCCTACCTCTTTTTTCT GTCTG | 331 4 |
| 28 | H45_16 2-189 | AGACAGAAAAAAGAGGTAGGGCG ACAGA | 2363 | TCTGTCGCCCTACCTCTTTTTTC TGTCT | 331 5 |
| 28 | H45_16 3-190 | GACAGAAAAAAGAGGTAGGGCGA CAGAT | 2364 | ATCTGTCGCCCTACCTCTTTTTT CTGTC | 331 6 |

**[Table 7-44]**

| | | | | | |
|---|---|---|---|---|---|
| 28 | H45_16 4-191 | ACAGAAAAAAGAGGTAGGGCGAC AGATC | 2365 | GATCTGTCGCCCTACCTCTTTTT TCTGT | 331 7 |
| 28 | H45_16 5-192 | CAGAAAAAAGAGGTAGGGCGACA GATCT | 2366 | AGATCTGTCGCCCTACCTCTTTT TTCTG | 331 8 |
| 28 | H45_16 6-193 | AGAAAAAAGAGGTAGGGCGACAG ATCTA | 2367 | TAGATCTGTCGCCCTACCTCTTT TTTCT | 331 9 |
| 28 | H45_16 7-194 | GAAAAAAGAGGTAGGGCGACAGA TCTAA | 2368 | TTAGATCTGTCGCCCTACCTCTT TTTTC | 332 0 |
| 28 | H45_16 8-195 | AAAAAAGAGGTAGGGCGACAGAT CTAAT | 2369 | ATTAGATCTGTCGCCCTACCTCT TTTTT | 332 1 |
| 28 | H45_16 9-196 | AAAAAGAGGTAGGGCGACAGATC TAATA | 2370 | TATTAGATCTGTCGCCCTACCTC TTTTT | 332 2 |
| 28 | H45_17 0-197 | AAAAGAGGTAGGGCGACAGATCT AATAG | 2371 | CTATTAGATCTGTCGCCCTACCT CTTTT | 332 3 |
| 28 | H45_17 1-198 | AAAGAGGTAGGGCGACAGATCTA ATAGG | 2372 | CCTATTAGATCTGTCGCCCTACC TCTTT | 332 4 |
| 28 | H45_17 2-199 | AAGAGGTAGGGCGACAGATCTAA TAGGA | 2373 | TCCTATTAGATCTGTCGCCCTAC CTCTT | 332 5 |
| 28 | H45_17 3-200 | AGAGGTAGGGCGACAGATCTAAT AGGAA | 2374 | TTCCTATTAGATCTGTCGCCCTA CCTCT | 332 6 |
| 28 | H45_17 4-201 | GAGGTAGGGCGACAGATCTAATA GGAAT | 2375 | ATTCCTATTAGATCTGTCGCCCT ACCTC | 332 7 |
| 28 | H45_17 5-202 | AGGTAGGGCGACAGATCTAATAG GAATG | 2376 | CATTCCTATTAGATCTGTCGCCC TACCT | 332 8 |
| 28 | H45_17 6-203 | GGTAGGGCGACAGATCTAATAGG AATGA | 2377 | TCATTCCTATTAGATCTGTCGCC CTACC | 332 9 |
| 28 | H45 17 7-204 | GTAGGGCGACAGATCTAATAGGA ATGAA | 2378 | TTCATTCCTATTAGATCTGTCGC CCTAC | 333 0 |
| 28 | H45_17 8-205 | TAGGGCGACAGATCTAATAGGAA TGAAA | 2379 | TTTCATTCCTATTAGATCTGTCG CCCTA | 333 1 |
| 28 | H45_17 9-206 | AGGGCGACAGATCTAATAGGAAT GAAAA | 2380 | TTTTCATTCCTATTAGATCTGTC GCCCT | 333 2 |
| 28 | H45_18 0-207 | GGGCGACAGATCTAATAGGAATG AAAAC | 2381 | GTTTTCATTCCTATTAGATCTGT CGCCC | 333 3 |
| 28 | H45_18 1-208 | GGCGACAGATCTAATAGGAATGA AAACA | 2382 | TGTTTTCATTCCTATTAGATCTG TCGCC | 333 4 |

**[Table 7-45]**

| | | | | | |
|---|---|---|---|---|---|
| 28 | H45_18 2-209 | GCGACAGATCTAATAGGAATGAA AACAT | 2383 | ATGTTTTCATTCCTATTAGATCT GTCGC | 333 5 |
| 28 | H45_18 3-210 | CGACAGATCTAATAGGAATGAAA ACATT | 2384 | AATGTTTTCATTCCTATTAGATC TGTCG | 333 6 |
| 28 | H45_18 4-211 | GACAGATCTAATAGGAATGAAAA CATTT | 2385 | AAATGTTTTCATTCCTATTAGAT CTGTC | 333 7 |
| 28 | H45_18 5-212 | ACAGATCTAATAGGAATGAAAAC ATTTT | 2386 | AAAATGTTTTCATTCCTATTAGA TCTGT | 333 8 |
| 28 | H45_18 6-213 | CAGATCTAATAGGAATGAAAACA TTTTA | 2387 | TAAAATGTTTTCATTCCTATTAG ATCTG | 333 9 |
| 28 | H45_18 7-214 | AGATCTAATAGGAATGAAAACAT TTTAG | 2388 | CTAAAATGTTTTCATTCCTATTA GATCT | 334 0 |
| 28 | H45_18 8-215 | GATCTAATAGGAATGAAAACATT TTAGC | 2389 | GCTAAAATGTTTTCATTCCTATT AGATC | 334 1 |
| 28 | H45_18 9-216 | ATCTAATAGGAATGAAAACATTT TAGCA | 2390 | TGCTAAAATGTTTTCATTCCTAT TAGAT | 334 2 |
| 28 | H45_19 0-217 | TCTAATAGGAATGAAAACATTTT AGCAG | 2391 | CTGCTAAAATGTTTTCATTCCTA TTAGA | 334 3 |
| 28 | H45_19 1-218 | CTAATAGGAATGAAAACATTTTA GCAGA | 2392 | TCTGCTAAAATGTTTTCATTCCT ATTAG | 334 4 |
| 28 | H45_19 2-219 | TAATAGGAATGAAAACATTTTAG CAGAC | 2393 | GTCTGCTAAAATGTTTTCATTCC TATTA | 334 5 |
| 28 | H45_19 3-220 | AATAGGAATGAAAACATTTTAGC AGACT | 2394 | AGTCTGCTAAAATGTTTTCATTC CTATT | 334 6 |
| 28 | 1145_19 4-221 | ATAGGAATGAAAACATTTTAGCA GACTT | 2395 | AAGTCTGCTAAAATGTTTTCATT CCTAT | 334 7 |
| 28 | H45_19 5-222 | TAGGAATGAAAACATTTTAGCAG ACTTT | 2396 | AAAGTCTGCTAAAATGTTTTCAT TCCTA | 334 8 |
| 28 | H45_19 6-223 | AGGAATGAAAACATTTTAGCAGA CTTTT | 2397 | AAAAGTCTGCTAAAATGTTTTCA TTCCT | 334 9 |
| 28 | H45_19 7-224 | GGAATGAAAACATTTTAGCAGAC TTTTT | 2398 | AAAAAGTCTGCTAAAATGTTTTC ATTCC | 335 0 |
| 28 | H45_19 8-225 | GAATGAAAACATTTTAGCAGACT TTTTA | 2399 | TAAAAAGTCTGCTAAAATGTTTT CATTC | 335 1 |
| 28 | H45_19 9-226 | AATGAAAACATTTTAGCAGACTT TTTAA | 2400 | TTAAAAAGTCTGCTAAAATGTTT TCATT | 335 2 |

**[Table 7-46]**

| | | | | | |
|---|---|---|---|---|---|
| 28 | H45_20 0-227 | ATGAAAACATTTTAGCAGACTTT TTAAG | 2401 | CTTAAAAAGTCTGCTAAAATGTT TTCAT | 335 3 |
| 28 | H45_20 1-228 | TGAAAACATTTTAGCAGACTTTT TAAGC | 2402 | GCTTAAAAAGTCTGCTAAAATGT TTTCA | 335 4 |
| 28 | H45_20 2-229 | GAAAACATTTTAGCAGACTTTTT AAGCT | 2403 | AGCTTAAAAAGTCTGCTAAAATG TTTTC | 335 5 |
| 28 | H45_20 3-230 | AAAACATTTTAGCAGACTTTTTA AGCTT | 2404 | AAGCTTAAAAGTCTGCTAAAAT GTTTT | 335 6 |
| 28 | H45_20 4-231 | AAACATTTTAGCAGACTTTTTAA GCTTT | 2405 | AAAGCTTAAAAAGTCTGCTAAAA TGTTT | 335 7 |
| 28 | H45_20 5-232 | AACATTTTAGCAGACTTTTTAAG CTTTC | 2406 | GAAAGCTTAAAAAGTCTGCTAAA ATGTT | 335 8 |
| 28 | H45_20 6-233 | ACATTTTAGCAGACTTTTTAAGC TTTCT | 2407 | AGAAAGCTTAAAAAGTCTGCTAA AATGT | 335 9 |
| 28 | H45_20 7-234 | CATTTTAGCAGACTTTTTAAGCT TTCTT | 2408 | AAGAAAGCTTAAAAAGTCTGCTA AAATG | 336 0 |
| 28 | H45_20 8-235 | ATTTTAGCAGACTTTTTAAGCTT TCTTT | 2409 | AAAGAAAGCTTAAAAAGTCTGCT AAAAT | 336 1 |
| 28 | H45_20 9-236 | TTTTAGCAGACTTTTTAAGCTTT CTTTA | 2410 | TAAAGAAAGCTTAAAAAGTCTGC TAAAA | 336 2 |
| 28 | H45_21 0-237 | TTTAGCAGACTTTTTAAGCTTTC TTTAG | 2411 | CTAAAGAAAGCTTAAAAAGTCTG CTAAA | 336 3 |
| 28 | H45_21 1-238 | TTAGCAGACTTTTTAAGCTTTCT TTAGA | 2412 | TCTAAAGAAAGCTTAAAAAGTCT GCTAA | 336 4 |
| 28 | H45_21 2-239 | TAGCAGACTTTTTAAGCTTTCTT TAGAA | 2413 | TTCTAAAGAAAGCTTAAAAAGTC TGCTA | 336 5 |
| 28 | H45_21 3-240 | AGCAGACTTTTTAAGCTTTCTTT AGAAG | 2414 | CTTCTAAAGAAAGCTTAAAAAGT CTGCT | 336 6 |
| 28 | H45_21 4-241 | GCAGACTTTTTAAGCTTTCTTTA GAAGA | 2415 | TCTTCTAAAGAAAGCTTAAAAAG TCTGC | 336 7 |
| 28 | H45_21 5-242 | CAGACTTTTTAAGCTTTCTTTAG AAGAA | 2416 | TTCTTCTAAAGAAAGCTTAAAAA GTCTG | 336 8 |
| 28 | H45_21 6-243 | AGACTTTTTAAGCTTTCTTTAGA AGAAT | 2417 | ATTCTTCTAAAGAAAGCTTAAAA AGTCT | 336 9 |
| 28 | H45_21 7-244 | GACTTTTTAAGCTTTCTTTAGAA GAATA | 2418 | TATTCTTCTAAAGAAAGCTTAAA AAGTC | 337 0 |

**[Table 7-47]**

| | | | | | |
|---|---|---|---|---|---|
| 28 | H45_21 8-245 | ACTTTTTAAGCTTTCTTTAGAAG AATAT | 2419 | ATATTCTTCTAAAGAAAGCTTAA AAAGT | 337 1 |
| 28 | H45_21 9-246 | CTTTTTAAGCTTTCTTTAGAAGA ATATT | 2420 | AATATTCTTCTAAAGAAAGCTTA AAAAG | 337 2 |
| 28 | H45_22 0-247 | TTTTTAAGCTTTCTTTAGAAGAA TATTT | 2421 | AAATATTCTTCTAAAGAAAGCTT AAAAA | 337 3 |
| 29 | H45_15 5-183 | AGCTGTCAGACAGAAAAAAGAGG TAGGGC | 2422 | GCCCTACCTCTTTTTTCTGTCTG ACAGCT | 337 4 |
| 29 | H45_15 6-184 | GCTGTCAGACAGAAAAAAGAGGT AGGGCG | 2423 | CGCCCTACCTCTTTTTCTGTCT GACAGC | 337 5 |
| 29 | H45_15 7-185 | CTGTCAGACAGAAAAAAGAGGTA GGGCGA | 2424 | TCGCCCTACCTCTTTTTTCTGTC TGACAG | 337 6 |
| 29 | H45_15 8-186 | TGTCAGACAGAAAAAAGAGGTAG GGCGAC | 2425 | GTCGCCCTACCTCTTTTTTCTGT CTGACA | 337 7 |
| 29 | H45_15 9-187 | GTCAGACAGAAAAAGAGGTAGG GCGACA | 2426 | TGTCGCCCTACCTCTTTTTTCTG TCTGAC | 337 8 |
| 29 | H45_16 0-188 | TCAGACAGAAAAAGAGGTAGGG CGACAG | 2427 | CTGTCGCCCTACCTCTTTTTCT GTCTGA | 337 9 |
| 29 | H45_16 1-189 | CAGACAGAAAAAAGAGGTAGGGC GACAGA | 2428 | TCTGTCGCCCTACCTCTTTTTC TGTCTG | 338 0 |
| 29 | H45_16 2-190 | AGACAGAAAAAAGAGGTAGGGCG ACAGAT | 2429 | ATCTGTCGCCCTACCTCTTTTTT CTGTCT | 338 1 |
| 29 | H45_16 3-191 | GACAGAAAAAGAGGTAGGGCGA CAGATC | 2430 | GATCTGTCGCCCTACCTCTTTTT TCTGTC | 338 2 |
| 29 | H45_16 4-192 | ACAGAAAAAAGAGGTAGGGCGAC AGATCT | 2431 | AGATCTGTCGCCCTACCTCTTTT TTCTGT | 338 3 |
| 29 | H45_16 5-193 | CAGAAAAAAGAGGTAGGGCGACA GATCTA | 2432 | TAGATCTGTCGCCCTACCTCTTT TTTCTG | 338 4 |
| 29 | H45_16 6-194 | AGAAAAAAGAGGTAGGGCGACAG ATCTAA | 2433 | TTAGATCTGTCGCCCTACCTCTT TTTTCT | 338 5 |
| 29 | H45_16 7-195 | GAAAAAAGAGGTAGGGCGACAGA TCTAAT | 2434 | ATTAGATCTGTCGCCCTACCTCT TTTTTC | 338 6 |
| 29 | H45_16 8-196 | AAAAAAGAGGTAGGGCGACAGAT CTAATA | 2435 | TATTAGATCTGTCGCCCTACCTC TTTTTT | 338 7 |
| 29 | H45_16 9-197 | AAAAAGAGGTAGGGCGACAGATC TAATAG | 2436 | CTATTAGATCTGTCGCCCTACCT CTTTTT | 338 8 |

**[Table 7-48]**

| | | | | | |
|---|---|---|---|---|---|
| 29 | H45_17 0-198 | AAAAGAGGTAGGGCGACAGATCT AATAGG | 2437 | CCTATTAGATCTGTCGCCCTACC TCTTTT | 338 9 |
| 29 | H45_17 1-199 | AAAGAGGTAGGGCGACAGATCTA ATAGGA | 2438 | TCCTATTAGATCTGTCGCCCTAC CTCTTT | 339 0 |
| 29 | H45_17 2-200 | AAGAGGTAGGGCGACAGATCTAA TAGGAA | 2439 | TTCCTATTAGATCTGTCGCCCTA CCTCTT | 339 1 |
| 29 | H45_17 3-201 | AGAGGTAGGGCGACAGATCTAAT AGGAAT | 2440 | ATTCCTATTAGATCTGTCGCCCT ACCTCT | 339 2 |
| 29 | H45_17 4-202 | GAGGTAGGGCGACAGATCTAATA GGAATG | 2441 | CATTCCTATTAGATCTGTCGCCC TACCTC | 339 3 |
| 29 | H45_17 5-203 | AGGTAGGGCGACAGATCTAATAG GAATGA | 2442 | TCATTCCTATTAGATCTGTCGCC CTACCT | 339 4 |
| 29 | H45_17 6-204 | GGTAGGGCGACAGATCTAATAGG AATGAA | 2443 | TTCATTCCTATTAGATCTGTCGC CCTACC | 339 5 |
| 29 | H45_17 7-205 | GTAGGGCGACAGATCTAATAGGA ATGAAA | 2444 | TTTCATTCCTATTAGATCTGTCG CCCTAC | 339 6 |
| 29 | H45_17 8-206 | TAGGGCGACAGATCTAATAGGAA TGAAAA | 2445 | TTTTCATTCCTATTAGATCTGTC GCCCTA | 339 7 |
| 29 | H45_17 9-207 | AGGGCGACAGATCTAATAGGAAT GAAAAC | 2446 | GTTTTCATTCCTATTAGATCTGT CGCCCT | 339 8 |
| 29 | H45_18 0-208 | GGGCGACAGATCTAATAGGAATG AAAACA | 2447 | TGTTTTCATTCCTATTAGATCTG TCGCCC | 339 9 |
| 29 | H45_18 1-209 | GGCGACAGATCTAATAGGAATGA AAACAT | 2448 | ATGTTTTCATTCCTATTAGATCT GTCGCC | 340 0 |
| 29 | H45_18 2-210 | GCGACAGATCTAATAGGAATGAA AACATT | 2449 | AATGTTTTCATTCCTATTAGATC TGTCGC | 340 1 |
| 29 | H45_18 3-211 | CGACAGATCTAATAGGAATGAAA ACATTT | 2450 | AAATGTTTTCATTCCTATTAGAT CTGTCG | 340 2 |
| 29 | H45_18 4-212 | GACAGATCTAATAGGAATGAAAA CATTTT | 2451 | AAAATGTTTTCATTCCTATTAGA TCTGTC | 340 3 |
| 29 | H45_18 5-213 | ACAGATCTAATAGGAATGAAAAC ATTTTA | 2452 | TAAAATGTTTTCATTCCTATTAG ATCTGT | 340 4 |
| 29 | H45_18 6-214 | CAGATCTAATAGGAATGAAAACA TTTTAG | 2453 | CTAAAATGTTTTCATTCCTATTA GATCTG | 340 5 |
| 29 | H45_18 7-215 | AGATCTAATAGGAATGAAAACAT TTTAGC | 2454 | GCTAAAATGTTTTCATTCCTATT AGATCT | 340 6 |

**[Table 7-49]**

| | | | | | |
|---|---|---|---|---|---|
| 29 | H45_18 8-216 | GATCTAATAGGAATGAAAACATT TTAGCA | 2455 | TGCTAAAATGTTTTCATTCCTAT TAGATC | 340 7 |
| 29 | H45_18 9-217 | ATCTAATAGGAATGAAAACATTT TAGCAG | 2456 | CTGCTAAAATGTTTTCATTCCTA TTAGAT | 340 8 |
| 29 | H45_19 0-218 | TCTAATAGGAATGAAAACATTTT AGCAGA | 2457 | TCTGCTAAAATGTTTTCATTCCT ATTAGA | 340 9 |
| 29 | H45_19 1-219 | CTAATAGGAATGAAAACATTTTA GCAGAC | 2458 | GTCTGCTAAAATGTTTTCATTCC TATTAG | 341 0 |
| 29 | H45_19 2-220 | TAATAGGAATGAAAACATTTTAG CAGACT | 2459 | AGTCTGCTAAAATGTTTTCATTC CTATTA | 341 1 |
| 29 | H45_19 3-221 | AATAGGAATGAAAACATTTTAGC AGACTT | 2460 | AAGTCTGCTAAAATGTTTTCATT CCTATT | 341 2 |
| 29 | H45_19 4-222 | ATAGGAATGAAAACATTTTAGCA GACTTT | 2461 | AAAGTCTGCTAAAATGTTTTCAT TCCTAT | 341 3 |
| 29 | H45_19 5-223 | TAGGAATGAAAACATTTTAGCAG ACTTTT | 2462 | AAAAGTCTGCTAAAATGTTTTCA TTCCTA | 341 4 |
| 29 | H45_19 6-224 | AGGAATGAAAACATTTTAGCAGA CTTTTT | 2463 | AAAAAGTCTGCTAAAATGTTTTC ATTCCT | 341 5 |
| 29 | H45_19 7-225 | GGAATGAAAACATTTTAGCAGAC TTTTTA | 2464 | TAAAAAGTCTGCTAAAATGTTTT CATTCC | 341 6 |
| 29 | H45_19 8-226 | GAATGAAAACATTTTAGCAGACT TTTTAA | 2465 | TTAAAAAGTCTGCTAAAATGTTT TCATTC | 341 7 |
| 29 | H45_19 9-227 | AATGAAAACATTTTAGCAGACTT TTTAAG | 2466 | CTTAAAAAGTCTGCTAAAATGTT TTCATT | 341 8 |
| 29 | H45_20 0-228 | ATGAAAACATTTTAGCAGACTTT TTAAGC | 2467 | GCTTAAAAAGTCTGCTAAAATGT TTTCAT | 341 9 |
| 29 | H45_20 1-229 | TGAAAACATTTTAGCAGACTTTT TAAGCT | 2468 | AGCTTAAAAAGTCTGCTAAAATG TTTTCA | 342 0 |
| 29 | H45_20 2-230 | GAAAACATTTTAGCAGACTTTTT AAGCTT | 2469 | AAGCTTAAAAAGTCTGCTAAAAT GTTTTC | 342 1 |
| 29 | H45_20 3-231 | AAAACATTTTAGCAGACTTTTTA AGCTTT | 2470 | AAAGCTTAAAAAGTCTGCTAAAA TGTTTT | 342 2 |
| 29 | H45_20 4-232 | AAACATTTTAGCAGACTTTTTAA GCTTTC | 2471 | GAAAGCTTAAAAAGTCTGCTAAA ATGTTT | 342 3 |
| 29 | H45_20 5-233 | AACATTTTAGCAGACTTTTTAAG CTTTCT | 2472 | AGAAAGCTTAAAAAGTCTGCTAA AATGTT | 342 4 |

**[Table 7-50]**

| | | | | | |
|---|---|---|---|---|---|
| 29 | H45_20 6-234 | ACATTTTAGCAGACTTTTTAAGC TTTCTT | 2473 | AAGAAAGCTTAAAAAGTCTGCTA AAATGT | 342 5 |
| 29 | H45_20 7-235 | CATTTTAGCAGACTTTTTAAGCT TTCTTT | 2474 | AAAGAAAGCTTAAAAAGTCTGCT AAAATG | 342 6 |
| 29 | H45_20 8-236 | ATTTTAGCAGACTTTTTAAGCTT TCTTTA | 2475 | TAAAGAAAGCTTAAAAAGTCTGC TAAAAT | 342 7 |
| 29 | H45_20 9-237 | TTTTAGCAGACTTTTTAAGCTTT CTTTAG | 2476 | CTAAAGAAAGCTTAAAAAGTCTG CTAAAA | 342 8 |
| 29 | H45_21 0-238 | TTTAGCAGACTTTTTAAGCTTTC TTTAGA | 2477 | TCTAAAGAAAGCTTAAAAAGTCT GCTAAA | 342 9 |
| 29 | H45_21 1-239 | TTAGCAGACTTTTTAAGCTTTCT TTAGAA | 2478 | TTCTAAAGAAAGCTTAAAAAGTC TGCTAA | 343 0 |
| 29 | H45_21 2-240 | TAGCAGACTTTTTAAGCTTTCTT TAGAAG | 2479 | CTTCTAAAGAAAGCTTAAAAAGT CTGCTA | 343 1 |
| 29 | H45_21 3-241 | AGCAGACTTTTTAAGCTTTCTTT AGAAGA | 2480 | TCTTCTAAAGAAAGCTTAAAAAG TCTGCT | 343 2 |
| 29 | H45_21 4-242 | GCAGACTTTTTAAGCTTTCTTTA GAAGAA | 2481 | TTCTTCTAAAGAAAGCTTAAAAA GTCTGC | 343 3 |
| 29 | H45_21 5-243 | CAGACTTTTTAAGCTTTCTTTAG AAGAAT | 2482 | ATTCTTCTAAAGAAAGCTTAAAA AGTCTG | 343 4 |
| 29 | H45_21 6-244 | AGACTTTTTAAGCTTTCTTTAGA AGAATA | 2483 | TATTCTTCTAAAGAAAGCTTAAA AAGTCT | 343 5 |
| 29 | H45_21 7-245 | GACTTTTTAAGCTTTCTTTAGAA GAATAT | 2484 | ATATTCTTCTAAAGAAAGCTTAA AAAGTC | 343 6 |
| 29 | H45_21 8-246 | ACTTTTTAAGCTTTCTTTAGAAG AATATT | 2485 | AATATTCTTCTAAAGAAAGCTTA AAAAGT | 343 7 |
| 29 | H45_21 9-247 | CTTTTTAAGCTTTCTTTAGAAGA ATATTT | 2486 | AAATATTCTTCTAAAGAAAGCTT AAAAAG | 343 8 |
| 29 | H45_22 0-248 | TTTTTAAGCTTTCTTTAGAAGAA TATTTC | 2487 | GAAATATTCTTCTAAAGAAAGCT TAAAAA | 343 9 |
| 30 | H45_15 4-183 | CAGCTGTCAGACAGAAAAAAGAG GTAGGGC | 2488 | GCCCTACCTCTTTTTTCTGTCTG ACAGCTG | 344 0 |
| 30 | H45_15 5-184 | AGCTGTCAGACAGAAAAAAGAGG TAGGGCG | 2489 | CGCCCTACCTCTTTTTCTGTCT GACAGCT | 344 1 |
| 30 | H45_15 6-185 | GCTGTCAGACAGAAAAAAGAGGT AGGGCGA | 2490 | TCGCCCTACCTCTTTTTCTGTC TGACAGC | 344 2 |

**[Table 7-51]**

| | | | | | |
|---|---|---|---|---|---|
| 30 | H45_15 7-186 | CTGTCAGACAGAAAAAGAGGTA GGGCGAC | 2491 | GTCGCCCTACCTCTTTTTTCTGT CTGACAG | 344 3 |
| 30 | H45_15 8-187 | TGTCAGACAGAAAAAGAGGTAG GGCGACA | 2492 | TGTCGCCCTACCTCTTTTTCTG TCTGACA | 344 4 |
| 30 | H45_15 9-188 | GTCAGACAGAAAAAAGAGGTAGG GCGACAG | 2493 | CTGTCGCCCTACCTCTTTTTCT GTCTGAC | 344 5 |
| 30 | H45_16 0-189 | TCAGACAGAAAAAAGAGGTAGGG CGACAGA | 2494 | TCTGTCGCCCTACCTCTTTTTTC TGTCTGA | 344 6 |
| 30 | H45_16 1-190 | CAGACAGAAAAAAGAGGTAGGGC GACAGAT | 2495 | ATCTGTCGCCCTACCTCTTTTTT CTGTCTG | 344 7 |
| 30 | H45_16 2-191 | AGACAGAAAAAAGAGGTAGGGCG ACAGATC | 2496 | GATCTGTCGCCCTACCTCTTTTT TCTGTCT | 344 8 |
| 30 | H45_16 3-192 | GACAGAAAAAGAGGTAGGGCGA CAGATCT | 2497 | AGATCTGTCGCCCTACCTCTTTT TTCTGTC | 344 9 |
| 30 | H45_16 4-193 | ACAGAAAAAAGAGGTAGGGCGAC AGATCTA | 2498 | TAGATCTGTCGCCCTACCTCTTT TTTCTGT | 345 0 |
| 30 | H45_16 5-194 | CAGAAAAAAGAGGTAGGGCGACA GATCTAA | 2499 | TTAGATCTGTCGCCCTACCTCTT TTTTCTG | 345 1 |
| 30 | H45_16 6-195 | AGAAAAAAGAGGTAGGGCGACAG ATCTAAT | 2500 | ATTAGATCTGTCGCCCTACCTCT TTTTTCT | 345 2 |
| 30 | H45_16 7-196 | GAAAAAAGAGGTAGGGCGACAGA TCTAATA | 2501 | TATTAGATCTGTCGCCCTACCTC TTTTTTC | 345 3 |
| 30 | H45_16 8-197 | AAAAAAGAGGTAGGGCGACAGAT CTAATAG | 2502 | CTATTAGATCTGTCGCCCTACCT CTTTTTT | 345 4 |
| 30 | H45_16 9-198 | AAAAAGAGGTAGGGCGACAGATC TAATAGG | 2503 | CCTATTAGATCTGTCGCCCTACC TCTTTTT | 345 5 |
| 30 | H45_17 0-199 | AAAAGAGGTAGGGCGACAGATCT AATAGGA | 2504 | TCCTATTAGATCTGTCGCCCTAC CTCTTTT | 345 6 |
| 30 | H45_17 1-200 | AAAGAGGTAGGGCGACAGATCTA ATAGGAA | 2505 | TTCCTATTAGATCTGTCGCCCTA CCTCTTT | 345 7 |
| 30 | H45_17 2-201 | AAGAGGTAGGGCGACAGATCTAA TAGGAAT | 2506 | ATTCCTATTAGATCTGTCGCCCT ACCTCTT | 345 8 |
| 30 | H45_17 3-202 | AGAGGTAGGGCGACAGATCTAAT AGGAATG | 2507 | CATTCCTATTAGATCTGTCGCCC TACCTCT | 345 9 |
| 30 | H45_17 4-203 | GAGGTAGGGCGACAGATCTAATA GGAATGA | 2508 | TCATTCCTATTAGATCTGTCGCC CTACCTC | 346 0 |

**[Table 7-52]**

| | | | | | |
|---|---|---|---|---|---|
| 30 | H45_17 5-204 | AGGTAGGGCGACAGATCTAATAG GAATGAA | 2509 | TTCATTCCTATTAGATCTGTCGC CCTACCT | 346 1 |
| 30 | H45_17 6-205 | GGTAGGGCGACAGATCTAATAGG AATGAAA | 2510 | TTTCATTCCTATTAGATCTGTCG CCCTACC | 346 2 |
| 30 | H45_17 7-206 | GTAGGGCGACAGATCTAATAGGA ATGAAAA | 2511 | TTTTCATTCCTATTAGATCTGTC GCCCTAC | 346 3 |
| 30 | H45_17 8-207 | TAGGGCGACAGATCTAATAGGAA TGAAAAC | 2512 | GTTTTCATTCCTATTAGATCTGT CGCCCTA | 346 4 |
| 30 | H45_17 9-208 | AGGGCGACAGATCTAATAGGAAT GAAAACA | 2513 | TGTTTTCATTCCTATTAGATCTG TCGCCCT | 346 5 |
| 30 | H45_18 0-209 | GGGCGACAGATCTAATAGGAATG AAAACAT | 2514 | ATGTTTTCATTCCTATTAGATCT GTCGCCC | 346 6 |
| 30 | H45_18 1-210 | GGCGACAGATCTAATAGGAATGA AAACATT | 2515 | AATGTTTTCATTCCTATTAGATC TGTCGCC | 346 7 |
| 30 | H45_18 2-211 | GCGACAGATCTAATAGGAATGAA AACATTT | 2516 | AAATGTTTTCATTCCTATTAGAT CTGTCGC | 346 8 |
| 30 | H45_18 3-212 | CGACAGATCTAATAGGAATGAAA ACATTTT | 2517 | AAAATGTTTTCATTCCTATTAGA TCTGTCG | 346 9 |
| 30 | H45_18 4-213 | GACAGATCTAATAGGAATGAAAA CATTTTA | 2518 | TAAAATGTTTTCATTCCTATTAG ATCTGTC | 347 0 |
| 30 | H45_18 5-214 | ACAGATCTAATAGGAATGAAAAC ATTTTAG | 2519 | CTAAAATGTTTTCATTCCTATTA GATCTGT | 347 1 |
| 30 | H45_18 6-215 | CAGATCTAATAGGAATGAAAACA TTTTAGC | 2520 | GCTAAAATGTTTTCATTCCTATT AGATCTG | 347 2 |
| 30 | H45_18 7-216 | AGATCTAATAGGAATGAAAACAT TTTAGCA | 2521 | TGCTAAAATGTTTTCATTCCTAT TAGATCT | 347 3 |
| 30 | H45_18 8-217 | GATCTAATAGGAATGAAAACATT TTAGCAG | 2522 | CTGCTAAAATGTTTTCATTCCTA TTAGATC | 347 4 |
| 30 | H45_18 9-218 | ATCTAATAGGAATGAAAACATTT TAGCAGA | 2523 | TCTGCTAAAATGTTTTCATTCCT ATTAGAT | 347 5 |
| 30 | H45_19 0-219 | TCTAATAGGAATGAAAACATTTT AGCAGAC | 2524 | GTCTGCTAAAATGTTTTCATTCC TATTAGA | 347 6 |
| 30 | H45_19 1-220 | CTAATAGGAATGAAAACATTTTA GCAGACT | 2525 | AGTCTGCTAAAATGTTTTCATTC CTATTAG | 347 7 |
| 30 | H45_19 2-221 | TAATAGGAATGAAAACATTTTAG CAGACTT | 2526 | AAGTCTGCTAAAATGTTTTCATT CCTATTA | 347 8 |

**[Table 7-53]**

| | | | | | |
|---|---|---|---|---|---|
| 30 | H45_19 3-222 | AATAGGAATGAAAACATTTTAGC AGACTTT | 2527 | AAAGTCTGCTAAAATGTTTTCAT TCCTATT | 347 9 |
| 30 | H45_19 4-223 | ATAGGAATGAAAACATTTTAGCA GACTTTT | 2528 | AAAAGTCTGCTAAAATGTTTTCA TTCCTAT | 348 0 |
| 30 | H45_19 5-224 | TAGGAATGAAAACATTTTAGCAG ACTTTTT | 2529 | AAAAAGTCTGCTAAAATGTTTTC ATTCCTA | 348 1 |
| 30 | H45_19 6-225 | AGGAATGAAAACATTTTAGCAGA CTTTTTA | 2530 | TAAAAAGTCTGCTAAAATGTTTT CATTCCT | 348 2 |
| 30 | H45_19 7-226 | GGAATGAAAACATTTTAGCAGAC TTTTTAA | 2531 | TTAAAAAGTCTGCTAAAATGTTT TCATTCC | 348 3 |
| 30 | H45_19 8-227 | GAATGAAAACATTTTAGCAGACT TTTTAAG | 2532 | CTTAAAAAGTCTGCTAAAATGTT TTCATTC | 348 4 |
| 30 | H45_19 9-228 | AATGAAAACATTTTAGCAGACTT TTTAAGC | 2533 | GCTTAAAAAGTCTGCTAAAATGT TTTCATT | 348 5 |
| 30 | H45_20 0-229 | ATGAAAACATTTTAGCAGACTTT TTAAGCT | 2534 | AGCTTAAAAAGTCTGCTAAAATG TTTTCAT | 348 6 |
| 30 | H45_20 1-230 | TGAAAACATTTTAGCAGACTTTT TAAGCTT | 2535 | AAGCTTAAAAAGTCTGCTAAAAT GTTTTCA | 348 7 |
| 30 | H45_20 2-231 | GAAAACATTTTAGCAGACTTTTT AAGCTTT | 2536 | AAAGCTTAAAAAGTCTGCTAAAA TGTTTTC | 348 8 |
| 30 | H45_20 3-232 | AAAACATTTTAGCAGACTTTTTA AGCTTTC | 2537 | GAAAGCTTAAAAAGTCTGCTAAA ATGTTTT | 348 9 |
| 30 | H45_20 4-233 | AAACATTTTAGCAGACTTTTTAA GCTTTCT | 2538 | AGAAAGCTTAAAAAGTCTGCTAA AATGTTT | 349 0 |
| 30 | H45_20 5-234 | AACATTTTAGCAGACTTTTTAAG CTTTCTT | 2539 | AAGAAAGCTTAAAAAGTCTGCTA AAATGTT | 349 1 |
| 30 | H45_20 6-235 | ACATTTTAGCAGACTTTTTAAGC TTTCTTT | 2540 | AAAGAAAGCTTAAAAAGTCTGCT AAAATGT | 349 2 |
| 30 | H45_20 7-236 | CATTTTAGCAGACTTTTTAAGCT TTCTTTA | 2541 | TAAAGAAAGCTTAAAAAGTCTGC TAAAATG | 349 3 |
| 30 | H45_20 8-237 | ATTTTAGCAGACTTTTTAAGCTT TCTTTAG | 2542 | CTAAAGAAAGCTTAAAAAGTCTG CTAAAAT | 349 4 |
| 30 | H45_20 9-238 | TTTTAGCAGACTTTTTAAGCTTT CTTTAGA | 2543 | TCTAAAGAAAGCTTAAAAAGTCT GCTAAAA | 349 5 |
| 30 | H45_21 0-239 | TTTAGCAGACTTTTTAAGCTTTC TTTAGAA | 2544 | TTCTAAAGAAAGCTTAAAAAGTC TGCTAAA | 349 6 |

**[Table 7-54]**

| | | | | | |
|---|---|---|---|---|---|
| 30 | H45_21 1-240 | TTAGCAGACTTTTTAAGCTTTCT TTAGAAG | 2545 | CTTCTAAAGAAAGCTTAAAAAGT CTGCTAA | 349 7 |
| 30 | H45_21 2-241 | TAGCAGACTTTTTAAGCTTTCTT TAGAAGA | 2546 | TCTTCTAAAGAAAGCTTAAAAAG TCTGCTA | 349 8 |
| 30 | H45_21 3-242 | AGCAGACTTTTTAAGCTTTCTTT AGAAGAA | 2547 | TTCTTCTAAAGAAAGCTTAAAAA GTCTGCT | 349 9 |
| 30 | H45_21 4-243 | GCAGACTTTTTAAGCTTTCTTTA GAAGAAT | 2548 | ATTCTTCTAAAGAAAGCTTAAAA AGTCTGC | 350 0 |
| 30 | H45_21 5-244 | CAGACTTTTTAAGCTTTCTTTAG AAGAATA | 2549 | TATTCTTCTAAAGAAAGCTTAAA AAGTCTG | 350 1 |
| 30 | H45_21 6-245 | AGACTTTTTAAGCTTTCTTTAGA AGAATAT | 2550 | ATATTCTTCTAAAGAAAGCTTAA AAAGTCT | 350 2 |
| 30 | H45_21 7-246 | GACTTTTTAAGCTTTCTTTAGAA GAATATT | 2551 | AATATTCTTCTAAAGAAAGCTTA AAAAGTC | 350 3 |
| 30 | H45_21 8-247 | ACTTTTTAAGCTTTCTTTAGAAG AATATTT | 2552 | AAATATTCTTCTAAAGAAAGCTT AAAAAGT | 350 4 |
| 30 | H45_21 9-248 | CTTTTTAAGCTTTCTTTAGAAGA ATATTTC | 2553 | GAAATATTCTTCTAAAGAAAGCT TAAAAAG | 350 5 |
| 30 | H45_22 0-249 | TTTTTAAGCTTTCTTTAGAAGAA TATTTCA | 2554 | TGAAATATTCTTCTAAAGAAAGC TTAAAAA | 350 6 |

In one embodiment, the third antisense oligomer of the present invention comprises a base sequence complementary to:
(a) any one base sequence selected from the group consisting of SEQ ID NOs: 1603 to 2554;
(b) a base sequence that hybridizes under stringent conditions to a base sequence complementary to any one base sequence selected from the group consisting of SEQ ID NOs: 1603 to 2554;
(c) a base sequence that has at least 85% identity with any one base sequence selected from the group consisting of SEQ ID NOs: 1603 to 2554, and has a length within ±15% of the length of the any one base sequence selected; or
(d) a partial base sequence of any one base sequence selected from the group consisting of the base sequences (a), (b), and (c) .

In one embodiment, the third antisense oligomer comprises or consists of a base sequence complementary to:
(a) any one base sequence selected from the group consisting of SEQ ID NOs: 1611 to 1654, 1664 to 1707, 1718 to 1761, 1773 to 1816, 1829 to 1872, 1886 to 1929, 1944 to 1987, 2003 to 2046, 2063 to 2106, 2124 to 2167, 2186 to 2229, 2249 to 2292, 2313 to 2356, 2378 to 2421, 2444 to 2487, and 2511 to 2554;
(b) a base sequence that hybridizes under stringent conditions to a base sequence complementary to any one base sequence selected from the group consisting of SEQ ID NOs: 1611 to 1654, 1664 to 1707, 1718 to 1761, 1773 to 1816, 1829 to 1872, 1886 to 1929, 1944 to 1987, 2003 to 2046, 2063 to 2106, 2124 to 2167, 2186 to 2229, 2249 to 2292, 2313 to 2356, 2378 to 2421, 2444 to 2487, and 2511 to 2554;
(c) a base sequence that has at least 85% identity with any one base sequence selected from the group consisting of SEQ ID NOs: 1611 to 1654, 1664 to 1707, 1718 to 1761, 1773 to 1816, 1829 to 1872, 1886 to 1929, 1944 to 1987, 2003 to 2046, 2063 to 2106, 2124 to 2167, 2186 to 2229, 2249 to 2292, 2313 to 2356, 2378 to 2421, 2444 to 2487, and 2511 to 2554, and has a length within ±15% of the length of the any one base sequence selected; or
(d) a partial base sequence of any one base sequence selected from the group consisting of the base sequences (a), (b), and (c) .

In one embodiment, the third antisense oligomer comprises or consists of a base sequence complementary to:
(a) any one base sequence selected from the group consisting of SEQ ID NOs: 1614 to 1654, 1667 to 1707, 1721 to 1761, 1776 to 1816, 1832 to 1872, 1889 to 1929, 1947 to 1987, 2006 to 2046, 2066 to 2106, 2127 to 2167, 2189 to 2229, 2252 to 2292, 2316 to 2356, 2381 to 2421, 2447 to 2487, and 2514 to 2554;
(b) a base sequence that hybridizes under stringent conditions to a base sequence complementary to any one base sequence selected from the group consisting of SEQ ID NOs: 1614 to 1654, 1667 to 1707, 1721 to 1761, 1776 to 1816, 1832 to 1872, 1889 to 1929, 1947 to 1987, 2006 to 2046, 2066 to 2106, 2127 to 2167, 2189 to 2229, 2252 to 2292, 2316 to 2356, 2381 to 2421, 2447 to 2487, and 2514 to 2554;
(c) a base sequence that has at least 85% identity with any one base sequence selected from the group consisting of SEQ ID NOs: 1614 to 1654, 1667 to 1707, 1721 to 1761, 1776 to 1816, 1832 to 1872, 1889 to 1929, 1947 to 1987, 2006 to 2046, 2066 to 2106, 2127 to 2167, 2189 to 2229, 2252 to 2292, 2316 to 2356, 2381 to 2421, 2447 to 2487, and 2514 to 2554, and has a length within ±15% of the length of the any one base sequence selected; or
(d) a partial base sequence of any one base sequence selected from the group consisting of the base sequences (a), (b), and (c).

In one embodiment, the third antisense oligomer comprises or consists of a base sequence complementary to:
(a) any one base sequence selected from the group consisting of SEQ ID NOs: 1617 to 1654, 1670 to 1707, 1724 to 1761, 1779 to 1816, 1835 to 1872, 1892 to 1929, 1950 to 1987, 2009 to 2046, 2069 to 2106, 2130 to 2167, 2192 to 2229, 2255 to 2292, 2319 to 2356, 2384 to 2421, 2450 to 2487, and 2517 to 2554;
(b) a base sequence that hybridizes under stringent conditions to a base sequence complementary to any one base sequence selected from the group consisting of SEQ ID NOs: 1617 to 1654, 1670 to 1707, 1724 to 1761, 1779 to 1816, 1835 to 1872, 1892 to 1929, 1950 to 1987, 2009 to 2046, 2069 to 2106, 2130 to 2167, 2192 to 2229, 2255 to 2292, 2319 to 2356, 2384 to 2421, 2450 to 2487, and 2517 to 2554;
(c) a base sequence that has at least 85% identity with any one base sequence selected from the group consisting of SEQ ID NOs: 1617 to 1654, 1670 to 1707, 1724 to 1761, 1779 to 1816, 1835 to 1872, 1892 to 1929, 1950 to 1987, 2009 to 2046, 2069 to 2106, 2130 to 2167, 2192 to 2229, 2255 to 2292, 2319 to 2356, 2384 to 2421, 2450 to 2487, and 2517 to 2554, and has a length within ±15% of the length of the any one base sequence selected; or
(d) a partial base sequence of any one base sequence selected from the group consisting of the base sequences (a), (b), and (c).

Herein, the base sequence (c) is a mutant type of the base sequence (a), and examples of such a mutant type also include:
(c-1) a base sequence that has at least 85% identity with any one base sequence selected from the group consisting of SEQ ID NOs: 1603 to 2554, and has a length within ±15% of the length of the any one base sequence selected,
(c-2) a base sequence that has at least 86% identity with any one base sequence selected from the group consisting of SEQ ID NOs: 1603 to 2554, and has a length within ±14% of the length of the any one base sequence selected,
(c-3) a base sequence that has at least 87% identity with any one base sequence selected from the group consisting of SEQ ID NOs: 1603 to 2554, and has a length within ±13% of the length of the any one base sequence selected,
(c-4) a base sequence that has at least 88% identity with any one base sequence selected from the group consisting of SEQ ID NOs: 1603 to 2554, and has a length within ±12% of the length of the any one base sequence selected,
(c-5) a base sequence that has at least 89% identity with any one base sequence selected from the group consisting of SEQ ID NOs: 1603 to 2554, and has a length within ±11% of the length of the any one base sequence selected,
(c-6) a base sequence that has at least 90% identity with any one base sequence selected from the group consisting of SEQ ID NOs: 1603 to 2554, and has a length within ±10% of the length of the any one base sequence selected,
(c-7) a base sequence that has at least 91% identity with any one base sequence selected from the group consisting of SEQ ID NOs: 1603 to 2554, and has a length within ±9% of the length of the any one base sequence selected,
(c-8) a base sequence that has at least 92% identity with any one base sequence selected from the group consisting of SEQ ID NOs: 1603 to 2554, and has a length within ±8% of the length of the any one base sequence selected,
(c-9) a base sequence that has at least 93% identity with any one base sequence selected from the group consisting of SEQ ID NOs: 1603 to 2554, and has a length within ±7% of the length of the any one base sequence selected,
(c-10) a base sequence that has at least 94% identity with any one base sequence selected from the group consisting of SEQ ID NOs: 1603 to 2554, and has a length within ±6% of the length of the any one base sequence selected,
(c-11) a base sequence that has at least 95% identity with any one base sequence selected from the group consisting of SEQ ID NOs: 1603 to 2554, and has a length within ±5% of the length of the any one base sequence selected,
(c-12) a base sequence that has at least 96% identity with any one base sequence selected from the group consisting of SEQ ID NOs: 1603 to 2554, and has a length within ±4% of the length of the any one base sequence selected,
(c-13) a base sequence that has at least 97% identity with any one base sequence selected from the group consisting of SEQ ID NOs: 1603 to 2554, and has a length within ±3% of the length of the any one base sequence selected,
(c-14) a base sequence that has at least 98% identity with any one base sequence selected from the group consisting of SEQ ID NOs: 1603 to 2554, and has a length within ±2% of the length of the any one base sequence selected,
(c-15) a base sequence that has at least 99% identity with any one base sequence selected from the group consisting of SEQ ID NOs: 1603 to 2554, and has a length within ±1% of the length of the any one base sequence selected, and
(c-16) a base sequence that has at least 99.5% identity with any one base sequence selected from the group consisting of SEQ ID NOs: 1603 to 2554, and has a length within ±0.5% of the length of the any one base sequence selected.

In one embodiment, the third antisense oligomer comprises or consists of:
(a) any one base sequence selected from the group consisting of SEQ ID NOs: 2555 to 3506; or
(b) a base sequence that has at least 85% identity with any one base sequence selected from the group consisting of SEQ ID NOs: 2555 to 3506, and has a length within ±15% of the length of the any one base sequence selected.

Herein, the base sequence (b) is a mutant type of the base sequence (a), and examples of such a mutant type also include:
(b-1) a base sequence that has at least 85% identity with any one base sequence selected from the group consisting of SEQ ID NOs: 2555 to 3506, and has a length within ±15% of the length of the any one base sequence selected,
(b-2) a base sequence that has at least 86% identity with any one base sequence selected from the group consisting of SEQ ID NOs: 2555 to 3506, and has a length within ±14% of the length of the any one base sequence selected,
(b-3) a base sequence that has at least 87% identity with any one base sequence selected from the group consisting of SEQ ID NOs: 2555 to 3506, and has a length within ±13% of the length of the any one base sequence selected,
(b-4) a base sequence that has at least 88% identity with any one base sequence selected from the group consisting of SEQ ID NOs: 2555 to 3506, and has a length within ±12% of the length of the any one base sequence selected,
(b-5) a base sequence that has at least 89% identity with any one base sequence selected from the group consisting of SEQ ID NOs: 2555 to 3506, and has a length within ±11% of the length of the any one base sequence selected,
(b-6) a base sequence that has at least 90% identity with any one base sequence selected from the group consisting of SEQ ID NOs: 2555 to 3506, and has a length within ±10% of the length of the any one base sequence selected,
(b-7) a base sequence that has at least 91% identity with any one base sequence selected from the group consisting of SEQ ID NOs: 2555 to 3506, and has a length within ±9% of the length of the any one base sequence selected,
(b-8) a base sequence that has at least 92% identity with any one base sequence selected from the group consisting of SEQ ID NOs: 2555 to 3506, and has a length within ±8% of the length of the any one base sequence selected,
(b-9) a base sequence that has at least 93% identity with any one base sequence selected from the group consisting of SEQ ID NOs: 2555 to 3506, and has a length within ±7% of the length of the any one base sequence selected,
(b-10) a base sequence that has at least 94% identity with any one base sequence selected from the group consisting of SEQ ID NOs: 2555 to 3506, and has a length within ±6% of the length of the any one base sequence selected,
(b-11) a base sequence that has at least 95% identity with any one base sequence selected from the group consisting of SEQ ID NOs: 2555 to 3506, and has a length within ±5% of the length of the any one base sequence selected,
(b-12) a base sequence that has at least 96% identity with any one base sequence selected from the group consisting of SEQ ID NOs: 2555 to 3506, and has a length within ±4% of the length of the any one base sequence selected,
(b-13) a base sequence that has at least 97% identity with any one base sequence selected from the group consisting of SEQ ID NOs: 2555 to 3506, and has a length within ±3% of the length of the any one base sequence selected,
(b-14) a base sequence that has at least 98% identity with any one base sequence selected from the group consisting of SEQ ID NOs: 2555 to 3506, and has a length within ±2% of the length of the any one base sequence selected,
(b-15) a base sequence that has at least 99% identity with any one base sequence selected from the group consisting of SEQ ID NOs: 2555 to 3506, and has a length within ±1% of the length of the any one base sequence selected, and
(b-16) a base sequence that has at least 99.5% identity with any one base sequence selected from the group consisting of SEQ ID NOs: 2555 to 3506, and has a length within ±0.5% of the length of the any one base sequence selected.

In one embodiment, the third antisense oligomer of the present invention comprises or consists of any one base sequence selected from the group consisting of SEQ ID NOs: 2555 to 3506.

In one embodiment, the third antisense oligomer comprises or consists of a base sequence selected from the group consisting of SEQ ID NOs: 3060, 3065, 3077, 3082, 3087, 3090, 3096, 3108, 3119, and 3320. In one embodiment, the third antisense oligomer comprises or consists of a base sequence selected from the group consisting of SEQ ID NOs: 3077, 3082, 3087, 3090, 3096, 3108, and 3119. In one embodiment, the third antisense oligomer comprises or consists of a base sequence selected from the group consisting of SEQ ID NOs: 3082, 3087, 3090, 3096, 3108, and 3119.

A combination of the first unit oligomer and the second unit oligomer comprised in the first antisense oligomer of the present invention, and the second antisense oligomer of the present invention (optionally the third antisense oligomer of the present invention) is not limited, and any combination can be used.

In one embodiment, the first antisense oligomer comprises the first unit oligomer and the second unit oligomer from the 5' ends in this order, and
the first unit oligomer comprises a base sequence of SEQ ID NO: 1201, the second unit oligomer comprises a base sequence of SEQ ID NO: 151, and the second antisense oligomer comprises or consists of a base sequence of SEQ ID NO: 4950,
the first unit oligomer comprises a base sequence of SEQ ID NO: 1201, the second unit oligomer comprises a base sequence of SEQ ID NO: 201, and the second antisense oligomer comprises or consists of a base sequence of SEQ ID NO: 4950,
the first unit oligomer comprises a base sequence of SEQ ID NO: 1201, the second unit oligomer comprises a base sequence of SEQ ID NO: 203, and the second antisense oligomer comprises or consists of a base sequence of SEQ ID NO: 4950,
the first unit oligomer comprises a base sequence of SEQ ID NO: 1201, the second unit oligomer comprises a base sequence of SEQ ID NO: 205, and the second antisense oligomer comprises or consists of a base sequence of SEQ ID NO: 4950,
the first unit oligomer comprises a base sequence of SEQ ID NO: 1239, the second unit oligomer comprises a base sequence of SEQ ID NO: 114, and the second antisense oligomer comprises or consists of a base sequence of SEQ ID NO: 4950,
the first unit oligomer comprises a base sequence of SEQ ID NO: 1224, the second unit oligomer comprises a base sequence of SEQ ID NO: 124, and the second antisense oligomer comprises or consists of a base sequence of SEQ ID NO: 4950,
the first unit oligomer comprises a base sequence of SEQ ID NO: 1180, the second unit oligomer comprises a base sequence of SEQ ID NO: 151, and the second antisense oligomer comprises or consists of a base sequence of SEQ ID NO: 4950,
the first unit oligomer comprises a base sequence of SEQ ID NO: 1190, the second unit oligomer comprises a base sequence of SEQ ID NO: 151, and the second antisense oligomer comprises or consists of a base sequence of SEQ ID NO: 4950,
the first unit oligomer comprises a base sequence of SEQ ID NO: 1212, the second unit oligomer comprises a base sequence of SEQ ID NO: 151, and the second antisense oligomer comprises or consists of a base sequence of SEQ ID NO: 4950,
the first unit oligomer comprises a base sequence of SEQ ID NO: 1222, the second unit oligomer comprises a base sequence of SEQ ID NO: 151, and the second antisense oligomer comprises or consists of a base sequence of SEQ ID NO: 4950,
the first unit oligomer comprises a base sequence of SEQ ID NO: 1201, the second unit oligomer comprises a base sequence of SEQ ID NO: 151, and the second antisense oligomer comprises or consists of a base sequence of SEQ ID NO: 4698,
the first unit oligomer comprises a base sequence of SEQ ID NO: 1201, the second unit oligomer comprises a base sequence of SEQ ID NO: 151, and the second antisense oligomer comprises or consists of a base sequence of SEQ ID NO: 4702,
the first unit oligomer comprises a base sequence of SEQ ID NO: 1201, the second unit oligomer comprises a base sequence of SEQ ID NO: 151, and the second antisense oligomer comprises or consists of a base sequence of SEQ ID NO: 4752,
the first unit oligomer comprises a base sequence of SEQ ID NO: 1201, the second unit oligomer comprises a base sequence of SEQ ID NO: 151, and the second antisense oligomer comprises or consists of a base sequence of SEQ ID NO: 4923,
the first unit oligomer comprises a base sequence of SEQ ID NO: 1201, the second unit oligomer comprises a base sequence of SEQ ID NO: 151, and the second antisense oligomer comprises or consists of a base sequence of SEQ ID NO: 4926,
the first unit oligomer comprises a base sequence of SEQ ID NO: 1201, the second unit oligomer comprises a base sequence of SEQ ID NO: 151, and the second antisense oligomer comprises or consists of a base sequence of SEQ ID NO: 4936,
the first unit oligomer comprises a base sequence of SEQ ID NO: 1201, the second unit oligomer comprises a base sequence of SEQ ID NO: 151, and the second antisense oligomer comprises or consists of a base sequence of SEQ ID NO: 4977, or
the first unit oligomer comprises a base sequence of SEQ ID NO: 1180, the second unit oligomer comprises a base sequence of SEQ ID NO: 151, and the second antisense oligomer comprises or consists of a base sequence of SEQ ID NO: 4977.

In one embodiment, the first antisense oligomer comprises the first unit oligomer and the second unit oligomer from the 5' ends in this order, the first unit oligomer comprises any one base sequence selected from SEQ ID NOs: 907 to 1602, the second unit oligomer comprises any one base sequence selected from SEQ ID NOs: 106 to 210, and the second antisense oligomer comprises any one base sequence selected from SEQ ID NOs: 4299 to 5090. In one embodiment, the first antisense oligomer comprises the first unit oligomer and the second unit oligomer from the 5' ends in this order, the first unit oligomer comprises a base sequence of SEQ ID No: 1201, the second unit oligomer comprises a base sequence of SEQ ID NO: 151, and the second antisense oligomer comprises a base sequence of SEQ ID NO: 4950 or 4880 (preferably 4950) .

In one embodiment, the first antisense oligomer comprises the first unit oligomer and the second unit oligomer from the 5' ends in this order, and
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4950, and the third antisense oligomer comprises or consists of a base sequence of SEQ ID NO: 3082,
the first unit oligomer comprises or consists of a base sequence of SEQ ID NO: 1201, the second unit oligomer comprises or consists of a base sequence of SEQ ID NO: 201, the second antisense oligomer comprises or consists of a base sequence of SEQ ID NO: 4950, and the third antisense oligomer comprises or consists of a base sequence of SEQ ID NO: 3082,
the first unit oligomer comprises or consists of a base sequence of SEQ ID NO: 1201, the second unit oligomer comprises or consists of a base sequence of SEQ ID NO: 203, the second antisense oligomer comprises or consists of a base sequence of SEQ ID NO: 4950, and the third antisense oligomer comprises or consists of a base sequence of SEQ ID NO: 3082,
the first unit oligomer comprises or consists of a base sequence of SEQ ID NO: 1201, the second unit oligomer comprises or consists of a base sequence of SEQ ID NO: 205, the second antisense oligomer comprises or consists of a base sequence of SEQ ID NO: 4950, and the third antisense oligomer comprises or consists of a base sequence of SEQ ID NO: 3082,
the first unit oligomer comprises or consists of a base sequence of SEQ ID NO: 1239, the second unit oligomer comprises or consists of a base sequence of SEQ ID NO: 114, the second antisense oligomer comprises or consists of a base sequence of SEQ ID NO: 4950, and the third antisense oligomer comprises or consists of a base sequence of SEQ ID NO: 3082,
the first unit oligomer comprises or consists of a base sequence of SEQ ID NO: 1224, the second unit oligomer comprises or consists of a base sequence of SEQ ID NO: 124, the second antisense oligomer comprises or consists of a base sequence of SEQ ID NO: 4950, and the third antisense oligomer comprises or consists of a base sequence of SEQ ID NO: 3082,
the first unit oligomer comprises or consists of a base sequence of SEQ ID NO: 1180, the second unit oligomer comprises or consists of a base sequence of SEQ ID NO: 151, the second antisense oligomer comprises or consists of a base sequence of SEQ ID NO: 4950, and the third antisense oligomer comprises or consists of a base sequence of SEQ ID NO: 3082,
the first unit oligomer comprises or consists of a base sequence of SEQ ID NO: 1190, the second unit oligomer comprises or consists of a base sequence of SEQ ID NO: 151, the second antisense oligomer comprises or consists of a base sequence of SEQ ID NO: 4950, and the third antisense oligomer comprises or consists of a base sequence of SEQ ID NO: 3082,
the first unit oligomer comprises or consists of a base sequence of SEQ ID NO: 1212, the second unit oligomer comprises or consists of a base sequence of SEQ ID NO: 151, the second antisense oligomer comprises or consists of a base sequence of SEQ ID NO: 4950, and the third antisense oligomer comprises or consists of a base sequence of SEQ ID NO: 3082,
the first unit oligomer comprises or consists of a base sequence of SEQ ID NO: 1222, the second unit oligomer comprises or consists of a base sequence of SEQ ID NO: 151, the second antisense oligomer comprises or consists of a base sequence of SEQ ID NO: 4950, and the third antisense oligomer comprises or consists of a base sequence of SEQ ID NO: 3082,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4950, and the third antisense oligomer comprises or consists of a base sequence of SEQ ID NO: 3060,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4950, and the third antisense oligomer comprises or consists of a base sequence of SEQ ID NO: 3065,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4950, and the third antisense oligomer comprises or consists of a base sequence of SEQ ID NO: 3077,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4950, and the third antisense oligomer comprises or consists of a base sequence of SEQ ID NO: 3087,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4950, and the third antisense oligomer comprises or consists of a base sequence of SEQ ID NO: 3090,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4950, and the third antisense oligomer comprises or consists of a base sequence of SEQ ID NO: 3096,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4950, and the third antisense oligomer comprises or consists of a base sequence of SEQ ID NO: 3108,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4950, and the third antisense oligomer comprises or consists of a base sequence of SEQ ID NO: 3119,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4950, and the third antisense oligomer comprises or consists of a base sequence of SEQ ID NO: 3320,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4698, and the third antisense oligomer comprises or consists of a base sequence of SEQ ID NO: 3082,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4702, and the third antisense oligomer comprises or consists of a base sequence of SEQ ID NO: 3082,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4752, and the third antisense oligomer comprises or consists of a base sequence of SEQ ID NO: 3082,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4923, and the third antisense oligomer comprises or consists of a base sequence of SEQ ID NO: 3082,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4926, and the third antisense oligomer comprises or consists of a base sequence of SEQ ID NO: 3082,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4936, and the third antisense oligomer comprises or consists of a base sequence of SEQ ID NO: 3082,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4977, and the third antisense oligomer comprises or consists of a base sequence of SEQ ID NO: 3082,
the first unit oligomer comprises or consists of a base sequence of SEQ ID NO: 1201, the second unit oligomer comprises or consists of a base sequence of SEQ ID NO: 151, the second antisense oligomer comprises or consists of a base sequence of SEQ ID NO: 4977, and the third antisense oligomer comprises or consists of a base sequence of SEQ ID NO: 3096, or
the first unit oligomer comprises or consists of a base sequence of SEQ ID NO: 1180, the second unit oligomer comprises or consists of a base sequence of SEQ ID NO: 151, the second antisense oligomer comprises or consists of a base sequence of SEQ ID NO: 4977, and the third antisense oligomer comprises or consists of a base sequence of SEQ ID NO: 3096.

In one embodiment, the first antisense oligomer comprises the first unit oligomer and the second unit oligomer from the 5' ends in this order, the first unit oligomer comprises any one base sequence selected from SEQ ID NOs: 907 to 1602, the second unit oligomer comprises any one base sequence selected from the SEQ ID NOs: 106 to 210, the second antisense oligomer comprises any one base sequence selected from SEQ ID NOs: 4299 to 5090, and the third antisense oligomer comprises any one base sequence selected from SEQ ID NOs: 2555 to 3506. In one embodiment, the first antisense oligomer comprises the first unit oligomer and the second unit oligomer from the 5' ends in this order, the first unit oligomer comprises a base sequence of SEQ ID NO: 1201, the second unit oligomer comprises a base sequence of SEQ ID NO: 151, the second antisense oligomer comprises a base sequence of SEQ ID NO: 4950 or 4880 (preferably 4950), and the third antisense oligomer comprises a base sequence of SEQ ID NO: 3082, 3090, or 3096.

The antisense oligomer of the present invention (including the linked-type antisense oligomer of the present invention) may be an oligonucleotide, morpholino oligomer or peptide nucleic acid (PNA) oligomer (hereinafter, also referred to as the "antisense oligonucleotide of the present invention", the "antisense morpholino oligomer of the present invention", or the "antisense peptide nucleic acid oligomer of the present invention").

The antisense oligonucleotide of the present invention is an antisense oligomer composed of nucleotides as constituent units. Such nucleotides may be any of ribonucleotides, deoxyribonucleotides and modified nucleotides.

The modified nucleotide refers to one having fully or partly modified nucleobases, sugar moieties and/or phosphate-binding regions, which constitute the ribonucleotide or deoxyribonucleotide.

The nucleobase includes, for example, adenine, guanine, hypoxanthine, cytosine, thymine, uracil, and modified bases thereof. Examples of such modified bases include, but not limited to, pseudouracil, 3-methyluracil, dihydrouracil, 5-alkylcytosines *(e.g.,* 5-methylcytosine), 5-alkyluracils *(e.g.,* 5-ethyluracil), 5-halouracils *(e.g.,* 5-bromouracil), 6-azapyrimidine, 6-alkylpyrimidines *(e.g.,* 6-methyluracil), 2-thiouracil, 4-thiouracil, 4-acetylcytosine, 5-(carboxyhydroxymethyl) uracil, 5'-carboxymethylaminomethyl-2-thiouracil, 5-carboxymethylaminomethyluracil, 1-methyladenine, 1-methylhypoxanthine, 2,2-dimethylguanine, 3-methylcytosine, 2-methyladenine, 2-methylguanine, N6-methyladenine, 7-methylguanine, 5-methoxyaminomethyl-2-thiouracil, 5-methylaminomethyluracil, 5-methylcarbonylmethyluracil, 5-methyloxyuracil, 5-methyl-2-thiouracil, 2-methylthio-N6-isopentenyladenine, uracil-5-oxyacetic acid, 2-thiocytosine, purine, 2,6-diaminopurine, 2-aminopurine, isoguanine, indole, imidazole, xanthine, etc.

Modification of the sugar moiety may include, for example, modifications at the 2'-position of ribose and modifications of the other positions of the sugar. The modification at the 2'-position of ribose includes a modification of replacing the 2'-OH of ribose with -OR, - R, -R'OR, -SH, -SR, -NH₂, -NHR, -NR₂, -N₃, -CN, -F, -Cl, - Br or -I, wherein R represents an alkyl or an aryl and R' represents an alkylene.

The modification for the other positions of the sugar includes, for example, replacement of O at the 4' position of ribose or deoxyribose with S, bridging between 2' and 4' positions of the sugar, *e.g.,* LNA (locked nucleic acid) or ENA (2'-O,4'-C-ethylene-bridged nucleic acids), but is not limited thereto.

A modification of the phosphate-binding region includes, for example, a modification of replacing phosphodiester bond with phosphorothioate bond, phosphorodithioate bond, alkyl phosphonate bond, phosphoramidate bond or boranophosphate bond (cf., *e.g*., Enya et al: Bioorganic & Medicinal Chemistry, 2008, 18, 9154-9160) (cf., *e.g*., Japan Domestic Re-Publications of PCT Application Nos. 2006/129594 and 2006/038608).

As used herein, the alkyl is preferably a straight or branched alkyl having 1 to 6 carbon atoms. Specific examples include methyl, ethyl, *n*-propyl, isopropyl, *n-*butyl, isobutyl, sec-butyl, *tert*-butyl, *n*-pentyl, isopentyl, neopentyl, tert-pentyl, *n*-hexyl and isohexyl. The alkyl may optionally be substituted. Examples of such substituents are a halogen, an alkoxy, cyano and nitro. The alkyl may be substituted with 1 to 3 substituents.

As used herein, the cycloalkyl is preferably a cycloalkyl having 3 to 12 carbon atoms. Specific examples include cyclopropyl, cyclobutyl, cyclopentyl, cyclohexyl, cycloheptyl, cyclooctyl, cyclodecyl and cyclododecyl.

As used herein, the halogen includes fluorine, chlorine, bromine and iodine.

As used herein, the alkoxy is a straight or branched alkoxy having 1 to 6 carbon atoms such as methoxy, ethoxy, n-propoxy, isopropoxy, n-butoxy, isobutoxy, *sec-*butoxy, *tert*-butoxy, *n*-pentyloxy, isopentyloxy, *n-*hexyloxy, isohexyloxy, etc. Among others, an alkoxy having 1 to 3 carbon atoms is preferred.

As used herein, the aryl is preferably an aryl having 6 to 10 carbon atoms. Specific examples include phenyl, **α**-naphthyl and **β**-naphthyl. Among others, phenyl is preferred. The aryl may optionally be substituted. Examples of such substituents are an alkyl, a halogen, an alkoxy, cyano and nitro. The aryl may be substituted with one to three of such substituents.

As used herein, the alkylene is preferably a straight or branched alkylene having 1 to 6 carbon atoms. Specific examples include methylene, ethylene, trimethylene, tetramethylene, pentamethylene, hexamethylene, 2-(ethyl) trimethylene and 1-(methyl) tetramethylene.

As used herein, the acyl includes a straight or branched alkanoyl or aroyl. Examples of the alkanoyl include formyl, acetyl, 2-methylacetyl, 2,2-dimethylacetyl, propionyl, butyryl, isobutyryl, pentanoyl, 2,2-dimethylpropionyl, hexanoyl, etc. Examples of the aroyl include benzoyl, toluoyl and naphthoyl. The aroyl may optionally be substituted at substitutable positions and may be substituted with an alkyl(s).

Preferably, the antisense oligonucleotide of the present invention is the antisense oligomer of the present invention having a group represented by general formula below as a constituent unit wherein the -OH group at position 2' of ribose is substituted with methoxy and the phosphate-binding region is a phosphorothioate bond: 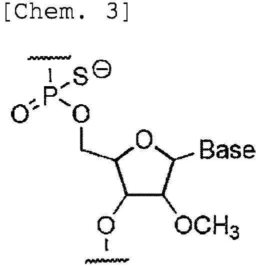 wherein Base represents a nucleobase.

The antisense oligonucleotide of the present invention may be easily synthesized using various automated synthesizer *(e.g.,* AKTA oligopilot plus 10/100 (GE Healthcare)). Alternatively, the synthesis may also be entrusted to a third-party organization *(e.g.,* Promega Corp. or Takara Co.), etc.

The antisense morpholino oligomer of the present invention is an antisense oligomer comprising the constituent unit represented by general formula below: 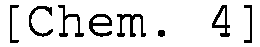 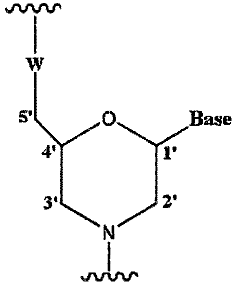
wherein Base has the same significance as defined above, and,
W represents a group shown by any one of the following groups: 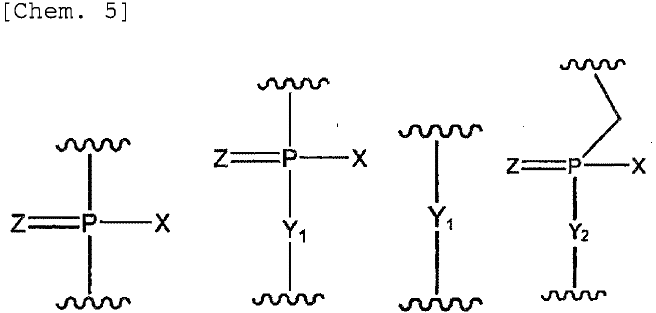
wherein X represents -CH₂R¹, -O-CH₂R¹, -S-CH₂R¹, -NR²R³, or F;
   R¹ represents H or an alkyl;
   R² and R³, which may be the same or different, each represents H, an alkyl, a cycloalkyl, or an aryl;
   Y₁ represents O, S, CH₂, or NR¹;
   Y₂ represents O, S, or NR¹;
   Z represents O or S.

Examples of morpholino monomer compounds that are used in synthesis of the antisense morpholino oligomer of the present invention include, but not limited to, the following morpholino monomer compound (A), morpholino monomer compound (C), morpholino monomer compound (T), and morpholino monomer compound (G) shown in Table 8.

### [Table 8]

**Table 8**

| **Morpholino monomer compound (A)** | **Morpholino monomer compound (C)** | **Morpholino monomer compound (T)** | **Morpholino monomer compound (G)** |
|---|---|---|---|
| 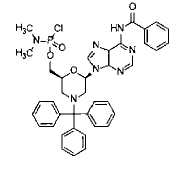 | 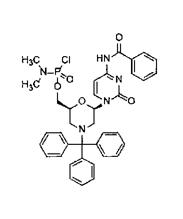 | 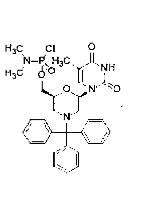 | 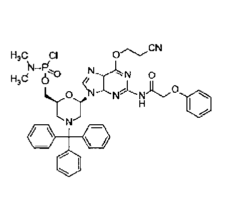 |

In the present invention, preferably, the morpholino oligomer is an oligomer having a group represented by general formula below as a constituent unit (phosphorodiamidate morpholino oligomer (hereinafter referred to as "PMO")). 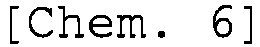 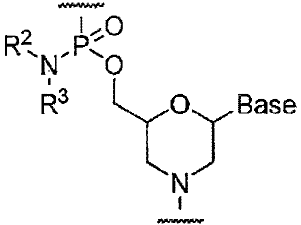 wherein Base, R² and R³ have the same significance as defined above.

The morpholino oligomer may be produced by the procedure described in, *e.g.,* WO 1991/009033 or WO 2009/064471. In particular, PMO can be produced by the procedure described in WO 2009/064471 or WO2013/100190.

The antisense peptide nucleic acid oligomer of the present invention is an antisense oligomer having a group represented by general formula below as a constituent unit: 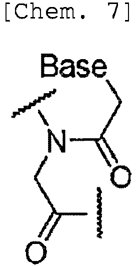 wherein Base has the same significance as defined above.

The peptide nucleic acid oligomer can be produced in accordance with, *e.g.,* the following literatures:
1)P. E. Nielsen, M. Egholm, R. H. Berg, O. Buchardt, Science, 254, 1497 (1991)2)M. Egholm, O. Buchardt, P. E. Nielsen, R. H. Berg, JACS, 114, 1895 (1992)3)K. L. Dueholm, M. Egholm, C. Behrens, L. Christensen, H. F. Hansen, T. Vulpius, K. H. Petersen, R. H. Berg, P. E. Nielsen, O. Buchardt, J. Org. Chem., 59, 5767 (1994) 4)L. Christensen, R. Fitzpatrick, B. Gildea, K. H. Petersen, H. F. Hansen, T. Koch, M. Egholm, O. Buchardt, P. E. Nielsen, J. Coull, R. H. Berg, J. Pept. Sci., 1, 175 (1995)5)T. Koch, H. F. Hansen, P. Andersen, T. Larsen, H. G. Batz, K. Otteson, H. Orum, J. Pept. Res., 49, 80 (1997)

The antisense oligomer of the present invention (including the linked-type antisense oligomer of the present invention) may be in the form of a pharmaceutically acceptable salt thereof, in the form of a hydrate thereof, or in the form of a hydrate of the pharmaceutically acceptable salt.

Examples of the pharmaceutically acceptable salt of the antisense oligomer of the present invention are alkali metal salts such as salts of sodium, potassium and lithium; alkaline earth metal salts such as salts of calcium and magnesium; metal salts such as salts of aluminum, iron, zinc, copper, nickel, cobalt, etc.; ammonium salts; organic amine salts such as salts of t-octylamine, dibenzylamine, morpholine, glucosamine, phenylglycine alkyl ester, ethylenediamine, *N-*methylglucamine, guanidine, diethylamine, triethylamine, dicyclohexylamine, *N,N'* -dibenzylethylenediamine, chloroprocaine, procaine, diethanolamine, *N-*benzylphenethylamine, piperazine, tetramethylammonium, tris(hydroxymethyl)aminomethane; hydrohalide salts such as salts of hydrofluorates, hydrochlorides, hydrobromides and hydroiodides; inorganic acid salts such as nitrates, perchlorates, sulfates, phosphates, etc.; lower alkane sulfonates such as methanesulfonates, trifluoromethanesulfonates and ethanesulfonates; arylsulfonates such as benzenesulfonates and p-toluenesulfonates; organic acid salts such as acetates, malates, fumarates, succinates, citrates, tartarates, oxalates, maleates, etc.; and, amino acid salts such as salts of glycine, lysine, arginine, ornithine, glutamic acid and aspartic acid. These salts may be produced by known methods. Alternatively, the antisense oligomer of the present invention may be in the form of a hydrate thereof.

The third antisense oligomer of the present invention may have a function as a suppressor antisense oligomer. In the present invention, a suppressor antisense oligomer means an antisense oligomer which suppresses single exon skipping (hereinafter, referred to as "single skipping"). The suppressor antisense oligomer can suppress single skipping and thereby enhance an effect of multi-exon skipping by an antisense oligomer. Accordingly, a combination of the present invention comprising the third antisense oligomer may have a higher effect of multi-exon skipping as compared with one not comprising the third antisense oligomer.

Specifically, the third antisense oligomer of the present invention can suppress single skipping of any one exon selected from the group consisting of the 45th exon to the 55th exon in human dystrophin pre-mRNA. More specifically, the third antisense oligomer of the present invention can suppress single skipping of the 45th exon in human dystrophin pre-mRNA.

The third antisense oligomer of the present invention can suppress single skipping by, for example, targeting the site of a splicing silencer sequence, a branch site sequence, or a splice site sequence in human dystrophin pre-mRNA and inhibiting splicing. The third antisense oligomer of the present invention reduces the efficiency of single skipping of an intended exon as compared with a control.

In one embodiment, the third antisense oligomer of the present invention targets a recognition sequence of heterogeneous nuclear ribonucleoprotein A1 (hnRNP A1) that is a splicing silencer sequence. A splicing silencer sequence refers to a base sequence element that functions to suppress recognition of an exon in pre-mRNA. A target sequence of the third antisense oligomer has been herein described.

Whether the suppressor antisense oligomer enhances a multi-exon skipping effect or not can be confirmed by providing (i) an experimental system for multi-exon skipping using only the antisense oligomer of the present invention alone and (ii) an experimental system for multi-exon skipping using the antisense oligomer and the suppressor antisense oligomer of the present invention such that the other conditions are the same therebetween, and observing the difference between a multi-exon skipping effect obtained in the experimental system (ii) and a multi-exon skipping effect obtained in the experimental system (i).

### [Method for producing PMO]

The antisense oligomer of the present invention may be PMO. An aspect of PMO is, for example, the compound represented by general formula (I) below (hereinafter, referred to as PMO (I)). 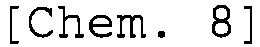 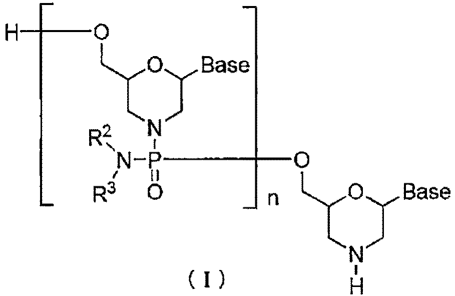 wherein Base, R² and R³ have the same significance as defined above; and,
n is a given integer of 1 to 99, preferably a given integer of 18 to 28.

PMO (I) can be produced in accordance with a known method (cf., e.g., WO2009/064471 or WO2013/100190).

In the antisense oligomer of the present invention, the 5' end may be a group represented by any of chemical structures (1) to (3) below, and preferably is (3)-OH.

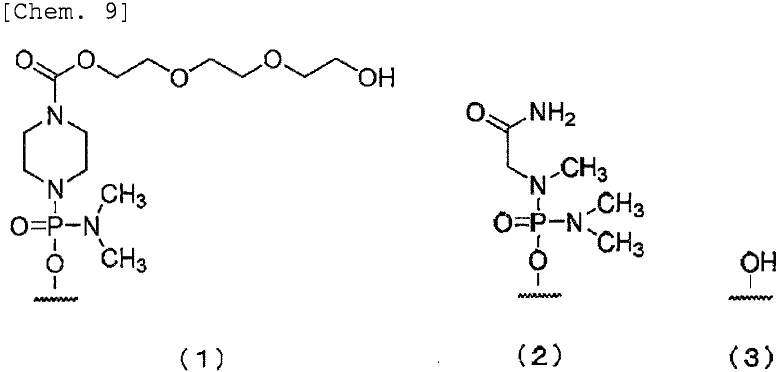

Hereinafter, the groups shown by (1), (2) and (3) above are referred to as "Group (1)," "Group (2)" and "Group (3)," respectively.

The antisense oligomer of the present invention may be in the form of a complex formed together with a functional peptide for purpose of improving effectiveness (for example, a cell-penetrating peptide for purpose of improving transport efficiency to a target cell) or an antibody fragment (for example, a Fab of an antibody to a muscle cell specific receptor such as a transferrin receptor) (International Publications WO2008/036127, WO2009/005793, WO2012/150960, WO2016/187425, WO2018/118662, WO2011/013700, WO2018/118599, and WO2018/118627, Japanese Patent Laid-Open No. 2022-47613, J. D. Ramsey, N. H. Flynn, Pharmacology & Therapeutics 154, 78-86 (2015), M. K. Tsoumpra et al., EBioMedicine, 45, 630-645 (2019), International Publications WO2020/028832, WO2021/142307, WO2021/142313, WO2022/020107, and WO2022/020108). A binding site is not especially limited, and it is preferable that the 5' end or the 3' end of the antisense oligomer is bonded to the amino terminal or carboxyl terminal of a functional peptide or an antibody fragment.

In another aspect, the antisense oligomer of the present invention and a functional peptide or an antibody fragment may form a complex via a linker. The linker is not especially limited, and it is preferable that the 5' end or the 3' end of the antisense oligomer is bonded to one end of the linker, and that the amino terminal or the carboxyl terminal of the functional peptide or the antibody fragment is bounded to the other end of the linker. An additional amino acid may be present between the functional peptide or the antibody fragment and the linker.

### Medical use

In one embodiment, the present invention provides a pharmaceutical composition comprising the first antisense oligomer and the second antisense oligomer of the present invention (also including a pharmaceutically acceptable salt thereof, or a hydrate thereof) (hereinafter, also referred to as the "pharmaceutical composition of the present invention"). The pharmaceutical composition of the present invention may further comprise the third antisense oligomer of the present invention (also including a pharmaceutically acceptable salt thereof, or a hydrate thereof) and/or a pharmaceutically acceptable carrier.

In one embodiment, the present invention provides a pharmaceutical combination of a pharmaceutical composition comprising the first antisense oligomer of the present invention and a pharmaceutical composition comprising the second antisense oligomer of the present invention (hereinafter, also referred to as the "pharmaceutical combination of the present invention"). The pharmaceutical combination of the present invention may further comprise the third antisense oligomer and/or a pharmaceutically acceptable carrier.

The pharmaceutical composition of the present invention comprises any combination of the antisense oligomers of the present invention. The pharmaceutical combination of the present invention also comprises any combination of the antisense oligomers of the present invention. Details of the combinations of the antisense oligomers are as described herein.

In one embodiment, the antisense oligomers in the combination of the present invention are comprised in one pharmaceutical composition to be simultaneously administered. In another embodiment, the antisense oligomers in the combination of the present invention are comprised in a plurality of pharmaceutical compositions (pharmaceutical combination of the present invention) to be separately (simultaneously or sequentially) administered. As used herein, the term "simultaneously" administering a plurality of pharmaceutical compositions means that a plurality of pharmaceutical compositions are administered at the same time. As used herein, the term "sequentially" administering a plurality of pharmaceutical compositions means that these are administered at different times. Specifically, one pharmaceutical composition may be administered before or after another pharmaceutical composition, and an administration interval in this case is not limited, but may be, for example, a few minutes, a few hours, or a few days.

The pharmaceutical composition of the present invention and the pharmaceutical combination of the present invention can each be used for the treatment of, for example, Duchenne muscular dystrophy, Becker muscular dystrophy, limb-girdle muscular dystrophy (LGMD), congenital muscular dystrophy, Emery-Dreifuss muscular dystrophy, facioscapulohumeral muscular dystrophy, oculopharyngeal muscular dystrophy, cerebral autosomal dominant arteriopathy with subcortical infarct and leukoencephalopathy (CADASIL), and Alport's syndrome. The pharmaceutical combination of the present invention and the pharmaceutical composition of the present invention can each be administered to a human patient and in particular, a human patient with muscular dystrophy. The patient to receive the pharmaceutical combination of the present invention or the pharmaceutical composition of the present invention may be a human patient having a mutation that is the target of skipping of two or more exons selected from the group consisting of exons 45 to 55 in the dystrophin gene. Herein, the mutation that is the target of exon skipping is not limited, and an example includes a patient having deletion of exon (for example, having deletion of exon 46, exon 46 to 47, exon 46 to 48, exon 46 to 50, exon 46 to 51, exon 46 to 52, exon 46 to 53, exon 46 to 55, exon 47 to 50, exon 47 to 52, exon 48 to 50, exon 48 to 52, exon 48 to 54, exon 49 to 50, exon 49 to 52, exon 49 to 54, exon 50, exon 50 to 52, exon 51, exon 51 to 53, exon 52, exon 53, or exon 53 to 54) in the dystrophin gene.

One aspect of the present invention provides a method for treatment of muscular dystrophy, which comprises administering to a patient with muscular dystrophy a combination of the antisense oligomer of the present invention. Another aspect of the present invention provides a method for treatment of muscular dystrophy, which comprises administering to a patient with muscular dystrophy the pharmaceutical composition of the present invention or the pharmaceutical combination of the present invention.

The method for treatment may involve performing skipping of any two or more numerically consecutive exons selected from the group consisting of the 45th exon to the 55th exon in human dystrophin pre-mRNA. In the method for treatment, the patient with muscular dystrophy may be a patient having a mutation that is the target of exon 45 to 55 skipping in the dystrophin gene. The patient may be a human and may be a human patient having a mutation that is the target of exon 45 to 55 skipping in the dystrophin gene.

The present invention further provides use of a combination of the antisense oligomer of the present invention, or the pharmaceutical composition of the present invention or the pharmaceutical combination of the present invention in manufacturing of a medicament for the treatment of muscular dystrophy.

The present invention further provides a combination of the antisense oligomer of the present invention, or the pharmaceutical composition of the present invention or the pharmaceutical combination of the present invention for use in the treatment of muscular dystrophy. The treatment may involve performing skipping of any two or more numerically consecutive exons selected from the group consisting of the 45th exon to the 55th exon in human dystrophin pre-mRNA. In the treatment, the patient with muscular dystrophy may be a patient having a mutation that is the target of exon 45 to 55 skipping in the dystrophin gene. The patient may be a human and may be a human patient having a mutation that is the target of exon 45 to 55 skipping in the dystrophin gene.

Administration route for the combination of the antisense oligomer of the present invention, or the pharmaceutical composition of the present invention or the pharmaceutical combination of the present invention is not particularly limited so long as it is pharmaceutically acceptable route for administration, and can be chosen depending upon method of treatment. In view of easiness in delivery to muscle tissues, preferred are intravenous administration, intraarterial administration, intramuscular administration, subcutaneous administration, oral administration, tissue administration, transdermal administration, etc. Also, dosage forms which are available for the composition of the present invention are not particularly limited, and include, for example, various injections, oral agents, drips, inhalations, ointments, lotions, etc.

In administration of the antisense oligomer of the present invention to patients with muscular dystrophy, preferably, the composition of the present invention contains a carrier to promote delivery of the oligomer to muscle tissues. Such a carrier is not particularly limited as far as it is pharmaceutically acceptable, and examples include cationic carriers such as cationic liposomes, cationic polymers, etc., or carriers using viral envelope. The cationic liposomes are, for example, liposomes composed of 2-O-(2-diethylaminoethyl)carabamoyl-1,3-O-dioleoylglycerol and phospholipids as the essential constituents (hereinafter referred to as "liposome A"), Oligofectamine (registered trademark) (manufactured by Invitrogen Corp.), Lipofectin (registered trademark) (manufactured by Invitrogen Corp.), Lipofectamine (registered trademark) (manufactured by Invitrogen Corp.), Lipofectamine 2000 (registered trademark) (manufactured by Invitrogen Corp.), DMRIE-C (registered trademark) (manufactured by Invitrogen Corp.), GeneSilencer (registered trademark) (manufactured by Gene Therapy Systems), TransMessenger (registered trademark) (manufactured by QIAGEN, Inc.), TransIT TKO (registered trademark) (manufactured by Mirus) and Nucleofector II (Lonza). Among others, liposome A is preferred. Examples of cationic polymers are JetSI (registered trademark) (manufactured by Qbiogene, Inc.) and Jet-PEI (registered trademark) (polyethylenimine, manufactured by Qbiogene, Inc.). An example of carriers using viral envelop is GenomeOne (registered trademark) (HVJ-E liposome, manufactured by Ishihara Sangyo). Alternatively, the medical devices described in Japanese Patent Nos. 2924179 and the cationic carriers described in Japanese Domestic RePublication PCT Nos. 2006/129594 and 2008/096690 may be used as well.

A concentration of the antisense oligomer of the present invention contained in the pharmaceutical composition of the present invention and/or the pharmaceutical combination of the present invention may vary depending on kind of the carrier, etc., and is appropriately in a range of 0.1 nM to 100 µM, preferably in a range of 1 nM to 10 µM, and more preferably in a range of 10 nM to 1 µM. A weight ratio of the antisense oligomer of the present invention contained in the composition of the present invention and the carrier (carrier/antisense oligomer of the present invention) may vary depending on property of the oligomer, type of the carrier, etc., and is appropriately in a range of 0.1 to 100, preferably in a range of 1 to 50, and more preferably in a range of 10 to 20.

In one embodiment, the antisense oligomers in the combination of the present invention are comprised in one pharmaceutical composition to be simultaneously administered. In another embodiment, the antisense oligomers in the combination of the present invention are comprised in a plurality of pharmaceutical compositions (pharmaceutical combination of the present invention) to be separately (simultaneously or sequentially) administered. When the antisense oligomers in the combination of the present invention are comprised in one or a plurality of pharmaceutical compositions, concentrations of the antisense oligomers are as follows.

The pharmaceutical composition of the present invention and/or the pharmaceutical combination of the present invention may be in the form of an aqueous solution. In this case, the pharmaceutical composition of the present invention and/or the pharmaceutical combination of the present invention may comprise the antisense oligomer of the present invention in a concentration of 2.5 to 500 mg/mL, 5 to 450 mg/mL, 10 to 400 mg/mL, 15 to 350 mg/mL, 20 to 300 mg/mL, 20 to 250 mg/mL, 20 to 200 mg/mL, 20 to 150 mg/mL, 20 to 100 mg/mL, 20 to 50 mg/mL, 20 to 40 mg/mL, 20 to 30 mg/mL, 23 to 27 mg/mL, 24 to 26 mg/mL, or 25 mg/mL. The pharmaceutical composition of the present invention and/or the pharmaceutical combination of the present invention may comprise the antisense oligomer of the present invention in a concentration of 10 to 100 mg/mL, 15 to 95 mg/mL, 20 to 80 mg/mL, 25 to 75 mg/mL, 30 to 70 mg/mL, 35 to 65 mg/mL, 40 to 60 mg/mL, 45 to 55 mg/mL, 47 to 53 mg/mL, 48 to 52 mg/mL, 49 to 51 mg/mL, or 50 mg/mL.

The pharmaceutical composition of the present invention and/or the pharmaceutical combination of the present invention may be in a dry form. In this case, in order to prepare the pharmaceutical composition of the present invention and/or the pharmaceutical combination of the present invention in an aqueous solution form, for example, 125 mg or 250 mg of the antisense oligomer of the present invention in a dry form may be mixed with 0.5 mL to 100 mL of water (which corresponds to a concentration of 1.25 mg/mL to 250 mg/mL or 2.5 mg/mL to 500 mg/mL of the antisense oligomer of the present invention), preferably with 1 mL to 50 mL of water (which corresponds to a concentration of 2.5 mg/mL to 125 mg/mL or 5 mg/mL to 250 mg/mL of the antisense oligomer of the present invention), more preferably with 5 mL to 10 mL of water (which corresponds to a concentration of 12.5 mg/mL to 25 mg/mL or 25 mg/mL to 50 mg/mL of the antisense oligomer of the present invention) for use.

When the antisense oligomers in the combination of the present invention are comprised in one or a plurality of pharmaceutical compositions, a total concentration of the antisense oligomers is as follows.

When the pharmaceutical composition of the present invention and/or the pharmaceutical combination of the present invention is in an aqueous solution form, the pharmaceutical composition of the present invention and/or the pharmaceutical combination of the present invention may comprise the antisense oligomers of the present invention in a total concentration of 2.5 to 500 mg/mL, 5 to 450 mg/mL, 10 to 400 mg/mL, 15 to 350 mg/mL, 20 to 300 mg/mL, 20 to 250 mg/mL, 20 to 200 mg/mL, 20 to 150 mg/mL, 20 to 100 mg/mL, 20 to 50 mg/mL, 20 to 40 mg/mL, 20 to 30 mg/mL, 23 to 27 mg/mL, 24 to 26 mg/mL, or 25 mg/mL, or 5 to 1000 mg/mL, 10 to 900 mg/mL, 20 to 800 mg/mL, 30 to 700 mg/mL, 40 to 600 mg/mL, 40 to 500 mg/mL, 40 to 400 mg/mL, 40 to 300 mg/mL, 40 to 200 mg/mL, 40 to 100 mg/mL, 40 to 80 mg/mL, 40 to 60 mg/mL, 46 to 54 mg/mL, 48 to 52 mg/mL, or 50 mg/mL, or 7.5 to 1500 mg/mL, 15 to 1350 mg/mL, 30 to 1200 mg/mL, 45 to 1150 mg/mL, 60 to 900 mg/mL, 60 to 750 mg/mL, 60 to 600 mg/mL, 60 to 450 mg/mL, 60 to 300 mg/mL, 60 to 150 mg/mL, 60 to 120 mg/mL, 60 to 90 mg/mL, 69 to 81 mg/mL, 72 to 78 mg/mL, or 75 mg/mL. Alternatively, the pharmaceutical composition of the present invention and/or the pharmaceutical combination of the present invention may comprise the antisense oligomers of the present invention in a total concentration of 10 to 100 mg/mL, 15 to 95 mg/mL, 20 to 80 mg/mL, 25 to 75 mg/mL, 30 to 70 mg/mL, 35 to 65 mg/mL, 40 to 60 mg/mL, 45 to 55 mg/mL, 47 to 53 mg/mL, 48 to 52 mg/mL, 49 to 51 mg/mL, or 50 mg/mL, or 20 to 200 mg/mL, 30 to 190 mg/mL, 40 to 160 mg/mL, 50 to 150 mg/mL, 60 to 140 mg/mL, 70 to 130 mg/mL, 80 to 120 mg/mL, 90 to 110 mg/mL, 94 to 106 mg/mL, 96 to 104 mg/mL, 98 to 102 mg/mL, or 100 mg/mL, or 30 to 300 mg/mL, 45 to 285 mg/mL, 60 to 240 mg/mL, 75 to 225 mg/mL, 90 to 210 mg/mL, 105 to 195 mg/mL, 120 to 180 mg/mL, 130 to 165 mg/mL, 141 to 159 mg/mL, 144 to 156 mg/mL, 147 to 153 mg/mL, or 150 mg/mL.

When the pharmaceutical composition of the present invention and/or the pharmaceutical combination of the present invention is in a dry form, in order to prepare the pharmaceutical composition of the present invention and/or the pharmaceutical combination of the present invention in an aqueous solution form, for example, 125 mg or 250 mg of the antisense oligomer of the present invention in a dry form may be mixed with 0.5 mL to 100 mL of water (which corresponds to a total concentration of 1.25 mg/mL to 250 mg/mL or 2.5 mg/mL to 500 mg/mL of the antisense oligomers of the present invention), preferably with 1 mL to 50 mL of water (which corresponds to a total concentration of 2.5 mg/mL to 125 mg/mL or 5 mg/mL to 250 mg/mL of the antisense oligomers of the present invention), more preferably with 5 mL to 10 mL of water (which correspond to a total concentration of 12.5 mg/mL to 25 mg/mL or 25 mg/mL to 50 mg/mL of the antisense oligomers of the present invention), or for example, 250 mg or 500 mg in total of the antisense oligomers of the present invention in a dry form may be mixed with 0.5 mL to 100 mL of water (which corresponds to a total concentration of 2.5 mg/mL to 500 mg/mL or 5 mg/mL to 1000 mg/mL of the antisense oligomers of the present invention), preferably with 1 mL to 50 mL of water (which corresponds to a total concentration of 5 mg/mL to 250 mg/mL or 10 mg/mL to 500 mg/mL of the antisense oligomers of the present invention), more preferably with 5 mL to 10 mL of water (which correspond to a total concentration of 25 mg/mL to 50 mg/mL or 50 mg/mL to 100 mg/mL of the antisense oligomers of the present invention), or for example, 375 mg or 750 mg in total of the antisense oligomers of the present invention in a dry form may be mixed with 0.5 mL to 100 mL of water (which corresponds to a total concentration of 3.75 mg/mL to 750 mg/mL or 7.5 mg/mL to 150 mg/mL of the antisense oligomers of the present invention), preferably with 1 mL to 50 mL of water (which corresponds to a total concentration of 7.5 mg/mL to 375 mg/mL or 15 mg/mL to 750 mg/mL of the antisense oligomers of the present invention), more preferably with 5 mL to 10 mL of water (which corresponds to a total concentration of 37.5 mg/mL to 75 mg/mL or 75 mg/mL to 150 mg/mL of the antisense oligomers of the present invention) for use.

In addition to the antisense oligomer of the present invention and the carrier described above, pharmaceutically acceptable additives may also be optionally formulated in the pharmaceutical composition of the present invention and/or the pharmaceutical combination of the present invention. Examples of such additives are emulsification aids (e.g., fatty acids having 6 to 22 carbon atoms and their pharmaceutically acceptable salts, albumin and dextran), stabilizers *(e.g.,* cholesterol, phosphatidic acid, mannitol, and sorbitol), isotonizing agents *(e.g.,* sodium chloride, glucose, maltose, lactose, sucrose, and trehalose), and pH controlling agents *(e.g.,* hydrochloric acid, sulfuric acid, phosphoric acid, acetic acid, sodium hydroxide, potassium hydroxide and triethanolamine). One or more of these additives can be used. The content of the additive in the composition of the present invention is appropriately 90 wt% or less, preferably 70 wt% or less and more preferably, 50 wt% or less.

The pharmaceutical composition of the present invention and/or the pharmaceutical combination of the present invention can be prepared by adding the antisense oligomer of the present invention to a carrier dispersion and adequately stirring the mixture. Additives may be added at an appropriate step either before or after addition of the antisense oligomer of the present invention. An aqueous solvent that can be used in adding the antisense oligomer of the present invention is not particularly limited as far as it is pharmaceutically acceptable, and examples are injectable water or injectable distilled water, electrolyte fluid such as physiological saline, etc., and sugar fluid such as glucose fluid, maltose fluid, etc. A person skilled in the art can appropriately choose conditions for pH and temperature for such matter.

The pharmaceutical composition of the present invention and/or the pharmaceutical combination of the present invention may be prepared into, *e.g.,* a liquid form and its lyophilized preparation. The lyophilized preparation can be prepared by lyophilizing the composition of the present invention in a liquid form in a conventional manner. The lyophilization can be performed, for example, by appropriately sterilizing the composition of the present invention in a liquid form, dispensing an aliquot into a vial container, performing preliminary freezing for 2 hours at conditions in a range of about -40°C to -20°C, performing a primary drying in a range of about 0°C to 10°C under reduced pressure, and then performing a secondary drying in a range of about 15°C to 25°C under reduced pressure. In general, the lyophilized preparation of the composition of the present invention can be obtained by replacing the content of the vial with nitrogen gas and capping.

The lyophilized preparation of the pharmaceutical composition of the present invention and/or the pharmaceutical combination of the present invention can be used in general upon reconstitution by adding an optional suitable solution (reconstitution liquid) and redissolving the preparation. Such a reconstitution liquid includes injectable water, physiological saline and other infusion fluids. A volume of the reconstitution liquid may vary depending on the intended use, etc., is not particularly limited, and is suitably 0.5-fold to 2-fold greater than the volume prior to lyophilization or no more than 500 mL.

It is desired to control a dose of the pharmaceutical composition of the present invention and/or the pharmaceutical combination of the present invention to be administered, by taking the following factors into account: the type and dosage form of the antisense oligomer of the present invention contained; patients' conditions including age, body weight, etc.; administration route; and the characteristics and extent of the disease. A single dose calculated as the amount of the antisense oligomer of the present invention can be 0.1 mg to 1 g per kg body weight, preferably 1 mg to 100 mg per kg body weight, more preferably 1 mg to 90 mg per kg body weight, and further preferably 1 mg to 80 mg per kg body weight. The frequency of administration may be once per 1 to 3 days, once per week, or once per 2 to 3 weeks. This numerical range may vary occasionally depending on type of the target disease, administration route and target molecule. Therefore, a dose or frequency of administration lower than the range may be sufficient in some occasion and conversely, a dose or frequency of administration higher than the range may be required occasionally.

In still another aspect of the pharmaceutical composition of the present invention and/or the pharmaceutical combination of the present invention, there is provided a pharmaceutical composition comprising a vector capable of expressing the antisense oligomer of the present invention and the carrier described above. Such an expression vector may be a vector capable of expressing a plurality of the antisense oligomers of the present invention of the present invention. The composition may be formulated with pharmaceutically acceptable additives as in the case with the composition of the present invention containing the antisense oligomer of the present invention. A concentration of the expression vector contained in the composition may vary depending upon type of the career, etc., and is appropriately in a range of 0.1 nM to 100 µM, preferably in a range of 1 nM to 10 µM, and more preferably in a range of 10 nM to 1 µM. A weight ratio of the expression vector contained in the composition and the carrier (carrier/expression vector) may vary depending on property of the expression vector, type of the carrier, etc., and is appropriately in a range of 0.1 to 100, preferably in a range of 1 to 50, and more preferably in a range of 10 to 20. The content of the carrier contained in the composition is the same as in the case with the composition of the present invention containing the antisense oligomer of the present invention, and a method for producing the same is also the same as in the case with the composition of the present invention.

### Examples

Hereinafter, the present invention will be described in more detail with reference to Examples and Test Examples below, but is not limited thereto.

### [Example 1: Production of antisense oligomers]

In accordance with the method described in Example 1 of International Publication WO2013/100190, antisense oligomers shown in Table 9 (PMO Nos. 1 to 5 (SEQ ID NOs: 5098 to 5102)) were synthesized. Theoretical values and actual values measured by ESI-TOF-MS of the molecular weights of the antisense oligomers are also shown. The 5' end of each PMO is Group (1) below. The synthesized PMO was dissolved in water for injection (manufactured by Otsuka Pharmaceutical Factory, Inc.).

### [Table 9]

**Table 9**

| PMO No. | **SEQ ID NO:** | **Target base sequence** | **Base sequence of PMO** | **Molecular weight** | |
|---|---|---|---|---|---|
| | | | | **Theoretical value** | **Actual value** |
| 1 | 5098 | M45_(-66)-(-61)_19-40 | TGACAACAGCTTGACGCTGCCCGTT TAA | 9247. 21 | 9247. 66 |
| 2 | 5099 | M55_(-4) -24 | AAAGCAGCCTCTTGCTCACTTACTC TGC | 9158. 18 | 9158. 3 |
| 3 | 5100 | M45_183-206 | AGCTCTGCTAAAAAGTCTCTGTCA | 7906. 75 | 7906. 12 |
| 4 | 5101 | M45_197-220 | TTAAAGGATAGTGTAGCTCTGCTA | 8001. 77 | 8001. 77 |
| 5 | 5102 | M45_191-214 | GATAGTGTAGCTCTGCTAAAAAGT | 8010. 78 | 8010. 48 |

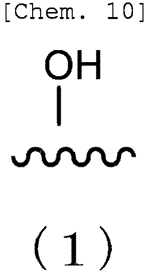

The target base sequence of the antisense oligomer of the present invention was described as "Ma_{1_}b₁-c₁", "Ma_{2_}b₂-c_{2_}Ma_{3_}b₃-c₃".

"Ma₁" represents the ath exon of the mouse dystrophin gene, "b₁" represents the 5'-terminal base of the target base sequence, and "c₁" represents the 3'-terminal base of the target base sequence.

When "b₁" and "c₁" are positive integers, "b₁" and "c₁" each represent a base number in the downstream direction when the 5'-terminal base of the ath exon is counted as the 1st base. On the other hand, when "b₁" and "c₁" are negative numbers, "b₁" and "c₁" each represent a base number in the upstream direction when the 3'-terminal base of the (a - 1)th intron is counted as the 1st base.

For example, "M55_(-4)-24" means a base sequence in which the 5' end of the target base sequence is the 4th base in the upstream direction from the 3' end of the 54th intron and the 3' end of the target base sequence is the 24th base in the downstream direction from the 5' end of the 55th exon.

"Ma_{2_}b₂-c₂" which is the first part of "Ma₂_b₂-c_{2_}Ma_{3_}b₃-c₃" means the target base sequence of a 3' unit oligomer constituting the antisense oligomer, and the second part "Ma_{3_}b₃-c₃" means the target base sequence of a 5' unit oligomer constituting the antisense oligomer.

When "Ma₂" and "Ma₃" are the same, the "_{_}Ma₃" part may be omitted.

For example, "M45_(-66)-(-61)_19-40" or "M45_(-66)-(-61)_M45_19-40" means a base sequence in which the target base sequence of the 3' unit oligomer constituting the antisense oligomer is "M45_(-66)-(-61)" and the target base sequence of the 5' unit oligomer constituting the antisense oligomer is "M45_19-40".

### [Example 2: Test on multi-exon skipping activity of antisense oligomer]

### <Test Example 1>

### Assay of exon 45 to 55 multi-exon skipping in model mouse-derived cultured cells - (1): induction of multi-exon skipping (total addition concentration: 30 µM) Procedures

H2K-mdx52 cells (immortalized myoblasts established from a crossbred individual of a mdx52 mouse, that is, Duchenne muscular dystrophy model, and a H-2kb-tsA58 transgenic mouse) were seeded in a 0.4% Gelatine-coated 48-well plate (manufactured by AGC Techno Glass Co., Ltd.) at 1 x 10⁴/well, and were cultured for 3 days under conditions of 37°C and 5% CO₂ in 0.5 mL of a growth medium (High glucose Dulbecco's Modified Eagle Medium (DMEM) (containing GlutaMax) (manufactured by Thermo Fisher Scientific) supplemented with 20% FBS (manufactured by Sigma Aldrich), 2% chick embryo extract (manufactured by US Biological, hereinafter the same), 2% L-glutamine (manufactured by Sigma Aldrich, hereinafter the same), 1% penicillin/streptomycin (manufactured by Sigma Aldrich, hereinafter the same), and 20 U/mL Recombinant Murine IFN-γ (manufactured by PeproTech)). After 48 hours, the growth medium was changed to a differentiation medium (DMEM supplemented with 5% horse serum (manufactured by Thermo Fisher Scientific), 2% L-glutamine, and 1% penicillin/streptomycin). After culturing for 3 days, transfection was performed with 30 µM PMO using 6 µM Endo-Porter (manufactured by Gene Tools, hereinafter the same). PMOs used here are shown in Table 10.

### [Table 10]

**Table 10**

| PMO No. | **Base sequence of PMO (5' to 3')** | **SEQ ID NO:** |
|---|---|---|
| 1 | TGACAACAGCTTGACGCTGCCCGTTTAA | 5098 |
| 2 | AAAGCAGCCTCTTGCTCACTTACTCTGC | 5099 |
| 3 | AGCTCTGCTAAAAAGTCTCTGTCA | 5100 |

After culturing for another 3 days, the resultant cells were washed once with PBS (manufactured by Takara Bio Inc.), and then, the total RNA was extracted with RNeasy Mini Kit (manufactured by Qiagen K. K.) . 350 µL of Buffer RLT (manufactured by Qiagen K. K.) containing 1% 2-mercaptoethanol (manufactured by Nacalai Tesque, Inc.) was added to the cells, and after the cells were allowed to stand at room temperature for a few minutes to lyse the cells, the lysate was collected into a QIAshredder homogenizer (manufactured by Qiagen K. K.). A homogenate was produced by centrifugation at 15,000 rpm for 2 minutes. The total RNA was extracted according to the protocol attached to RNeasy Mini Kit (manufactured by Qiagen K. K.). The concentration of the total RNA extracted was determined using a NanoDrop One C (manufactured by Thermo Fisher Scientific). One-Step RT-PCR was performed with 400 ng of the extracted total RNA using a QIAGEN One Step RT-PCR Kit (manufactured by Qiagen K. K.). A reaction solution was prepared in accordance with the protocol attached to the kit. Veriti 96 Well Thermal Cycler (manufactured by Thermo Fisher Scientific) was used as the thermal cycler. The RT-PCR program used was as follows.

50°C, 30 mins: reverse transcription reaction
95°C, 15 mins: polymerization activation, reverse transcriptase inactivation, cDNA thermal denaturation
[94°C, 10 seconds; 57°C, 30 seconds; 72°C, 1 minute] x 33 cycles: PCR amplification
72°C, 10 mins: final extension

The base sequences of the forward primer and reverse primer used for RT-PCR are given below.
Forward primer: 5'-cagttgaaaaatggcgacac-3' (SEQ ID NO: 5103)
Reverse primer 1: 5'-ttagctgctgctcatctcca-3' (SEQ ID NO: 5104)
Reverse primer 2: 5'-ttccagggatctcaggattt-3' (SEQ ID NO: 5105)

Transcripts (429 bp) having no skipping and transcripts (253 bp) having single exon skipping of exon 45 can be detected by a combination of the forward primer and the reverse primer 1, and transcripts (218 bp) having multi-exon skipping of exons 45 to 55 can be detected by a combination of the forward primer and the reverse primer 2.

The reaction product of the PCR above was analyzed using MultiNA (manufactured by Shimadzu Corp.).

The polynucleotide level "A" of the band with skipping of exons 45 to 55, the polynucleotide level "B" of the band with skipping of exon 45, and the polynucleotide level "C" of the band having no skipping were measured. Based on these measurement values of "A", "B", and "C", the skipping efficiencies of exon 45 to 55 skipping and exon 45 skipping were determined by the following equations: Skipping efficiency of exon 45 to 55 skipping (%) = A / (A + B + C) × 100 Skipping efficiency of exon 45 skipping (%) = B / (A + B + C) × 100

### Results

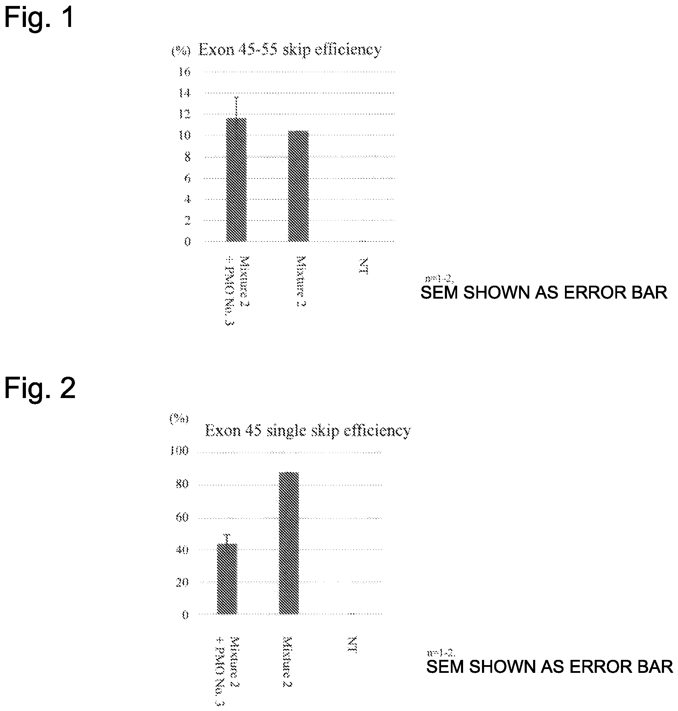

The results are shown in Figures 1 to 2.

As compared with the mixture of PMO No. 1 and PMO No. 2 (15 µM each, Mixture 2) used singly, the mixture additionally containing PMO No. 3 targeting hnRNP A1 (10 µM each, Mixture 2 + PMO No. 3) increased the skipping efficiency of exon 45 to 55 skipping (Figure 1), and reduced the skipping efficiency of exon 45 skipping (Figure 2).

### <Test Example 2>

### Assay of exon 45 to 55 multi-exon skipping in model mouse-derived cultured cells - (2): induction of multi-exon skipping (total addition concentration: 15 µM, mixing ratio changed)

### Procedures

H2K-mdx52 cells were seeded in a 0.4% Gelatine-coated 24-well plate at 5 x 10⁴/well and cultured for 48 hours under conditions of 37°C and 5% CO₂ in 1 mL of a growth medium, and then the growth medium was changed to a differentiation medium. After culturing for 3 days, transfection was performed with 15 µM PMO using 6 µM Endo-Porter.

A PMO shown in Table 11 was used in addition to those used in Test Example 1.

### [Table 11]

**Table 11**

| PMO No. | **Base sequence of PM0 (5' to 3')** | **SEQ ID NO:** |
|---|---|---|
| 4 | TTAAAGGATAGTGTAGCTCTGCTA | 5101 |

After culturing for another 3 days, the resultant cells were collected in the same manner as in Test Example 1, the total RNA was extracted, and subjected to One-Step RT-PCR, and the reaction product of the PCR thus obtained was analyzed to obtain the skipping efficiencies of exon 45 to 55 skipping and exon 45 skipping.

### (Results)

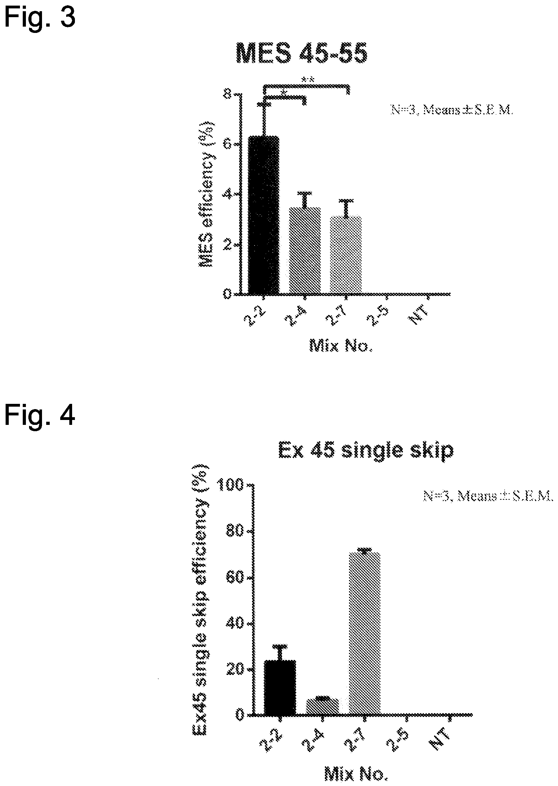

The results are shown in Figures 3 to 8.

As compared with the mixture of PMO No. 1 and PMO No. 2 (Mixture 2) used singly, the mixture additionally containing PMO No. 3 targeting hnRNP A1 (Mixture 2 + hnRNP A1) increased the skipping efficiency of exon 45 to 55 skipping (Figure 3), and reduced the skipping efficiency of exon 45 skipping (Figure 4).

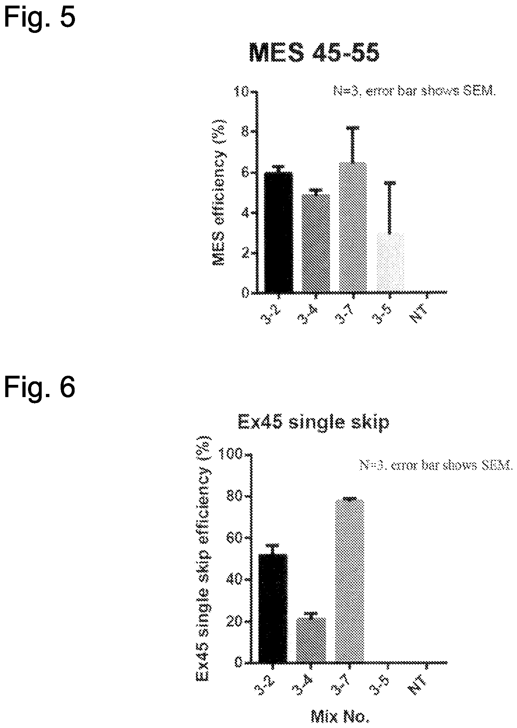

The mixture of Mixture 2 and PMO No. 4 targeting hnRNP A1 (Mixture 2 + PMO No. 4) caused exon 45 to 55 skipping (Figure 5), and reduced the skipping efficiency of exon 45 skipping (Figure 6).

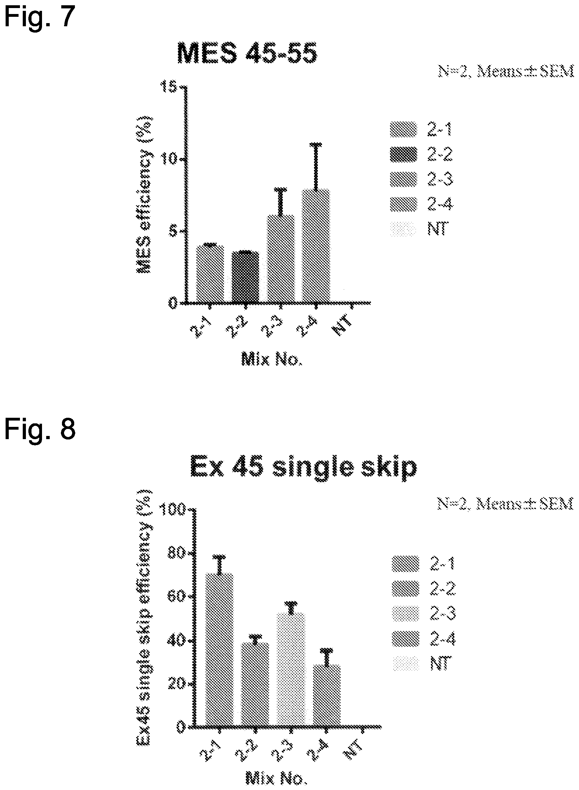

As a result of studying the case where the ratio between Mixture 2 and PMO No. 3 targeting hnRNP A1 was changed, when these were formulated at 3:1, the skipping efficiency of exon 45 to 55 skipping was the highest (Figure 7), and the skipping efficiency of exon 45 skipping was most largely suppressed (Figure 8).

### <Test Example 3>

### Assay of exon 45 to 55 multi-exon skipping in model mouse-derived cultured cells - (3): induction of multi-exon skipping (total addition concentration: 50 µM)

### Procedures

H2K-mdx52 cells were seeded in a 0.4% Gelatine-coated 24-well plate at 6.7 x 10⁴/well and cultured for 1 day under conditions of 37°C and 5% CO₂ in 2 mL of a growth medium. After culturing for 2 days, the growth medium was changed to a differentiation medium. After culturing for 3 days, transfection was performed with 50 µM PMO using 6 µM Endo-Porter. After culturing for another 3 days, the resultant cells were collected in the same manner as in Test Example 1, the total RNA was extracted, and subjected to One-Step RT-PCR, and the reaction product of the PCR thus obtained was analyzed to obtain skipping efficiencies of exon 45 to 55 skipping and exon 45 skipping.

### (Results)

The results are shown in Figures 9 and 10.

As compared with the mixture of PMO No. 1 and PMO No. 2 (Mix 2) used singly, the mixture additionally containing PMO No. 3 targeting hnRNP A1 (Mix 2 + hnRNP A1) increased the skipping efficiency of exon 45 to 55 skipping (Figure 9), and reduced the skipping efficiency of exon 45 skipping (Figure 10). Mix 2 had the same definition as Mixture 2 used in Test Example 1 and Test Example 2.

### <Test Example 4>

### Assay of exon 45 to 55 multi-exon skipping in model mouse-derived cultured cells - (4): restoration of dystrophin protein by multi-exon skipping

### Procedures

H2K-mdx52 cells were seeded in a 0.4% Gelatine-coated 24-well plate at 6.7 x 10⁴/well and cultured for 1 day under conditions of 37°C and 5% CO₂ in 2 mL of a growth medium. After culturing for 2 days, the growth medium was changed to a differentiation medium. After culturing for 3 days, transfection was performed with 50 µM PMO using 6 µM Endo-Porter. After culturing for another 3 days, the medium was changed to a differentiation medium, and after culturing for another 1 day, the resultant cells were collected with a cell lysis buffer, Pierce RIPA Buffer (Thermo Fisher Scientific) containing protease inhibitor cocktail, cOmplete, Mini (manufactured by Roche Diagnostics) added thereto. The cells were crushed with a sonicator, Bioruptor UCD-250 (manufactured by Sonicbio Co., Ltd.) (output: H, three times each for 30 seconds), and centrifuged (15,000 rpm, 4°C, 15 minutes) with a cooling centrifuge (TOMY MX-305, rotor: AR015-24, manufactured by Tomy Seiko Co., Ltd.) to obtain a supernatant as a cell lysate. Pierce BCA Protein Assay Kit (manufactured by Thermo Fisher Scientific) was used to measure an absorbance at 562 nm with a plate reader, Synergy HTX Multi-Mode Microplate Reader (manufactured by BioTek Instruments), and a protein concentration in the cell lysate was obtained with data analysis software, Gen5 version 2.09.2 (manufactured by BioTek Instruments). The cell lysate (in an amount corresponding to 30 µg of protein) was subjected to electrophoresis (150 V, 75 minutes) with polyacrylamide gel NuPAGE 3 to 8%, Tris-Acetate, 1.5 mm, Mini Protein Gel, 15-well (manufactured by Thermo Fisher Scientific). As a molecular weight marker, HiMark Pre-Stained Protein Standard (manufactured by Thermo Fisher Scientific) was used.

After the electrophoresis, transcription (4 mA/cm², 30 minutes) was conducted into Immobilon-P Transfer membrane (manufactured by Merck Millipore) by semi-dry blotting. Western blotting was conducted by using, as a primary antibody, a 100-fold diluted anti-dystrophin antibody (NCL-Dys1, manufactured by Leica Biosystems Newcastle Ltd.), and as a secondary antibody, a 2,500-fold diluted goat anti-mouse IgG (H + L) - Horseradish Peroxidase complex (manufactured by Bio-Rad Laboratories). After completing the antibody reaction, light emission was caused with ECL Prime Western Blotting Detection System (manufactured by Cytiva), and the light emission was detected with a chemiluminescence gel imaging apparatus, ChemiDoc Touch MP Imaging System (manufactured by Bio-Rad Laboratories) to take an image.

### (Results)

The results are shown in Figure 11.

In a negative control, or the mixture of PMO No. 1 and PMO No. 2 (Mix 2), the dystrophin protein was not expressed, but in the mixture additionally containing PMO No. 3 targeting hnRNP A1 (Mix 2 + hnRNP A1), the expression of the dystrophin protein corresponding to exon 45 to 55 skipping was confirmed (Figure 11). Mix 2 had the same definition as Mixture 2 used in Test Example 1 and Test Example 2.

### [Example 3: Production of Antisense Oligomer - (2)]

In the same manner as in Example 1, antisense oligomers shown in Table 12 (PMO Nos. 6 to 33) were synthesized. Theoretical values and actual values measured by ESI-TOR-MS of the molecular weights of the antisense oligomers are also shown. The 5' end of each PMO is Group (1) as in Example 1. The synthesized PMO was dissolved in water for injection (manufactured by Otsuka Pharmaceutical Factory, Inc.).

**[Table 12-1]**

| **Table 12** | | | | | |
|---|---|---|---|---|---|
| PMO No. | **SEQ ID NO:** | **Target base sequence** | **Base sequence of PMO** | **Molecular weight** | |
| | | | | **Theoretical value** | **Actual value** |
| 6 | 1201, 151 | H45_(-66)-(-61)_19-40 | TGACAACAGTTTGCCGCTGCCCGATTAA | 9247. 20 | 9247. 20 |
| 7 | 3082 | H45_183-206 | TTTTCATTCCTATTAGATCTGTCG | 7869. 71 | 7869. 72 |
| 8 | 4950 | H55_(-4)-24 | AAAGCAGCCTCTCGCTCACTCACCCTGC | 9113.17 | 9113.17 |
| 9 | 1201, 201 | H45_(-66)-(-57)_19-40 | TGACAACAGTTTGCCGCTGCCCAAAAGATT AA | 10603. 6 8 | 10603. 6 9 |
| 10 | 1201, 203 | H45_(-68)-(-59)_19-40 | TGACAACAGTTTGCCGCTGCCCAAGATTAA AC | 10579. 6 7 | 10579. 6 7 |
| 11 | 1201, 205 | H45_(-70)-(-61)_19-40 | TGACAACAGTTTGCCGCTGCCCGATTAAAC AG | 10595. 6 7 | 10595. 6 8 |
| 12 | 1239, 114 | H45_(-63)-(-62)_13-35 | ACAGTTTGCCGCTGCCCAATGCCAT | 8198.85 | 8198.49 |
| 13 | 1224, 124 | H45_(-66)-(-64)_(-3) 20 | CCAATGCCATCCTGGAGTTCCTGTAA | 8552. 97 | 8553.61 |
| 14 | 1180, 151 | H45_(-66)-(-61)_(-3)-19 | CAATGCCATCCTGGAGTTCCTGGATTAA | 9262. 21 | 9262. 21 |
| 15 | 1190, 151 | H45_(-66)-(-61)_8-29 | TGCCGCTGCCCAATGCCATCCTGATTAA | 9183. 19 | 9183.22 |
| 16 | 1212, 151 | H45_(-66)-(-61)_30-51 | ATTCAATGTTCTGACAACAGTTGATTAA | 9284. 22 | 9284. 25 |

**[Table 12-2]**

| | | | | | |
|---|---|---|---|---|---|
| 17 | 1222, 151 | H45_(-66)-(-61)_40-61 | CCCCAGTTGCATTCAATGTTCTGATTAA | 9212. 19 | 9212. 19 |
| 18 | 3060 | H45_161-184 | CGCCCTACCTCTTTTTTCTGTCTG | 7782. 68 | 7782. 67 |
| 19 | 3077 | H45_178-201 | ATTCCTATTAGATCTGTCGCCCTA | 7848. 72 | 7848. 99 |
| 20 | 3090 | H45_191-214 | CTAAAATGTTTTCATTCCTATTAG | 7886. 73 | 7886. 37 |
| 21 | 3096 | H45_197-220 | AGTCTGCTAAAATGTTTTCATTCC | 7887. 73 | 7887. 49 |
| 22 | 3108 | H45_209-232 | GAAAGCTTAAAAAGTCTGCTAAAA | 7996. 80 | 7996. 82 |
| 23 | 3119 | H45_220-243 | ATTCTTCTAAAGAAAGCTTAAAAA | 7946. 78 | 7946. 79 |
| 24 | 3065 | H45_166-189 | TCTGTCGCCCTACCTCTTTTTTCT | 7757. 67 | 7757. 89 |
| 25 | 3087 | H45_188-211 | AAATGTTTTCATTCCTATTAGATC | 7886. 73 | 7886. 24 |
| 26 | 3320 | H45_167-194 | TTAGATCTGTCGCCCTACCTCTTTTTTC | 9121. 14 | 9121. 27 |
| 27 | 4698 | H55_10-32 | TTTCTTCCAAAGCAGCCTCTCGC | 7479. 60 | 7479. 54 |
| 28 | 4702 | H55_14-36 | TGAGTTTCTTCCAAAGCAGCCTC | 7543. 62 | 7543. 28 |
| 29 | 4752 | H55_13-36 | TGAGTTTCTTCCAAAGCAGCCTCT | 7873. 73 | 7873. 59 |
| 30 | 4923 | H55_(-31)-(-4) | CAAAGGACCAAATGTTCAGATGCAATTA | 9312. 25 | 9312. 25 |
| 31 | 4926 | H55_(-28)-(-1) | CTGCAAAGGACCAAATGTTCAGATGCAA | 9313. 25 | 9313.24 |
| 32 | 4936 | H55_(-18)-10 | CTCACTCACCCTGCAAAGGACCAAATGT | 9185.21 | 9185. 24 |
| 33 | 4977 | H55_24-51 | TGTTGCAGTAATCTATGAGTTTCTTCCA | 9258. 19 | 9258. 19 |

A target base sequence of the antisense oligomer of the present invention was described as "Ha₁_b₁-c₁" or "Ha₂_b₂-c₂_Ha₃_b₃-c₃".

"Ha₁" represents the ath exon of the human dystrophin gene, "b₁" represents the 5'-terminal base of the target base sequence, and "c₁" represents the 3'-terminal base of the target base sequence.

When "b₁" and "c₁" are positive integers, "b₁" and "c₁" each represent a base number in the downstream direction when the 5'-terminal base of the ath exon is counted as the 1st base. On the other hand, when "b₁" and "c₁" are negative numbers, "b₁" and "c₁" each represent a base number in the upstream direction when the 3'-terminal base of the (a - 1)th intron is counted as the 1st base.

For example, "H55_(-18)-10" means a base sequence in which the 5' end of the target base sequence is the 18th base in the upstream direction from the 3' end of the 54th intron and the 3' end of the target base sequence is the 10th base in the downstream direction from the 5' end of the 55th intron.

"Ha₂_b₂-c₂" which is the first part of "Ha₂_b₂-c₂_Ha₃_b₃-c₃" means the target base sequence of a 3' unit oligomer constituting the antisense oligomer, and the second part "Ha₃_b₃-c₃" means the target base sequence of a 5' unit oligomer constituting the antisense oligomer.

When "Ha₂" and "Ha₃" are the same, the "_Ha₃" part may be omitted.

For example, "H45_(-66)-(-61)_19-40" or "H45_(-66)-(-61)_H45_19-40" means a base sequence in which the target base sequence of the 3' unit oligomer constituting the antisense oligomer is "H45_(-66)-(-61)" and the target base sequence of the 5' unit oligomer is "H45_19-40".

### [Example 4: Test on multi-exon skipping activity of antisense oligomer - (2)]

### <Test Example 1>

### Assay of exon 45 to 55 multi-exon skipping in normal human-derived myoblasts - (1): induction of multi-exon skipping

### Procedures

Normal human-derived myoblasts (manufactured by LONZA) were subjected to direct immunofluorescence staining with PE anti-human CD82 antibody (manufactured by BioLegend, hereinafter the same), and the resultant was sorted with Cell Sorter SH800S (manufactured by Sony, hereinafter the same) to obtain CD82-positive normal human-derived myoblasts. The CD82-positive normal human-derived myoblasts were seeded in a collagen I coat microplate 96-well (manufactured by AGC Techno Glass Co., Ltd.) coated with Corning(R) Matrigel Basement Membrane Matrix (manufactured by Corning, hereinafter the same) at 5 x 10⁴/well, and cultured for 1 day under conditions of 37°C and 5% CO₂ in 0.1 mL of a growth medium for normal human myoblasts (DMEM, high glucose, GlutaMAX(TM) Supplement, Pyruvate (manufactured by Thermo Fisher Scientific, hereinafter the same) supplemented with 20% fetal bovine serum (FBS) (manufactured by Corning, hereinafter the same), 0.1% hBFGF (manufactured by Sigma Aldrich), and 1% penicillin/streptomycin (P/S) (manufactured by Sigma Aldrich, hereinafter the same)). On the next day of the seeding, the medium was changed from the growth medium to 0.2 mL of a differentiation medium for normal human myoblasts (DMEM, High Glucose, GlutaMAX(TM) Supplement, Pyruvate supplemented with 2% horse serum (manufactured by Thermo Fisher Scientific), 1% ITS liquid medium supplement (100x) (manufactured by Sigma Aldrich), and P/S). After culturing for 7 days, transfection was performed with PMO using 6 µM Endo-Porter (manufactured by Gene Tools). PMOs used here are shown in Table 13 below. PMOs targeting the same position in the human dystrophin gene as PMO Nos. 1 to 3 targeting the mouse dystrophin gene were used.

### [Table 13]

**Table 13**

| PMO No. | **Base sequence of PMO (5' to 3')** | **SEQ ID NO:** |
|---|---|---|
| 6 | TGACAACAGTTTGCCGCTGCCCGATTAA | 1201, 151 |
| 7 | TTTTCATTCCTATTAGATCTGTCG | 3082 |
| 8 | AAAGCAGCCTCTCGCTCACTCACCCTGC | 4950 |

The used PMOs and concentrations thereof in the medium are shown in Table 14 below.

### [Table 14]

**Table 14**

| **Condition** | PMO No. 6 (µM) | PMO No. 7 (µM) | PM0 No. 8 (µM) |
|---|---|---|---|
| 1 | - | - | - |
| 2 | 10 | 10 | 10 |
| 3 | 20 | 20 | 20 |
| 4 | 30 | 30 | 30 |
| 5 | 30 | - | 30 |
| 6 | 30 | 30 | - |
| 7 | - | 30 | 30 |
| 8 | 60 | - | - |
| 9 | - | 60 | - |
| 10 | - | - | 60 |

After culturing for another 3 days, the medium was changed to 0.25 mL of a differentiation medium for normal human myoblasts. Seven days after the addition of PMO, the cells were washed once with PBS (manufactured by Takara Bio Inc.), and the total RNA was extracted with RNeasy Micro Kit (manufactured by Qiagen K. K.). 75 µL of Buffer RLT (manufactured by Qiagen K. K.) containing 1% 2-mercaptoethanol (manufactured by Nacalai Tesque, Inc.) was added to the cells, and after the cells were allowed to stand at room temperature for a few minutes to lyse the cells, the total RNA was extracted according to the protocol attached to RNeasy Mini Kit (manufactured by Qiagen K. K.). The concentration of the total RNA extracted was determined using a NanoDrop One C (manufactured by Thermo Fisher Scientific). One-Step RT-PCR was performed with 100 ng of the extracted total RNA using QIAGEN One Step RT-PCR Kit (manufactured by Qiagen K. K.). A reaction solution was prepared in accordance with the protocol attached to the kit. Veriti 96 Well Thermal Cycler (manufactured by Thermo Fisher Scientific) was used as the thermal cycler. The RT-PCR program used here was as follows.
50°C, 30 mins: reverse transcription reaction
95°C, 15 mins: polymerization activation, reverse transcriptase inactivation, cDNA thermal denaturation
[94°C, 30 seconds; 57°C, 30 seconds; 72°C, 1 minute] x 33 cycles: PCR amplification
72°C, 10 mins: final extension

The base sequences of forward primers and reverse primers used in the RT-PCR are shown in Table 15 below.

### [Table 15]

**Table 15**

| **Primer** | **Base sequence (5' to 3')** | **SEQ ID NO:** |
|---|---|---|
| **Forward primer** 1 | GTTGAGAAATGGCGGCGTTT | 5106 |
| **Forward primer** 2 | ATGACATACGCCCAAAGGTG | 5107 |
| **Reverse primer** 1 | TGTTGAGAGACTTTTTCCGAAGT | 5108 |
| **Reverse primer** 2 | ATTCACCCCCTGCTGAATTT | 5109 |

Transcripts (301 bp) having multi-exon skipping of exons 45 to 55 can be detected by a combination of the forward primer 1 and the reverse primer 1. Transcripts (245 bp) of a region of exons 37 to 38 not affected by skipping can be detected by a combination of the forward primer 2 and the reverse primer 2.

The reaction product of the PCR was analyzed with MultiNA (manufactured by Shimadzu Corporation). The polynucleotide level "A" of the band with skipping of exons 45 to 55, and the polynucleotide level "B" of the band having no skipping were measured. Based on these measurement values of "A" and "B", the skipping efficiency of exon 45 to 55 skipping was determined by the following equation: Skipping efficiency of exon 45 to 55 skipping (%) = A / B × 100

One-Step RT-PCR was performed for exon 45 skipping in the same manner as in the detection of exon 45 to 55 skipping by using primers shown in Table 16 below.

### [Table 16]

**Table 16**

| **Primer** | **Base sequence (5' to 3')** | **SEQ ID NO:** |
|---|---|---|
| **Forward primer** | ATTTGACAGATCTGTTGAGAAATGG | 5110 |
| **Reverse primer** | AGTTGCTGCTCTTTTCCAGGT | 5111 |

Transcripts (268 bp) having skipping of exon 45 and transcripts (444 bp) having no skipping can be detected by a combination of the forward primer and the reverse primer.

The reaction product of the PCR was analyzed with MultiNA (manufactured by Shimadzu Corporation). The polynucleotide level "A" of the band with skipping of exon 45, and the polynucleotide level "B" of the band having no skipping were measured. Based on these measurement values of "A" and "B", the skipping efficiency of exon 45 skipping was determined by the following equation: Skipping efficiency of exon 45 skipping (%) = A / (A + B) × 100

### Results

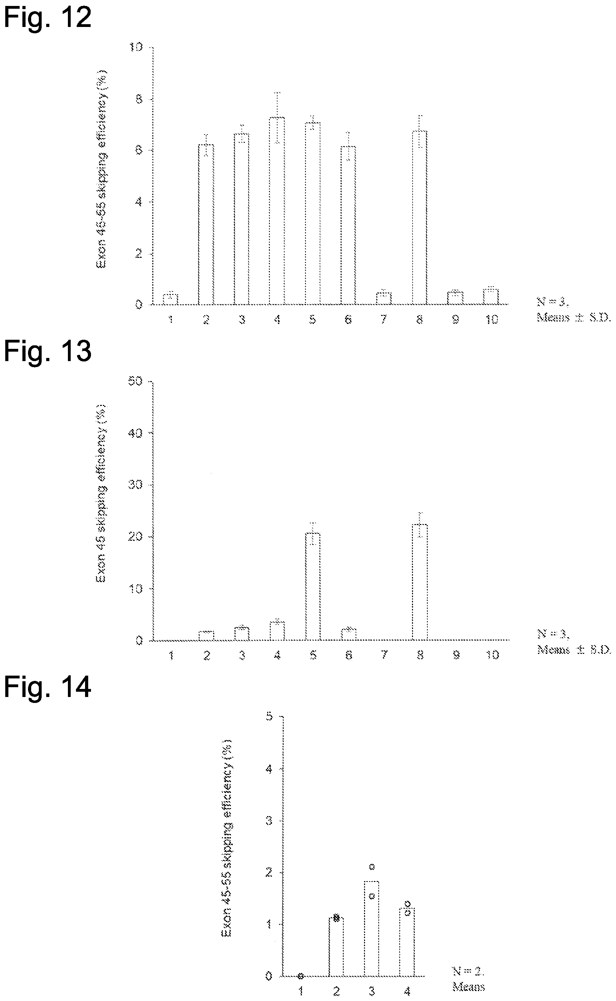

The results are shown in Figures 12 and 13. It was confirmed that in the normal human cultured cells, exon 45 to 55 skipping is inducted by PMO No. 6 used singly (condition 8) and the mixture containing PMO No. 6 (conditions 2 to 6) (Figure 12). On the other hand, the skipping efficiency of exon 45 skipping was reduced by the mixtures containing, in addition to PMO No. 6, PMO No. 7 targeting hnRNP A1 (conditions 2 to 4, and 6), and single skipping was thus suppressed (Figure 13).

### <Test Example 2>

### Assay of exon 45 to 55 multi-exon skipping in DMD patient-derived myoblasts with exon 48 to 50 deletion - (1): induction of multi-exon skipping

### Procedures

DMD patient-derived myoblasts with exon 48 to 50 deletion obtained from NCNP BioBank were subjected to direct immunofluorescence staining with PE anti-human CD82 antibody and APC anti-human CD56 antibody (manufactured by Milternyi Biotec, hereinafter the same), and the resultant was sorted with Cell Sorter SH800S to obtain CD56- and CD82-positive DMD patient-derived myoblasts with exon 48 to 50 deletion. The DMD patient-derived myoblasts with exon 48 to 50 deletion (CD56-positive and CD82-positive) were seeded in a Corning BioCoat collagen I 48-well transparent microplate coated with Corning(R) Matrigel Basement Membrane Matrix at 5 x 10⁴/well, and cultured for 1 day under conditions of 37°C and 5% CO₂ in 0.25 mL of a growth medium for DMD patient-derived myoblasts (Dulbecco's Modified Eagle Medium: Nutrient Mixture F-12 (DMD/F12) (manufactured by Thermo Fisher Scientific, hereinafter the same) supplemented with 20% fetal bovine serum (FBS) and 1% P/S). On the next day of the seeding, the medium was changed from the growth medium to 0.5 mL of a differentiation medium for DMD patient-derived myoblasts (DMEM/F12 supplemented with 2% horse serum, 1% ITS liquid medium supplement (100x), and 1% P/S). After culturing for 6 days in the differentiation medium, transfection was performed with PMO using 6 µM Endo-Porter. The same PMOs as those used in Text Example 1 were used, and concentrations thereof in the medium are shown in Table 17 below.

### [Table 17]

**Table 17**

| **Condition** | PMO No. 6 (µM) | PMO No. 7 (µM) | PMO No. 8 (µM) |
|---|---|---|---|
| 1 | - | - | - |
| 2 | 10 | 10 | 10 |
| 3 | 30 | 30 | 30 |
| 4 | 30 | - | 30 |

After culturing for another 3 days, the medium was changed to 0.5 mL of a differentiation medium. Seven days after the addition of PMO, the total RNA was extracted from the cells in the same manner as in Test Example 1 of Example 2, and One-Step RT-PCR was performed with 200 ng of the extracted total RNA in the same manner as in Test Example 1, and the reaction product of the PCR thus obtained was analyzed to obtain skipping efficiencies of exon 45 to 55 skipping and exon 45 skipping.

### Results

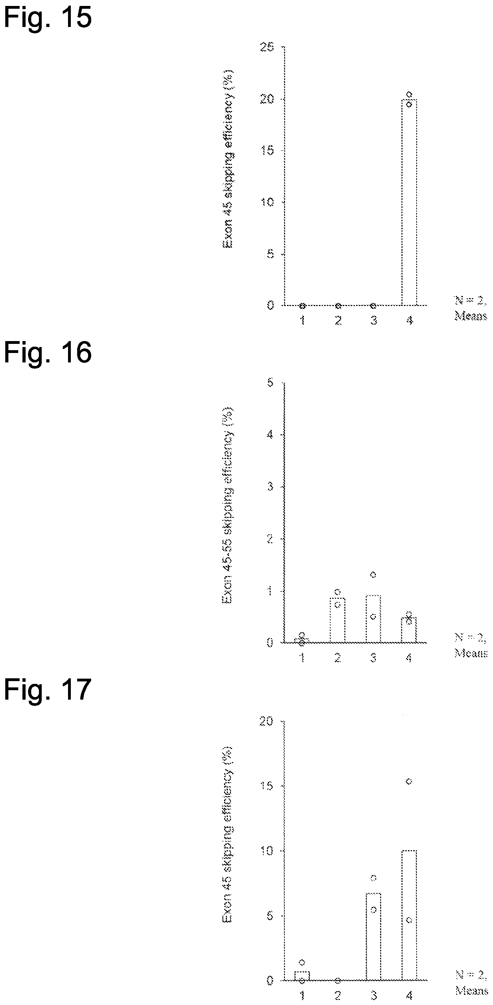

The results are shown in Figures 14 and 15.

In the DMD patient-derived myoblasts with exon 48 to 50 deletion, exon 45 to 55 skipping was confirmed to be caused by the mixture of PMO No. 6 and PMO No. 8 (condition 4). Exon 45 to 55 skipping was confirmed to be induced also by the mixtures further containing PMO NO. 7 targeting hnRNP A1 (conditions 2 and 3) (Figure 14), but the skipping efficiency of exon 45 skipping was reduced, and single skipping was thus suppressed (Figure 15) .

### <Test Example 3>

### Assay of exon 45 to 55 multi-exon skipping in DMD patient-derived myoblasts with exon 48 to 50 deletion - (2): induction of multi-exon skipping

### Procedures

DMD patient-derived myoblasts with exon 48 to 50 deletion (CD56-positive and CD82-positive) prepared in the same manner as in Test Example 2 were seeded in a Corning BioCoat collagen I 48-well transparent microplate coated with Corning(R) Matrigel Basement Membrane Matrix at 2 x 10⁴/well, and cultured for 3 days under conditions of 37°C and 5% CO₂ in 0.25 mL of a growth medium for DMD patient-derived myoblasts. 3 days after the seeding, the medium was changed from the growth medium to 0.5 mL of a differentiation medium for DMD patient-derived myoblasts. After culturing for 8 days in the differentiation medium, transfection was performed with PMO using 6 µM Endo-Porter. The same PMOs as those used in Text Examples 1 and 2 were used, and concentrations thereof in the medium are shown in Table 18 below.

### [Table 18]

**Table 18**

| **Condition** | PMO No. 6 (µM) | PMO No. 7 (µM) | PMO No. 8 (µM) |
|---|---|---|---|
| 1 | - | - | - |
| 2 | 20 | 20 | 20 |
| 3 | 30 | - | 30 |
| 4 | 60 | - | - |

After culturing for another 3 days, the medium was changed to 0.5 mL of a differentiation medium. Six days after the addition of PMO, the total RNA was extracted in the same manner as in Test Example 2, and One-Step RT-PCR was performed with 200 ng of the extracted total RNA in the same manner as in Test Examples 1 and 2, and the reaction product of the PCR thus obtained was analyzed to obtain skipping efficiencies of exon 45 to 55 skipping and exon 45 skipping.

### Results

The results are shown in Figures 16 and 17.

As compared with PMO No. 6 used singly (condition 4), the mixtures additionally containing PMO No. 8 or PMO No. 7 and PMO No. 8 in addition to PMO No. 6 (conditions 2 and 3) increased the skipping efficiency of exon 45 to 55 skipping (Figure 16). On the other hand, the mixture containing PMO No. 7 targeting hnRNP A1 (condition 2) reduced the skipping efficiency of exon 45 skipping, and single skipping was thus suppressed (Figure 17).

### <Test Example 4>

### Assay of exon 45 to 55 multi-exon skipping in DMD patient-derived myoblasts with exon 48 to 50 deletion - (3): restoration of dystrophin protein by multi-exon skipping

### Procedures

DMD patient-derived myoblasts with exon 48 to 50 deletion (CD56-positive and CD82-positive) were seeded in a collagen I coat microplate 24-well (manufactured by AGC Techno Glass Co., Ltd., hereinafter the same) coated with Corning(R) Matrigel Basement Membrane Matrix at 1.0 x 10⁵/well, and cultured for 1 day under conditions of 37°C and 5% CO₂ in 1 mL of a growth medium for DMD patient-derived myoblasts. On the next day of the seeding, the medium was changed from the growth medium for DMD patient derived myoblasts to a differentiation medium for DMD patient-derived myoblasts. After culturing for 6 days, transfection was performed with PMO using 6 µM Endo-Porter. After culturing for another 3 days, the medium was changed to a differentiation medium, and after culturing for 7 days after the addition of PMO, a cell lysate was prepared in the same manner as in Test Example 4 of Example 2, and a protein concentration in the cell lysate was obtained. Western blotting was performed in the same manner as in Test Example 4 of Example 2 except that the cell lysate was used in an amount corresponding to 30 µg of protein, that the electrophoresis time was 120 minutes, and that a 250-fold diluted anti-dystrophin antibody (NCL-DYS1) was used, and thus the dystrophin protein was detected. Samples subjected to the electrophoresis are shown in Table 19. As a positive control of dystrophin expression, a lysate of mouse C2C12 cells having been muscle differentiation cultured for 12 days (normal dystrophin control), and a lysate of skeletal muscle of exon 45 to 55 deletion transgenic mouse (exon 45 to 55 deletion dystrophin expression control) were used.

### [Table 19]

**Table 19**

| **Condition** | **Sample** |
|---|---|
| 1 | **Normal dystrophin control** |
| 2 | **Exon 45 to 55 deletion dystrophin expression control** |
| 3 | **No PMO added** |
| 4 | 30 µM PMO No. 6 + 30 µM PMO No. 7 +30 µM PM0 No. 8 |
| 5 | 30 µM PMO No. 6 + 30 µM PM0 No. 8 |
| 6 | 10 µM PMO No. 6 + 10 µM PMO No. 7 +10 µM PMO No. 8 |

### (Results)

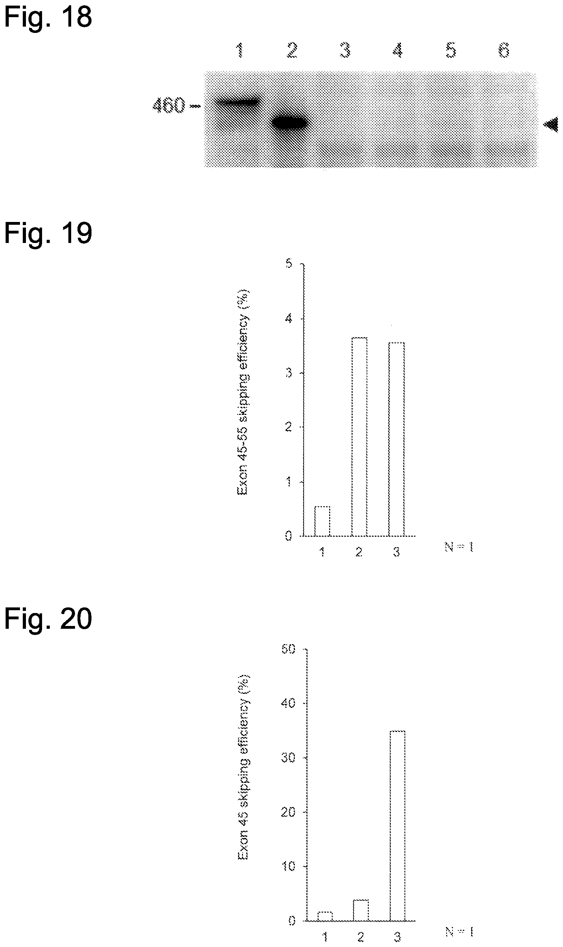

The results are shown in Figure 18.

In the negative control (condition 3), the dystrophin protein was not expressed, but expression of the dystrophin protein corresponding to exon 45 to 55 skipping caused by the mixture of PMO No. 6 and PMO No. 8 (condition 5) and the mixtures of PMO Nos. 6 to 8 (conditions 4 and 6) was confirmed (Figure 18: arrowhead).

### <Test Example 5>

### Assay of exon 45 to 55 multi-exon skipping in DMD patient-derived myoblasts with exon 46 to 51 deletion - (1): induction of multi-exon skipping

### Procedures

DMD patient-derived myoblasts with exon 46 to 51 deletion (CD56-positive and CD82-positive) obtained by sorting DMD patient-derived myoblasts with exon 46 to 51 deletion obtained from NCNP BioBank in the same manner as in Test Example 2 were seeded in a Corning BioCoat collagen I 48-well transparent microplate coated with Corning(R) Matrigel Basement Membrane Matrix at 5 x 10⁴/well, and cultured for 1 day under conditions of 37°C and 5% CO₂ in 0.25 mL of a growth medium for DMD patient-derived myoblasts. On the next day of the seeding, the medium was changed from the growth medium to 0.3 mL of a differentiation medium for DMD patient-derived myoblasts. After culturing for 6 days in the differentiation medium, transfection was performed with PMO using 6 µM Endo-Porter. The same PMOs as those used in Text Examples 1 to 4 were used, and concentrations thereof in the medium are shown in Table 20 below.

### [Table 20]

**Table 20**

| **Condition** | PM0 No. 6 (µM) | PMO No. 7 (µM) | PMO No. 8 (µM) |
|---|---|---|---|
| 1 | - | - | - |
| 2 | 30 | 30 | 30 |
| 3 | 30 | - | - |

After culturing for another 3 days, the medium was changed to 0.3 mL of a differentiation medium. Five days after the addition of PMO, the total RNA was extracted in the same manner as in Test Examples 2 to 3, One-Step RT-PCR was performed with 100 ng of the extracted total RNA in the same manner as in Test Examples 1 to 3, and the reaction product of the PCR thus obtained was analyzed to obtain skipping efficiency of exon 45 to 55 skipping. Besides, One-Step RT-PCR was performed in the same manner as in Test Examples 1 to 3 except that primers shown in Table 21 below were used, and the reaction product of the PCR thus obtained was analyzed to obtain skipping efficiency of exon 45 skipping. Transcripts (162 bp) having exon 45 skipping and transcripts (338 bp) having no skipping can be detected by a combination of the forward primer and the reverse primer.

### [Table 21]

**Table 21**

| **Primer** | **Base sequence (5' to 3')** | **SEQ ID NO:** |
|---|---|---|
| **Forward primer** | GAGAAATGGCGGCGTTTTCA | 5112 |
| **Reverse primer** | GGGACGCCTCTGTTCCAAAT | 5113 |

### Results

The results are shown in Figures 19 and 20.

In the DMD patient-derived myoblasts with exon 46 to 51 deletion, exon 45 to 55 skipping was confirmed to be induced by PMO No. 6 used singly (condition 3) and the mixture of PMO Nos. 6 to 8 (condition 2) (Figure 19).

The efficiency of exon 45 skipping was reduced by the mixture containing PMO Nos. 6 to 8 (condition 2) as compared with that by PMO No. 6 used singly (condition 3), and single skipping was thus suppressed (Figure 20).

### <Test Example 6>

### Assay of exon 45 to 55 multi-exon skipping in DMD patient-derived myoblasts with exon 46 to 51 deletion - (2): induction of multi-exon skipping

### Procedures

DMD patient-derived myoblasts with exon 46 to 51 deletion (CD56-positive and CD82-positive) prepared in the same manner as in Test Example 5 were seeded in a Corning BioCoat collagen I 48-well transparent microplate coated with Corning(R) Matrigel Basement Membrane Matrix at 6.3 x 10³/well, and cultured for 4 days under conditions of 37°C and 5% CO₂ in 0.25 mL of a growth medium for DMD patient-derived myoblasts. Four days after the seeding, the medium was changed from the growth medium to 0.3 mL of a differentiation medium for DMD patient-derived myoblasts. After culturing for 7 days in the differentiation medium, transfection was performed with PMO using 6 µM Endo-Porter. The same PMOs as those used in Text Examples 1 to 5 were used, and concentrations thereof in the medium are shown in Table 22 below.

### [Table 22]

**Table 22**

| **Condition** | PMO No. 6 (µM) | PMO No. 7 (µM) | PMO No. 8 (µM) |
|---|---|---|---|
| 1 | - | - | - |
| 2 | 20 | 20 | 20 |
| 3 | 30 | - | 30 |
| 4 | - | 30 | 30 |
| 5 | 30 | 30 | - |
| 6 | 60 | - | - |

After culturing for another 3 days, the medium was changed to 0.3 mL of a differentiation medium. Seven days after the addition of PMO, the total RNA was extracted in the same manner as in Test Examples 2 to 3 and 5, and One-Step RT-PCR was performed in the same manner as in Test Example 5 except that 100 ng of the extracted total RNA was used, and the reaction product of the PCR thus obtained was analyzed to obtain skipping efficiencies of exon 45 to 55 skipping and exon 45 skipping.

### Results

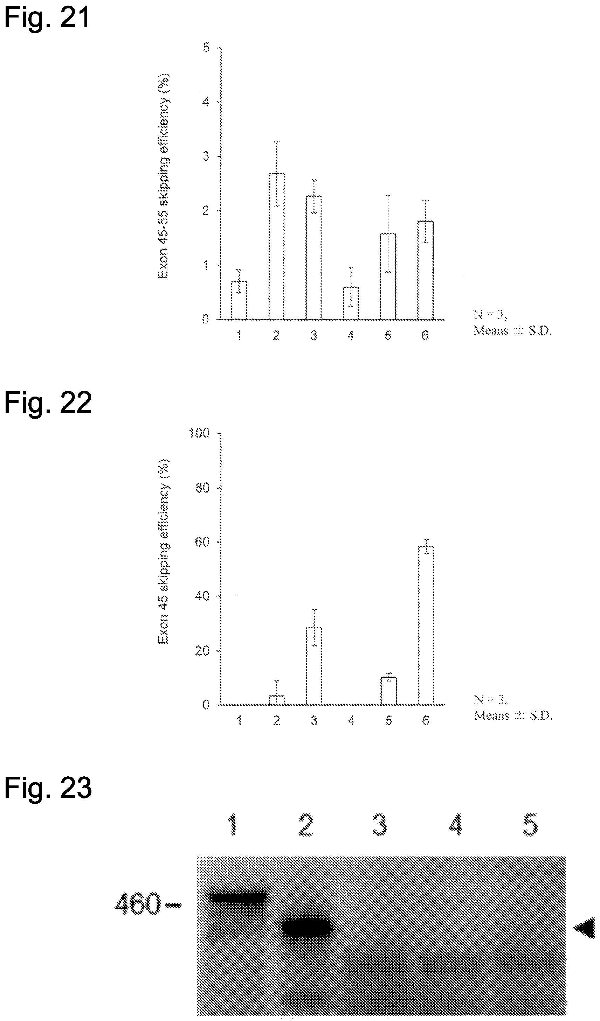

The results are shown in Figures 21 and 22. In the DMD patient-derived myoblasts with exon 46 to 51 deletion, exon 45 to 55 skipping was confirmed to be induced by PMO No. 6 used singly (condition 6) and the mixtures containing PMO No. 6 (conditions 2, 3 and 5) (Figure 21). The efficiency of exon 45 skipping was reduced by the mixture containing PMO No. 7 in addition to PMO No. 6 (condition 5) as compared with that by the mixture containing PMO No. 8 in addition to PMO No. 6 (condition 3), and single skipping was thus suppressed (Figure 22).

### <Test Example 7>

### Assay of exon 45 to 55 multi-exon skipping in DMD patient-derived myoblasts with exon 46 to 51 deletion - (4): restoration of dystrophin protein by multi-exon skipping

### Procedures

DMD patient-derived myoblasts with exon 46 to 51 deletion (CD56-positive and CD82-positive) were seeded in a collagen I coat microplate 24-well coated with Corning(R) Matrigel Basement Membrane Matrix at 8.0 x 10⁴/well, and cultured for 1 day under conditions of 37°C and 5% CO₂ in 1 mL of a growth medium for DMD patient-derived myoblasts. On the next day of the seeding, the medium was changed from the growth medium for DMD patient derived myoblasts to 1 mL of a differentiation medium for DMD patient-derived myoblasts. After culturing 4 days, the medium was changed, and after culturing for 7 days, transfection was performed with PMO using 6 µM Endo-Porter. After culturing for another 3 days, the medium was changed to a differentiation medium, and after culturing for 7 days after the addition of PMO, a cell lysate was prepared in the same manner as in Test Example 4 of Example 2 and Test Example 4 of the present example, and Western blotting was performed in the same manner as in Test Example 4 of Example 2 and Test Example 4 of the present example except that the cell lysate was used in an amount corresponding to 24 µg of protein to detect the dystrophin protein. Samples subjected to the electrophoresis are shown in Table 23 below.

### [Table 23]

**Table 23**

| **Condition** | **Sample** |
|---|---|
| 1 | **Normal dystrophin control** |
| 2 | **Exon 45 to 55 deletion dystrophin expression control** |
| 3 | **No PMO added** |
| 4 | 30 µM PMO No. 6 + 30 µM PMO No. 7 +30 µM PMO No. 8 |
| 5 | 30 µM PMO No. 6 + 30 µM PMO No. 8 |

### (Results)

The results are shown in Figure 23.

In the negative control (condition 3), the dystrophin protein was not expressed, but expression of the dystrophin protein corresponding to exon 45 to 55 skipping caused by the mixture of PMO No. 6 and PMO No. 8 (condition 5) and the mixture of PMO Nos. 6 to 8 (condition 4) was confirmed (Figure 23: arrowhead).

### <Test Example 8>

### Assay of exon 45 to 55 multi-exon skipping in DMD patient-derived myoblasts with exon 51 deletion - (1): study of first antisense oligomer - (1)

### Procedures

DMD patient-derived myoblasts with exon 51 deletion (CD-56 positive, CD-82 positive) obtained by sorting DMD patient-derived myoblasts with exon 51 deletion obtained from NCNP BioBank in the same manner as in Example 3 and 6 were seeded in a collagen I coat microplate 24-well (manufactured by AGC Techno Glass Co., Ltd.) coated with Corning(R) Matrigel Basement Membrane Matrix at 5 x 10⁴/well, and cultured for 3 days under conditions of 37°C and 5% CO₂ in 0.5 mL of a growth medium for DMD patient-derived myoblasts. Three days after the seeding, the medium was changed from the growth medium to 0.5 mL of a differentiation medium for DMD patient-derived myoblasts. After culturing for 4 days in the differentiation medium for DMD patient-derived myoblasts, transfection was performed with PMO using 6 µM Endo-Porter. In addition to the PMOs used in Text Examples 1 to 7, PMOs shown in Table 24 below were also used.

### [Table 24]

**Table 24**

| PMO No. | **Base sequence of PMO (5' to 3')** | **SEQ ID NO:** |
|---|---|---|
| 9 | TGACAACAGTTTGCCGCTGCCCAAAAGATTAA | 1201, 201 |
| 10 | TGACAACAGTTTGCCGCTGCCCAAGATTAAAC | 1201, 203 |
| 11 | TGACAACAGTTTGCCGCTGCCCGATTAAACAG | 1201, 205 |

The PMOs were added in concentrations in the medium shown in Table 25 below.

### [Table 25]

**Table 25**

| **Condition** | PMO |
|---|---|
| 1 | **No PMO added** |
| 2 | 30 µM PMO No. 6 + 30 µM PMO No. 8 |
| 3 | 20 µM PMO No. 6 + 20 µM PMO No. 7 + 20 µM PM0 No. 8 |
| 4 | 30 µM PMO No. 9 + 30 µM PMO No. 8 |
| 5 | 20 µM PMO No. 9 + 20 µM PMO No. 7 + 20 µM PMO No. 8 |
| 6 | 30 µM PMO No. 10 + 30 µM PMO No. 8 |
| 7 | 20 µM PMO No. 10 + 20 µM PMO No. 7 + 20 µM PMO No. 8 |
| 8 | 30 µM PMO No. 11 + 30 µM PMO No. 8 |
| 9 | 20 µM PMO No. 11 + 20 µM PMO No. 7 + 20 µM PMO No. 8 |

After culturing for another 3 days, the medium was changed to 0.5 mL of a differentiation medium for DMD patient-derived myoblasts. Seven days after the addition of PMO, the total RNA was extracted in the same manner as in Test Examples 2, 3, 5, and 6, One-Step RT-PCR was performed with 200 ng of the extracted total RNA in the same manner as in Test Examples 1 to 3, and the reaction product of the PCR thus obtained was analyzed to obtain skipping efficiencies of exon 45 to 55 skipping and exon 45 skipping.

### Results

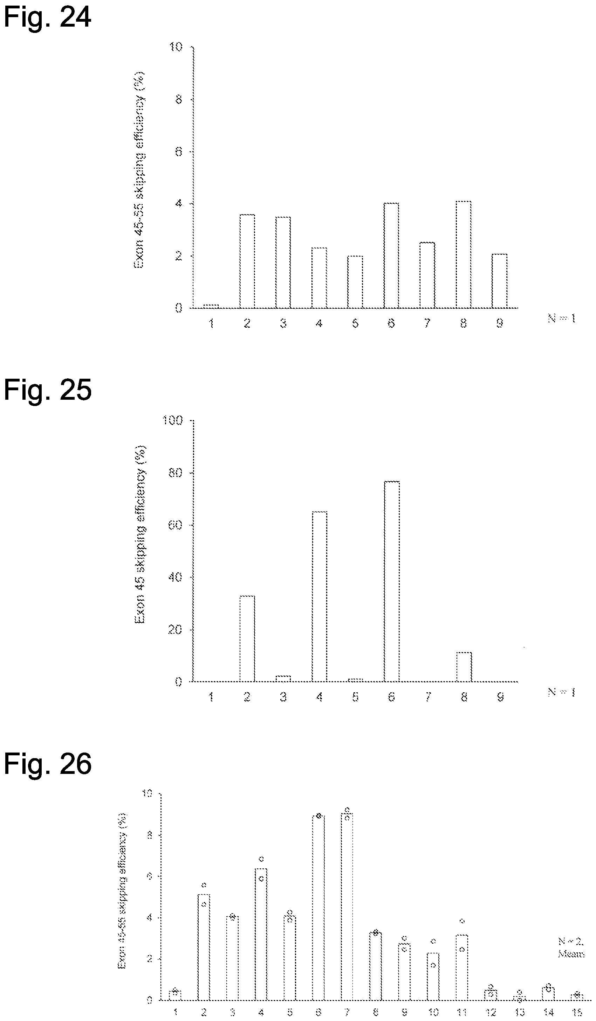

The results are shown in Figures 24 and 25. In the DMD patient-derived myoblasts with exon 51 deletion, exon 45 to 55 skipping was confirmed to be induced by the mixture of PMO No. 6 and PMO No. 8 (condition 2) (Figure 24). Exon 45 to 55 skipping was also confirmed to be induced by the mixture containing, in addition to PMO No. 6 and PMO No. 8, PMO No. 7 targeting hnRNPA1 (condition 3) (Figure 24). Besides, exon 45 to 55 skipping was confirmed to be induced in the conditions 4, 6 and 8 in which PMO Nos. 9 to 11 were added as the first antisense oligomer together with PMO No. 8. Exon 45 to 55 skipping was also confirmed to be induced in the conditions 5, 7, and 9 in which PMO No. 7 targeting hnRNP A1 was further added (Figure 24), but the skipping efficiency of exon 45 skipping was reduced, and single skipping was thus suppressed (Figure 25).

### <Test Example 9>

### Assay of exon 45 to 55 multi-exon skipping in DMD patient-derived myoblasts with exon 51 deletion - (2): study of first antisense oligomer - (2)

### Procedures

DMD patient-derived myoblasts with exon 51 deletion (CD-56 positive, CD-82 positive) prepared in the same manner as in Test Example 8 were seeded in a Corning BioCoat collagen I 48-well transparent microplate coated with Corning(R) Matrigel Basement Membrane Matrix at 5 x 10⁴/well, and cultured for 1 day under conditions of 37°C and 5% CO₂ in 0.25 mL of a growth medium for DMD patient-derived myoblasts. On the next day of the seeding, the medium was changed from the growth medium to 0.25 mL of a differentiation medium for DMD patient-derived myoblasts. After culturing for 7 days in the differentiation medium for DMD patient-derived myoblasts, transfection was performed with PMO using 6 µM Endo-Porter. In addition to the PMOs used in Text Examples 1 to 7, PMOs shown in Table 26 below were used.

### [Table 26]

**Table 26**

| PMO No. | **Base sequence of PMO (5' to 3')** | **SEQ ID NO:** |
|---|---|---|
| 12 | ACAGTTTGCCGCTGCCCAATGCCAT | 1239, 114 |
| 13 | CCAATGCCATCCTGGAGTTCCTGTAA | 1224, 124 |
| 14 | CAATGCCATCCTGGAGTTCCTGGATTAA | 1180, 151 |
| 15 | TGCCGCTGCCCAATGCCATCCTGATTAA | 1190, 151 |
| 16 | ATTCAATGTTCTGACAACAGTTGATTAA | 1212, 151 |
| 17 | CCCCAGTTGCATTCAATGTTCTGATTAA | 1222, 151 |

The PMOs were added in concentrations in the medium shown in Table 27 below.

### [Table 27]

**Table 27**

| **Condition** | PMO |
|---|---|
| 1 | **No PMO added** |
| 2 | 30 µM PMO No. 6 + 30 µM PMO No. 8 |
| 3 | 20 µM PMO No. 6 + 20 µM PMO No. 7 + 20 µM PMO No. 8 |
| 4 | 30 µM PMO No. 12 + 30 µM PMO No. 8 |
| 5 | 20 µM PMO No. 12 + 20 µM PMO No. 7 + 20 µM PMO No. 8 |
| 6 | 30 µM PMO No. 13 + 30 µM PMO No. 8 |
| 7 | 20 µM PMO No. 13 + 20 µM PM0 No. 7 + 20 µM PMO No. 8 |
| 8 | 30 µM PMO No. 14 + 30 µM PM0 No. 8 |
| 9 | 20 µM PMO No. 14 + 20 µM PMO No. 7 + 20 µM PMO No. 8 |
| 10 | 30 µM PMO No. 15 + 30 µM PMO No. 8 |
| 11 | 20 µM PMO No. 15 + 20 µM PMO No. 7 + 20 µM PMO No. 8 |
| 12 | 30 µM PMO No. 16 + 30 µM PMO No. 8 |
| 13 | 20 µM PMO No. 16 + 20 µM PM0 No. 7 + 20 µM PMO No. 8 |
| 14 | 30 µM PMO No. 17 + 30 µM PM0 No. 8 |
| 15 | 20 µM PMO No. 17 + 20 µM PM0 No. 7 + 20 µM PMO No. 8 |

After culturing for another 3 days, the medium was changed to 0.3 mL of a differentiation medium for DMD patient-derived myoblasts. Seven days after the addition of PMO, the total RNA was extracted in the same manner as in Test Examples 2, 3, 5, 6, and 8, One-Step RT-PCR was performed with 200 ng of the extracted total RNA in the same manner as in Test Examples 1 to 3 and 8, and the reaction product of the PCR thus obtained was analyzed to obtain skipping efficiencies of exon 45 to 55 skipping and exon 45 skipping.

### Results

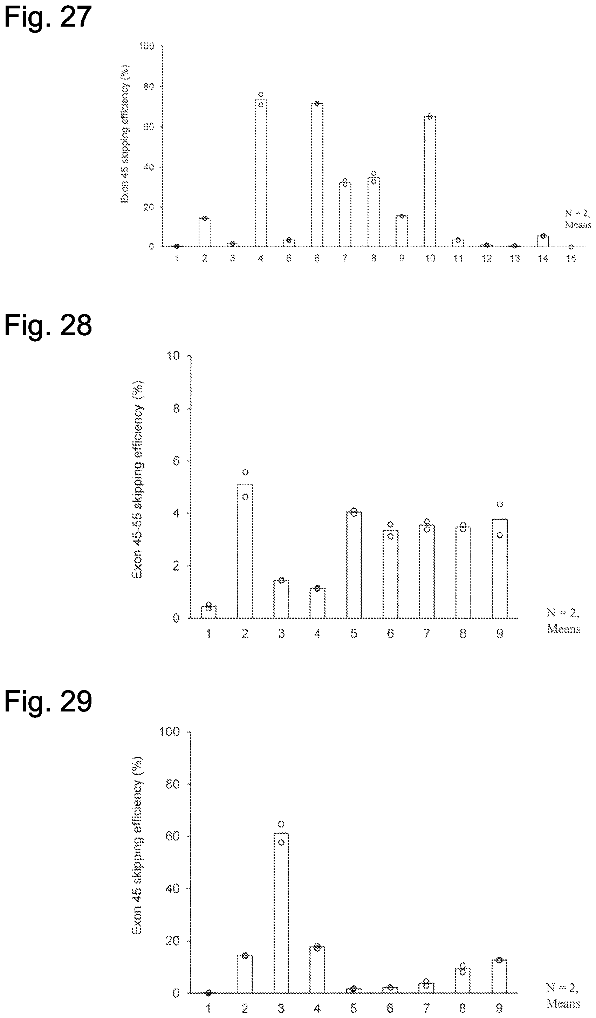

The results are shown in Figures 26 and 27. Exon 45 to 55 skipping was confirmed to be induced also in the conditions 4, 6, 8, and 10 in which PMO Nos. 12 to 17 were added as the first antisense oligomer together with PMO No. 8. Exon 45 to 55 skipping was confirmed to be induced also in the conditions 5, 7, 9, and 11 in which PMO No. 7 targeting hnRNP A1 was further added (Figure 26), but the skipping efficiency of exon 45 skipping was reduced, and single skipping was thus suppressed (Figure 27) .

### <Test Example 10>

### Assay of exon 45 to 55 multi-exon skipping in DMD patient-derived myoblasts with exon 51 deletion - (3): study of third antisense oligomer - (1)

### Procedures

DMD patient-derived myoblasts with exon 51 deletion (CD-56 positive, CD-82 positive) prepared in the same manner as in Test Examples 8 and 9 were seeded in a Corning BioCoat collagen I 48-well transparent microplate coated with Corning(R) Matrigel Basement Membrane Matrix at 5 x 10⁴/well, and cultured for 1 day under conditions of 37°C and 5% CO₂ in 0.25 mL of a growth medium for DMD patient-derived myoblasts. On the next day of the seeding, the medium was changed from the growth medium to 0.25 mL of a differentiation medium for DMD patient-derived myoblasts. After culturing for 7 days in the differentiation medium for DMD patient-derived myoblasts, transfection was performed with PMO using 6 µM Endo-Porter. In addition to the PMOs used in Text Examples 1 to 7, PMOs shown in Table 28 below were used.

### [Table 28]

**Table 28**

| PMO No. | **Base sequence of PMO (5' to 3')** | **SEQ ID NO:** |
|---|---|---|
| 18 | CGCCCTACCTCTTTTTTCTGTCTG | 3060 |
| 19 | ATTCCTATTAGATCTGTCGCCCTA | 3077 |
| 20 | CTAAAATGTTTTCATTCCTATTAG | 3090 |
| 21 | AGTCTGCTAAAATGTTTTCATTCC | 3096 |
| 22 | GAAAGCTTAAAAAGTCTGCTAAAA | 3108 |
| 23 | ATTCTTCTAAAGAAAGCTTAAAAA | 3119 |

The PMOs were added in concentrations in the medium shown in Table 29 below.

### [Table 29]

**Table 29**

| **Condition** | PMO |
|---|---|
| 1 | **No PMO added** |
| 2 | 30 µM PMO No. 6 + 30 µM PMO No. 8 |
| 3 | 20 µM PMO No. 6 + 20 µM PMO No. 18 + 20 µM PMO No. 8 |
| 4 | 20 µM PMO No. 6 + 20 µM PMO No. 19 + 20 µM PMO No. 8 |
| 5 | 20 µM PMO No. 6 + 20 µM PMO No. 7 + 20 µM PMO No. 8 |
| 6 | 20 µM PM0 No. 6 + 20 µM PMO No. 20 + 20 µM PM0 No. 8 |
| 7 | 20 µM PM0 No. 6 + 20 µM PM0 No. 21 + 20 µM PM0 No. 8 |
| 8 | 20 µM PM0 No. 6 + 20 µM PMO No. 22 + 20 µM PM0 No. 8 |
| 9 | 20 µM PM0 No. 6 + 20 µM PMO No. 23 + 20 µM PMO No. 8 |

After culturing for another 3 days, the medium was changed to 0.3 mL of a differentiation medium for DMD patient-derived myoblasts. Seven days after the addition of PMO, the total RNA was extracted in the same manner as in Test Examples 2, 3, 5, 6, 8, and 9, One-Step RT-PCR was performed with 200 ng of the extracted total RNA in the same manner as in Test Examples 1 to 3, 8, and 9, and the reaction product of the PCR thus obtained was analyzed to obtain skipping efficiencies of exon 45 to 55 skipping and exon 45 skipping.

### Results

The results are shown in Figures 28 and 29. As a result of adding PMO Nos. 20 to 23 as the third antisense oligomer to PMO No. 6 and PMO No. 8, exon 45 to 55 skipping was confirmed to be induced to the same extent as in a case where PMO No. 7 was added (conditions 5) (Figure 28). Exon 45 skipping was reduced, and single skipping tended to be suppressed (Figure 29). On the other hand, although exon 45 skipping efficiency was not reduced by the mixture containing PMO Nos. 18 and 19 (conditions 3 and 4) as compared with the mixture containing PMO No. 7 (condition 5), exon 45 to 55 skipping was confirmed to be more highly induced as compared with that in a negative control (condition 1).

### <Test Example 11>

### Assay of exon 45 to 55 multi-exon skipping in DMD patient-derived myoblasts with exon 51 deletion - (4): study of third antisense oligomer - (2)

### Procedures

DMD patient-derived myoblasts with exon 51 deletion (CD-56 positive, CD-82 positive) prepared in the same manner as in Test Examples 8 to 10 were seeded in a Corning BioCoat collagen I 48-well transparent microplate coated with Corning(R) Matrigel Basement Membrane Matrix at 5 x 10⁴/well, and cultured for 1 day under conditions of 37°C and 5% CO₂ in 0.25 mL of a growth medium for DMD patient-derived myoblasts. On the next day of the seeding, the medium was changed from the growth medium to 0.25 mL of a differentiation medium for DMD patient-derived myoblasts. After culturing for 3 days in the differentiation medium for DMD patient-derived myoblasts, transfection was performed with PMO using 6 µM Endo-Porter. In addition to the PMOs used in Text Examples 1 to 7, PMOs shown in Table 30 below were used.

### [Table 30]

**Table 30**

| PM0 No. | **Base sequence of PMO (5' to 3')** | **SEQ ID NO:** |
|---|---|---|
| 19 | ATTCCTATTAGATCTGTCGCCCTA | 3077 |
| 20 | CTAAAATGTTTTCATTCCTATTAG | 3090 |
| 21 | AGTCTGCTAAAATGTTTTCATTCC | 3096 |
| 24 | TCTGTCGCCCTACCTCTTTTTTCT | 3065 |
| 25 | AAATGTTTTCATTCCTATTAGATC | 3087 |
| 26 | TTAGATCTGTCGCCCTACCTCTTTTTTC | 3320 |

The PMOs were added in concentrations in the medium shown in Table 31 below.

### [Table 31]

**Table 31**

| **Condition** | PMO |
|---|---|
| 1 | **No PMO added** |
| 2 | 20 µM PMO No. 6 + 20 µM PMO No. 24 + 20 µM PMO No. 8 |
| 3 | 20 µM PM0 No. 6 + 20 µM PMO No. 19 + 20 µM PMO No. 8 |
| 4 | 20 µM PM0 No. 6 + 20 µM PM0 No. 7 + 20 µM PM0 No. 8 |
| 5 | 20 µM PMO No. 6 + 20 µM PMO No. 25 + 20 µM PMO No. 8 |
| 6 | 20 µM PMO No. 6 + 20 µM PMO No. 20 + 20 µM PMO No. 8 |
| 7 | 20 µM PMO No. 6 + 20 µM PMO No. 21 + 20 µ*M* PMO No. 8 |
| 8 | 20 µM PMO No. 6 + 20 µM PMO No. 26 + 20 µM PM0 No. 8 |

After culturing for another 3 days, the medium was changed to 0.3 mL of a differentiation medium for DMD patient-derived myoblasts. Seven days after the addition of PMO, the total RNA was extracted in the same manner as in Test Examples 2, 3, 5, 6, and 8 to 10, One-Step RT-PCR was performed with 200 ng of the extracted total RNA in the same manner as in Test Examples 1 to 3 and 8 to 10, and the reaction product of the PCR thus obtained was analyzed to obtain skipping efficiencies of exon 45 to 55 skipping and exon 45 skipping.

### Results

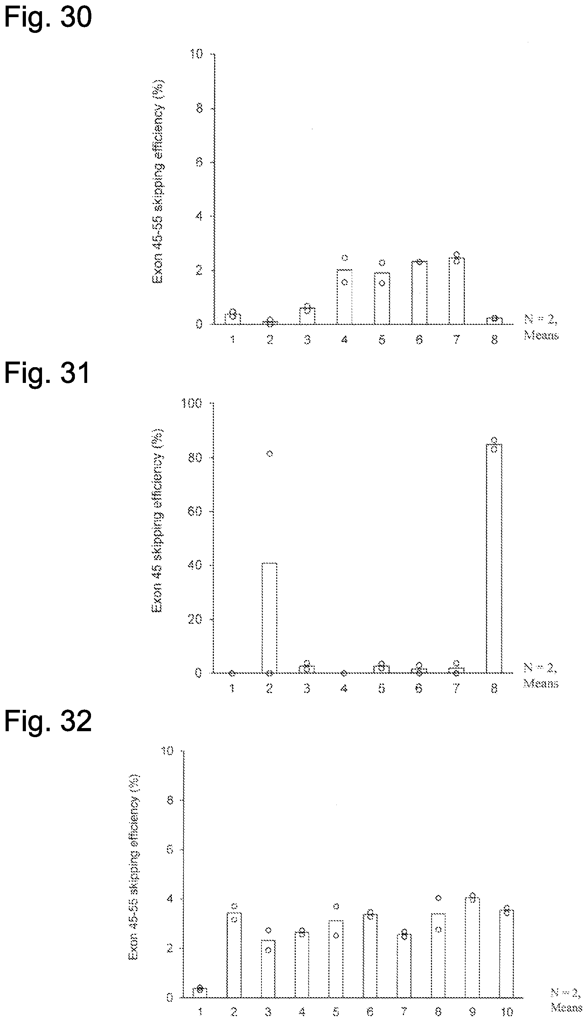

The results are shown in Figures 30 and 31. As a result of adding PMO Nos. 24 to 26 as the third antisense oligomer to PMO No. 6 and PMO No. 8 (conditions 2, 5, and 8), exon 45 to 55 skipping efficiency was reduced when PMO Nos. 24 and 26 were added (conditions 2 and 8) as compared with a case where PMO No. 7 was added (condition 4), but exon 45 to 55 skipping was confirmed to be induced to the same extent when PMO No. 25 was added (condition 5) (Figure 30). Exon 45 skipping efficiency was reduced when PMO No. 25 was added (condition 5), and single skipping was thus suppressed (Figure 31).

### <Test Example 12>

### Assay of exon 45 to 55 multi-exon skipping in DMD patient-derived myoblasts with exon 51 deletion - (5): study of second antisense oligomer - (1)

### Procedures

DMD patient-derived myoblasts with exon 51 deletion (CD-56 positive, CD-82 positive) prepared in the same manner as in Test Examples 8 to 11 were seeded in a Corning BioCoat collagen I 48-well transparent microplate coated with Corning(R) Matrigel Basement Membrane Matrix at 5 x 10⁴/well, and cultured for 1 day under conditions of 37°C and 5% CO₂ in 0.25 mL of a growth medium for DMD patient-derived myoblasts. On the next day of the seeding, the medium was changed from the growth medium to 0.25 mL of a differentiation medium for DMD patient-derived myoblasts. After culturing for 7 days in the differentiation medium for DMD patient-derived myoblasts, transfection was performed with PMO using 6 µM Endo-Porter.

In addition to the PMOs used in Text Examples 1 to 7, PMOs shown in Table 32 below were used.

### [Table 32]

**Table 32**

| PMO No. | **Base sequence of PMO (5' to 3')** | **SEQ ID NO:** |
|---|---|---|
| 27 | TTTCTTCCAAAGCAGCCTCTCGC | 4698 |
| 28 | TGAGTTTCTTCCAAAGCAGCCTC | 4702 |
| 29 | TGAGTTTCTTCCAAAGCAGCCTCT | 4752 |
| 30 | CAAAGGACCAAATGTTCAGATGCAATTA | 4923 |
| 31 | CTGCAAAGGACCAAATGTTCAGATGCAA | 4926 |
| 32 | CTCACTCACCCTGCAAAGGACCAAATGT | 4936 |
| 33 | TGTTGCAGTAATCTATGAGTTTCTTCCA | 4977 |

The PMOs were added in concentrations in the medium shown in Table 33 below.

### [Table 33]

**Table 33**

| **Condition** | PMO |
|---|---|
| 1 | **No PMO added** |
| 2 | 30 µM PMO No. 6 + 30 µM PMO No. 27 |
| 3 | 30 µM PMO No. 6 + 30 µM PMO No. 28 |
| 4 | 30 µM PMO No. 6 + 30 µM PMO No. 29 |
| 5 | 30 µM PMO No. 6 + 30 µM PMO No. 30 |
| 6 | 30 µM PMO No. 6 + 30 µM PMO No. 31 |
| 7 | 30 µM PMO No. 6 + 30 µM PMO No. 32 |
| 8 | 30 µM PMO No. 6 + 30 µM PMO No. 8 |
| 9 | 30 µM PMO No. 6 + 30 µM PMO No. 33 |
| 10 | 20 µM PMO No. 6 + 20 µM PMO No. 7 + 20 µM PMO No. 8 |

After culturing for another 3 days, the medium was changed to 0.3 mL of a differentiation medium for DMD patient-derived myoblasts. Seven days after the addition of PMO, the total RNA was extracted in the same manner as in Test Examples 2, 3, 5, 6, and 8 to 11, One-Step RT-PCR was performed with 200 ng of the extracted total RNA in the same manner as in Test Examples 1 to 3 and 8 to 11, and the reaction product of the PCR thus obtained was analyzed to obtain skipping efficiencies of exon 45 to 55 skipping and exon 45 skipping.

### Results

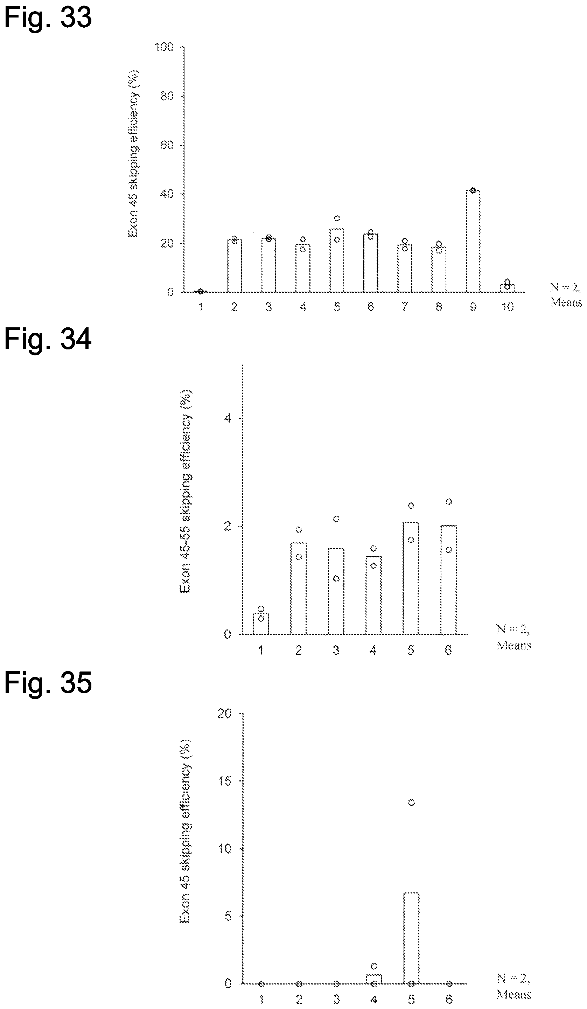

The results are shown in Figures 32 and 33.

As a result of adding PMO Nos. 27 to 33 as the second antisense oligomer together with PMO No. 6 (conditions 2 to 7, and 9), exon 45 to 55 skipping was confirmed to be induced to the same extent as in a case where PMO No. 8 was added (condition 8) (Figure 32). Even when the second antisense oligomer was changed, exon 45 skipping efficiency was not reduced, and single skipping was not suppressed (Figure 33).

### <Test Example 13>

### Assay of exon 45 to 55 multi-exon skipping in DMD patient-derived myoblasts with exon 51 deletion - (6): study of second antisense oligomer - (2)

### Procedures

DMD patient-derived myoblasts with exon 51 deletion (CD-56 positive, CD-82 positive) prepared in the same manner as in Test Examples 8 to 12 were seeded in a Corning BioCoat collagen I 48-well transparent microplate coated with Corning(R) Matrigel Basement Membrane Matrix at 5 x 10⁴/well, and cultured for 1 day under conditions of 37°C and 5% CO₂ in 0.25 mL of a growth medium for DMD patient-derived myoblasts. On the next day of the seeding, the medium was changed from the growth medium to 0.25 mL of a differentiation medium for DMD patient-derived myoblasts. After culturing for 3 days in the differentiation medium for DMD patient-derived myoblasts, transfection was performed with PMO using 6 µM Endo-Porter. The PMOs used in Text Example 12 were used in concentrations in the medium shown in Table 34 below.

### [Table 34]

**Table 34**

| **Condition** | PMO |
|---|---|
| 1 | **No PMO added** |
| 2 | 20 µM PMO No. 6 + 20 µM PMO No. 7 + 20 µM PMO No. 27 |
| 3 | 20 µM PM0 No. 6 + 20 µM PMO No. 7 + 20 µM PMO No. 28 |
| 4 | 20 µM PMO No. 6 + 20 µM PMO No. 7 + 20 µM PMO No. 29 |
| 5 | 20 µM PM0 No. 6 + 20 µM PMO No. 7 + 20 µM PMO No. 32 |
| 6 | 20 µM PMO No. 6 + 20 µM PMO No. 7 + 20 µM PMO No. 8 |

After culturing for another 3 days, the medium was changed to 0.3 mL of a differentiation medium for DMD patient-derived myoblasts. Seven days after the addition of PMO, the total RNA was extracted in the same manner as in Test Examples 2, 3, 5, 6, and 8 to 12, One-Step RT-PCR was performed with 200 ng of the extracted total RNA in the same manner as in Test Examples 1 to 3 and 8 to 12, and the reaction product of the PCR thus obtained was analyzed to obtain skipping efficiencies of exon 45 to 55 skipping and exon 45 skipping.

### Results

The results are shown in Figures 34 and 35. As a result of respectively adding PMO Nos. 27 to 29 and 32 together with PMO No. 6 and PMO No. 7 (conditions 2 to 5), exon 45 to 55 skipping was confirmed to be induced to the same extent as in a case where PMO No. 8 was added (condition 6) (Figure 34). The induction of exon 45 skipping was little confirmed excluding a case where PMO No. 32 was added (condition 5), and single skipping was thus suppressed (Figure 35).

### <Test Example 14>

### Assay of exon 45 to 55 multi-exon skipping in DMD patient-derived myoblasts with exon 51 deletion - (7): study of second antisense oligomer - (3)

### Procedures

DMD patient-derived myoblasts with exon 51 deletion (CD-56 positive, CD-82 positive) prepared in the same manner as in Test Examples 8 to 13 were seeded in a Corning BioCoat collagen I 48-well transparent microplate coated with Corning(R) Matrigel Basement Membrane Matrix at 5 x 10⁴/well, and cultured for 1 day under conditions of 37°C and 5% CO₂ in 0.25 mL of a growth medium for DMD patient-derived myoblasts. On the next day of the seeding, the medium was changed from the growth medium to 0.25 mL of a differentiation medium for DMD patient-derived myoblasts. After culturing for 7 days in the differentiation medium for DMD patient-derived myoblasts, transfection was performed with PMO using 6 µM Endo-Porter. The PMOs used in Text Example were added in concentrations in the medium shown in Table 35 below.

### [Table 35]

**Table 35**

| **Condition** | PMO |
|---|---|
| 1 | **No PMO added** |
| 2 | 30 µM PMO No. 6 + 30 µM PMO No. 8 |
| 3 | 20 µM PMO No. 6 + 20 µM PMO No. 7 + 20 µM PMO No. 30 |
| 4 | 20 µM PMO No. 6 + 20 µM PMO No. 7 + 20 µM PMO No. 31 |
| 5 | 20 µM PMO No. 6 + 20 µM PM0 No. 7 + 20 µM PMO No. 8 |
| 6 | 20 µM PMO No. 6 + 20 µM PM0 No. 7 + 20 µM PMO No. 33 |

After culturing for another 3 days, the medium was changed to 0.3 mL of a differentiation medium for DMD patient-derived myoblasts. Seven days after the addition of PMO, the total RNA was extracted in the same manner as in Test Examples 2, 3, 5, 6, and 8 to 13, One-Step RT-PCR was performed with 200 ng of the extracted total RNA in the same manner as in Test Examples 1 to 3 and 8 to 13, and the reaction product of the PCR thus obtained was analyzed to obtain skipping efficiencies of exon 45 to 55 skipping and exon 45 skipping.

### Results

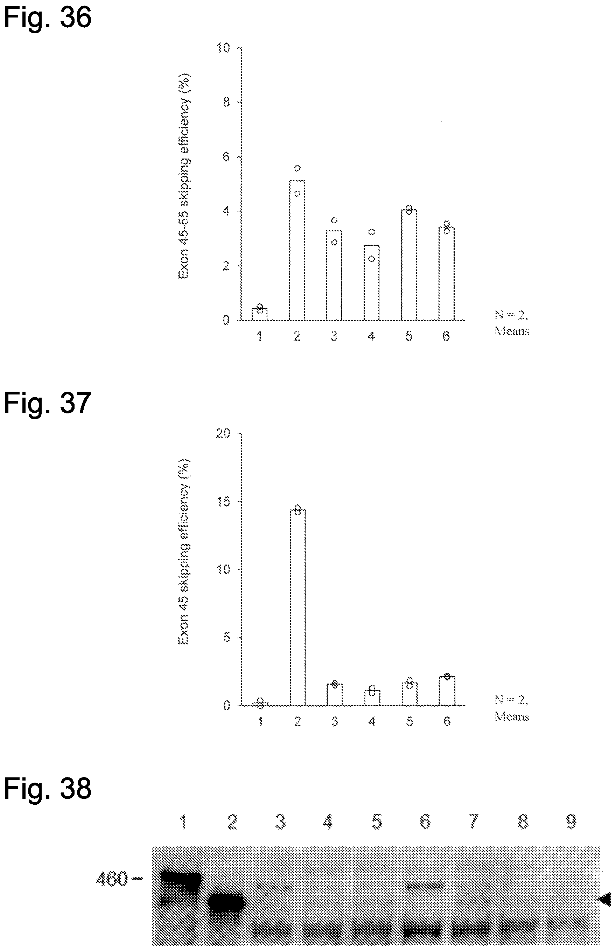

The results are shown in Figures 36 and 37.

As a result of respectively adding PMO No. 30, 31, or 33 as the second antisense oligomer together with PMO No. 6 and PMO No. 7 (conditions 3, 4, and 6), exon 45 to 55 skipping was confirmed to the same extent as in a case where PMO No. 8 was added (condition 5) (Figure 36). As compared with a case where only PMO No. 6 and PMO No. 8 were added (condition 2), the skipping efficiency of exon 45 skipping was all reduced, and single skipping was thus suppressed (Figure 37).

### <Test Example 15>

### Assay of exon 45 to 55 multi-exon skipping in DMD patient-derived myoblasts with exon 51 deletion - (8): restoration of dystrophin protein by multi-exon skipping

### Procedures

DMD patient-derived myoblasts with exon 51 deletion (CD56-positive and CD82-positive) were seeded in a collagen I coat microplate 24-well (manufactured by AGC Techno Glass Co., Ltd.) coated with Corning(R) Matrigel Basement Membrane Matrix at 2.0 x 10⁵/well, and cultured for 1 day under conditions of 37°C and 5% CO₂ in 1 mL of a growth medium for DMD patient-derived myoblasts. On the next day of the seeding, the medium was changed from the growth medium for DMD patient derived myoblasts to a differentiation medium for DMD patient-derived myoblasts. After culturing for 3 days in the differentiation medium, transfection was performed with PMO using 6 µM Endo-Porter. After culturing for 3 days, the medium was changed to a differentiation medium. After culturing for 7 days or 11 days after the addition of PMO, Western blotting was performed in the same manner as in Test Examples 4 and 7 of the present example to detect the dystrophin protein. Samples subjected to electrophoresis are shown in Table 36. As a positive control of dystrophin expression, a lysate of mouse C2C12 cells having been muscle differentiation cultured for 12 days (normal dystrophin control), and a lysate of skeletal muscle of exon 45 to 55 deletion transgenic mouse (exon 45 to 55 deletion dystrophin expression control) were used.

### [Table 36]

**Table 36**

| **Condition** | **Sample** | **Collection date** |
|---|---|---|
| 1 | **Normal dystrophin control** | |
| 2 | **Exon 45 to 55 deletion dystrophin expression control** | |
| 3 | **No PMO added** | **7 days after PMO addition** |
| 4 | 30 µM PM0 No. 6 + 30 µM PM0 No. 7 +30 µM PMO No. 8 | |
| 5 | 30 µM PM0 No. 6 + 30 µM PMO No. 8 | |
| 6 | **No PMO added** | **11 days after PMO addition** |
| 7 | 30 µM PMO No. 6 + 30 µM PM0 No. 7 +30 µM PMO No. 8 | |
| 8 | 30 µM PMO No. 6 + 30 µM PMO No. 8 | |
| 9 | 30 µM PM0 No. 6 | |

### (Results)

The results are shown in Figure 38. In the negative control (conditions 3 and 6), the band was not confirmed in the same position (Figure 38: arrowhead) as the band of exon 45 to 55 deletion dystrophin-positive control (condition 2), but in the samples transfected with a cocktail of PMOs (conditions 4, 5, 7, and 8), expression of the dystrophin protein corresponding to exon 45 to 55 skipping was confirmed (Figure 38: arrowhead).

### <Test Example 16>

### Assay of exon 45 to 55 multi-exon skipping in DMD patient-derived myoblasts with exon 51 deletion - (9): restoration of dystrophin protein by multi-exon skipping - (2)

### Procedures

DMD patient-derived myoblasts with exon 51 deletion (CD56-positive and CD82-positive) prepared in the same manner as in Test Examples 8 to 15 were seeded in a collagen I coat microplate 24-well (manufactured by AGC Techno Glass Co., Ltd.) coated with Corning(R) Matrigel Basement Membrane Matrix at 2.0 x 10⁵/well, and cultured for 1 day under conditions of 37°C and 5% CO₂ in 1 mL of a growth medium for DMD patient-derived myoblasts. On the next day of the seeding, the medium was changed from the growth medium for DMD patient-derived myoblasts to a differentiation medium for DMD patient-derived myoblasts. After culturing for 7 days in the differentiation medium, transfection was performed with PMO using 6 µM Endo-Porter. The PMOs used here are shown in Table 37 below.

### [Table 37]

**Table 37**

| PM0 No. | **Base sequence of PMO (5' to 3')** | **SEQ ID NO:** |
|---|---|---|
| 6 | TGACAACAGTTTGCCGCTGCCCGATTAA | 1201, 151 |
| 7 | TTTTCATTCCTATTAGATCTGTCG | 3082 |
| 8 | AAAGCAGCCTCTCGCTCACTCACCCTGC | 4950 |
| 14 | CAATGCCATCCTGGAGTTCCTGGATTAA | 1180, 151 |
| 21 | AGTCTGCTAAAATGTTTTCATTCC | 3096 |
| 33 | TGTTGCAGTAATCTATGAGTTTCTTCCA | 4977 |

After culturing for another 3 days, the medium was changed to a differentiation medium. After culturing for 7 days after the addition of PMO, Western blotting was performed in the same manner as in Test Examples 4, 7, and 15 of the present example to detect the dystrophin protein. Samples subjected to the electrophoresis are shown in Table 38 below.

### [Table 38]

**Table 38**

| **Condition** | **Sample** |
|---|---|
| 1 | **Normal dystrophin control** |
| 2 | **Exon 45 to 55 deletion dystrophin expression control** |
| 3 | **No PMO added** |
| 4 | 30 µM PMO No. 14 + 30 µM PMO No. 8 |
| 5 | 20 µM PMO No. 14 + 20 µM PMO No. 7 + 20 µM PMO No. 8 |
| 6 | 30 µM PMO No. 6 + 30 µM PMO No. 33 |
| 7 | 20 µM PM0 No. 6 + 20 µM PM0 No. 21 + 20 µM PM0 No. 33 |
| 8 | 20 µM PM0 No. 14 + 20 µM PMO No. 21 + 20 µM PMO No. 33 |

### (Results)

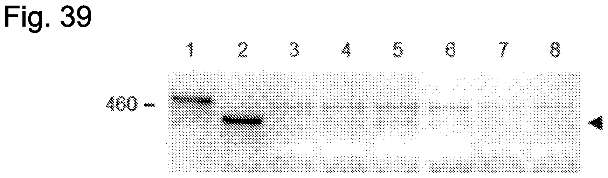

The results are shown in Figure 39.

In the negative control (condition 3), the band was not confirmed in the same position as the band of exon 45 to 55 deletion dystrophin-positive control (condition 2), but in the samples transfected with a cocktail of PMOs (conditions 4, 5, and 6), expression of the dystrophin protein corresponding to exon 45 to 55 skipping was confirmed (Figure 39: arrowhead).

## Claims

1. A combination of antisense oligomers or pharmaceutically acceptable salts thereof, or hydrates thereof which cause simultaneous skipping of any two or more numerically consecutive exons selected from the group consisting of the 45th exon to the 55th exon in human dystrophin pre-mRNA,
the combination comprising:
(i) a first antisense oligomer or a pharmaceutically acceptable salt thereof, or a hydrate thereof, comprising:
a first unit oligomer comprising a base sequence complementary to a base sequence consisting of a base sequence of 11 bases in the upstream direction from the 3' end of the 44th intron and a base sequence of 69 bases in the downstream direction from the 5' end of the 45th exon in the human dystrophin pre-mRNA, or a partial base sequence thereof; and
a second unit oligomer comprising a base sequence complementary to a base sequence of from the 52nd to 75th bases in the upstream direction from the 3' end of the 44th intron in the human dystrophin pre-mRNA, or a partial base sequence thereof; and
(ii) a second antisense oligomer or a pharmaceutically acceptable salt thereof, or a hydrate thereof, comprising a base sequence complementary to a base sequence consisting of a base sequence of 33 bases in the upstream direction from the 3' end of the 54th intron and a base sequence of 53 bases in the downstream direction from the 5' end of the 55th exon in the human dystrophin pre-mRNA, or a partial base sequence thereof.

2. The combination according to claim 1, wherein
the first unit oligomer comprises a base sequence complementary to consecutive 15 to 30 bases of a base sequence consisting of a base sequence of 11 bases in the upstream direction from the 3' end of the 44th intron and a base sequence of 69 bases in the downstream direction from the 5' end of the 45th exon in the human dystrophin pre-mRNA,
the second unit oligomer comprises a base sequence complementary to consecutive 1 to 10 bases of a base sequence of from the 52nd to 75th bases in the upstream direction from the 3' end of the 44th intron in the human dystrophin pre-mRNA, and
the second antisense oligomer comprises a base sequence complementary to consecutive 15 to 30 bases of a base sequence consisting of a base sequence of 33 bases in the upstream direction from the 3' end of the 54th intron and a base sequence of 53 bases in the downstream direction from the 5' end of the 55th exon in the human dystrophin pre-mRNA.

3. The combination according to claim 1 or 2, wherein
the first unit oligomer comprises a base sequence complementary to:
(a) any one base sequence selected from the group consisting of SEQ ID NOs: 211 to 906;
(b) a base sequence that hybridizes under stringent conditions to a base sequence complementary to any one base sequence selected from the group consisting of SEQ ID NOs: 211 to 906;
(c) a base sequence that has at least 85% identity with any one base sequence selected from the group consisting of SEQ ID NOs: 211 to 906, and has a length within ±15% of the length of the any one base sequence selected; or
(d) a partial base sequence of any one base sequence selected from the group consisting of the base sequences (a), (b), and (c), and/or
the second unit oligomer comprises a base sequence complementary to:
(a) any one base sequence selected from the group consisting of SEQ ID NOs: 1 to 105;
(b) a base sequence that hybridizes under stringent conditions to a base sequence complementary to any one base sequence selected from the group consisting of SEQ ID NOs: 1 to 105;
(c) a base sequence that has at least 85% identity with any one base sequence selected from the group consisting of SEQ ID NOs: 1 to 105, and has a length within ±15% of the length of the any one base sequence selected; or
(d) a partial base sequence of any one base sequence selected from the group consisting of the base sequences (a), (b), and (c) .

4. The combination according to any one of claims 1 to 3, wherein
the second antisense oligomer comprises a base sequence complementary to:
(a) any one base sequence selected from the group consisting of SEQ ID NOs: 3507 to 4298;
(b) a base sequence that hybridizes under stringent conditions to a base sequence complementary to any one base sequence selected from the group consisting of SEQ ID NOs: 3507 to 4298;
(c) a base sequence that has at least 85% identity with any one base sequence selected from the group consisting of SEQ ID NOs: 3507 to 4298, and has a length within ±15% of the length of the any one base sequence selected; or
(d) a partial base sequence of any one base sequence selected from the group consisting of the base sequences (a), (b), and (c) .

5. The combination according to any one of claims 1 to 4, wherein the first antisense oligomer comprises the first unit oligomer and the second unit oligomer from the 5' ends in this order, the first unit oligomer comprises any one base sequence selected from SEQ ID NOs: 907 to 1602, the second unit oligomer comprises any one base sequence selected from SEQ ID NOs: 106 to 210, and the second antisense oligomer comprises any one base sequence selected from SEQ ID NOs: 4299 to 5090.

6. The combination according to any one of claims 1 to 5, wherein the first unit oligomer comprises any one base sequence selected from the group consisting of SEQ ID NOs: 1180, 1190, 1201, 1212, 1222, 1224, and 1239.

7. The combination according to any one of claims 1 to 6, wherein the second unit oligomer comprises any one base sequence selected from the group consisting of SEQ ID NOs: 114, 124, 151, 201, 203, and 205.

8. The combination according to claim 6 or 7, wherein
the first antisense oligomer comprises the first unit oligomer and the second unit oligomer from the 5' ends in this order, and
the first unit oligomer comprises a base sequence of SEQ ID NO: 1201, and the second unit oligomer comprises a base sequence of SEQ ID NO: 151,
the first unit oligomer comprises a base sequence of SEQ ID NO: 1201, and the second unit oligomer comprises a base sequence of SEQ ID NO: 201,
the first unit oligomer comprises a base sequence of SEQ ID NO: 1201, and the second unit oligomer comprises a base sequence of SEQ ID NO: 203,
the first unit oligomer comprises a base sequence of SEQ ID NO: 1201, and the second unit oligomer comprises a base sequence of SEQ ID NO: 205,
the first unit oligomer comprises a base sequence of SEQ ID NO: 1239, and the second unit oligomer comprises a base sequence of SEQ ID NO: 114,
the first unit oligomer comprises a base sequence of SEQ ID NO: 1224, and the second unit oligomer comprises a base sequence of SEQ ID NO: 124,
the first unit oligomer comprises a base sequence of SEQ ID NO: 1180, and the second unit oligomer comprises a base sequence of SEQ ID NO: 151,
the first unit oligomer comprises a base sequence of SEQ ID NO: 1190, and the second unit oligomer comprises a base sequence of SEQ ID NO: 151,
the first unit oligomer comprises a base sequence of SEQ ID NO: 1212, and the second unit oligomer comprises a base sequence of SEQ ID NO: 151, or
the first unit oligomer comprises a base sequence of SEQ ID NO: 1222, and the second unit oligomer comprises a base sequence of SEQ ID NO: 151.

9. The combination according to any one of claims 1 to 8, wherein the second antisense oligomer comprises a base sequence selected from the group consisting of SEQ ID NOs: 4698, 4702, 4752, 4923, 4926, 4936, 4950, and 4977.

10. The combination according to any one of claims 1 to 9, wherein
the first antisense oligomer comprises the first unit oligomer and the second unit oligomer from the 5' ends in this order, and
the first unit oligomer comprises a base sequence of SEQ ID NO: 1201, the second unit oligomer comprises a base sequence of SEQ ID NO: 151, and the second antisense oligomer comprises a base sequence of SEQ ID NO: 4950,
the first unit oligomer comprises a base sequence of SEQ ID NO: 1201, the second unit oligomer comprises a base sequence of SEQ ID NO: 201, and the second antisense oligomer comprises a base sequence of SEQ ID NO: 4950,
the first unit oligomer comprises a base sequence of SEQ ID NO: 1201, the second unit oligomer comprises a base sequence of SEQ ID NO: 203, and the second antisense oligomer comprises a base sequence of SEQ ID NO: 4950,
the first unit oligomer comprises a base sequence of SEQ ID NO: 1201, the second unit oligomer comprises a base sequence of SEQ ID NO: 205, and the second antisense oligomer comprises a base sequence of SEQ ID NO: 4950,
the first unit oligomer comprises a base sequence of SEQ ID NO: 1239, the second unit oligomer comprises a base sequence of SEQ ID NO: 114, and the second antisense oligomer comprises a base sequence of SEQ ID NO: 4950,
the first unit oligomer comprises a base sequence of SEQ ID NO: 1224, the second unit oligomer comprises a base sequence of SEQ ID NO: 124, and the second antisense oligomer comprises a base sequence of SEQ ID NO: 4950,
the first unit oligomer comprises a base sequence of SEQ ID NO: 1180, the second unit oligomer comprises a base sequence of SEQ ID NO: 151, and the second antisense oligomer comprises a base sequence of SEQ ID NO: 4950,
the first unit oligomer comprises a base sequence of SEQ ID NO: 1190, the second unit oligomer comprises a base sequence of SEQ ID NO: 151, and the second antisense oligomer comprises a base sequence of SEQ ID NO: 4950,
the first unit oligomer comprises a base sequence of SEQ ID NO: 1212, the second unit oligomer comprises a base sequence of SEQ ID NO: 151, and the second antisense oligomer comprises a base sequence of SEQ ID NO: 4950,
the first unit oligomer comprises a base sequence of SEQ ID NO: 1222, the second unit oligomer comprises a base sequence of SEQ ID NO: 151, and the second antisense oligomer comprises a base sequence of SEQ ID NO: 4950,
the first unit oligomer comprises a base sequence of SEQ ID NO: 1201, the second unit oligomer comprises a base sequence of SEQ ID NO: 151, and the second antisense oligomer comprises a base sequence of SEQ ID NO: 4698,
the first unit oligomer comprises a base sequence of SEQ ID NO: 1201, the second unit oligomer comprises a base sequence of SEQ ID NO: 151, and the second antisense oligomer comprises a base sequence of SEQ ID NO: 4702,
the first unit oligomer comprises a base sequence of SEQ ID NO: 1201, the second unit oligomer comprises a base sequence of SEQ ID NO: 151, and the second antisense oligomer comprises a base sequence of SEQ ID NO: 4752,
the first unit oligomer comprises a base sequence of SEQ ID NO: 1201, the second unit oligomer comprises a base sequence of SEQ ID NO: 151, and the second antisense oligomer comprises a base sequence of SEQ ID NO: 4923,
the first unit oligomer comprises a base sequence of SEQ ID NO: 1201, the second unit oligomer comprises a base sequence of SEQ ID NO: 151, and the second antisense oligomer comprises a base sequence of SEQ ID NO: 4926,
the first unit oligomer comprises a base sequence of SEQ ID NO: 1201, the second unit oligomer comprises a base sequence of SEQ ID NO: 151, and the second antisense oligomer comprises a base sequence of SEQ ID NO: 4936,
the first unit oligomer comprises a base sequence of SEQ ID NO: 1201, the second unit oligomer comprises a base sequence of SEQ ID NO: 151, and the second antisense oligomer comprises a base sequence of SEQ ID NO: 4977, or
the first unit oligomer comprises a base sequence of SEQ ID NO: 1180, the second unit oligomer comprises a base sequence of SEQ ID NO: 151, and the second antisense oligomer comprises a base sequence of SEQ ID NO: 4977.

11. The combination according to any one of claims 5 to 10, wherein the first unit oligomer comprises a base sequence of SEQ ID NO: 1201, the second unit oligomer comprises a base sequence of SEQ ID NO: 151, and the second antisense oligomer comprises a base sequence of SEQ ID NO: 4950.

12. The combination according to any one of claims 1 to 11, further comprising:
(iii) a third antisense oligomer or a pharmaceutically acceptable salt thereof, or a hydrate thereof, comprising a base sequence complementary to a base sequence consisting of a base sequence of 23 bases in the upstream direction from the 3' end of the 45th exon and a base sequence of 73 bases in the downstream direction from the 5' end of the 45th intron in the human dystrophin pre-mRNA, or a partial base sequence thereof.

13. The combination according to claim 12, wherein the third antisense oligomer comprises a base sequence complementary to consecutive 15 to 30 bases of a base sequence consisting of a base sequence of 23 bases in the upstream direction from the 3' end of the 45th exon and a base sequence of 73 bases in the downstream direction from the 5' end of the 45th intron in the human dystrophin pre-mRNA.

14. The combination according to claim 12 or 13, wherein
the third antisense oligomer comprises a base sequence complementary to:
(a) any one base sequence selected from the group consisting of SEQ ID NOs: 1603 to 2554;
(b) a base sequence that hybridizes under stringent conditions to a base sequence complementary to any one base sequence selected from the group consisting of SEQ ID NOs: 1603 to 2554;
(c) a base sequence that has at least 85% identity with any one base sequence selected from the group consisting of SEQ ID NOs: 1603 to 2554, and has a length within ±15% of the length of the any one base sequence selected; or
(d) a partial base sequence of any one base sequence selected from the group consisting of the base sequences (a), (b), and (c) .

15. The combination according to (14), wherein the third antisense oligomer comprises a base sequence complementary to:
(a) any one base sequence selected from the group consisting of SEQ ID NOs: 1611 to 1654, 1664 to 1707, 1718 to 1761, 1773 to 1816, 1829 to 1872, 1886 to 1929, 1944 to 1987, 2003 to 2046, 2063 to 2106, 2124 to 2167, 2186 to 2229, 2249 to 2292, 2313 to 2356, 2378 to 2421, 2444 to 2487, and 2511 to 2554;
(b) a base sequence that hybridizes under stringent conditions to a base sequence complementary to any one base sequence selected from the group consisting of SEQ ID NOs: 1611 to 1654, 1664 to 1707, 1718 to 1761, 1773 to 1816, 1829 to 1872, 1886 to 1929, 1944 to 1987, 2003 to 2046, 2063 to 2106, 2124 to 2167, 2186 to 2229, 2249 to 2292, 2313 to 2356, 2378 to 2421, 2444 to 2487, and 2511 to 2554;
(c) a base sequence that has at least 85% identity with any one base sequence selected from the group consisting of SEQ ID NOs: 1611 to 1654, 1664 to 1707, 1718 to 1761, 1773 to 1816, 1829 to 1872, 1886 to 1929, 1944 to 1987, 2003 to 2046, 2063 to 2106, 2124 to 2167, 2186 to 2229, 2249 to 2292, 2313 to 2356, 2378 to 2421, 2444 to 2487, and 2511 to 2554, and has a length within ±15% of the length of the any one base sequence selected; or
(d) a partial base sequence of any one base sequence selected from the group consisting of the base sequences (a), (b), and (c) .

16. The combination according to claim 15, wherein the third antisense oligomer comprises a base sequence complementary to:
(a) any one base sequence selected from the group consisting of SEQ ID NOs: 1617 to 1654, 1670 to 1707, 1724 to 1761, 1779 to 1816, 1835 to 1872, 1892 to 1929, 1950 to 1987, 2009 to 2046, 2069 to 2106, 2130 to 2167, 2192 to 2229, 2255 to 2292, 2319 to 2356, 2384 to 2421, 2450 to 2487, and 2517 to 2554;
(b) a base sequence that hybridizes under stringent conditions to a base sequence complementary to any one base sequence selected from the group consisting of SEQ ID NOs: 1617 to 1654, 1670 to 1707, 1724 to 1761, 1779 to 1816, 1835 to 1872, 1892 to 1929, 1950 to 1987, 2009 to 2046, 2069 to 2106, 2130 to 2167, 2192 to 2229, 2255 to 2292, 2319 to 2356, 2384 to 2421, 2450 to 2487, and 2517 to 2554;
(c) a base sequence that has at least 85% identity with any one base sequence selected from the group consisting of SEQ ID NOs: 1617 to 1654, 1670 to 1707, 1724 to 1761, 1779 to 1816, 1835 to 1872, 1892 to 1929, 1950 to 1987, 2009 to 2046, 2069 to 2106, 2130 to 2167, 2192 to 2229, 2255 to 2292, 2319 to 2356, 2384 to 2421, 2450 to 2487, and 2517 to 2554, and has a length within ±15% of the length of the any one base sequence selected; or
(d) a partial base sequence of any one base sequence selected from the group consisting of the base sequences (a), (b), and (c) .

17. The combination according to any one of claims 1 to 14, wherein the third antisense oligomer comprises a base sequence selected from the group consisting of SEQ ID NOs: 3060, 3065, 3077, 3082, 3087, 3090, 3096, 3108, 3119, and 3320.

18. The combination according to any one of claims 12 to 17, wherein the first antisense oligomer comprises the first unit oligomer and the second unit oligomer from the 5' ends in this order, the first unit oligomer comprises any one base sequence selected from SEQ ID NOs: 907 to 1602, the second unit oligomer comprises any one base sequence selected from SEQ ID NOs: 106 to 210, the second antisense oligomer comprises any one base sequence selected from SEQ ID NOs: 4299 to 5090, and the third antisense oligomer comprises any one base sequence selected from SEQ ID NOs: 2555 to 3506.

19. The combination according to any one of claims 1 to 18, wherein
the first antisense oligomer comprises the first unit oligomer and the second unit oligomer from the 5' ends in this order, and
the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3082,
the first unit oligomer comprises a base sequence of SEQ ID NO: 1201, the second unit oligomer comprises a base sequence of SEQ ID NO: 201, the second antisense oligomer comprises a base sequence of SEQ ID NO: 4950, and the third antisense oligomer comprises a base sequence of SEQ ID NO: 3082,
the first unit oligomer comprises a base sequence of SEQ ID NO: 1201, the second unit oligomer comprises a base sequence of SEQ ID NO: 203, the second antisense oligomer comprises a base sequence of SEQ ID NO: 4950, and the third antisense oligomer comprises a base sequence of SEQ ID NO: 3082,
the first unit oligomer comprises a base sequence of SEQ ID NO: 1201, the second unit oligomer comprises a base sequence of SEQ ID NO: 205, the second antisense oligomer comprises a base sequence of SEQ ID NO: 4950, and the third antisense oligomer comprises a base sequence of SEQ ID NO: 3082,
the first unit oligomer comprises a base sequence of SEQ ID NO: 1239, the second unit oligomer comprises a base sequence of SEQ ID NO: 114, the second antisense oligomer comprises a base sequence of SEQ ID NO: 4950, and the third antisense oligomer comprises a base sequence of SEQ ID NO: 3082,
the first unit oligomer comprises a base sequence of SEQ ID NO: 1224, the second unit oligomer comprises a base sequence of SEQ ID NO: 124, the second antisense oligomer comprises a base sequence of SEQ ID NO: 4950, and the third antisense oligomer comprises a base sequence of SEQ ID NO: 3082,
the first unit oligomer comprises a base sequence of SEQ ID NO: 1180, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3082,
the first unit oligomer comprises a base sequence of SEQ ID NO: 1190, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3082,
the first unit oligomer comprises a base sequence of SEQ ID NO: 1212, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3082,
the first unit oligomer comprises a base sequence of SEQ ID NO: 1222, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3082,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3060,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3065,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3077,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3087,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3090,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3096,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3108,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3119,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3320,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4698, and the third antisense oligomer comprises a base sequence of SEQ ID NO: 3082,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4702, and the third antisense oligomer comprises a base sequence of SEQ ID NO: 3082,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4752, and the third antisense oligomer comprises a base sequence of SEQ ID NO: 3082,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4923, and the third antisense oligomer comprises a base sequence of SEQ ID NO: 3082,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4926, and the third antisense oligomer comprises a base sequence of SEQ ID NO: 3082,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4936, and the third antisense oligomer comprises a base sequence of SEQ ID NO: 3082,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4977, and the third antisense oligomer comprises a base sequence of SEQ ID NO: 3082,
the first unit oligomer comprises a base sequence of SEQ ID NO: 1201, the second unit oligomer comprises a base sequence of SEQ ID NO: 151, the second antisense oligomer comprises a base sequence of SEQ ID NO: 4977, and the third antisense oligomer comprises a base sequence of SEQ ID NO: 3096, or
the first unit oligomer comprises a base sequence of SEQ ID NO: 1180, the second unit oligomer comprises a base sequence of SEQ ID NO: 151, the second antisense oligomer comprises a base sequence of SEQ ID NO: 4977, and the third antisense oligomer comprises a base sequence of SEQ ID NO: 3096.

20. The combination according to claim 18 or 19, wherein the first unit oligomer comprises a base sequence of SEQ ID NO: 1201, the second unit oligomer comprises a base sequence of SEQ ID NO: 151, the second antisense oligomer comprises a base sequence of SEQ ID NO: 4950, and the third antisense oligomer comprises a base sequence of SEQ ID NO: 3082, 3090, or 3096.

21. The combination according to any one of claims 1 to 20, the combination causing skipping of all exons from the 45th exon to the 55th exon in the human dystrophin pre-mRNA.

22. The combination according to any one of claims 1 to 11, wherein the first and second antisense oligomers are oligonucleotides, or the combination according to any one of claims 12 to 21, wherein the first to third antisense oligomers are oligonucleotides.

23. The combination according to claim 22, wherein a sugar moiety and/or a phosphate-binding region of at least one base constituting the oligonucleotide is modified.

24. The combination according to claim 22 or 23, wherein the sugar moiety of at least one base constituting the oligonucleotide is a ribose in which a 2'-OH group is replaced by any one group selected from the group consisting of -OR, -R, -R'OR, -SH, -SR, -NH₂, -NHR, -NR₂, -N₃, -CN, -F, -Cl, -Br, and -I (wherein R is an alkyl or an aryl and R' is an alkylene).

25. The combination according to any one of claims 22 to 24, wherein the phosphate-binding region of at least one base constituting the oligonucleotide is any one selected from the group consisting of a phosphorothioate bond, a phosphorodithioate bond, an alkylphosphonate bond, a phosphoramidate bond, and a boranophosphate bond.

26. The combination according to any one of claims 1 to 11, wherein the first and second antisense oligomers are morpholino oligomers, or the combination according to any one of claims 12 to 21, wherein the first to third antisense oligomers are oligonucleotides.

27. The combination according to claim 26, wherein the first to third antisense oligomers are phosphorodiamidate morpholino oligomers.

28. The combination according to claim 26 or 27, wherein the 5' end of each of the first to third antisense oligomers is a group represented by any one of the following chemical formulae (1) to (3):

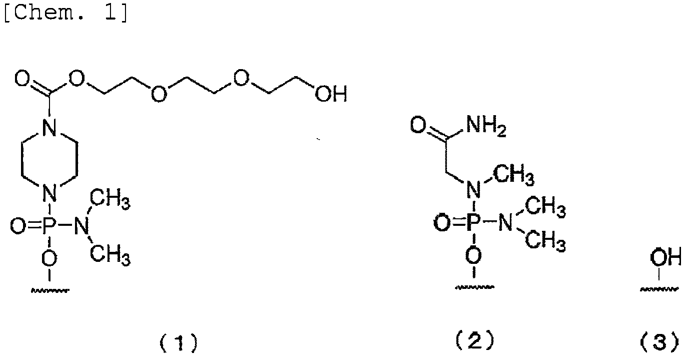

29. (a) A pharmaceutical composition comprising the first and second antisense oligomers according to any one of claims 1 to 28, or pharmaceutically acceptable salts thereof, or hydrates thereof, or
(b) a pharmaceutical combination comprising (i) a pharmaceutical composition comprising the first antisense oligomer according to any one of claims 1 to 28, or a pharmaceutically acceptable salt thereof, or a hydrate thereof, and (ii) a pharmaceutical composition comprising the second antisense oligomer according to any one of claims 1 to 28, or a pharmaceutically acceptable salt thereof, or a hydrate thereof.

30. (a) A pharmaceutical composition comprising the first to third antisense oligomers according to any one of claims 12 to 28, or pharmaceutically acceptable salts thereof, or hydrates thereof, or
(b) a pharmaceutical combination comprising (i) a pharmaceutical composition comprising the first antisense oligomer according to any one of claims 12 to 28, or a pharmaceutically acceptable salt thereof, or a hydrate thereof, (ii) a pharmaceutical composition comprising the second antisense oligomer according to any one of claims 12 to 28, or a pharmaceutically acceptable salt thereof, or a hydrate thereof, and (iii) a pharmaceutical composition comprising the third antisense oligomer according to any one of claims 12 to 28, or a pharmaceutically acceptable salt thereof, or a hydrate thereof.

31. The pharmaceutical composition or the pharmaceutical combination according to claim 29 or 30, wherein the pharmaceutical composition further comprises a pharmaceutically acceptable carrier.

32. The pharmaceutical composition or the pharmaceutical combination according to any one of claims 29 to 31 for treatment of muscular dystrophy.

33. The pharmaceutical composition or the pharmaceutical combination according to any one of claims 29 to 32 for being administered to a human patient.

34. A method for treatment of muscular dystrophy, comprising administering to a patient with muscular dystrophy (i) the first and second antisense oligomers according to any one of claims 1 to 28, or pharmaceutically acceptable salts thereof, or hydrates thereof, (ii) the first to third antisense oligomers according to any one of claims 12 to 28, or pharmaceutically acceptable salts thereof, or hydrates thereof, or (iii) the pharmaceutical composition or the pharmaceutical combination according to any one of claims 29 to 33.

35. The method for treatment according to claim 34,
wherein the muscular dystrophy patient is a patient with a mutation that is a target of exon 45 to 55 skipping in dystrophin gene.

36. The method for treatment according to claim 34 or 35, wherein the patient is a human.
